(19) Europäisches Patentamt
European Patent Office
Office européen des brevets

(11) EP 4 707 392 A1

(12) EUROPEAN PATENT APPLICATION
published in accordance with Art. 153(4) EPC

(43) Date of publication:
**11.03.2026 Bulletin 2026/11**

(21) Application number: **24810324.4**

(22) Date of filing: **17.05.2024**

(51) International Patent Classification (IPC):
***C12N 15/113*** *(2010.01)* ***A61K 31/713*** *(2006.01)*
***A61K 31/712*** *(2006.01)* ***C07H 21/02*** *(2006.01)*

(52) Cooperative Patent Classification (CPC):
**A61K 31/712; A61K 31/713; C07H 21/02; C12N 15/113**

(86) International application number:
**PCT/CN2024/093907**

(87) International publication number:
**WO 2024/240080 (28.11.2024 Gazette 2024/48)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **19.05.2023 CN 202310574400**

(71) Applicant: **Rigerna Therapeutics (Beijing) Co., Ltd.**
**Beijing 102629 (CN)**

(72) Inventors:
- **HUANG, Yuanyu**
  **Beijing 102629 (CN)**
- **LI, Haitao**
  **Beijing 102629 (CN)**
- **HAN, Xiaofeng**
  **Beijing 102629 (CN)**
- **GAO, Yongxin**
  **Beijing 102629 (CN)**

(74) Representative: **Acapo Onsagers AS**
**Edvard Griegs vei 1**
**5059 Bergen (NO)**

Remarks:
The complete document including Reference Table(s) and the Sequence Listing(s) can be downloaded from the EPO website

# (54) DOUBLE-STRANDED OLIGONUCLEOTIDE, CONJUGATE THEREOF, AND USE THEREOF

(57) Provided are a preparation method for a modified double-stranded oligonucleotide, an oligonucleotide conjugate or a composition, and use. Provided is a modified double-stranded oligonucleotide. Specific modification of a single site or a plurality of sites is carried out on a specific site of a sense strand and/or an antisense strand of an oligonucleotide, so that the modified oligonucleotide can have good pharmaceutical activity and have good application prospects in the development of RNA interference (RNAi)-based drugs.

EP 4 707 392 A1

## Description

### CROSS-REFERENCE TO RELATED APPLICATIONS

The present disclosure claims the priority to the Chinese patent application with the filing No. 202310574400.2, filed on May 19, 2023 with the China National Intellectual Property Administration, and entitled "DOUBLE-STRANDED OLIGONUCLEOTIDE, CONJUGATE THEREOF, AND USE THEREOF", the contents of which are incorporated herein by reference in entirety.

### TECHNICAL FIELD

The present disclosure relates to the field of oligonucleotides, and specifically to a preparation method for a modified double-stranded oligonucleotide, an oligonucleotide conjugate or a composition and use thereof.

### BACKGROUND ART

It is well known in the art that double-stranded oligonucleotides (dsRNAs) direct gene-specific post-transcriptional silencing, and have been employed as active pharmaceutical ingredients with proven efficacy.

It is widely acknowledged that the development of RNA interference (RNAi)-based drugs requires double-stranded oligonucleotide molecules with good gene-silencing properties or inhibitory activities, which typically need modifications. For example, siRNA duplexes comprising alternative modifications are used, and it is common to stabilize siRNA in serum through different combinations of 2'-OMe, 2'-F and phosphorothioate modifications. However, different modifications usually lead to variations in activity of double-stranded oligonucleotides or even loss of activity. For example, Tai, W. ("Chemical modulation of siRNA lipophilicity for efficient delivery." J Control Release 307: 98-107) proposed that one or more MOE modifications impair siRNA activity.

In the proprietary medicine of double-stranded oligonucleotides, the development of modification strategies for double-stranded oligonucleotides with optimal pharmaceutical activity remains an ongoing pursuit in the art.

### SUMMARY

The present disclosure aims at solving one of the technical problems in the related art at least to some extent.

In view of this, in one aspect, the present disclosure provides a double-stranded oligonucleotide (dsRNA) for inhibiting expression of a target gene, wherein the double-stranded oligonucleotide comprises a sense strand and an antisense strand, each having 17 to 35 nucleotides, each nucleotide being a modified or unmodified nucleotide; the sense strand and the antisense strand are at least partially reversely complementary to form a double-stranded region, and the double-stranded region has a nucleotide with sterically bulky modification and/or nucleotide with disubstitution modification, wherein composition of the double-stranded region is as represented by following Formula (I):

SS: $5' - (N)_{a'} - (X)_{p'} - (N)_{b'} - (X)_{q'} - (N)_{c'} - (X)_{r'} - (N)_{d'} -3'$
AS:

$$3' - (N)_a - (X)_p - (N)_b - (X)_q - (N)_c -5' \qquad (I).$$

Herein, SS represents the sense strand, and AS represents the antisense strand.

In Formula (I), each X independently represents a nucleotide in which 2'-hydroxyl group of ribose is substituted with a sterically bulky group or a nucleotide in which 2'-position is substituted with disubstituent (i.e., 2'-hydroxyl group and hydrogen are both substituted), wherein the 2'-sterically bulky group is selected from groups with a greater steric bulk than 2'-methoxy; and each substituent in the disubstitution is independently selected from $C_1$-$C_6$ alkyl or halogen.

In some embodiments, when the X represents a nucleotide in which 2'-hydroxyl group is substituted with a sterically bulky group, the sterically bulky group is independently selected from $2'-(O)_{m1}(CH_2)_n(O)_{m2}R_1$, wherein each of m1 and m2 is independently 0 or 1, and n is an integer selected from 0 to 6; $R_1$ is selected from substituted or unsubstituted $C_1$-$C_6$ alkyl, or $-Si(R_2)_3$, wherein each $R_2$ is independently selected from substituted or unsubstituted $C_1$-$C_6$ alkyl, and substituted or unsubstituted $C_1$-$C_6$ alkoxy, wherein the substituent comprises one or more substituents selected from the group consisting of halogen, $C_1$-$C_6$ alkyl, $C_1$-$C_6$ alkoxy, hydroxyl group, amino group, cycloalkyl having up to 6 carbon atoms, aryl having up to 12 carbon atoms, and heteroaryl having up to 12 carbon atoms.

In some embodiments, m1=0, n=0, m2=1, and $R1=Si(R_2)_3$.

In some embodiments, m1=1, m2=0, and n is a positive integer.

In some embodiments, each X is a nucleotide with modification independently selected from 2'-O$(CH_2)_n$O$R_1$ or 2'-$R_3$-2'-$R_4$. Herein, n is 1 or 2, and substituents $R_1$ and $R_2$ are as defined in the above. Herein, each of $R_3$ and $R_4$ is independently selected from $C_1$-$C_6$ alkyl or halogen. Optionally, the halogen is fluorine. Optionally, the $C_1$-$C_6$ alkyl is selected from methyl group or ethyl group.

In Formula (I), each N independently represents an unmodified nucleotide, or a nucleotide in which 2'-position of ribose is modified and which is not X (i.e., not being the abovementioned 2'-modification in X). Optionally, the modified nucleotide is selected from the group consisting of 2'-O-alkyl, 2'-alkyl, 2'-substituted alkyl, 2'-halo, 2'-deoxy (i.e., 2'-H modification), and a nucleotide analog. Herein, the alkyl has 1-6 carbon atoms, and the nucleotide analog is selected from the group consisting of ENA, BNA, LNA, GNA and UNA; optionally, the 2'-substituted alkyl has 1-6 carbon atoms in total. In the present disclosure, the alkyl group comprises branched-chain or straight-chain alkyl group.

Preferably, each N is independently selected from nucleotide modified with 2'-OMe, nucleotide modified with 2'-F or nucleotide modified with 2'-deoxy.

In Formula I, each of a, a', p, p', b, b', q, q', c, c', r', and d' in the general structural formula of the double-stranded region independently represents the number of nucleotides, wherein

a' is an integer selected from 3 to 8; p' is an integer selected from 0 to 3; b' is an integer selected from 4 to 13; q' is an integer selected from 0 to 4; c' is an integer selected from 3 to 9; r' is an integer selected from 0 to 3; d' is an integer selected from 0 to 9; a is an integer selected from 4 to 7; p is an integer selected from 0 to 1; b is an integer selected from 4 to 8; q is an integer selected from 0 to 4; c is an integer selected from 6 to 10; and p', q', r', p and q are not simultaneously 0, and optionally, $0 \leq q'+r' \leq 4$.

In some embodiments of the present disclosure, in a double-stranded oligonucleotide molecule comprising the double-stranded region represented by the above Formula (I), in a direction from 5'-end to 3'-end, a nucleotide sequence of the strand sense and/or antisense strand has at least one modified nucleotide X, i.e., a nucleotide with sterically bulky modification and/or a nucleotide modified with disubstituent, at a specific position.

In some embodiments of the present disclosure, in the direction from the 5'-end to the 3'-end, the nucleotide with sterically bulky modification and/or modified with disubstituent is located at a specific position of the antisense strand in the double-stranded region represented by Formula (I), the specific position being at least one of positions 10 and 15.

In some embodiments of the present disclosure, the specific position further comprises at least one of positions 8-10.

In some embodiments of the present disclosure, the nucleotide with sterically bulky modification and/or modified with disubstituent is located at a specific position of the sense strand in the double-stranded region represented by Formula (I), wherein the specific position is opposite to at least one of positions 2, 8, 12 and 15 (in the direction from the 5'-end to the 3'-end) of the antisense strand (i.e., at an opposite site).

In some embodiments of the present disclosure, the modified nucleotide X on the sense strand is located at a site opposite to any of positions 2, 8, and 15 of the antisense strand complementary thereto.

In some embodiments, optionally, counting from the 5'-end, X is located at position 15 of the antisense strand, and the antisense strand comprises at least one fluoro modification at position 9 or position 12, and/or a position of the sense strand opposite to position 10 of the antisense strand is a fluoro modification.

In some embodiments of the present disclosure, (N)a comprises at least one nucleotide modified with fluoro.

In some embodiments of the present disclosure, (N)c comprises at least two fluoro-modified nucleotides.

In some embodiments of the present disclosure, when q'+c'+r'+d'=9, $0 \leq q'+r' \leq 4$.

In some embodiments of the present disclosure, in the direction from the 5'-end to the 3'-end the first four nucleotides of (N)b' on the sense strand comprise at least two fluoro-modified nucleotides.

In some embodiments of the present disclosure, each X is a nucleotide with modification independently selected from 2'-O-methoxyethyl (MOE), 2'-O-TBDMS, 2'-O-TOM, and 2'-O-$CH_2$-O-$R_5$, $R_5$ being substituted or unsubstituted $C_1$-$C_3$ alkyl; optionally, each of the substituents is one or more independently selected from the following substituents: halogen, $C_1$-$C_3$ alkyl, $C_1$-$C_3$ alkoxy, or amino group; and optionally, the halogen is fluorine. Herein, MOE is methoxyethyl, TBDMS is *tert*-butyldimethylsilyl, and TOM is tris(isopropyl)silyloxymethyl.

In some embodiments of the present disclosure, the nucleotide in which the 2'-position is substituted with a disubstituent is selected from nucleotide modified with [2'-halo-2'-halo] or [2'-halo-2'-alkyl] or [2'-alkyl-2'-alkyl], e.g., from nucleotide modified with difluoro [2'-F-2'-F]. Optionally, the alkyl is $C_1$-$C_3$ alkyl.

In some embodiments of the present disclosure, the antisense strand further comprises one or two phosphorothioate linkage modifications.

In some embodiments of the present disclosure, the double-stranded oligonucleotide consists of a double-stranded region and a overhang region, that is, the double-stranded oligonucleotide may further comprise one or more overhang regions at 3'-end or 5'-end or both ends of one strand, wherein the overhang region may have a length of 1-6 nucleotides.

In some embodiments of the present disclosure, the double-stranded oligonucleotide molecule comprising the overhang region can be represented by following Formula (II):

SS: $5'\text{-}(T)_{t1}\text{-}(N)_{a'}\text{-}(X)_{p'}\text{-}(N)_{b'}\text{-}(X)_{q'}\text{-}(N)_{c'}\text{-}(X)_{r'}\text{-}(N)_{d'}\text{-}(T)_{t2}\text{-}3'$

AS:

$$3'\text{-}(T)_{t1}\text{-}(N)_a\text{-}(X)_p\text{-}(N)_b\text{-}(X)_q\text{-}(N)_c\text{-}(T)_{t2}\text{-}5' \quad \text{(II).}$$

Herein, SS represents the sense strand, AS represents the antisense strand; T represents a nucleotide of the overhang, each of t1 and t2 is an integer independently selected from 0 to 6, and cannot be simultaneously 0; and the remaining substituents are as defined for the above Formula (I). Herein, each nucleotide T of the overhang independently represents a modified or unmodified nucleotide, wherein the modified nucleotide is selected from the group consisting of 2'-O-alkyl (2'-alkoxy), 2'-alkyl, 2'-substituted alkyl, 2'-halogen, 2'-deoxy, abasic nucleotide, and inverted nucleotide; and optionally, the alkyl has 1-6 carbon atoms, preferably, 1-3 carbon atoms. The substituent is selected from one or more of C1-C6 alkyl, C1-C6 alkoxy, halogen, and amino group.

In some embodiments of the present disclosure, the antisense strand of the double-stranded oligonucleotide has a nucleotide overhang at the 3'-end, and the 5'-end is blunt; and the overhang comprises 1-3 nucleotides. In this case, the double-stranded oligonucleotide molecule can be represented by following Formula (IIa):

SS: $5'\text{-}(N)_{a'}\text{-}(X)_{p'}\text{-}(N)_{b'}\text{-}(X)_{q'}\text{-}(N)_{c'}\text{-}(X)_{r'}\text{-}(N)_{d'}\text{-}3'$

AS:

$$3'\text{-}(T)_{t1}\text{-}(N)_a\text{-}(X)_p\text{-}(N)_b\text{-}(X)_q\text{-}(N)_c\text{-}5' \quad \text{(IIa).}$$

Herein, t1 is an integer selected from 1 to 3, and the remaining substituents are as defined for the above Formula II.

In some embodiments of the present disclosure, the overhang region (T)t1 has a phosphorothioate group between nucleotides. In an embodiment, the double-stranded oligonucleotide of the present disclosure may further have two blunt ends at two ends of its duplex.

In some embodiments of the present disclosure, the double-stranded oligonucleotide of the present disclosure has a double-stranded region with 19-21 nucleotides (nt) in length.

In some embodiments of the present disclosure, each strand of the double-stranded oligonucleotide comprises 17-35 nucleotides.

In some embodiments of the present disclosure, each strand of the double-stranded oligonucleotide comprises 17-25 nucleotides.

In some embodiments of the present disclosure, each strand of the double-stranded oligonucleotide comprises 17-21 nucleotides.

In some embodiments of the present disclosure, each strand of the double-stranded oligonucleotide comprises 19-23 nucleotides.

In some embodiments of the present disclosure, each strand of the double-stranded oligonucleotide comprises 19-21 nucleotides.

In some embodiments of the present disclosure, the nucleotide X modified with Formula I and Formula II is represented by following Formula (A), or is a tautomer of Formula (A):

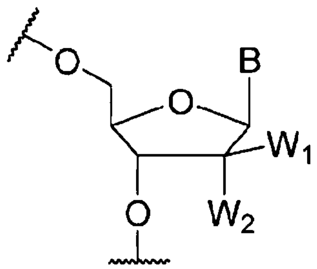

(A)

Herein, B is selected from nucleotide bases, comprising: uracil or derivatives thereof, thymine or derivatives thereof, cytosine or derivatives thereof, 5-methylcytosine or derivatives thereof, adenine or derivatives thereof, and guanine or derivatives thereof.

In some embodiments of the present disclosure, when the modified nucleotide X is a nucleotide modified with a 2'-sterically bulky group, each of $W_1$ and $W_2$ is independently selected from H, -O-MOE, -O-TBDMS, -O-TOM, and -O-

$CH_2$-O-R groups, wherein R is substituted or unsubstituted $C_1$-$C_3$ alkyl; and one of $W_1$ and $W_2$ is H, and $W_1$ and $W_2$ are different.

In some embodiments of the present disclosure, when the modified nucleotide X is a nucleotide modified with 2'-disubstituent, both $W_1$ and $W_2$ are halogens, or one of $W_1$ and $W_2$ is halogen, and the other is $C_1$-$C_6$ alkyl.

In some embodiments of the present disclosure, the halogen is fluorine (F).

In some embodiments of the present disclosure, both $W_1$ and $W_2$ are F.

In some embodiments of the present disclosure, one of $W_1$ and $W_2$ is F, and the other is $C_1$-$C_3$ alkyl.

In some embodiments of the present disclosure, the overhang (T)t1 of the AS strand comprises at least one nucleotide substituted with 2'-F-2'-Me.

In some embodiments of the present disclosure, the double-stranded oligonucleotide further comprises at least one ligand.

In some embodiments of the present disclosure, the ligand includes liver-targeting ligands and non-liver-targeting ligands.

In some embodiments of the present disclosure, the ligand is a liver-targeting ASGPR ligand, and the ASGPR ligand comprises GalNAc attached through a branched linker or a derivative thereof.

In another aspect, the present disclosure further provides a double-stranded oligonucleotide, comprising a sense strand and an antisense strand, each having 17 to 35 nucleotides, wherein each nucleotide is a modified or unmodified nucleotide, the sense strand and the antisense strand are at least partially reversely complementary to form a double-stranded region, wherein the double-stranded oligonucleotide comprises at least one nucleotide modified with 2'-disubstituent, and each substituent in the 2'-disubstituent is independently selected from methyl or fluorine.

Herein, when the double-stranded region of the double-stranded oligonucleotide comprises a nucleotide modified with 2'-disubstituent, the nucleotide modified with 2'-disubstituent is selected from 2'-difluoro substituted nucleotides; or, when the double-stranded oligonucleotide comprises a nucleotide modified with 2'-disubstituent at positions outside the double-stranded region, the nucleotide modified with 2'-disubstituent is selected from nucleotides substituted with 2'-F-2'-Me.

In some embodiments of the present disclosure, the double-stranded oligonucleotide has one or more of features as follows:

(1) when the double-stranded oligonucleotide comprises a 2'-difluoro substituted nucleotide, the 2'-difluoro substituted nucleotide is located at at least one of positions 7, 8, 9, and 10 of the sense strand counting from the 5'-end;
(2) when the double-stranded oligonucleotide comprises a 2'-difluoro substituted nucleotide, the 2'-difluoro substituted nucleotide is located at at least one of positions 2, 4, 6, 8, 9, 10, 12, 14 and 16 of the antisense strand counting from the 5'-end;
(3) when the double-stranded oligonucleotide comprises a nucleotide substituted with 2'-F-2'-Me, the nucleotide substituted with 2'-F-2'-Me is located at a position outside the double-stranded region of the oligonucleotide; and
(4) when the double-stranded oligonucleotide comprises a nucleotide substituted with 2'-F-2'-Me, the nucleotide substituted with 2'-F-2'-Me is located at a overhang at the 3'-end of the antisense strand.

In some embodiments of the present disclosure, in feature (1), when the double-stranded oligonucleotide comprises a 2'-difluoro substituted nucleotide, the 2'-difluoro substituted nucleotide is located at at least two or at least three of positions 7, 8, 9 and 10 of the sense strand counting from the 5'-end.

In some embodiments of the present disclosure, in feature (2), when the double-stranded oligonucleotide comprises a 2'-difluoro substituted nucleotide, the 2'-difluoro substituted nucleotide is located at at least two of positions 2, 4, 6, 8, 9, 10, 12, 14 and 16 of the antisense strand counting from the 5'-end.

In another aspect, the present disclosure further provides an oligonucleotide conjugate, comprising the double-stranded oligonucleotide provided by the present disclosure and a conjugating group conjugated to the double-stranded oligonucleotide. The conjugating group comprises a linker and a pharmaceutically acceptable targeting group and/or a delivery assisting group, the targeting group is selected from ligands capable of binding to a cell surface receptor, and the delivery assisting group is selected from groups capable of increasing the biocompatibility of the oligonucleotide conjugate in a delivery target organ or tissue.

In another aspect, the present disclosure further provides a pharmaceutical composition, comprising the double-stranded oligonucleotide provided by the present disclosure or a conjugate thereof, and a pharmaceutically acceptable carrier.

In another aspect, the present disclosure further provides use of the double-stranded oligonucleotide, the pharmaceutical composition and/or the oligonucleotide conjugate of the present disclosure in the preparation of a medicine for treating and/or preventing diseases or conditions associated with level of mRNA expressed by a target gene.

In another aspect, the present disclosure further provides a method for treating and/or preventing diseases or conditions associated with level of mRNA expressed by a target gene, comprising administering the double-stranded

oligonucleotide, the pharmaceutical composition and/or the oligonucleotide conjugate of the present disclosure to a subject in need thereof.

In another aspect, the present disclosure further provides a method for delivering the double-stranded oligonucleotide of the present disclosure to a specific target in a subject via subcutaneous or intravenous administration.

In another aspect, the present disclosure further provides a method for regulating an expression level of a target gene in a cell, comprising contacting an effective amount of the double-stranded oligonucleotide, the pharmaceutical composition and/or the oligonucleotide conjugate of the present disclosure with the cell.

In addition, the present disclosure further provides a kit, comprising the double-stranded oligonucleotide, the pharmaceutical composition and/or the oligonucleotide conjugate of the present disclosure.

Additional aspects and advantages of the present disclosure will be partially given in the following description, partially become apparent from the following description, or be comprehended through practice of the present disclosure.

**Advantageous effects**

The double-stranded oligonucleotide and/or oligonucleotide conjugate of the present disclosure have good tolerance through specific modification at single or multiple sites of the sense strand and/or antisense strand, can well maintain the pharmaceutical activity of the oligonucleotide, and have relatively high activity of regulating expression of target genes.

## BRIEF DESCRIPTION OF DRAWINGS

In order to more clearly illustrate technical solutions of embodiments of the present disclosure, drawings which need to be used in the embodiments will be briefly introduced below. It should be understood that the drawings merely show some embodiments of the present disclosure, and thus should not be considered as limitation to the scope. Those ordinarily skilled in the art still could obtain other relevant drawings according to these drawings, without using any inventive efforts.

FIG. 1 shows relative expression level of target gene CC3 mRNA after HepG2 was transfected by siRNA modified with TBDMS at different sites of a sense strand in Example 2 of the present disclosure;
FIG. 2 shows relative expression level of target gene CC3 mRNA after HepG2 was transfected by siRNA modified with TBDMS at different sites of an antisense strand in Example 2 of the present disclosure;
FIG. 3 shows expression level of serum CC3 protein in mice following administration of siRNA conjugates in Example 3 of the present disclosure;
FIG. 4 shows relative expression level of target gene ANGPTL3 mRNA following free uptake of siRNA modified with TBDMS at different sites of a sense strand by mouse primary hepatocytes in Example 4 of the present disclosure;
FIG. 5 shows relative expression level of target gene ANGPTL3 mRNA following free uptake of siRNA modified with TBDMS at different sites of an antisense strand by mouse primary hepatocytes in Example 4 of the present disclosure;
FIG. 6 shows expression level of ANGPTL3 mRNA in mice following administration of siRNA conjugates in Example 5 of the present disclosure;
FIG. 7 shows relative expression level of target gene FXI mRNA following free uptake of siRNA modified with TBDMS at different sites of a sense strand by mouse primary hepatocytes in Example 6 of the present disclosure;
FIG. 8 shows relative expression level of target gene FXI mRNA following free uptake of siRNA modified with TBDMS at different sites of an antisense strand by mouse primary hepatocytes in Example 6 of the present disclosure;
FIG. 9 shows expression level of CC3 mRNA in mice following administration of siRNA conjugates in Example 7 of the present disclosure;
FIG. 10 shows relative expression level of target gene CC3 mRNA after HepG2 was transfected by siRNA modified with MOE at different sites of a sense strand in Example 8 of the present disclosure;
FIG. 11 shows relative expression level of target gene CC3 mRNA after HepG2 was transfected by siRNA modified with MOE at different sites of an antisense strand in Example 8 of the present disclosure;
FIG. 12 shows relative expression level of target gene CC3 mRNA after HepG2 was transfected by siRNA modified with MOE at multiple sites of a sense strand in Example 9 of the present disclosure;
FIG. 13 shows relative expression level of target gene CC3 mRNA after HepG2 was transfected by siRNA modified with MOE at multiple sites of an antisense strand in Example 9 of the present disclosure;
FIG. 14 shows relative expression level of target gene CC3 mRNA after HepG2 was transfected by siRNA modified with MOE at multiple sites of a sense strand and an antisense strand in Example 9 of the present disclosure;
FIG. 15 shows expression level of CC3 mRNA in mice following administration of siRNA conjugates (RZ502031, RZ502033, RZ502034, RZ502035, and RZ502039) in Example 10 of the present disclosure;
FIG. 16 shows expression level of CC3 mRNA in mice following administration of siRNA conjugates (RZ502031,

RZ502036, RZ502049, and RZ502087) in Example 10 of the present disclosure;

FIG. 17 shows expression level of SOD1 mRNA in mice following administration of siRNA conjugates in Example 11 of the present disclosure;

FIGS. 18a-d show relative expression level of target gene CC3 mRNA after HepG2 was transfected by siRNA conjugates (different modification schemes of RZ002003, RZ002006, RZ002011 and RZ002031) in Example 12 of the present disclosure.

FIG. 19 shows relative expression level of target gene CC3 mRNA after Huh7 was transfected by siRNA conjugates (different modification schemes of RZ003017 and RZ003020) in Example 13 of the present disclosure.

FIG. 20 shows AGT protein expression level in serum of hREN×hAGT hypertensive mice following administration of siRNA conjugates (RZ003021, RZ003065, and RZ003066) in Example 14 of the present disclosure.

FIG. 21 shows relative expression level of target gene mRNA in LPA-targeting HDI mouse models following administration of siRNA conjugates (RZ001032, RZ001034, RZ001037, and RZ001039) in Example 15 of the present disclosure.

FIG. 22 shows LPA protein expression level in hApo(a) transgenic mice following administration of siRNA conjugates (RZ001032 and RZ001034) in Example 16 of the present disclosure.

FIG. 23 shows relative expression level of SOD1 mRNA in mice following administration of siRNA conjugates modified with NM062 and NM063 at different sites of an antisense strand in Example 17 of the present disclosure.

FIG. 24 shows relative expression level of target gene SOD1 mRNA following free uptake of siRNA conjugates modified with NM096, NM097, NM098 and NM099 at different sites of an antisense strand by mouse primary hepatocytes in Example 18 of the present disclosure;

FIG. 25 shows relative expression level of target gene SOD1 mRNA following free uptake of siRNA conjugate (RZ599051) modified with 2'-difluoro on a sense strand by mouse primary hepatocytes in Example 19 of the present disclosure.

FIG. 26 shows relative expression level of target gene SOD1 mRNA following free uptake of siRNA conjugates (RZ599055 and RZ599056) modified with 2'-difluoro on a sense strand by mouse primary hepatocytes in Example 20 of the present disclosure.

FIG. 27 shows relative expression level of target gene ANGPTL3 mRNA following free uptake of siRNA conjugates modified with 2'-difluoro on a sense strand by mouse primary hepatocytes in Example 21 of the present disclosure.

FIG. 28 shows relative expression level of target gene CFB mRNA following free uptake of siRNA conjugates modified with 2'-difluoro on a sense strand by mouse primary hepatocytes in Example 22 of the present disclosure.

FIG. 29 shows relative expression level of target gene CFB mRNA in mice following administration of siRNA conjugates modified with 2'-difluoro on a sense strand in Example 23 of the present disclosure.

FIG. 30 shows relative expression level of target gene ANGPTL3 mRNA following free uptake of siRNA conjugates modified with 2'-difluoro on an antisense strand by mouse primary hepatocytes in Example 24 of the present disclosure.

FIG. 31 shows relative expression level of target gene CFB mRNA following free uptake of siRNA conjugates (RZM11504 and RZM11505) modified with 2'-difluoro on an antisense strand by mouse primary hepatocytes in Example 25 of the present disclosure.

FIG. 32 shows relative expression level of target gene CFB mRNA following free uptake of siRNA conjugate (RZM11507) modified with 2'-difluoro on an antisense strand by mouse primary hepatocytes in Example 26 of the present disclosure.

FIG. 33 shows relative expression level of target gene CFB mRNA in mice following administration of siRNA conjugates modified with 2'-difluoro on an antisense strand in Example 27 of the present disclosure.

FIG. 34 shows relative expression level of target gene C4B mRNA in mice following administration of siRNA conjugates modified with 2'-difluoro on an antisense strand in Example 28 of the present disclosure.

FIG. 35 shows relative expression level of target gene SOD1 mRNA in mice following administration of siRNA conjugates comprising NM054 modification on an antisense strand in Example 29 of the present disclosure.

FIG. 36 shows relative expression level of target gene ANGPTL3 mRNA in mice following administration of siRNA conjugates comprising NM054 modification on an antisense strand in Example 30 of the present disclosure.

FIG. 37 shows CC3 protein expression level in cynomolgus monkey serum following administration of siRNA conjugates (RZ002099, RZ002101, RZ002106, and RZ002113) in Example 31 of the present disclosure.

FIG. 38 shows AGT protein expression level in cynomolgus monkey serum following administration of siRNA conjugate (RZ003069) in Example 32 of the present disclosure.

## DETAILED DESCRIPTION OF EMBODIMENTS

Embodiments of the present disclosure will be described in detail below. The embodiments described below are illustrative, merely for explaining the present disclosure, but should not be construed as limitation to the present disclosure.

It should be noted that the terms "first" and "second" are merely used for descriptive purpose, but should not be construed as indicating or implying importance in the relativity or implicitly indicating the number of a related technical feature. Thus, defining a feature with "first" or "second" may explicitly or implicitly mean that one or more such features are comprised. Further, in the description of the present disclosure, unless otherwise stated, "a plurality of (multiple)" means two or more.

Endpoints and any value of ranges disclosed herein are not limited to the precise ranges or values, and these ranges or values should be understood as encompassing values close to these ranges or values. For numerical ranges, endpoint values of various ranges can be combined with each other, endpoint values of various ranges and individual point values can be combined with each other, and individual point values can be combined with each other to obtain one or more new numerical ranges, which numerical ranges should be construed as being specifically disclosed herein.

In order to make the present disclosure easier to understand, certain technical and scientific terms are specifically defined below. Unless obviously defined otherwise in this document, all other technical and scientific terms used herein have the meanings as commonly understood by those of ordinary skill in the art to which the present disclosure belongs.

The term "containing" or "comprising" herein is an open-ended expression, i.e., comprising what is specified in the present disclosure, but not excluding contents in other aspects.

The term "optionally" or "optional" herein generally mean that the event or case subsequently described can but may not occur, and that the description comprises situations wherein the event or case occurs, and situations wherein the event or case does not occur.

The term "complement component 3" (CC3) herein refers to an intrinsic component involved in three complement activation pathways, and is a complement component with the highest plasma content. During complement activation, C3 can be cleaved by C3 convertase into small fragment C3a and large fragment C3b: C3a is free in liquid phase and has anaphylotoxin activity; C3b is a key component of C5 convertases of the three activation pathways and C3 convertase of alternative pathway, and can bind to immune complexes, microorganisms, high-molecular substances or cell membranes, thereby mediating opsonization and immune adherence effect.

The term "angiopoietin-like protein 3" (ANGPTL3) herein is a member of the angiopoietin-like protein family participating in regulating blood lipid metabolism, and the family thereof also comprises ANGPTL4 and ANGPTL8. Protein encoded by ANGPTL3 gene is only produced in liver, and further secreted into circulation system. In liver, ANGPTL3 is exclusively produced by hepatocytes, and its expression is regulated by hydroxysterol-activated liver X receptor (LXR).

The term "coagulation Factor XI" (FXI) herein is a plasma glycoprotein involved in a contact stage of a coagulation process (intrinsic coagulation pathway), and FXI is quite important for normal hemostasis *in vivo.*

The term "oligonucleotide" herein refers to a nucleic acid molecule (RNA or DNA), for example, having less than 100, 200 or 300 nucleotides in length.

The term "LNA" herein refers to locked nucleic acid. LNA is a modified RNA nucleotide. Ribose moiety of an LNA nucleotide is modified by linking 2'-hydroxyl to 4'-carbon of the same ribose by an additional bridge (e.g., methylene or ethylene bridge). The term "ENA" refers to ethylene bridged nucleic acid, and usually refers to restricted or inaccessible RNA. The term "BNA" refers to bridged nucleic acid. BNA may comprise a 5-membered, 6-membered, or even 7-membered bridged structure with "fixed" C3'-endo sugar condensation, and the bridge is typically incorporated at 2'-, 4'-positions of the ribose so as to provide a 2'-, 4'-BNA nucleotide (e.g., LNA or ENA). The term GNA refers to glycerol nucleic acid, and UNA refers to seco nucleotide (unlocked nucleotide).

Herein, unless particularly specified, capital letters C, G, U, A, and T represent base composition of nucleotides; lowercase letter m means that a nucleotide adjacent to the left of the letter m is a nucleotide modified with methoxy; lowercase letter f means that a nucleotide adjacent to the left of the letter f is a nucleotide modified with fluoro; and lowercase letter s means that two nucleotides adjacent to the left and right of the letter s are linked by a phosphorothioate group.

The "nucleotide modified with fluoro" herein refers to a nucleotide in which 2'-hydroxyl group of ribose group is substituted with fluorine. Unless specifically stated, fluoro modification refers to mono-fluoro modification, and "nucleotide-like conpound" or "nucleotide analog" refers to a group capable of replacing a nucleotide in a nucleic acid, but structurally distinct from adenine ribonucleotide, guanine ribonucleotide, cytosine ribonucleotide, uracil ribonucleotide or thymine deoxyribose nucleotide, for example, isonucleotide, bridged nucleic acid (BNA for short) or acyclic nucleotide. The "nucleotide modified with methoxy" refers to a nucleotide formed by substituting 2'-hydroxy group of ribose group with methoxy group. 2-methoxy herein may also be represented by 2'-OMe, and 2-methoxy and 2'-OMe herein are used interchangeably. Unless stated otherwise, in the context, the term "modified nucleotide" refers to a nucleotide in which 2'-position of ribose group is modified.

The nucleotide herein may refer to an independent nucleotide, or a nucleotide residue in an oligonucleotide; the nucleotide herein may be in singular or plural, and when in plural, it can refer to a class of nucleotides. The double-stranded oligonucleotide herein can also be represented by dsRNA, siRNA or double-stranded oligomeric nucleotide, and the double-stranded oligonucleotide, the double-stranded oligomeric nucleotide, dsRNA and siRNA herein are used interchangeably.

The "alkyl" herein refers to straight chain and branched chain having a specified number of carbon atoms, typically 1 to 20 carbon atoms. For example, $C_1$-$C_6$ alkyl encompasses straight-chain and branched-chain alkyl comprising 1 to 6 carbon atoms.

The siRNA molecule herein consists of two strands, in which a strand that binds to target mRNA is referred to as an antisense strand or a guide strand, and the other is referred to as a sense strand or a passenger strand. The term "antisense strand" refers to such a strand of siRNA that comprises a region fully or substantially complementary to a target sequence. The term "sense strand" refers to such a strand of siRNA that comprises a region substantially complementary to a region of the term antisense strand as defined herein. The term "complementary region" refers to a region on the antisense strand that is fully or substantially complementary to the target mRNA sequence. Under a condition that the complementary region is not fully complementary to the target sequence, mismatching (mispairing) may occur within internal or terminal region of a molecule. As used herein, the term "complementary" refers to the ability of a first polynucleotide to hybridize with a second polynucleotide under certain conditions, e.g., stringent conditions.

In the present context, expressions "duplex", "duplex region" and "double-stranded region" are used interchangeably and have the meaning well known to those skilled in the art, that is, a double-stranded structure formed by pairing a sense strand and an antisense strand pair in a complementary manner in a double-stranded nucleic acid molecule.

The expressions "complementary" and "reversely complementary" herein are used interchangeably, and have the meaning well known to those skilled in the art, that is, bases of one strand are respectively paired with bases of the other strand in a complementary manner in a double-stranded nucleic acid molecule.

Herein, unless particularly specified, the term "complementary" encompasses "substantially reversely complementary", "virtually reversely complementary" and "fully reversely complementary". "Substantially reversely complementary" means that there are no more than 3 base mismatches between two nucleotide sequence segments involved; "virtually reversely complementary" means that there is no more than one base mismatch between two nucleotide sequence segments; and "fully reversely complementary" means that there is no base mismatch between two nucleotide sequence segments.

Herein, unless otherwise stated, "conjugation" means that two or more chemical moieties each having a specific function are covalently linked to each other. Accordingly, "conjugate" refers to a compound formed by covalent linkage between various chemical moieties. Further, "oligonucleotide conjugate" refers to a compound formed by covalently linking one or more chemical moieties with specific functions to an oligonucleotide. In the context of the present disclosure, "conjugating molecule" should be understood as a specific compound that may be conjugated to an oligonucleotide through reaction, ultimately forming the oligonucleotide conjugate of the present disclosure.

The inventors, through repeated research and experimentation, have found that a double-stranded oligonucleotide can have good tolerance through specific modification at single or multiple sites among specific sites of a sense strand and/or an antisense strand, and can well maintain and even further elevate activity of a double-stranded oligonucleotide molecule.

In the first aspect, the present disclosure provides a modified double-stranded oligonucleotide molecule and reagents thereof that maintain and even enhance inhibitory activity, and a pharmaceutical composition suitable for therapeutic use.

In a specific embodiment, the present disclosure provides a double-stranded oligonucleotide (dsRNA) for inhibiting expression of a target gene, wherein the double-stranded oligonucleotide comprises a sense strand and an antisense strand, each having 17 to 35 nucleotides, each nucleotide being a modified or unmodified nucleotide; the sense strand and the antisense strand are at least partially reversely complementary to form a double-stranded region; in a direction from 5'-end to 3'-end, a nucleotide sequence of the strand sense and/or the antisense strand has a nucleotide with sterically bulky modification and/or a nucleotide modified with disubstituent at a specific position, wherein the nucleotide with sterically bulky modification refers to a nucleotide in which 2'-hydroxyl group of ribose of the nucleotide is substituted with a sterically bulky group, and the 2'-sterically bulky group is selected from groups having a greater steric bulk than 2'-O-methyl; the nucleotide modified with disubstituent refers to a nucleotide in which both 2'-hydroxyl group and hydrogen of ribose of the nucleotide are simultaneously substituted. Optionally, each substituent in the disubstituent is independently selected from $C_1$-$C_6$ alkyl or halogens.

In some embodiments, in the double-stranded oligonucleotide of the present disclosure, in the direction from the 5'-end to the 3'-end, at least one of nucleotides at position 8, 9, 10 and 15 of the antisense strand is a nucleotide with sterically bulky modification or a nucleotide modified with disubstituent; and/or, a modified nucleotide X is present or absent in the sense strand; when the sense strand comprises the modified nucleotide X, at least one nucleotide X is located at a site opposite to any of positions 2, 8 and 15 of the antisense strand (in the direction from the 5'-end to the 3'-end).

In some embodiments, the antisense strand and the sense strand of the double-stranded oligonucleotide are complementary to form a double-stranded region, a structure of which is as represented by following Formula (I):

SS: 5'- $(N)_{a'}$ - $(X)_{p'}$ - $(N)_{b'}$ - $(X)_{q'}$ - $(N)_{c'}$ - $(X)_{r'}$ - $(N)_{d'}$ -3'
AS:

3' - $(N)_a$ - $(X)_p$ - $(N)_b$ - $(X)_q$ - $(N)_c$ -5' (I).

Herein, SS represents the sense strand, and AS represents the antisense strand.

In Formula (I), each X independently represents a nucleotide in which 2'-hydroxyl group of ribose is substituted with a sterically bulky group or a nucleotide in which 2'-hydroxyl group and hydrogen are substituted with disubstituent, wherein the 2'-sterically bulky group is selected from groups with a greater steric bulk than 2'-methoxy; and each substituent in the disubstituent is independently selected from $C_1$-$C_6$ alkyl group or halogens.

In some embodiments, when X represents a nucleotide in which 2'-hydroxyl group of ribose is substituted with a sterically bulky group, it is independently selected from the following nucleotides modified at 2'-position: 2'-$(O)_{m1}(CH_2)_n(O)_{m2}R_1$, wherein each of m1 and m2 is independently 0 or 1, and n is an integer selected from 0 to 6; $R_1$ is selected from substituent-substituted or unsubstituted $C_1$-$C_6$ alkyl, and -$Si(R_2)_3$, wherein each $R_2$ is independently selected from substituted or unsubstituted $C_1$-$C_6$ alkyl, and substituted or unsubstituted $C_1$-$C_6$ alkoxy, wherein the substitution or substituent is selected from one or more of: halogen, $C_1$-$C_6$ alkyl, $C_1$-$C_6$ alkoxy, hydroxyl group, amino group, cycloalkyl having up to 6 carbon atoms, aryl having up to 12 carbon atoms or heteroaryl having up to 12 carbon atoms.

In some embodiments, each X is independently selected from following modified nucleotides: 2'-$O(CH_2)_nOR_1$ or 2'-$R_3$-2'-$R_4$. Herein, n is 1 or 2, $R_1$ is selected from substituent-substituted or unsubstituted $C_1$-$C_6$ alkyl, and -$Si(R_2)_3$, wherein each $R_2$ is independently selected from substituted or unsubstituted $C_1$-$C_6$ alkyl, and substituted or unsubstituted $C_1$-$C_6$ alkoxy, wherein the substituent is selected from halogen, $C_1$-$C_6$ alkyl, $C_1$-$C_6$ alkoxy, hydroxyl group, amino group, cycloalkyl having up to 6 carbon atoms, aryl having up to 12 carbon atoms or heteroaryl having up to 12 carbon atoms.

Herein, each of $R_3$ and $R_4$ is independently selected from $C_1$-$C_6$ alkyl or halogen. Optionally, the halogen is fluorine. Optionally, the $C_1$-$C_6$ alkyl is selected from methyl group or ethyl group.

In Formula I, each N independently represents an unmodified nucleotide, or a nucleotide which is different from X and in which 2'-position of ribose is modified. The modified nucleotide is selected from the group consisting of 2'-O-alkyl, 2'-alkyl, 2'-substituted alkyl, 2'-halo, 2'-deoxy, and a nucleotide analog. The nucleotide analog is selected from the group consisting of ENA, BNA, LNA, GNA and UNA; and optionally, the alkyl group or alkoxy group has 1-6 carbon atoms.

In some embodiments, each N is independently selected from nucleotide modified with 2'-OMe, nucleotide modified with 2'-F and nucleotide modified with 2'-H, wherein nucleotides modified with 2'-H and 2'-deoxy have the same meaning, i.e., 2'-hydroxy is substituted with H. In some other embodiments, each N is independently selected from nucleotides modified with 2'-OMe and 2'-F.

In some embodiments, each nucleotide X in which 2'-hydroxyl group is substituted with a sterically bulky group is independently selected from the following modified nucleotides: 2'-O-methoxyethyl (MOE), 2'-O-TBDMS, 2'-O-TOM, and 2'-O-$CH_2$-O-R groups, R being substituted or unsubstituted $C_1$-$C_3$ alkyl; and optionally, the substituents are independently selected from one or more of: halogens, $C_1$-$C_3$ alkyl, $C_1$-$C_3$ alkoxy, and amino groups.

In some embodiments, each X is independently selected from the following modified nucleotides : 2'-O-methoxyethyl (MOE), 2'-O-$CH_2$-O-$CH_2$-$CH_3$, and 2'-O-$CH_2$-O-$CH_2$-$CF_3$.

In some embodiments, the nucleotide in which the 2'-position is substituted with a disubstituent is nucleotide modified with [2'-halo-2'-halo] or [2'-halo-2'-alkyl] or [2'-alkyl-2'-alkyl], optionally nucleotide modified with [2'-F-2'-F] or [2'-fluoro-2'-alkyl]. Optionally, the alkyl is $C_1$-$C_3$ alkyl.

In Formula I, each of a, a', p, p', b, b', q, q', c, c', r', and d' independently represents the number of nucleotides. For example, when a is 3, (N)a represents 3 contiguous modified nucleotides N-N-N, wherein each N may be the same or different. Herein, a' is an integer from 3 to 8; p' is an integer from 0 to 3; b' is an integer from 4 to 13; q' is an integer from 0 to 4; c' is an integer from 3 to 9; r' is an integer from 0 to 3; d' is an integer from 0 to 9; a is an integer from 4 to 7; p is an integer from 0 to 1; b is an integer from 4 to 8; q is an integer from 0 to 4; c is an integer from 6 to 10; and p', q', r', p and q are not simultaneously 0.

Optionally, q' and r' are not simultaneously 0.

Optionally, $q'+c'+r'+d'=9$, and $0 \leq q'+r' \leq 4$.

Optionally, (N)a and (N)b each comprise at least one nucleotide modified with fluoro, and (N)c comprises at least two fluoro-modified nucleotides.

In some embodiments, the nucleotide X in the present disclosure may have a structure as represented by following Formula (A), or is a tautomer of Formula (A):

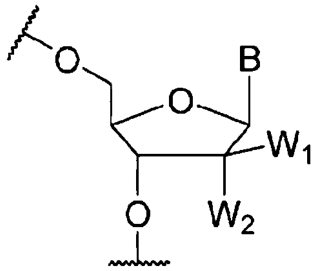

(A)

Herein, B is selected from nucleotide bases, including: uracil or derivatives thereof, thymine or derivatives thereof, cytosine or derivatives thereof, 5-methylcytosine or derivatives thereof, adenine or derivatives thereof, or guanine or derivatives thereof.

In some embodiments of the present disclosure, when the modified nucleotide X is a nucleotide modified with a 2'-sterically bulky group, each of $W_1$ and $W_2$ is independently selected from H, -O-MOE, -O-TBDMS, -O-TOM, and -O-$CH_2$-O-R groups, wherein R is substituted or unsubstituted $C_1$-$C_3$ alkyl; and one of $W_1$ and $W_2$ is H, and $W_1$ and $W_2$ are different.

In some embodiments of the present disclosure, when the modified nucleotide X is a nucleotide modified with 2'-disubstituent, both $W_1$ and $W_2$ are halogens, or one of $W_1$ and $W_2$ is halogen, and the other is $C_1$-$C_6$ alkyl.

In some embodiments of the present disclosure, the halogen is fluorine (F).

In some embodiments of the present disclosure, both $W_1$ and $W_2$ are F.

In some embodiments of the present disclosure, one of $W_1$ and $W_2$ is F, and the other is methyl or ethyl.

Herein, a wavy line is a linkage between nucleotides.

In some embodiments, in addition to the above double-stranded region, the double-stranded oligonucleotide of the present disclosure further comprises one or more overhang regions that may have a length of 1-6 nucleotides. The overhang may make one strand longer than the other, or make two strands have the same length. The overhang can form a mismatch with the target mRNA, or it can be complementary to a targeted gene sequence, or other sequences. For example, the sense strand and the antisense strand may be linked by an additional base to form a hairpin, or linked by other non-base linker. In an example, each of nucleotides in the overhang group may be independently a modified or unmodified nucleotide.

In an example, the overhang is present at 3'-end of the sense strand, the antisense strand or both. Optionally, the double-stranded oligonucleotide of the present disclosure comprises only one overhang. In another example, the 3'-overhang is present in the antisense strand. In another example, the 3'-overhang is present in the sense strand.

In some embodiments of the present disclosure, the double-stranded oligonucleotide molecule comprising the overhang region can be represented by following Formula II:

SS: $5'\text{-}(T)_{t1}\text{-}(N)_{a'}\text{-}(X)_{p'}\text{-}(N)_{b'}\text{-}(X)_{q'}\text{-}(N)_{c'}\text{-}(X)_{r'}\text{-}(N)_{d'}\text{-}(T)_{t2}\text{-}3'$
AS:

$$3'\text{-}(T)_{t1}\text{-}(N)_a\text{-}(X)_p\text{-}(N)_b\text{-}(X)_q\text{-}(N)_c\text{-}(T)_{t2}\text{-}5' \qquad \text{(II)}$$

Herein, SS represents the sense strand, AS represents the antisense strand; T represents the overhang nucleotide, each of t1 and t2 is independently an integer selected from 0 to 6, and cannot be simultaneously 0; and the remaining substituents are as defined for the above Formula (I).

Herein, each of the overhang nucleotides T independently represents a modified or unmodified nucleotide, wherein the modified nucleotide is selected from the group consisting of 2'-alkoxy, 2'-$C_1$-$C_6$ substituted alkoxy, 2'-alkyl, 2'-$C_1$-$C_6$ substituted alkyl, 2'-halo, 2'-deoxy, abasic nucleotide, and an inverted nucleotide; and optionally, the alkyl or alkoxy has 1-6 carbon atoms, preferably, 1-3 carbon atoms.

In some embodiments of the present disclosure, the antisense strand of the double-stranded oligonucleotide has a nucleotide overhang at the 3'-end; and the overhang comprises 1-3 nucleotides. In this case, the double-stranded oligonucleotide molecule can be represented by following Formula (IIa):

SS: $5'\text{-}(N)_{a'}\text{-}(X)_{p'}\text{-}(N)_{b'}\text{-}(X)_{q'}\text{-}(N)_{c'}\text{-}(X)_{r'}\text{-}(N)_{d'}\text{-}3'$
AS:

$$3'\text{-}(T)_{t1}\text{-}(N)_a\text{-}(X)_p\text{-}(N)_b\text{-}(X)_q\text{-}(N)_c\text{-}5' \qquad \text{(IIa)..}$$

Herein, t1 is an integer selected from 1 to 3, and the remaining substituents are as defined for the above Formula (II).

In some examples, the overhang group (T)t1 has a phosphorothioate group between two nucleotides. In an embodiment, the double-stranded oligonucleotide of the present disclosure may further have two blunt ends at two ends of its duplex.

In some embodiments, a double-stranded region of the double-stranded oligonucleotide of the present disclosure has 17-30 nucleotide pairs in length. For example, the double-stranded region has 17-25 nucleotide pairs, 17-21 nucleotide pairs, 17-19 nucleotide pairs, or 19-25 nucleotide pairs, 19-23 nucleotide pairs, 19-21 nucleotide pairs, or 21-25 nucleotide pairs, 21-23 nucleotide pairs.

In some embodiments, the length of the double-stranded region is selected from 17, 18, 19, 20, 21, 22, and 23 nucleotide pairs.

In an embodiment, the duplex region has 19 or 21 nucleotide pairs in length.

In some embodiments, in the double-stranded oligonucleotide of the present disclosure, the antisense strand and the sense strand in the double-stranded region represented by Formula (I) are complementary, encompassing: substantially reversely complementary, virtually reversely complementary or fully reversely complementary. In an embodiment, the antisense strand and the sense strand in the double-stranded region represented by Formula (I) are fully reversely complementary. In another embodiment, the antisense strand and the sense strand in the double-stranded region represented by Formula (I) comprise 1-3 base mismatches, thereby achieving substantially or virtually reverse complementarity.

In some embodiments, each strand of the double-stranded oligonucleotide represented by Formula (II) provided by the present disclosure can have 17-35 nucleotides in length. For example, each strand of the double-stranded oligonucleotide may have 17-23 nucleotides, 17-21 nucleotides, 17-19 nucleotides, or 19-25 nucleotides, 19-23 nucleotides, 19-21 nucleotides, and 21-23 nucleotides.

In some embodiments, in the double-stranded oligonucleotide molecule of the present disclosure, the nucleotide X is a nucleotide modified with a sterically bulky group, and one or more nucleotides X are present and only in the antisense strand. In some embodiments, in the direction from the 5'-end to the 3'-end, when the antisense strand comprises only one X, X may be located at any of positions 8-10 and position 15 of the antisense strand. Optionally, X may be located at position 10 or 15 of the antisense strand.

In some embodiments, in the double-stranded oligonucleotide molecule of the present disclosure, the nucleotide X is a nucleotide modified with a sterically bulky group, and one or more X are present and only in the sense strand. Optionally, the sense strand may comprise one or two X. Optionally, X in the sense strand may be located at a position opposite to any of positions 2, 8 and 15 (counting from the 5'-end) of the antisense strand. Herein, the opposite position refers to a position (i.e., opposite site) where a nucleotide on one strand in a duplex region is complementary to a corresponding nucleotide on the other strand.

In some embodiments, the nucleotide X is a nucleotide modified with a sterically bulky group; in a double-stranded oligonucleotide molecule comprising the double-stranded region structure of the above Formula (I) in the present disclosure, when the duplex is 19 nucleotide pairs (or the double-stranded oligonucleotide molecule is 19/21 nucleotides), at least one nucleotide X is present at positions 7-9 (in the 5'-3' direction) of the sense strand, and positions 7-9 of the sense strand are opposite to nucleotides at positions 11-13 (in the 5'-3' direction) of the antisense strand.

In some embodiments, the nucleotide X is a nucleotide modified with a sterically bulky group; in a double-stranded oligonucleotide molecule comprising the duplex structure of the above Formula (I) of the present disclosure, two or more nucleotides X are present and respectively located in both the antisense strand and the sense strand. In some embodiments, when the antisense strand comprises only one X, the sense strand may comprise one or two X. In some embodiments, X may be located at any of positions 8-10 and 15 (counting from the 5'-end) of the antisense strand; and meanwhile, X in the sense strand can be located at a position opposite to any of positions 2, 8, 12 and 15 (counting from the 5'-end) of the antisense strand.

In some embodiments, the nucleotide X is a nucleotide modified with a sterically bulky group; and in the double-stranded oligonucleotide molecule, at least one X is present and located at position 15 (counting from the 5'-end) of the antisense strand.

In some embodiments, the 2'-sterically bulky group is 2'-O-methoxyethyl (MOE). In some other embodiments, the 2'-sterically bulky group is 2'-O-$CH_2$-O-$CH_2$-$CH_3$, or 2'-O-$CH_2$-O-$CH_2$-$CF_3$.

In some embodiments, in the double-stranded oligonucleotide represented by Formula (II) of the present disclosure or a double-stranded oligonucleotide comprising the double-stranded region structure represented by Formula (I), each N is independently selected from nucleotide modified with 2'-OMe, nucleotide modified with 2'-F and nucleotide modified with 2'-deoxy; each X is independently selected from nucleotide modified with 2'-O-MOE, nucleotide modified with 2'-O-TBDMS, nucleotide modified with 2'-O-TOM, nucleotide modified with 2'-O-$CH_2$-O-$CH_2$-$CH_3$, and nucleotide

modified with 2'-O-$CH_2$-O-$CH_2$-$CF_3$, wherein each of a, a', p, p', b, b', q, q', c, c', r', and d' independently represents the number of nucleotides and is defined as follows: a' is an integer from 3 to 8; p' is an integer from 0 to 3; b' is an integer from 4 to 13; q' is an integer from 0 to 4; c' is an integer from 3 to 9; r' is an integer from 0 to 3; d' is an integer from 0 to 9; a is an integer from 4 to 7; p is an integer from 0 to 1; b is an integer from 4 to 8; q is an integer from 0 to 4; c is an integer from 6 to 10; and p', q', r', p and q are not simultaneously 0.

Optionally, q' and r' are not simultaneously 0.

Optionally, q'+c'+r'+d'=9, and 0≤q'+r'≤4.

Optionally, (N)a and (N)b each comprise at least one nucleotide modified with fluoro, and (N)c comprises at least two fluoro-modified nucleotides.

In an embodiment, when p'=0, that is, when (X)p' is absent, a'=6. In another embodiment, when the double-stranded oligonucleotide is SS/AS=21/23 nt, a'=8.

In an embodiment, when p'=1, X is located on the sense strand, and at any position opposite to positions 14-16 of the antisense strand (counting from the 5'-end).

In an embodiment, (X)p' comprises at least one X and one X is located at a position opposite to position 15 (counting from the 5'-end) of the antisense strand; and optionally, when p'=1, X is located at a position opposite to position 15 (counting from the 5'-end) of the antisense strand.

In an embodiment, when neither (X)q' nor (X)r' is present (that is, both q' and r' are 0), the sense strand comprises only (X)p', and then the sense strand SS of the double-stranded region is: 5'- $(N)_{a'}$ - $(X)_{p'}$ -$(N)_{k'}$ - $(N)_{d'}$ -3', wherein k'=7-22.

In an embodiment, the first four nucleotides (counting from the 5'-end) on the sense strand (N)b' are located at positions opposite to positions 10-13 (counting from the 5'-end) of the antisense strand, and the first four nucleotides comprise at least two fluoro-modified nucleotides.

In an embodiment, q' is preferably from 0 to 2, and further preferably from 0 to 1.

In an embodiment, when neither r' nor q' is 0, d' is not 0. For example, when r'=1 and q'=1, b' and c' may be 5, and d' is 1 (in this case, one X on the sense strand is located at position 12, and the other X is located at position 18, counting from the 5'-end).

In an embodiment, q' and r' are not simultaneously 0. Further, when q' and r' are not simultaneously 0, at least one X on the sense strand is located at a position opposite to a seed region (positions 1-8, counting from the 5'-end) of the antisense strand. In another embodiment, the sense strand comprises at least one X at position opposite to positions 2-8 (counting from the 5'-end) of the seed region of the antisense strand.

In an embodiment, d'=1-9.

In an embodiment, q'+c'+r'+d'=9, and 0≤q'+r'≤4.

In an embodiment, when (X)p is absent, a=5-7. For example, when dsRNA is SS/AS=21/23 nt, a=7.

In an embodiment: (N)a comprises at least one nucleotide modified with fluoro, preferably at position 16 (counting from the 5'-end) of the antisense strand.

In an embodiment, when p≠0 (i.e., p=1), X is located at position 15 (counting from the 5'-end) of the antisense strand.

In an embodiment, when (X)q is absent, b is 8 (i.e., when q=0, b=8).

In an embodiment, when (X)q is absent, b+c=14.

In an embodiment: (N)b comprises at least one nucleotide modified with fluoro, preferably at position 14 (counting from the 5'-end) of the antisense strand.

In an embodiment, when (X)q is absent, c is 10.

In a preferred embodiment, (N)c comprises at least two fluoro-modified nucleotides; and further preferably, at least two fluoro-modified nucleotides are present and respectively located at positions 2 and 6 (counting from the 5'-end) of the antisense strand.

In some embodiments, in the double-stranded oligonucleotide represented by Formula (II) of the present disclosure or the double-stranded oligonucleotide comprising the double-stranded region represented by the above Formula (I), sequences of the antisense strand and the sense strand are sufficiently complementary to each other so as to form a double-stranded region of 17-23 base pairs, wherein each N is independently nucleotide modified with 2'-OMe, nucleotide modified with 2'-F or nucleotide modified with 2'-deoxy; each X is independently selected from nucleotides modified with 2'-O-MOE, 2'-O-TBDMS, 2'-O-TOM, 2'-O-$CH_2$-O-$CH_2$-$CH_3$, and 2'-O-$CH_2$-O-$CH_2$-$CF_3$, each of a', p, p', b, b', q, q', c, c', r', and d' independently represents the number of nucleotides, further defined as follows:

a' is an integer from 3 to 8; p' is an integer from 0 to 2; b' is an integer from 4 to 12; q' is an integer from 0 to 2; c' is an integer from 3 to 8; r' is an integer from 0 to 2; d' is an integer from 1 to 9; a is an integer from 4 to 7; p is 0 or 1; b is an integer from 4 to 8; q is an integer from 0 to 2; and c is an integer from 6 to 10; and

1) (N)a and (N)b each comprise at least one nucleotide modified with fluoro, and (N)c comprises at least two fluoro-modified nucleotides; q'+c'+r'+d'=9, and 0≤q'+r'≤4; and
2) the first four nucleotides (counting from the 5'-end) of (N)b' comprise at least two fluoro-modified nucleotides.

In some embodiments, in the double-stranded oligonucleotide represented by Formula (II) of the present disclosure or the double-stranded oligonucleotide comprising the double-stranded region represented by the above Formula (I), sequences of the antisense strand and the sense strand are sufficiently complementary to each other so as to form a duplex region of 19-21 base pairs, wherein each N is independently selected from nucleotide modified with 2'-OMe, nucleotide modified with 2'-F and nucleotide modified with 2'-deoxy; each X is independently selected from nucleotides modified with 2'-O-MOE, 2'-O-TBDMS, 2'-O-TOM, 2'-O-$CH_2$-O-$CH_2$-$CH_3$, and 2'-O-$CH_2$-O-$CH_2$-$CF_3$, each of a', p, p', b, b', q, q', c, c', r', and d' independently represents the number of nucleotides: a' is an integer from 3 to 8; p' is 0 or 1; b' is an integer from 4 to 13; q' is 0 or 1; c' is an integer from 3 to 9; r' is 0 or 1; d' is an integer from 1 to 8; a is an integer from 4 to 7; p is 1; b is an integer from 4 to 8; q is 0 or 1; and c is an integer from 6 to 10; and

1) (N)a comprises at least one nucleotide modified with fluoro, and position 16 (counting from the 5'-end) of the antisense strand is a nucleotide modified with fluoro;
2) (N)b comprises at least one nucleotide modified with fluoro, and position 14 (counting from the 5'-end) of the antisense strand is a nucleotide modified with fluoro;
3) (N)c comprises at least two fluoro-modified nucleotides, and positions 2 and 6 (counting from the 5'-end) of the antisense strand are both fluoro-modified nucleotides;
4) $q'+c'+r'+d'=9$, $0 \leq q'+r' \leq 2$; and optionally, q' and r' are not simultaneously 0;
5) the sense strand comprises at least one X at a position opposite to the seed region (positions 1-8, counting from the 5'-end) of the antisense strand; and
6) the first four nucleotides (based on the 5'-3' direction) of (N)b' are located at positions opposite (opposite sites) to positions 10-13 of the antisense strand (counting from the 5'-end), and the first four nucleotides comprise at least two fluoro-modified nucleotides, and optionally comprise one or at least one nucleotide modified with non-fluoro. The nucleotide modified with non-fluoro may be selected from one or more of nucleotide X with 2'-bulky modification, nucleotide modified with 2'-deoxy, and nucleotide modified with 2'-methoxy.

Optionally, the nucleotide modified with non-fluoro is the nucleotide X with 2'-bulky modification, and X is located in the first three nucleotides of (N)b'. Further optionally, X is located in the first two nucleotides of (N)b'.

In an embodiment, the first four nucleotides (counting from the 5'-end) of (N)b' comprise two or three fluoro-modified nucleotides, and one nucleotide X with 2'-bulky modification or nucleotide modified with disubstituent as described in the present disclosure.

In another embodiment, the first three nucleotides (counting from the 5'-end) of (N)b' comprise two fluoro-modified nucleotides, and one nucleotide modified with disubstituent or nucleotide modified with 2'-deoxy.

In an embodiment, the duplex region of the double-stranded oligonucleotide represented by Formula (I) is 19 nucleotide pairs, wherein the first four nucleotides (counting from the 5'-end) of (N)b' region on the sense strand are located at positions 7-10; in this case, the nucleotide modified with non-fluoro may be located at any of positions 7, 8, 9 and 10. Exemplarily, the nucleotide X modified with disubstituent or nucleotide modified with 2'-deoxy (DNA) is located at any of positions 7, 8, and 9 of the sense strand. Optionally, the nucleotide X modified with disubstituent is located at position 8 of the sense strand.

In an embodiment, when q=0, b+c=14. In another embodiment, when q=0, b=8 and c=6. In another embodiment, when q=0, b=4 and c=10.

In some embodiments, in the double-stranded oligonucleotide molecule of the present disclosure, one or more X are present and located only in the antisense strand. In some embodiments, in the direction from the 5'-end to the 3'-end, when the antisense strand comprises only one X, X is located at any of positions 8-10 and position 15 of the antisense strand. Optionally, X is located at position 15 of the antisense strand.

In some embodiments, in the double-stranded oligonucleotide molecule of the present disclosure, one or more X are present and located only in the sense strand. Optionally, the sense strand may comprise one or two X. Exemplarily, X in the sense strand may be located at a position opposite to any of positions 2, 8 and 15 (counting from the 5'-end) of the antisense strand. Herein, the opposite position refers to a position where a nucleotide on one strand in a duplex region is complementary to a corresponding nucleotide on the other strand. Exemplarily, in the 5'-3' direction, in a structure consisting of 19/21 nucleotide pairs, position 5 of the SS strand is opposite to a nucleotide at position 15 of the AS strand, and position 12 of the SS strand is opposite to a nucleotide at position 8 of the AS strand. This is well-known in the art.

In some embodiments, in the double-stranded oligonucleotide molecule of the present disclosure, two or more X are present and respectively located in both the antisense strand and the sense strand. In some embodiments, X may be located at any of positions 8-10 and position 15 (counting from the 5'-end) of the antisense strand; meanwhile, X in the sense strand may be located at a position opposite to any of positions 2, 8 and 15 (counting from the 5'-end) of the antisense strand.

In some embodiments, in the double-stranded oligonucleotide represented by Formula (II) of the present disclosure or the double-stranded oligonucleotide comprising the double-stranded region represented by the above

Formula (I), the sense strand has one or more or all of the following features:

1) the sense strand comprises at least one X, and one X is located at a position opposite to position 15 (counting from the 5'-end) of the antisense strand;
2) four nucleotides on the sense strand at positions opposite to positions 10-13 (counting from the 5'-end) of the antisense strand comprise at least two fluoro-modified nucleotides; and
3) optionally, the sense strand comprises at least one X at a position opposite to the seed region (positions 1-8, counting from the 5'-end) of the antisense strand.

And/or, the antisense strand of the double-stranded oligonucleotide has one or more or all of the following features:

1) position 2, position 6, position 14 and position 16 (counting from the 5'-end) of the antisense strand are all fluoro-modified nucleotides;
2) nucleotides at other positions of the antisense strand are all nucleotides modified with non-fluoro; and
3) X is absent or at least one X is present in the antisense strand; and when X is present on the antisense strand, X is optionally located at any of positions 8, 9, 10 and 15 of the antisense strand.

Optionally, the antisense strand comprises one X nucleotide located at position 15.
Optionally, the double-stranded region of the double-stranded oligonucleotide comprises 19-23 base pairs (for example, 19 or 20 or 21 base pairs) formed by sequences of the antisense strand and the sense strand that are sufficiently complementary to each other.
In some other embodiments, the double-stranded oligonucleotide represented by Formula (II) of the present disclosure or the double-stranded oligonucleotide comprising the double-stranded region represented by the above Formula (I) has at least two or at least three or all of the following features:

1) the sense strand comprises at least one X nucleotide; and at least one X is located at a position opposite to any of positions 15, 8 and 2 (counting from the 5'-end) of the antisense strand;
2) four nucleotides on the sense strand located at sites opposite to positions 10-13 (counting from the 5'-end) of the antisense strand comprise at least two fluoro-modified nucleotides;
3) position 2, position 6, position 14 and position 16 (counting from the 5'-end) of the antisense strand are all fluoro-modified nucleotides, and nucleotides at other positions are all nucleotides modified with non-fluoro;
4) at least one of position 8, position 9, position 10, and position 15 of the antisense strand is X nucleotide; and
5) optionally, the sense strand comprises at least one X at a position opposite to the seed region (positions 1-8, counting from the 5'-end) of the antisense strand.

Optionally, the above duplex region comprises 19-21 base pairs (for example, 19 or 20 or 21 base pairs) formed by sequences of the antisense strand and the sense strand that are sufficiently complementary to each other. Exemplarily, the double-stranded oligonucleotide may comprise a sense strand that is 19-21 nucleotides in length and an antisense strand that is 19-23 nucleotides in length.
In another embodiments, the double-stranded oligonucleotide represented by Formula (II) of the present disclosure or the double-stranded oligonucleotide comprising the double-stranded region represented by the above Formula (I) has all of the following features:

1) the sense strand comprises one or two X nucleotides; and optionally, at least one X is located at a position opposite to any of positions 15, 8 and 2 (counting from the 5'-end) of the antisense strand;
2) four nucleotides on the sense strand at positions opposite to positions 10-13 (counting from the 5'-end) of the antisense strand comprise at least two fluoro-modified nucleotides;
3) position 2, position 6, position 14 and position 16 (counting from the 5'-end) of the antisense strand are all fluoro-modified nucleotides, and nucleotides at other positions are all nucleotides modified with non-fluoro; and
4) at least one of position 8, position 9, position 10, and position 15 of the antisense strand is nucleotide X.

Optionally, the sense strand comprises one nucleotide X at a position opposite to any of positions 15, 8 and 2 (counting from the 5'-end) of the antisense strand.
Optionally, position 15 of the antisense strand is nucleotide X.
In another embodiment, the double-stranded oligonucleotide represented by Formula (II) of the present disclosure or the double-stranded oligonucleotide comprising the double-stranded region represented by the above Formula (I) has all of the following features:

1) the sense strand comprises one X at a position opposite to any of positions 15, 8 and 2 (counting from the 5'-end) of the antisense strand;
2) four nucleotides on the sense strand at positions opposite to positions 10-13 (counting from the 5'-end) of the antisense strand comprise at least two fluoro-modified nucleotides;
3) position 2, position 6, position 14 and position 16 (counting from the 5'-end) of the antisense strand are all fluoro-modified nucleotides, and nucleotides at other positions are all nucleotides modified with non-fluoro;
4) at least one of position 10 and position 15 of the antisense strand is X nucleotide; and/or, the antisense strand comprises only one X nucleotide at position 15 (counting from the 5'-end); and
5) optionally, the duplex region comprises 19-23 base pairs.

In an embodiment, in the double-stranded oligonucleotide molecule of the present disclosure, in the direction from the 5'-end to the 3'-end, the $15^{th}$ nucleotide of the antisense strand in the duplex region is X nucleotide, and nucleotides on the sense strand at positions opposite to positions 2 and 8 (counting from the 5'-end) of the antisense strand are also all X nucleotides.

In an embodiment, the double-stranded oligonucleotide molecule of the present disclosure further has phosphorothioate linkage modification, for example, having one or two phosphorothioate linkage modifications in nucleotides at positions 1-5 (counting from the 5'-end) of the sense strand, and/or one or two phosphorothioate linkage modifications at position 18-23 (counting from the 5'-end of the antisense strand) of the antisense strand.

In some embodiments, the double-stranded oligonucleotide of the present disclosure is as represented by Formula (II), wherein each nucleotide in the duplex region is a modified nucleotide.

In some other embodiments, the double-stranded oligonucleotide of the present disclosure is as represented by Formula (IIa), wherein each nucleotide of the duplex region is a modified nucleotide, the overhang is located at the 3'-end of the antisense strand, and the overhang nucleotide sequence has a length of 1 to 3 nucleotides. Exemplarily, a ratio of lengths of the sense strand and the antisense strand of the double-stranded oligonucleotide provided by the present disclosure may be 19/19, 19/21, 19/22, 20/20, 21/21, 21/22, 21/23, 22/22, 22/24, 22/25, 23/25, etc. In some embodiments, each of the sense strand and the antisense strand of the double-stranded oligonucleotide of the present disclosure has 19/21 nucleotides or 21/23 nucleotides in length.

In some embodiments, the overhang is 2 nucleotides. For example, the overhang is dTdT (contiguous thymine deoxyribonucleotides), UU.

In some embodiments, the double-stranded oligonucleotide represented by the above Formula (II) comprises a sense strand that is 19-21 nucleotides in length and an antisense strand that is 19-23 nucleotides in length, wherein sequences of the antisense strand and the sense strand are complementary to each other so as to form a duplex region of 17-21 base pairs. Moreover, the double-stranded oligonucleotide has a nucleotide overhang at the 3'-end of the antisense strand. And optionally, the antisense strand or the sense strand each independently comprises 1-4 phosphorothioate internucleotide linkages, for example, comprising, but not limited to, 1, 2, 3 or 4 phosphorothioate internucleotide linkages; and/or the sense strand of the double-stranded oligonucleotide is conjugated to a ligand.

For the nucleotide overhang, exemplarily, there is an overhang of 2-4 nucleotides at the 3'-end of the antisense strand and a blunt end at the 5'-end of the antisense strand.

In some embodiments, in the double-stranded oligonucleotide represented by the above Formula (II) of the present disclosure, in the direction from the 5'-end to the 3'-end, 1) at least one of nucleotides at position 8, 9, 10 or 15 of the antisense strand is X nucleotide; and position 2, position 6, position 14 and position 16 (counting from the 5'-end) of the antisense strand are all fluoro-modified nucleotides; 2) the sense strand comprises one or two X nucleotides, and at least one X nucleotide on the sense strand is located at a position opposite to any of positions 2, 8, 12, and 15 (counting from the 5'-end) of the antisense strand; and four nucleotides on the sense strand at positions opposite to positions 10-13 (counting from the 5'-end) of the antisense strand comprise at least two fluoro-modified nucleotides.

In some embodiments, at least one of phosphate groups in a phosphate-sugar backbone in the sense strand and the antisense strand of the double-stranded oligonucleotide provided by the present disclosure is a sulfo-modified phosphate group (i.e., phosphorothioate group). The phosphorothioate group herein is a phosphorothioate group formed by substituting at least one oxygen atom in a phosphodiester bond in the phosphate group with a sulfur atom. In some embodiments, the phosphate group with a modified group is a phosphorothioate group formed by substituting one oxygen atom in a phosphodiester bond with a sulfur atom.

In some embodiments, in the double-stranded oligonucleotide of the present disclosure, the phosphorothioate group is present at an end of the sense strand and/or a terminal region of the antisense strand, wherein the terminal region comprises 3 or 4 or 5 contiguous nucleotides at the 5'-end and the 3'-end. In some other embodiments, on the basis that an end nucleotide of a single strand is the first nucleotide, the phosphorothioate group is present between any two contiguous nucleotides of the first to third nucleotide sequences.

Exemplarily, on the basis that an end nucleotide of a single strand is the first nucleotide, the phosphorothioate group may be present in one or more of any of the following positions in the terminal region of the sense strand and/or the

antisense strand: between the first and second nucleotides at the 5'-end, between the second and third nucleotides at the 5'-end, between the first and second nucleotides at the 3'-end, and between the second and third nucleotides at the 3'-end.

In some embodiments, the 5'-end nucleotide of the antisense strand is a 5'-phosphate nucleotide or a nucleotide modified with 5'-phosphate analog. In an embodiment, the nucleotide modified with 5'-phosphate analog is a nucleotide comprising a vinyl phosphate modification (a nucleotide modified with vinyl phosphate) (e.g., 5'-VP), or is a phosphorothioate modified nucleotide. Optionally, the 5'-VP may be 5'-E-VP, 5'-Z-VP, or a combination thereof.

The double-stranded oligonucleotide of the present disclosure may be various double-stranded oligonucleotides that regulate gene expression. In some embodiments, it may be a double-stranded oligonucleotide that inhibits or down-regulates gene expression, such as siRNA. In some embodiments, it may be a double-stranded oligonucleotide that activates or up-regulates gene expression, such as saRNA.

In an embodiments, in the double-stranded oligonucleotide of the present disclosure:

1) in the 5'-3' direction of the antisense strand, at least one of nucleotides at positions 10 and 15 of the antisense strand is a nucleotide selected from following modified nucleotides: 2'-O-MOE, 2'-O-TBDMS, 2'-O-TOM, 2'-O-$CH_2$-O-$CH_2$-$CH_3$, and 2'-O-$CH_2$-O-$CH_2$-$CF_3$; and position 2, position 6, position 14 and position 16 of the antisense strand are all fluoro-modified nucleotides; and remaining positions of the antisense strand are nucleotides modified with methoxy;
2) on the sense strand, a position opposite to any of positions 2, 8 and 15 (counting from the 5'-end) of the antisense strand is a nucleotide selected from following modified nucleotides: 2'-O-MOE, 2'-O-TBDMS, 2'-O-TOM, 2'-O-$CH_2$-O-$CH_2$-$CH_3$, and 2'-O-$CH_2$-O-$CH_2$-$CF_3$; and four nucleotides on the sense strand at positions opposite to positions 10-13 (counting from the 5'-end of the antisense strand) of the antisense strand comprise at least two fluoro-modified nucleotides; and remaining positions of the sense strand are nucleotides modified with methoxy; and
3) the sense strand and the antisense strand each of comprise at least one phosphorothioate group, and the phosphorothioate group is located in a terminal region of each single strand. Herein, the terminal region comprises a length interval of 3-5 nucleotides.

In an embodiment, the double-stranded oligonucleotide further comprises a nucleotide modified with 5'-VP.

In an embodiment, each of the sense strand and the antisense strand of the double-stranded oligonucleotide of the present disclosure has 17-30 nucleotides. In an example, the sense strand has 19-21 nucleotides, and the antisense strand has 19-25 nucleotides. In an example, the sense strand has 19 or 21 nucleotides, and the antisense strand has 21 or 23 nucleotides.

In an embodiment, a nucleotide at position 1 from the 5'-end of the antisense strand in the duplex of the double-stranded oligonucleotide is selected from: A, dA, dU, U or dT.

In an embodiment, the antisense strand of the double-stranded oligonucleotide (dsRNA) of the present disclosure is 100% complementary to a target RNA, and can inhibit its expression by RNA interference. In another embodiment, the antisense strand of the double-stranded oligonucleotide of the present disclosure is at least 95%, at least 90%, at least 85%, at least 80% complementary to the target RNA, as measured by percentage of base pairs.

The double-stranded oligonucleotide dsRNA described in the present disclosure may be represented by Formula (II), or comprises the duplex region represented by Formula (I).

In an embodiment, the double-stranded oligonucleotide of the present disclosure comprises a sense strand and an antisense strand, where:

(a) the sense strand therein has:

(i) 19-21 nucleotides in length;
(ii) an ASGPR ligand attached to the 3'-end, wherein the ASGPR ligand is GalNAc or a derivative thereof;
(iii) at least one position opposite to position 2, 8, or 15 (counting from the 5'-end) of the antisense strand being a nucleotide modified with a 2'-bulky group;
(iv) four nucleotides at positions opposite to positions 10-13 (counting from the 5'-end) of the antisense strand comprising at least two nucleotides modified with 2'-fluoro; and optionally, the remaining positions being independently selected from one or at least one of nucleotide modified with 2'-OMe, nucleotide modified with 2'-deoxy or nucleotide modified with a 2'-bulky group, wherein the 2'-bulky group modification is selected from one or more of 2'-O-MOE, 2'-O-TBDMS, 2'-O-TOM, 2'-O-$CH_2$-O-$CH_2$-$CH_3$, and 2'-O-$CH_2$-O-$CH_2$-$CF_3$;

and
(b) the antisense strand therein has:

(i) 21-23 nucleotides in length;

(ii) at least one of nucleotides at position 10 or 15 of the antisense strand being a nucleotide modified with a 2'-bulky group;

(iii) position 2, position 6, position 14 and position 16 of the antisense strand being all nucleotides modified with 2'-fluoro; and, optionally, the remaining positions of the antisense strand being one or at least one selected from one or at least one of nucleotide modified with 2'-OMe, nucleotide modified with 2'-deoxy or nucleotide modified with a 2'-bulky group, wherein the 2'-bulky group modification is selected from one or more of 2'-O-MOE, 2'-O-TBDMS, 2'-O-TOM, 2'-O-$CH_2$-O-$CH_2$-$CH_3$, and 2'-O-$CH_2$-O-$CH_2$-$CF_3$;

(iv) at least one phosphorothioate linkage between nucleotides in the terminal region; and

(v) optionally, a overhang consisting of 2-3 nucleotides at the 3'-end of the antisense strand, and a blunt end at the 5'-end of the antisense strand.

In an embodiment, the above double-stranded oligonucleotide of the present disclosure comprises phosphorothioate linkages between nucleotide positions 1 and 2, between nucleotide positions 2 and 3, between nucleotide positions 19 and 20, and between nucleotide positions 20 and 21 of the antisense strand (counting from the 5'-end). In an embodiment, the 3'-end of the antisense strand has a overhang consisting of two nucleotides, and the 5'-end of the antisense strand is a blunt end.

In an embodiment, the double-stranded oligonucleotide of the present disclosure comprises: counting from the 5'-end, nucleotide modified with a 2'-bulky group at position 15 of the antisense strand; and nucleotide modified with a 2'-bulky group on the sense strand at any position opposite to positions 2, 8, and 15 of the antisense strand, wherein the 2'-bulky group provides a greater steric bulk to the nucleotide where it is located than 2'-OMe modification.

In an embodiment, position 15 of the antisense strand has a nucleotide modified with a 2'-bulky group; and a nucleotide on the sense strand at a position opposite to position 15 of the antisense strand is a nucleotide modified with a 2'-bulky group.

In an embodiment, position 15 of the antisense strand has a nucleotide modified with a 2'-bulky group; and a nucleotide on the sense strand at a position opposite to position 8 of the antisense strand is a nucleotide modified with a 2'-bulky group.

In an embodiment, position 15 of the antisense strand has a nucleotide modified with a 2'-bulky group; and a nucleotide on the sense strand at a position opposite to position 2 of the antisense strand is a nucleotide modified with a 2'-bulky group.

In an embodiment, position 15 of the antisense strand has a nucleotide modified with a 2'-bulky group; and nucleotides on the sense strand at positions opposite to position 2 and position 8 of the antisense strand are nucleotides modified with a 2'-bulky group.

In optional embodiments, the sense strand comprises a nucleotide modified with a 2'-bulky group at a position opposite to the seed region of the antisense strand (wherein the seed region of the antisense strand is located at positions 1-8, counting from the 5'-end).

Optionally, the above 2'-bulky group is selected from one or more of 2'-O-MOE, 2'-O-TBDMS, 2'-O-TOM, 2'-O-$CH_2$-O-$CH_2$-$CH_3$, and 2'-O-$CH_2$-O-$CH_2$-$CF_3$.

Exemplarily, when the sense strand is 21 nucleotides in length, the nucleotide modified with a 2'-bulky group is located at positions 7, 14, and 20 counting from the 5'-end (positions opposite to positions 15, 8, and 2 of the antisense strand, respectively).

Exemplarily, when the sense strand is 19 nucleotides in length, the nucleotide modified with a 2'-bulky group is located at positions 5, 12, and 18 counting from the 5'-end (positions opposite to positions 15, 8, and 2 of the antisense strand, respectively).

The double-stranded oligonucleotide provided by the present disclosure can be used alone, or form a pharmaceutical composition with a pharmaceutically acceptable carrier, or form an oligonucleotide conjugate by binding to a conjugating molecule, or form other forms. When in use, an effective amount of the double-stranded oligonucleotide, the pharmaceutical composition or the oligonucleotide conjugate can be contacted with a cell, so as to regulate expression of a target gene, or the double-stranded oligonucleotide, the pharmaceutical composition or conjugate is administered to a subject to regulate expression of a target gene, so as to achieve a purpose of treating a pathologic condition or disease associated with an expression level of the target gene.

In another aspect, the present disclosure provides a double-stranded oligonucleotide, comprising a sense strand and an antisense strand, each having 17 to 35 nucleotides, wherein each nucleotide is a modified or unmodified nucleotide, the sense strand and the antisense strand are at least partially reversely complementary to form a double-stranded region, wherein the double-stranded oligonucleotide comprises at least one nucleotide modified with 2'-disubstituent, and each substituent in the 2'-disubstituent is independently selected from methyl or fluorine.

Herein, when the double-stranded region of the double-stranded oligonucleotide comprises a nucleotide modified with 2'-disubstituent, the nucleotide modified with 2'-disubstituent is selected from 2'-difluoro substituted nucleotide; or, when the double-stranded oligonucleotide comprises a nucleotide modified with 2'-disubstituent at

positions outside the double-stranded region, the nucleotide modified with 2'-disubstituent is selected from nucleotide substituted with 2'-F-2'-Me.

In some embodiments, the double-stranded oligonucleotide has one or more of features as follows:

(1) when the double-stranded oligonucleotide comprises a 2'-difluoro substituted nucleotide substituted, the 2'-difluoro substituted nucleotide is located at at least one of positions 7, 8, 9, and 10 of the sense strand counting from the 5'-end;
(2) when the double-stranded oligonucleotide comprises a 2'-difluoro substituted nucleotide, the 2'-difluoro substituted nucleotide is located at at least one of positions 2, 4, 6, 8, 9, 10, 12, 14 and 16 of the antisense strand counting from the 5'-end;
(3) when the double-stranded oligonucleotide comprises a nucleotide substituted with 2'-F-2'-Me, the nucleotide substituted with 2'-F-2'-Me is located at a position outside the double-stranded region of the oligonucleotide; and
(4) when the double-stranded oligonucleotide comprises a nucleotide substituted with 2'-F-2'-Me, the nucleotide substituted with 2'-F-2'-Me is located at a overhang at the 3'-end of the antisense strand.

In some embodiments, in feature (1), when the double-stranded oligonucleotide comprises a 2'-difluoro substituted nucleotide, the 2'-difluoro substituted nucleotide is located at at least two or at least three of positions 7, 8, 9 and 10 of the sense strand counting from the 5'-end.

In some embodiments, in feature (2), when the double-stranded oligonucleotide comprises a 2'-difluoro substituted nucleotide, the 2'-difluoro substituted nucleotide is located at at least two of positions 2, 4, 6, 8, 9, 10, 12, 14 and 16 of the antisense strand counting from the 5'-end. In another aspect, the present disclosure provides a double-stranded oligonucleotide conjugate, comprising the double-stranded oligonucleotide provided by the present disclosure or the double-stranded oligonucleotide obtained according to the method of the present disclosure, and a conjugating group conjugated to the double-stranded oligonucleotide. The present disclosure can further improve blood stability and targeting property of the double-stranded oligonucleotide of the present disclosure by forming a pharmaceutical composition with a suitable carrier or forming an oligonucleotide conjugate with a suitable conjugating molecule. The conjugating molecule may be a conjugating molecule targeting a tissue such as liver, lung, and kidney.

In some embodiments, the conjugating group comprises a linker and a pharmaceutically acceptable targeting group; the double-stranded oligonucleotide, the linker and the targeting group or the delivery assisting group is sequentially linked covalently or non-covalently; and each targeting group is selected from ligands capable of binding to a cell surface receptor. Generally, there are 1-6 targeting groups. In an embodiment, there are 2-4 targeting groups. A conjugation site between the double-stranded oligonucleotide and the conjugating group may be at the 3'-end or the 5'-end of the sense strand of the double-stranded oligonucleotide, or at the 5'-end of the antisense strand, or in an internal sequence of the double-stranded oligonucleotide. In some specific embodiments, the conjugation site between the double-stranded oligonucleotide and the conjugating group is at the 3'-end of the sense strand of the double-stranded oligonucleotide.

The abovementioned oligonucleotide conjugate can be synthesized by methods already described in detail in the prior art. For example, WO2015006740A2 describes preparation methods for a variety of siRNA conjugates in detail.

In yet another aspect, the present disclosure provides a conjugate of a double-stranded oligonucleotide, wherein the conjugate comprises the double-stranded oligonucleotide described herein and a targeting group, so as to provide tissue specific targeting.

In some embodiments, the conjugate of the present disclosure specifically targets a hepatocyte surface receptor, thereby specifically targeting a hepatic tissue. In some embodiments, the targeting group of the present disclosure is a ligand that has affinity to an asialoglycoprotein receptor, so as to specifically target the asialoglycoprotein receptor (ASGPR) on a surface of hepatocytes. Exemplarily, the targeting group is a ligand group from N-acetyl-galactosamine (GalNAc) or derivatives thereof.

In an embodiment, the double-stranded oligonucleotide provided by the present disclosure is linked to at least one ASGPR ligand. Exemplarily, the ASGPR ligand is GalNAc or a derivative thereof attached through a bivalent or trivalent branched linker.

In some embodiments, the ASGPR ligand is attached to the 5'-end or the 3'-end of the sense strand or the antisense strand of the double-stranded oligonucleotide through a linker to form a conjugate. Exemplarily, the conjugate has following structural formula (B):

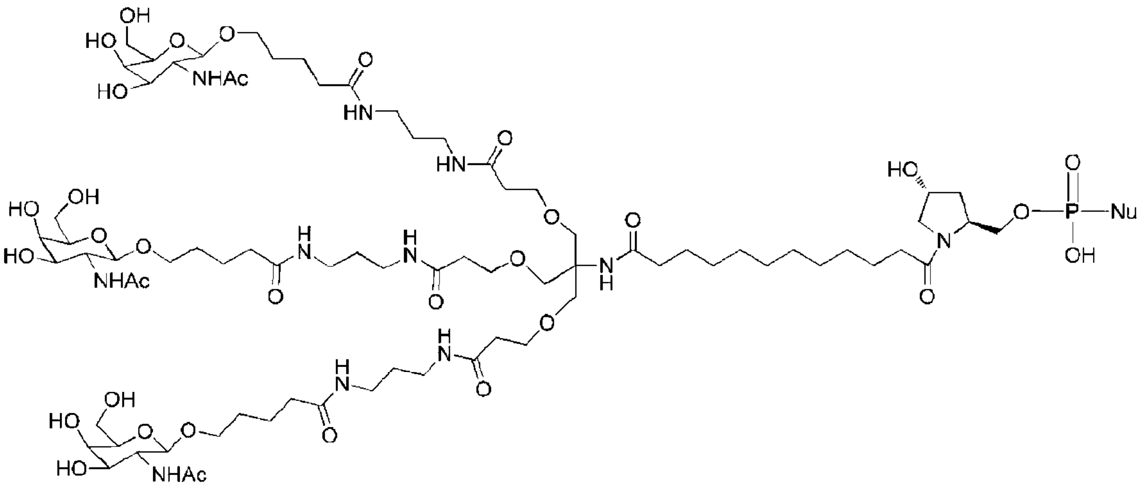

(B)

Herein, Nu represents the double-stranded oligonucleotide of the present disclosure or other nucleic acid-based pharmaceutically active molecules (e.g., single-stranded oligonucleotide).

In an embodiment, the ligand may be conjugated at the 3'-end or the 5'-end of the sense strand of the double-stranded oligonucleotide.

The present disclosure further provides use of the double-stranded oligonucleotide and a reagent thereof. In an embodiment, the use is for inhibiting expression of a target gene in a subject. The subject may be any animal, preferably mammals such as mice, rats, sheep, cattle, dogs, cats, or humans.

In some embodiments, the present disclosure further provides a pharmaceutical composition, comprising the double-stranded oligonucleotide of the present disclosure and a pharmaceutically acceptable carrier. The pharmaceutically acceptable carrier may be any available carrier.

In some embodiments, contents of the double-stranded oligonucleotide and the pharmaceutically acceptable carrier in the pharmaceutical composition are not particularly required. The pharmaceutical composition can further comprise other pharmaceutically acceptable auxiliary materials, which may be one or more of various preparations or compounds conventionally used in the art. For example, the other pharmaceutically acceptable auxiliary materials may comprise at least one of a pH buffer, a protective agent and an osmotic pressure regulator.

In some embodiments, the present disclosure provides a method for treating and/or preventing diseases or conditions associated with level of mRNA expressed by a target gene, comprising administering an effective amount of the double-stranded oligonucleotide, the pharmaceutical composition and/or the oligonucleotide conjugate of the present disclosure to a subject in need thereof. In some embodiments, the disease or condition associated with the level of mRNA expressed by the target gene is hepatitis, hepatic fibrosis, liver proliferative diseases and/or dyslipidemia. In some embodiments, the dyslipidemia is hypercholesterolemia, hypertriglyceridemia or atherosclerosis.

In some embodiments, the present disclosure provides use of the double-stranded oligonucleotide, the pharmaceutical composition comprising the double-stranded oligonucleotide or the oligonucleotide conjugate in preparation of a medicine for treating and/or preventing an induced pathologic condition or disease (e.g., overexpression of a gene) associated with level of mRNA expressed by a target gene.

In some embodiments, the target gene is an abnormally expressed gene in hepatocytes. In some embodiments, the target gene is an endogenous gene expressed in liver. Endogenous genes expressed in liver comprise, but are not limited to, ApoB, ApoC, ANGPTL3, PCSK9, SCD1, TIMP-1, Col1A1, FVII, STAT3, p53, HBV, HCV, and other genes.

In some embodiments, the disease is selected from chronic liver disease, hepatitis, hepatic fibrosis, liver proliferative diseases and dyslipidemia.

In some embodiments, the dyslipidemia is hypercholesterolemia, hypertriglyceridemia or atherosclerosis.

In some embodiments, the present disclosure provides a method for modulating gene expression, comprising contacting an effective amount of the double-stranded oligonucleotide, the pharmaceutical composition or the oligonucleotide conjugate with a cell expressing the gene.

In some embodiments, the present disclosure provides a method for treating a pathologic condition or disease caused by abnormal gene expression, comprising administering an effective amount of the double-stranded oligonucleotide, the pharmaceutical composition or the oligonucleotide conjugate to a subject. In some embodiments, the abnormal expression is overexpression, and correspondingly, the regulation is to inhibit the overexpression. In some embodiments, the regulation refers to inhibiting expression of a target gene in hepatocytes, wherein mRNA expressed by the target gene

is selected from mRNA expressed by a hepatitis B virus (HBV) gene, mRNA expressed by an angiopoietin-like protein 3 (ANGPTL3) gene or mRNA expressed by an apolipoprotein C3 (ApoC3) gene.

In some embodiments, the double-stranded oligonucleotide, the pharmaceutical composition or the oligonucleotide conjugate of the present disclosure can also be used for treating other liver diseases, comprising cancer, hepatocellular carcinoma (HCC), liver metastasis or hepatoblastoma.

In some embodiments, any suitable route known in the art can be used for administration to the subject, comprising, but is not limited to, oral or parenteral route, e.g., intravenous administration, intramuscular administration, subcutaneous administration, transdermal administration, endotracheal administration (aerosol), pulmonary administration, nasal administration, rectal administration and topical administration (comprising buccal administration and sublingual administration). Administration may be performed once or multiple times weekly, monthly or annually.

In some embodiments, the present disclosure further provides a method for delivering the double-stranded oligonucleotide, the pharmaceutical composition or the oligonucleotide conjugate to a specific target in a subject via subcutaneous or intravenous administration.

The present disclosure provides a kit, comprising the double-stranded oligonucleotide as described above, the pharmaceutical composition as described above and/or the oligonucleotide conjugate as described above.

In some embodiments, the kit herein can provide the double-stranded oligonucleotide in a container. In some embodiments, the kit herein may comprise a container that provides a pharmaceutically acceptable excipient. In some embodiments, the kit can further comprise other ingredients such as a stabilizer or a preservative. In some embodiments, the kit herein may comprise at least one other therapeutic agent in a container other than the container providing the double-stranded oligonucleotide herein.

In the kit of the present disclosure, the double-stranded oligonucleotide and the pharmaceutically acceptable carrier and/or auxiliary material, as well as the double-stranded oligonucleotide composition and/or conjugate, and/or the pharmaceutically acceptable auxiliary material, may be provided in any form, such as in a liquid form, a dried form, or a lyophilized form. In some embodiments, the double-stranded oligonucleotide and the pharmaceutically acceptable carrier and/or auxiliary material and the double-stranded oligonucleotide composition and/or conjugate and optional pharmaceutically acceptable auxiliary material are substantially pure and/or sterilized. In some embodiments, sterilized water may be provided in the kit of the present disclosure.

Unless otherwise stated, meanings of base composition and modifications described in various examples herein are as follows: capital letters A, U, G, C, and T represent base composition of nucleotides; lowercase letter m means that a nucleotide represented by a preceding letter thereof is a nucleotide modified with 2'-methoxy; lowercase letter f means that a nucleotide represented by a preceding letter thereof is a nucleotide modified with 2'-fluoro; lowercase letter d means that a nucleotide represented by a preceding letter thereof is a 2'-deoxyribonucleotide; brackets plus capital letters TBDMS, i.e., (TBDMS), mean that a nucleotide represented by a preceding letter thereof is a nucleotide modified with 2'-O-*tert*-butyldimethylsilyl; brackets plus capital letters TOM, i.e., (TOM), mean that a nucleotide represented by a preceding letter thereof is a nucleotide modified with 2'-O-tris(isopropyl)silyloxymethyl; brackets plus capital letters MOE, i.e., (MOE), mean that a nucleotide represented by a preceding letter thereof is a nucleotide modified with 2'-O-methoxyethyl; and lowercase letter s means that nucleotides represented by two preceding and following letters thereof are linked by a phosphorothioate linkage.

In the present disclosure, compound L96-PS has a structural formula as represented by Formula (C):

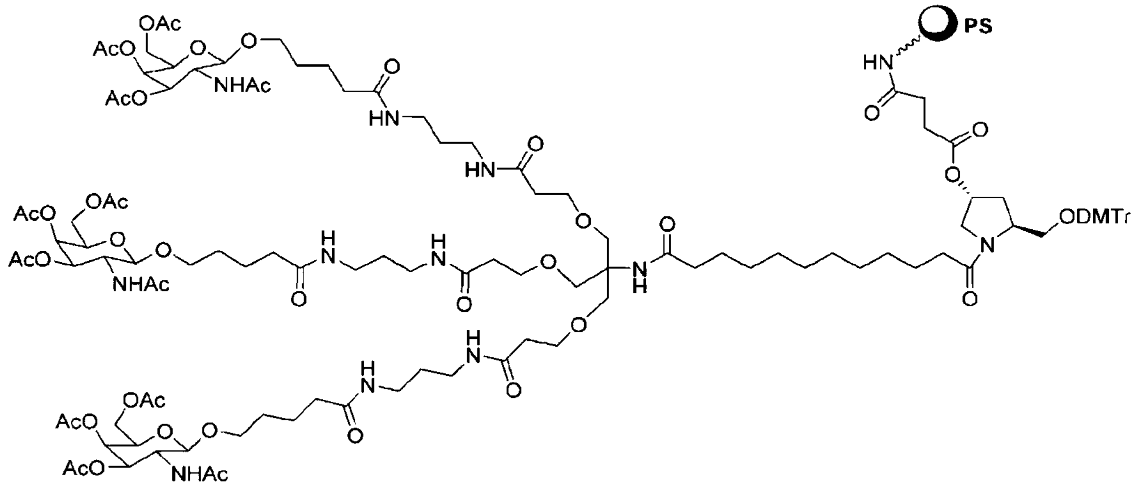

(C)

Herein, PS represents a polystyrene resin solid-phase carrier, and compound L96-PS is purchased from Asymchem Laboratories (Tianjin) Co., Ltd., with a load amount of 120±12 μmol/g (a detection method being UV/HPLC).

Unless otherwise stated, reagents and consumable materials (Table 1) and instrument and equipment (Table 2) used in the present disclosure are all products commercially available from the following manufacturers.

Table 1

| Name | Manufacturer |
|---|---|
| HepG2 cell | Wuhan Pricella Biotechnology Co., Ltd. |
| DMEM medium | M&C GENE TECHNOLOGY(BEIJING) LTD. |
| Opti-MEM medium | GIBCO |
| Fetal bovine serum (FBS) | Merck Ltd |
| Lipofectamine 3000 | Thermo Fisher Scientific |
| Hanwei RNA extraction kit | Zhejiang Hanwei Science and Technology Co. Ltd. |
| Reverse Transcription System | Promega Corporation |
| SYBR Select Master Mix | ABI |
| Deoxyribonuclease I from bovine pancreas | SIGMA |
| Collagenase from Clostridium histolyticum | SIGMA |
| RNALater | Thermo Fisher Scientific |

Table 2

| Name | Manufacturer |
|---|---|
| Full-automatic nucleic acid extractor | Zhejiang Hanwei Science and Technology Co. Ltd. |
| High-speed refrigerated centrifuge | Eppendorf |
| NANODROP OneC | Thermo Fisher Scientific |
| Gradient PCR amplifier | Eppendorf |
| Fluorescence quantitative PCR instrument | ABI StepOne Plus |
| Gel imager | Shanghai Tanon Science & Technology Co.,Ltd. |
| Electrophoresis apparatus | BEIJING LIUYI INSTRUMENT PLANT |
| $CO_2$ thermostatic incubator | Thermo Fisher Scientific |
| Biological safety cabinet | Esco Lifesciences |
| Tissuelyser type II full-automatic tissue homogenizer | Shanghai Jingxin Industrial Development Co., Ltd. |

Unless otherwise stated, dsRNA (referred to as siRNA in examples) sequences used in the present disclosure were all entrusted to be synthesized by Suzhou Biosyntech Co., Ltd.; PCR primer synthesis was entrusted to be completed by Beijing Tsingke Biotech Co., Ltd.; experimental animals C57BL/6J mice and ICR mice were both purchased from SPF (Beijing) Biotechnology Co., Ltd.; and blood biochemical standard measurement was entrusted to be completed by Beijing Sinogenetic Biotechnology Co., Ltd.

Unless otherwise stated, Real-time PCR detection data involved in *in vivo*/*in vitro* siRNA activity experiments in various examples of the present disclosure are all used for relative quantitative calculation of target gene mRNA in various test groups by a ΔΔCt method, and a calculation method is summarized as follows:

$$\Delta Ct \text{ (test group)} = Ct \text{ (target gene in test group)} - Ct \text{ (internal reference gene in test group)};$$

ΔCt (control group)= Ct (target gene in control group) - Ct (internal reference gene in control group);

$$\Delta\Delta Ct \text{ (test group)} = \Delta Ct \text{ (test group)} - \Delta Ct \text{ (control group mean)};$$

and

$$\Delta\Delta Ct \text{ (control group)} = \Delta Ct \text{ (control group)} - \Delta Ct \text{ (control group mean)}.$$

Taking the control group as benchmark, expression level of the target gene mRNA in the test group is normalized, and remaining expression level of the target gene mRNA in the control group is defined as 100%.

Relative remaining expression level of target gene mRNA in test group = $2^{-\Delta\Delta Ct}$ (test group) × 100%

Inhibition rate of target gene mRNA in test group = 100% - relative expression level of target gene mRNA in test group

*In vivo*/*in vitro* activity experimental data are all expressed by $\overline{X}\pm SD$ (or X±STDEV), and experimental data are all plotted and analyzed using GraphPad prism 8.0 software. Herein, ±SD values and ±STDEV values both represent standard deviations.

Proportions of the reagents in various examples of the present disclosure are all calculated as volume ratio (v/v), unless otherwise stated.

Solutions of the present disclosure will be illustrated below in conjunction with examples. Those skilled in the art could understand that the following examples are merely used for illustrating the present disclosure, but should not be considered as limitation to the scope of the present disclosure. Wherein specific techniques or conditions are not specified in the examples, they are carried out according to techniques or conditions described in documents in the art or according to product specifications. If manufacturers of reagents or apparatuses used are not specified, all of them are conventional products commercially available.

**Preparation of nucleoside monomer analogs with sterically bulky modification**

Reagents used in preparation examples of the present disclosure and reagent sources are as listed in Table 3.

Table 3

| Reagent Name | Abbreviation | CAS No. |
|---|---|---|
| Uridine | Uridine | 58-96-8 |
| 1,3-dichloro-1,1,3,3-tetraisopropyldisiloxane | - | 69304-37-6 |
| N-iodosuccinimide | NIS | 516-12-1 |
| Trifluoromethanesulfonic acid | TfOH | 1493-13-6 |
| Ammonium fluoride | $NH_4F$ | - |
| 4,4'-dimethoxytrityl chloride/4,4'-dimethoxy triphenylchloromethane | DMTrCl | 40615-36-9 |
| Pyridine | Py | 110-86-1 |
| Bis(diisopropylamino)(2-cyanoethoxy)phosphine | - | 102691-36-1 |
| 4,5-dicyanoimidazole | DCI | 1122-28-7 |
| Trifluoroethanol | - | 75-89-8 |

**Preparation Example 1: Synthesis of compound NM062**

A synthesis route of compound NM062 was as follows:

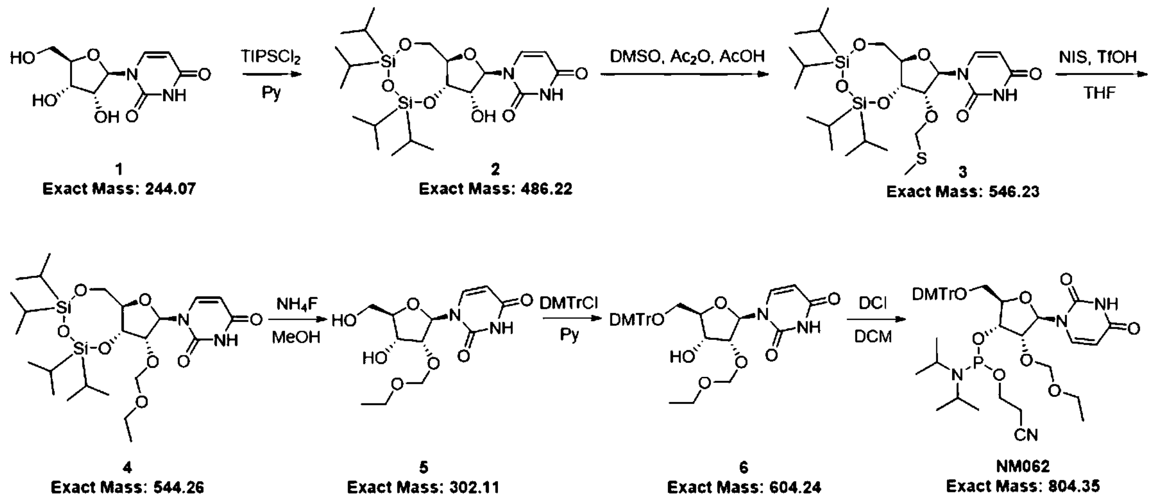


1 Exact Mass: 244.07

2 Exact Mass: 486.22

3 Exact Mass: 546.23

4 Exact Mass: 544.26

5 Exact Mass: 302.11

6 Exact Mass: 604.24

NM062 Exact Mass: 804.35

(1-1) Synthesis of compound 2

Compound 1 (10 g, 40.97 mmol, uridine) was dissolved in 100 ml of pyridine, and 1,3-dichloro-1,1,3,3-tetraisopropyldisiloxane (14.22 g, 45.07 mmol) was added, followed by stirring at 25 °C for 16 hours. After reaction was completed, reaction solution was concentrated to remove solvent, diluted by adding 300 ml of dichloromethane, and washed first with 100 ml of saturated aqueous ammonium chloride solution twice (2×100 ml), and then with 100 ml of saturated aqueous sodium chloride solution twice (2×100 ml). Organic phases were dried over anhydrous sodium sulfate, filtered, concentrated, and subjected to normal phase column chromatography purification (elution gradient: petroleum ether/ethyl acetate=3/1, volume ratio v/v), to provide compound 2 (11.5 g, in a yield of 57.7%). MS ESI (*m*/*z*) = 487.27 $[M+H]^+$.

(1-2) Synthesis of compound 3

At 25 °C, compound 2 (11 g, 22.62 mmol) was dissolved in 77 ml of dimethyl sulphoxide, and 77 ml of acetic acid and 38 ml of acetic anhydride were added, followed by stirring at 25 °C for 16 hours. After reaction was completed, 500 ml of ethyl acetate was added for dilution, 100 ml of saturated aqueous sodium bicarbonate solution was used for washing twice (2×100 ml), and then 100 ml of saturated aqueous sodium chloride solution was used for washing twice (2×100 ml). Organic phases were dried over anhydrous sodium sulfate, filtered, concentrated, and subjected to normal phase column chromatography purification (elution gradient: petroleum ether/ethyl acetate=3/1, v/v), to provide compound 3 (11.22 g, in a yield of 90.81%). MS ESI (*m*/*z*) = 546.23 $[M+H]^+$.

(1-3) Synthesis of compound 4

At 25 °C, compound 3 (5 g, 9.15 mmol) was dissolved in 50 ml of tetrahydrofuran, and ethanol (4.21 g, 91.5 mmol) was added. Nitrogen replacement was performed three times. Reaction system was cooled to -40 °C using a dry ice acetonitrile bath, and a solution of N-iodosuccinimide (2.47 g, 10.98 mmol) in tetrahydrofuran (5 ml) and a solution of trifluoromethanesulfonic acid (2.75 g, 18.3 mmol) in tetrahydrofuran (5 ml) were added dropwise at -40 °C. Reaction solution was stirred at -40 °C for 1 hour under nitrogen. After completion, reaction was quenched by adding 8 ml of triethylamine, 10 mass% aqueous solution of sodium thiosulfate was added, followed by four times of extraction with 150 ml of dichloromethane (4×150 ml). Organic phases were combined, washed twice with 80 ml of saturated aqueous sodium bicarbonate solution (2×80 ml) and once with 100 ml of saturated brine (1×100 ml), dried over anhydrous sodium sulfate, filtered, concentrated, and subjected to normal phase column chromatography purification (elution gradient: petroleum ether/ethyl acetate=4/1, v/v), to provide compound 4 (4.78 g, in a yield of 95.96%). MS ESI (*m*/*z*) = 544.26 $[M+H]^+$.

(1-4) Synthesis of compound 5

At 25 °C, compound 4 (4 g, 7.35 mmol) was dissolved in 32 ml of methanol, and ammonium fluoride (1.36 g, 36.75 mmol) was added, followed by stirring at 60 °C for 16 hours. After reaction was completed, solvent was removed by spin-drying, followed by normal phase column chromatography purification (elution gradient: dichloromethane/methanol=20/1, v/v), to provide compound 5 (2.05 g, 92.34% yield). MS ESI (*m*/*z*) = 303.2 $[M+H]^+$.

(1-5) Synthesis of compound 6

Compound 5 (2 g, 6.62 mmol) was dissolved in 20 ml of pyridine in ice bath, and cooled to 0 °C in ice bath, and 4,4'-dimethoxytrityl chloride (2.77 g, 7.94 mmol) was added portion-wise. Reaction solution was stirred at 0 °C for 1 hour. After completion, reaction was quenched by adding 2 ml of methanol, solvent was removed by spin-drying, 50 ml of saturated aqueous ammonium chloride solution was added, and 50 ml of ethyl acetate was used for extraction three times (3×50 ml). Organic phases were combined, washed with 100 ml of saturated brine once (1×100 ml), dried over anhydrous sodium sulfate, filtered, concentrated, and subjected to normal phase column chromatography purification (elution gradient: petroleum ether/ethyl acetate=1/1, v/v), to provide compound 6 (2.28 g, in a yield of 55.67%). MS ESI (*m/z*) = 604.3 $[M+H]^+$.the

(1-6) Synthesis of compound NM062with

Compound 6 (1.5 g, 2.48 mmol) was repeatedly dried three times by azeotrope with 10 ml of acetonitrile (3×10 ml), then dissolved in 15 ml of dichloromethane, followed by addition of a solution (15 ml) of 3-{[bis(diisopropylamino) phosphoryl]oxy}propanenitrile (899.6 mg, 2.98 mmol, repeatedly dried three times by azeotrope with 10 ml of acetonitrile (3×10 ml)) in dichloromethane, and then addition of 1*H*-imidazole-4,5-dicarbonitrile (374.8 mg, 1.984 mmol). Nitrogen replacement was performed three times, followed by stirring at 25 °C for 1 hour under nitrogen. After reaction was completed, reaction solution was diluted by adding 30 ml of saturated aqueous sodium bicarbonate solution, and extracted three times with 20 ml of dichloromethane (3×20 ml). Organic phases were combined, washed with 30 ml of saturated brine twice (2×30 ml), dried over anhydrous sodium sulfate, filtered, concentrated, and subjected to reverse phase column chromatography purification (elution gradient: water/acetonitrile=1/9, v/v), to provide compound NM062 (1.7 g, in a yield of 85.14%). MS ESI (*m/z*) = 604.3 $[M+H]^+$.

$^1H$ NMR (400 MHz, DMSO-$d_6$) δ = 11.41 (s, 1H), 7.75 (dd, *J* = 8.2, 10.6 Hz, 1H), 7.43 - 7.35 (m, 2H), 7.35 - 7.29 (m, 2H), 7.29 - 7.21 (m, 5H), 6.90 (dd, *J* = 6.1, 7.8 Hz, 4H), 5.88 (dd, *J* = 2.3, 4.2 Hz, 1H), 5.35 (dd, *J* = 8.5, 9.6 Hz, 1H), 4.81 - 4.68 (m, 2H), 4.48 - 4.33 (m, 2H), 4.19 - 4.04 (m, 1H), 3.74 (d, *J* = 2.0 Hz, 7H), 3.58 - 3.44 (m, 4H), 3.39 - 3.31 (m, 2H), 3.30 (br s, 1H), 2.78 (t, *J* = 5.9 Hz, 1H), 2.63 (t, *J* = 5.9 Hz, 1H), 1.16 - 1.03 (m, 12H), 0.98 (d, *J* = 6.7 Hz, 3H).

**Preparation Example 2: Synthesis of compound NM063**

A synthesis route of compound NM063 was as follows:

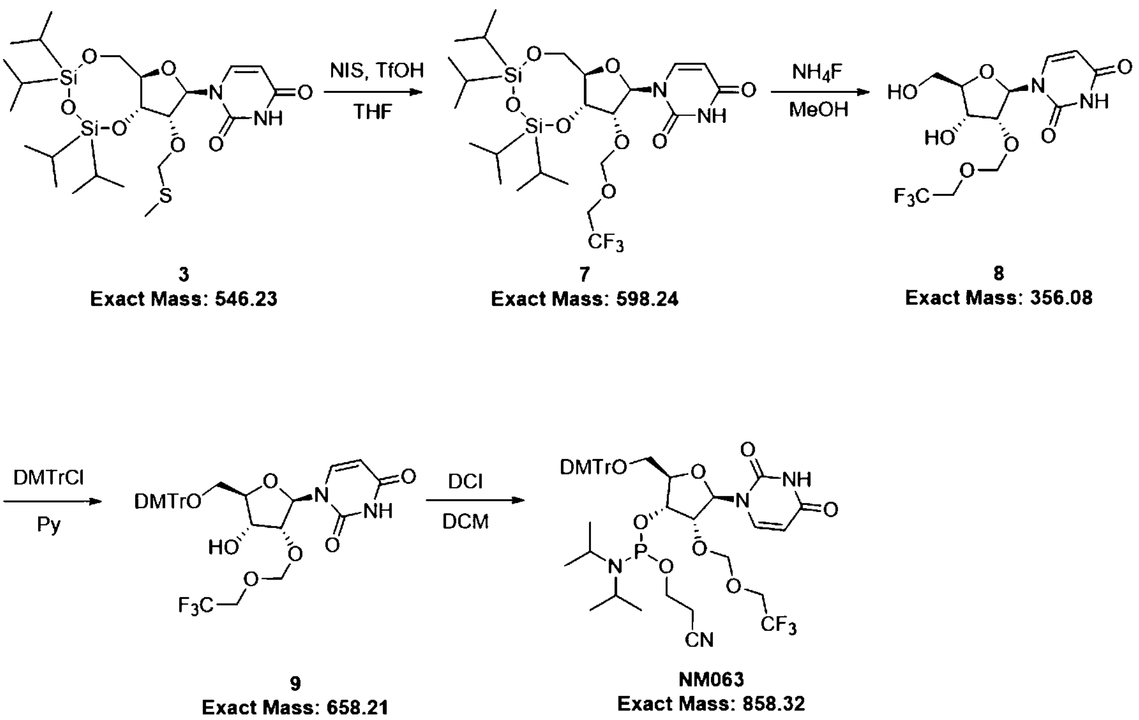


3
Exact Mass: 546.23

7
Exact Mass: 598.24

8
Exact Mass: 356.08

9
Exact Mass: 658.21

NM063
Exact Mass: 858.32

(2-1) Synthesis of compound 7

At 25 °C, compound 3 (5 g, 9.15 mmol) was dissolved in 50 ml of tetrahydrofuran, and trifluoroethanol (9.15 g, 91.5 mmol) was added. Nitrogen replacement was performed three times. A dry ice acetonitrile bath was used for cooling to -40 °C. A solution of N-iodosuccinimide (2.47 g, 10.98 mmol) in tetrahydrofuran (5 ml) and a solution of trifluoromethanesulfonic acid (2.75 g, 18.3 mmol) in tetrahydrofuran (5 ml) were added dropwise at -40 °C, followed by stirring at -40 °C for 1 hour under nitrogen. After completion, reaction was quenched by adding 80 ml of trimethylamine to reaction solution, and 10 mass% aqueous solution of sodium thiosulfate was added, followed by four times of extraction with 150 ml of dichloromethane (4×150 ml). Organic phases were combined, washed twice with 80 ml of saturated aqueous sodium bicarbonate solution (2×80 ml) and once with 100 ml of saturated brine (1×100 ml), dried over anhydrous sodium sulfate, filtered, concentrated, and subjected to normal phase column chromatography purification (elution gradient: petroleum ether/ethyl acetate=4/1, v/v), to provide compound 7 (3.04 g, in a yield of 55.51%). MS ESI (*m*/*z*) = 599.23 $[M+H]^+$.

(2-2) Synthesis of compound 8

At 25 °C, compound 7 (3 g, 5.01 mmol) was dissolved in methanol (32 ml), followed by addition of ammonium fluoride (926.85 mg, 25.05 mmol). Reaction solution was stirred at 60 °C for 16 hours. After reaction was completed, solvent was removed by spin-drying, followed by normal phase column chromatography purification (elution gradient: dichloromethane/methanol = 20/1, v/v), to provide compound 8 (1.51 g, 83.43% yield). MS ESI (*m*/*z*) = 356.26 $[M+H]^+$.

(2-3) Synthesis of compound 9

Compound 8 (1.51 g, 4.24 mmol) was dissolved in pyridine (15 ml) in ice bath, and cooled to 0 °C in the ice bath, and 4,4'-dimethoxytrityl chloride (1.73 g, 5.09 mmol) was added portion-wise. Reaction solution was stirred at 0 °C for 1 hour. After completion, reaction was quenched by adding methanol (2 ml), solvent was removed by spin-drying, a saturated aqueous ammonium chloride solution (50 ml) was added, and ethyl acetate (3×50 ml) was used for extraction three times. Organic phases were combined, washed with saturated brine (100 ml), dried over anhydrous sodium sulfate, filtered, concentrated, and subjected to normal phase column chromatography purification (elution gradient: petroleum ether/ethyl acetate=1/1, v/v), to provide compound 9 (2.08 g, in a yield of 74.53%). MS ESI (*m*/*z*) = 681.32 $[M+Na]^+$.

(2-4) Synthesis of compound NM063

Compound 9 (1.9 g, 2.89 mmol) was repeatedly dried with by azeotrope with 10 ml of acetonitrile acetonitrile (3×10 mL) and dissolved in dichloromethane (15 mL), followed by addition of a solution of 3-{[bis(diisopropylamino) phosphoryl]oxylpropanenitrile (1.307 g, 3.46 mmol) dried with acetonitrile by azeotrop (3×10 mL) in dichloromethane (15 mL), and 1*H*-imidazole-4,5-dicarbonitrile (272.5 mg, 2.312 mmol). Nitrogen replacement was performed three times. Reaction solution was stirred at 25 °C for 1 hour under nitrogen. After reaction was completed, saturated aqueous sodium bicarbonate solution (30 mL) was added for dilution, and dichloromethane (3×20 mL) was used for extraction. Organic phases were combined, washed with saturated brine twice (2×30 mL), dried over anhydrous sodium sulfate, filtered, concentrated, and subjected to reverse phase column chromatography purification (C18 chromatographic column, elution gradient: water/acetonitrile=1/9, v/v), to provide compound NM063 (2.07 g, in a yield of 83.55%). MS ESI (*m*/*z*) = 859.43 $[M+H]^+$.

$^1H$ NMR (400 MHz, DMSO-$d_6$) δ = 11.41 (s, 1H), 7.77 (dd, *J* = 6.0, 7.9 Hz, 1H), 7.43 - 7.35 (m, 2H), 7.35 - 7.18 (m, 7H), 6.95 - 6.85 (m, 4H), 5.90 - 5.83 (m, 1H), 5.28 (d, *J* = 8.1 Hz, 1H), 4.99 - 4.86 (m, 2H), 4.53 - 4.40 (m, 2H), 4.26 - 4.02 (m, 3H), 3.85 - 3.59 (m, 8H), 3.52 (qd, *J* = 6.7, 10.4 Hz, 2H), 3.43 - 3.32 (m, 2H), 2.81 - 2.75 (m, 1H), 2.63 (t, *J* = 5.9 Hz, 1H), 1.16 - 1.04 (m, 9H), 0.97 (d, *J* = 6.7 Hz, 3H).

**Preparation Example 3: Synthesis of compound NM096**

In the present preparation example, a synthesis route of compound NM096 was as follows:

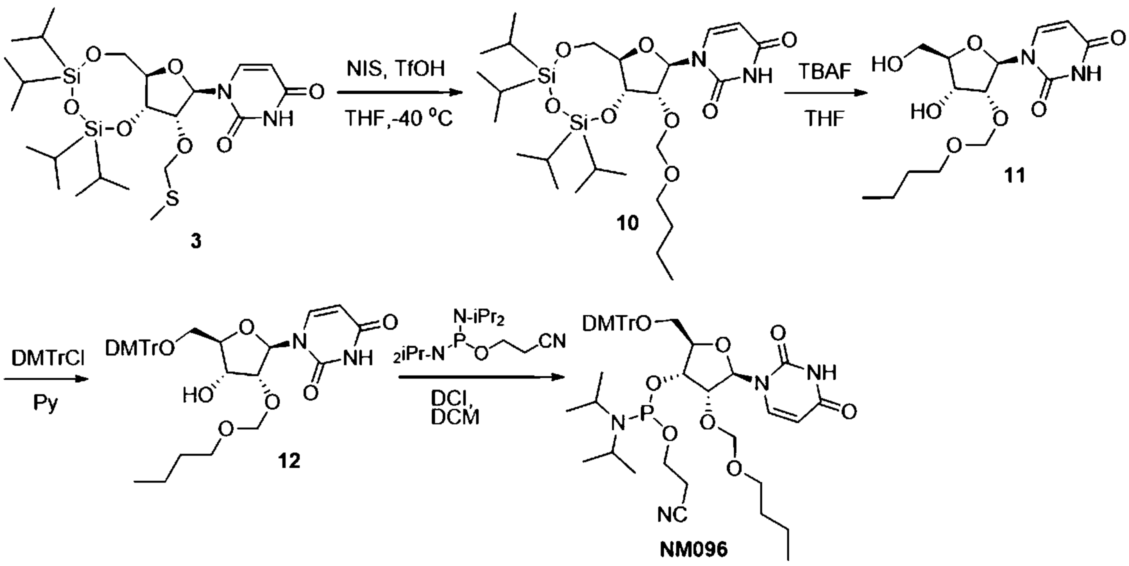


3 10 11 12 NM096

(3-1) Synthesis of compound 10

At 25 °C, compound 3 (3 g, 5.49 mmol) was dissolved in tetrahydrofuran (30 mL), and n-butanol (4.06 g, 54.92 mmol, 10 eq, CAS No. 71-36-3) was added. Nitrogen replacement was performed three times. Reaction system was cooled by dry ice to -40 °C, and a solution of N-iodosuccinimide (1.48 g, 6.59 mmol, 1.2 eq, abbreviated as NIS, CAS No. 516-12-1) in tetrahydrofuran (5 ml) and a solution of trifluoromethanesulfonic acid (1.64 g, 10.98 mmol, 2 eq, abbreviated as TfOH, CAS No. 1493-13-6) in tetrahydrofuran (5 ml) were added dropwise. Reaction system was stirred at -40 °C for 1 hour, and quenched by adding triethylamine (6 mL). After reaction was completed, reaction solution was concentrated, extracted by adding dichloromethane (2×50 mL), washed with a saturated aqueous sodium bicarbonate (2×50 mL) and a saturated aqueous sodium chloride solution (30 ml), dried over anhydrous sodium sulfate, filtered, concentrated, and subjected to column chromatography purification (eluent: petroleum ether/ethyl acetate=3/1, volume ratio v/v), to provide compound 10 (3.05 g, in a yield of 96.95%) as a white solid. MS ESI (*m*/*z*) = 573.3 $[M + H]^+$.

(3-2) Synthesis of compound 11

At 25 °C, compound 10 (3.05 g, 5.33 mmol, 1 eq) was dissolved in tetrahydrofuran (30 ml), followed by addition of a solution of tetrabutylammonium fluoride (CAS No. 429-41-4) in tetrahydrofuran (10.66 mL, 1 M, 10.66 mmol, 2 eq). Reaction solution was stirred at 25 °C for 3 hours. After reaction was completed, the reaction solution was concentrated, and subjected to column chromatography purification (eluent: dichloromethane/methanol=10/1, v/v), to provide compound 11 (1.7 g, in a yield of 98.27%) as a white solid. MS ESI (*m*/*z*) = 353.4 $[M + Na]^+$.

(3-3) Synthesis of compound 12

At 25 °C, compound 11 (1.7 g, 5.15 mmol, 1 eq) was dissolved in pyridine (17 ml), cooled to 0 °C in ice bath, and 4,4'-dimethoxytrityl chloride (2.27 g, 6.69 mmol, 1.3 eq, abbreviated as DMTrCl, CAS No. 40615-36-9) was added portion-wise. Reaction solution was stirred at 25 °C for 1 hour, and quenched by adding methanol. After reaction was completed, the reaction solution was concentrated, diluted by adding ethyl acetate (50 mL), washed with a saturated aqueous ammonium chloride solution (2×30 mL) and a saturated brine (30 ml), dried over anhydrous sodium sulfate, filtered, concentrated, and subjected to column chromatography purification (eluent: petroleum ether/ethyl acetate=1/1, v/v), to provide compound 12 (2.44 g, in a yield of 74.89%) as a yellow solid. MS ESI (m/z) = 633.3 $[M + H]^+$.

(3-3) Synthesis of compound NM096

Compound 12 (2.44 g, 3.85 mmol, 1 eq) was repeatedly dried by azeotrope with acetonitrile (3×30 mL) and then dissolved in dichloromethane (25 mL), followed by addition of a solution of bis(diisopropylamino)(2-cyanoethoxy) phosphine (1.63 g, 5.78 mmol, 1.5 eq, CAS No. 102691-36-1) dried by azeotrope with acetonitrile (3×10 mL) in dichloromethane (25 mL), and addition of 1H-imidazole-4,5-dicarbonitrile (364.2 mg, 3.08 mmol, 0.8 eq, CAS No. 1122-28-7). Nitrogen replacement was performed three times. Reaction system was stirred at 25 °C for 1 hour under nitrogen. After reaction was completed, reaction solution was diluted by adding a saturated aqueous sodium bicarbonate

solution (50 ml), and extracted with dichloromethane (3×30 ml). Organic phases were combined, washed with a saturated aqueous sodium chloride solution (2×30 ml), dried over anhydrous sodium sulfate, filtered, concentrated, and subjected to column chromatography purification (eluent: petroleum ether/ethyl acetate=2/1, v/v), to provide compound NM096 (2.7 g, in a yield of 84.11%) as a white solid. MS ESI (*m*/*z*) = 833.4 [M + H]$^+$.

$^1$H NMR (400 MHz, DMSO-d6) δ 11.36 (s, 1H), 7.72 (dd, J = 11.8, 8.1 Hz, 1H), 7.42 - 7.29 (m, 4H), 7.29 - 7.20 (m, 5H), 6.89 (dd, J = 8.6, 5.8 Hz, 4H), 5.89 (dd, J = 5.3, 3.1 Hz, 1H), 5.37 (t, J = 7.7 Hz, 1H), 4.79 - 4.66 (m, 2H), 4.40 (dp, J = 19.0, 4.8 Hz, 2H), 4.12 (dq, J = 20.3, 3.8 Hz, 1H), 3.74 (d, J = 2.1 Hz, 7H), 3.71 - 3.58 (m, 1H), 3.53 (dq, J = 9.8, 6.9 Hz, 2H), 3.49 - 3.40 (m, 2H), 3.39 - 3.32 (m, 1H), 2.78 (t, J = 6.1 Hz, 1H), 2.63 (t, J = 5.9 Hz, 1H), 1.42 (dq, J = 10.1, 6.8 Hz, 2H), 1.26 (dq, J = 14.3, 7.3 Hz, 2H), 1.20 - 1.06 (m, 10H), 0.99 (d, J = 6.7 Hz, 3H), 0.83 (td, J = 7.4, 3.5 Hz, 3H).

**Preparation Example 4: Synthesis of compound NM097**

In the present preparation example, a synthesis route of compound NM097 was as follows:

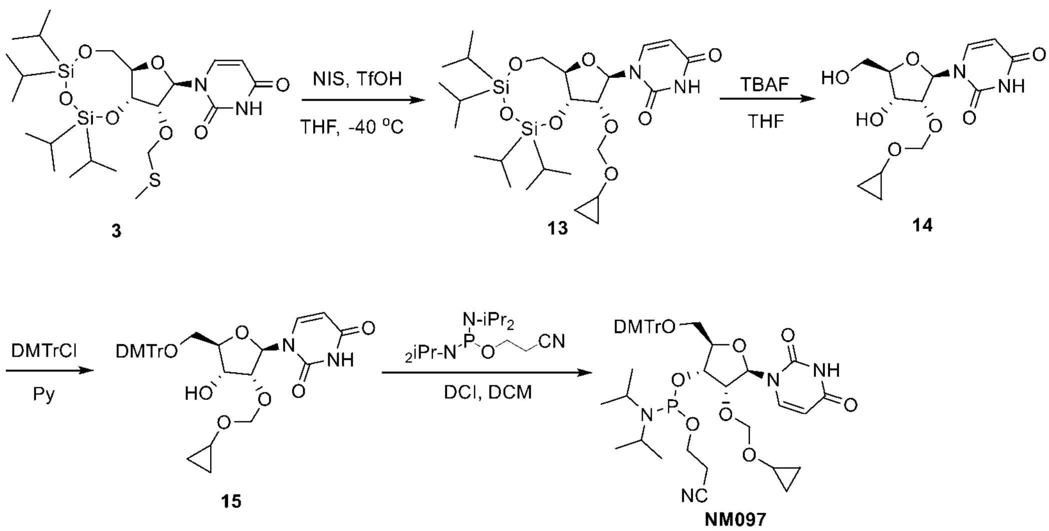


3 13 14 15 NM097

(4-1) Synthesis of compound 13

At 25 °C, compound 3 (3 g, 5.49 mmol, 1 eq) was dissolved in tetrahydrofuran (30 mL), and cyclopropanol (3.18 g, 54.92 mmol, 10 eq) was added. Nitrogen replacement was performed three times. Reaction system was cooled to -40 °C using dry ice, and a solution of NIS (1.48 g, 6.59 mmol, 1.2 eq) in tetrahydrofuran (5 ml) and a solution of TfOH (1.64 g, 10.98 mmol, 2 eq) in tetrahydrofuran (5 ml) were added dropwise. Reaction system was stirred at -40 °C for 1 hour, and quenched by adding triethylamine (6 mL). After reaction was completed, reaction solution was concentrated, and extracted by adding dichloromethane (2×50 mL). Organic phases were combined, washed with a saturated aqueous sodium bicarbonate (2×50 mL) and a saturated aqueous sodium chloride solution (30 ml), dried over anhydrous sodium sulfate, filtered, concentrated, and subjected to column chromatography purification (eluent: petroleum ether/ethyl acetate=3/1, v/v), to provide compound 13 (2.59 g, in a yield of 84.78%) as a white solid. MS ESI (m/z) = 557.3 [M + H]$^+$.

(4-2) Synthesis of compound 14

At 25 °C, compound 13 (2.59 g, 4.66 mmol, 1 eq) was dissolved in tetrahydrofuran (30 ml), followed by addition of a solution of tetrabutylammonium fluoride in tetrahydrofuran (9.32 mL, 1 M, 9.32 mmol, 2 eq). Reaction solution was stirred at 25 °C for 3 hours. After reaction was completed, reaction solution was concentrated, and subjected to column chromatography purification (eluent: dichloromethane/methanol=20/1, v/v), to provide compound 14 (1.45 g, in a yield of 99.3%) as a white solid. MS ESI (m/z) = 337.1 [M + Na]$^+$.

(4-3) Synthesis of compound 15

At 25 °C, compound 14 (1.45 g, 4.62 mmol, 1 eq) was dissolved in pyridine (15 ml), cooled to 0 °C in ice bath, and DMTrCl (2.05 g, 6.00 mmol, 1.3 eq) was added portion-wise. Reaction solution was stirred at 25 °C for 1 hour, and quenched by adding methanol. After reaction was completed, the reaction solution was concentrated, diluted by adding ethyl acetate (50 mL), washed with a saturated aqueous ammonium chloride solution (2×30 mL) and a saturated aqueous

sodium chloride solution (30 ml), dried over anhydrous sodium sulfate, filtered, concentrated, and subjected to column chromatography purification (eluent: petroleum ether/ethyl acetate=1/1, v/v), to provide compound 15 (1.69 g, in a yield of 59.5%) as a yellow solid. MS ESI (m/z) = 617.2 $[M + H]^+$.

(4-4) Synthesis of compound NM097

Compound 15 (1.69 g, 2.74 mmol, 1 eq) was repeatedly dried by azeotrope with acetonitrile (3×10 mL) and dissolved in dichloromethane (20 mL), followed by addition of a solution of bis(diisopropylamino)(2-cyanoethoxy) phosphine (1.24 g, 4.11 mmol, 1.5 eq) dried by azeotrope with acetonitrile (3×10 mL) in dichloromethane (25 mL), and addition of 1*H*-imidazole-4,5-dicarbonitrile (258.9 mg, 2.19 mmol, 0.8 eq). Nitrogen replacement was performed three times. Reaction solution was stirred at 25 °C for 1 hour under nitrogen. After reaction was completed, the reaction solution was diluted by adding a saturated aqueous sodium bicarbonate solution (50 ml), and extracted with dichloromethane (3×30 ml). Organic phases were combined, washed with a saturated aqueous sodium chloride solution (2×30 ml), dried over anhydrous sodium sulfate, filtered, concentrated, and subjected to column chromatography purification (eluent: petroleum ether/ethyl acetate=1/1, v/v), to provide compound NM097 (1.7 g, in a yield of 75.96%) as a white solid. MS ESI (m/z) = 817.2 $[M + H]^+$.

$^1H$ NMR (400 MHz, DMSO-d6) δ 11.40 (s, 1H), 7.76 (dd, J = 10.9, 8.1 Hz, 1H), 7.42 - 7.29 (m, 4H), 7.29 - 7.21 (m, 5H), 6.89 (td, J = 6.6, 3.2 Hz, 4H), 5.90 (dt, J = 7.1, 3.6 Hz, 1H), 5.40 - 5.24 (m, 1H), 4.94 - 4.68 (m, 2H), 4.56 - 4.37 (m, 2H), 4.13 (dt, J = 22.0, 4.1 Hz, 1H), 3.74 (d, J = 2.1 Hz, 7H), 3.71 - 3.44 (m, 4H), 3.35 (t, J = 3.6 Hz, 1H), 3.30 (d, J = 4.8 Hz, 1H), 2.78 (t, J = 5.9 Hz, 1H), 2.63 (t, J = 5.9 Hz, 1H), 1.15 - 1.08 (m, 9H), 0.98 (d, J = 6.8 Hz, 3H), 0.56 - 0.36 (m, 4H).

**<u>Preparation Example</u>** <u>5</u>: **<u>Synthesis of compound NM098</u>**

In the present preparation example, a synthesis route of compound NM098 was as follows:

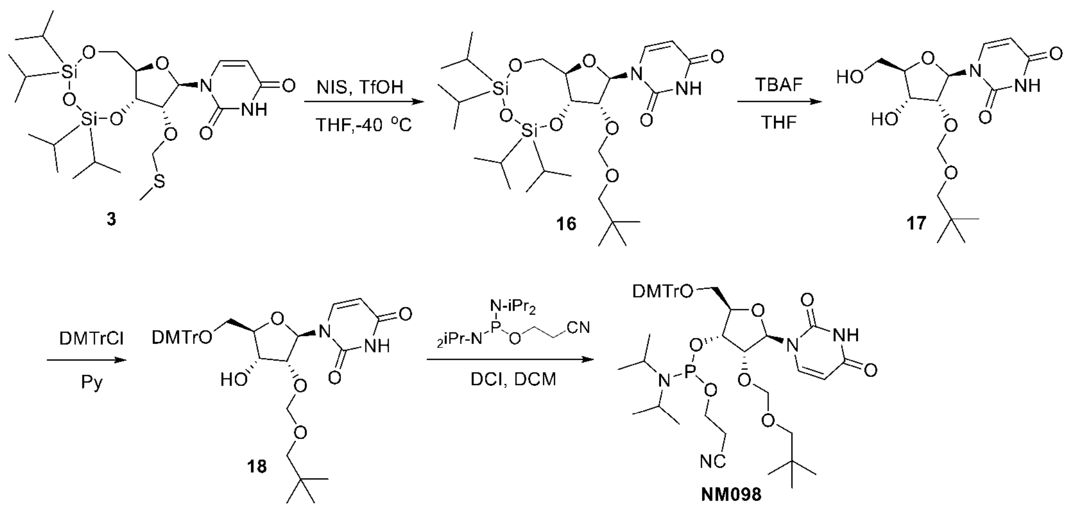


3 16 17 18 NM098

(5-1) Synthesis of compound 16

At 25 °C, compound 3 (3 g, 5.49 mmol, 1 eq) was dissolved in tetrahydrofuran (30 mL), followed by addition of neopentyl alcohol (4.83 g, 54.92 mmol, 10 eq, CAS No. 75-84-3). Nitrogen replacement was performed three times. Reaction system was cooled to -40 °C using dry ice, and a solution of NIS (1.48 g, 6.59 mmol, 1.2 eq) in tetrahydrofuran (5 ml) and a solution of TfOH (1.64 g, 10.98 mmol, 2 eq) in tetrahydrofuran (5 ml) were added dropwise. Reaction solution was stirred at -40 °C for 1 hour, and quenched by adding triethylamine (6 mL). After reaction was completed, the reaction solution was concentrated, and extracted by adding dichloromethane (2×50 mL). Organic phases were combined, washed with a saturated aqueous sodium bicarbonate (2×50 mL) and a saturated aqueous sodium chloride solution (30 ml), dried over anhydrous sodium sulfate, filtered, concentrated, and subjected to column chromatography purification (eluent: petroleum ether/ethyl acetate=3/1, v/v), to provide compound 16 (3.06 g, in a yield of 95.03%) as a white solid. MS ESI (*m*/*z*) = 587.3 $[M + H]^+$.

(5-2) Synthesis of compound 17

At 25 °C, compound 16 (3.06 g, 5.22 mmol, 1 eq) was dissolved in tetrahydrofuran (30 ml), followed by addition of

a solution of tetrabutylammonium fluoride in tetrahydrofuran (10.44 mL, 1 M, 10.44 mmol, 2 eq). Reaction solution was stirred at 25 °C for 3 hours. After reaction was completed, the reaction solution was concentrated, and subjected to column chromatography purification (eluent: dichloromethane/methanol=10/1, v/v), to provide compound 17 (1.78 g, in a yield of 99.1%) as a white solid. MS ESI (*m/z*) = 367.1 [M + Na]$^+$.

(5-3) Synthesis of compound 18

At 25 °C, compound 17 (1.78 g, 5.17 mmol, 1 eq) was dissolved in pyridine (15 ml), and cooled to 0 °C in ice bath. DMTrCl (2.28 g, 6.72 mmol, 1.3 eq) was added portion-wise. Reaction solution was stirred at 25 °C for 1 hour, and quenched by adding methanol. After reaction was completed, the reaction solution was concentrated, diluted by adding ethyl acetate (50 mL), washed with a saturated aqueous ammonium chloride solution (2×30 mL) and a saturated aqueous sodium chloride solution (30 ml), dried over anhydrous sodium sulfate, filtered, concentrated, and subjected to column chromatography purification (eluent: petroleum ether/ethyl acetate=1/1, v/v), to provide compound 18 (2.50 g, in a yield of 74.85%) as a yellow solid. MS ESI (*m/z*) = 647.3 [M + H]$^+$.

(5-4) Synthesis of compound NM098

Compound 18 (2.50 g, 3.87 mmol, 1 eq) was repeatedly dried by azeotrope with acetonitrile (3×10 mL) and dissolved in dichloromethane (30 mL), followed by addition of a solution of bis(diisopropylamino)(2-cyanoethoxy) phosphine (1.75 g, 5.80 mmol, 1.5 eq) dried by azeotrope with acetonitrile (3×10 mL) in dichloromethane (25 mL), and addition of 1*H*-imidazole-4,5-dicarbonitrile (364.9 mg, 3.10 mmol, 0.8 eq). Nitrogen replacement was performed three times. Reaction solution was stirred at 25 °C for 1 hour under nitrogen. After reaction was completed, the reaction solution was diluted by adding a saturated aqueous sodium bicarbonate solution (50 ml), and extracted with dichloromethane (3×30 ml). Organic phases were combined, washed with a saturated aqueous sodium chloride solution (2×30 ml), dried over anhydrous sodium sulfate, filtered, concentrated, and subjected to column chromatography purification (eluent: petroleum ether/ethyl acetate=1/1, v/v), to provide compound NM098 (2.84 g, in a yield of 86.87%) as a white solid. MS ESI (m/z) = 847.4 [M + H]$^+$.

$^1$H NMR (400 MHz, DMSO-$d_6$) δ 11.41 (s, 1H), 7.72 (dd, *J* = 9.6, 8.1 Hz, 1H), 7.43 - 7.30 (m, 4H), 7.30 - 7.20 (m, 5H), 6.89 (td, *J* = 6.8, 3.3 Hz, 4H), 5.89 (dd, *J* = 4.6, 2.4 Hz, 1H), 5.38 (d, *J* = 8.0 Hz, 1H), 4.85 - 4.66 (m, 2H), 4.38 (dp, *J* = 15.0, 4.9, 4.4 Hz, 2H), 4.12 (dd, *J* = 20.1, 3.7 Hz, 1H), 3.74 (d, *J* = 2.2 Hz, 7H), 3.70 - 3.46 (m, 3H), 3.42 - 3.32 (m, 1H), 3.28 (t, *J* = 5.4 Hz, 1H), 3.19 - 3.08 (m, 2H), 2.78 (td, *J* = 5.9, 2.1 Hz, 1H), 2.62 (t, *J* = 5.9 Hz, 1H), 1.15 - 1.08 (m, 9H), 0.98 (d, *J* = 6.7 Hz, 3H), 0.82 (d, *J* = 5.9 Hz, 9H).

**Preparation Example 6: Synthesis of compound NM099**

In the present preparation example, a synthesis route of compound NM099 was as follows:

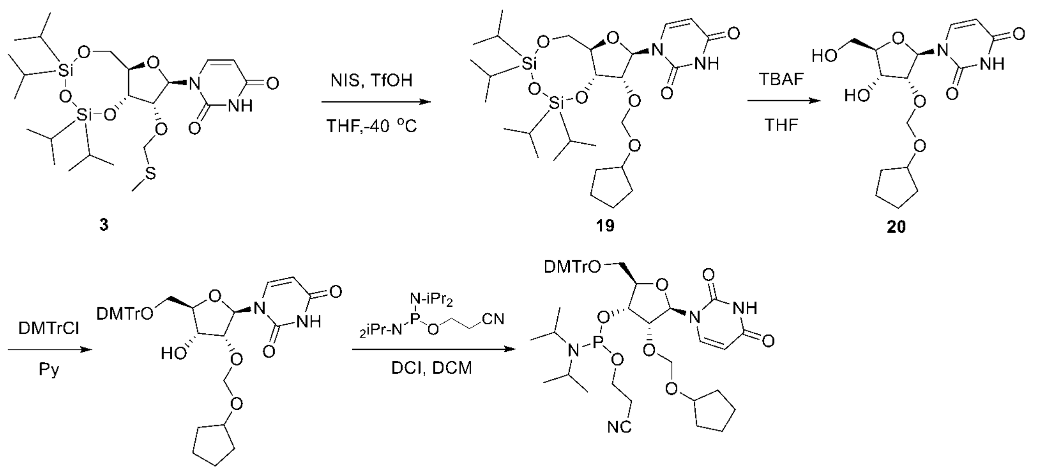


3 19 20

21 NM099

(6-1) Synthesis of compound 19

At 25 °C, compound 3 (3 g, 5.49 mmol, 1 eq) was dissolved in tetrahydrofuran (30 mL), followed by addition of cyclopentanol (4.74 g, 54.92 mmol, 10 eq). Nitrogen replacement was performed three times. Reaction system was cooled

to -40 °C using dry ice, and a solution of NIS (1.48 g, 6.59 mmol, 1.2 eq) in tetrahydrofuran (5 ml) and a solution of TfOH (1.64 g, 10.98 mmol, 2 eq) in tetrahydrofuran (5 ml) were added dropwise. Reaction system was stirred at -40 °C for 1 hour, and quenched by adding triethylamine (6 mL). After reaction was completed, reaction solution was concentrated, and extracted by adding dichloromethane (2×50 mL), washed with a saturated aqueous sodium bicarbonate (2×50 mL) and a saturated aqueous sodium chloride solution (30 ml), dried over anhydrous sodium sulfate, filtered, concentrated, and subjected to column chromatography purification (eluent: petroleum ether/ethyl acetate=4/1, v/v), to provide compound 19 (3.20 g, in a yield of 99.6%) as a white solid. MS ESI (m/z) = 585.3 $[M + H]^+$.

(6-2) Synthesis of compound 20

At 25 °C, compound 19 (3.20 g, 5.48 mmol, 1 eq) was dissolved in tetrahydrofuran (30 ml), followed by addition of a solution of tetrabutylammonium fluoride in tetrahydrofuran (10.95 mL, 1 M, 10.95 mmol, 2 eq). Reaction solution was stirred at 25 °C for 3 hours. After reaction was completed, the reaction solution was concentrated, and subjected to column chromatography purification (eluent: dichloromethane/methanol=20/1, v/v), to provide compound 20 (1.87 g, in a yield of 99.3%) as a white solid. MS ESI (m/z) = 365.1 $[M + Na]^+$.

(6-3) Synthesis of compound 21

At 25 °C, compound 20 (1.87 g, 5.47 mmol, 1 eq) was dissolved in pyridine (20 ml), and cooled to 0 °C in ice bath. DMTrCl (2.41 g, 7.11 mmol, 1.3 eq) was added portion-wise. Reaction solution was stirred at 25 °C for 1 hour, and quenched by adding methanol. After reaction was completed, the reaction solution was concentrated, diluted by adding ethyl acetate (50 mL), washed with a saturated aqueous ammonium chloride solution (2×30 mL) and a saturated aqueous sodium chloride solution (30 ml), dried over anhydrous sodium sulfate, filtered, concentrated, and subjected to column chromatography purification (eluent: petroleum ether/ethyl acetate=1/1), to provide compound 21 (2.18 g, in a yield of 61.93%) as a yellow solid. MS-ESI (m/z) = 645.3 $[M + H]^+$.

(6-4) Synthesis of compound NM099

Compound 21 (2.18 g, 3.38 mmol, 1 eq) was repeatedly dried by azeotrope with acetonitrile three times (10 mL each time) and then dissolved in dichloromethane (20 mL), followed by addition of a solution of bis(diisopropylamino)(2-cyanoethoxy)phosphine (1.53 g, 5.08 mmol, 1.5 eq) dried by azeotrope with acetonitrile (3×10 mL) in dichloromethane (25 mL), and addition of 1*H*-imidazole-4,5-dicarbonitrile (319.7 mg, 2.71 mmol, 0.8 eq). Nitrogen replacement was performed three times. Reaction system was stirred at 25 °C for 1 hour under nitrogen. After reaction was completed, the reaction solution was diluted by adding a saturated aqueous sodium bicarbonate solution (50 ml), and extracted with dichloromethane (3×30 ml). Organic phases were combined, washed with a saturated aqueous sodium chloride solution (2×30 ml), dried over anhydrous sodium sulfate, filtered, concentrated, and subjected to column chromatography purification (eluent: petroleum ether/ethyl acetate=2/1), to provide compound NM099 (2.28 g, in a yield of 79.80%) as a white solid. MS ESI (m/z) = 845.3 $[M + H]^+$.

$^1$H NMR (400 MHz, DMSO-d6) δ 11.40 (s, 1H), 7.72 (dd, J = 12.3, 8.1 Hz, 1H), 7.35 (dt, J = 21.9, 6.3 Hz, 4H), 7.26 (t, J = 8.9 Hz, 5H), 6.89 (dd, J = 8.6, 5.7 Hz, 4H), 5.90 (d, J = 5.6 Hz, 1H), 5.47 - 5.34 (m, 1H), 4.72 (dq, J = 14.7, 7.1 Hz, 2H), 4.49 - 4.31 (m, 2H), 4.18 - 4.07 (m, 2H), 3.74 (d, J = 2.0 Hz, 7H), 3.70 - 3.46 (m, 3H), 3.35 (q, J = 7.5, 4.9 Hz, 1H), 3.29 (d, J = 3.2 Hz, 1H), 2.78 (t, J = 6.0 Hz, 1H), 2.63 (t, J = 5.9 Hz, 1H), 1.72 - 1.35 (m, 8H), 1.12 (td, J = 6.7, 4.4 Hz, 9H), 0.99 (d, J = 6.7 Hz, 3H).

## Preparation Example 7: Synthesis of compound NM054

In the present preparation example, a synthesis route of compound NM054 was as follows:

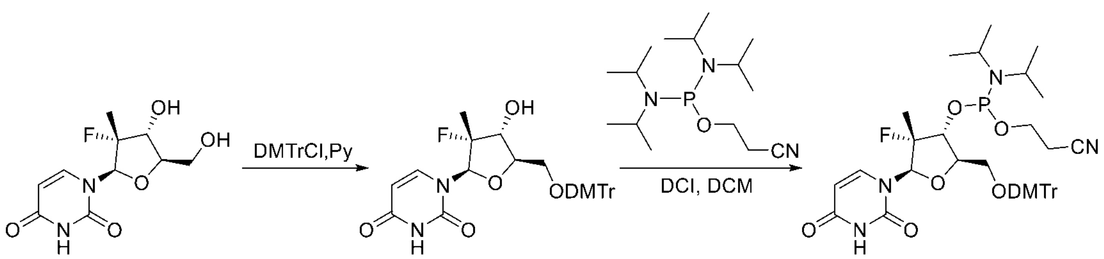


NM054-1 NM054-2 NM054

(7-1) Synthesis of compound NM054-2

To a 500 ml reaction kettle, compound NM054-1 (3 g, 11.54 mmol, 1.0 eq, (2'R)-2'-deoxy-2'-fluoro-2'-methyluridine, CAS No. 863329-66-2) and pyridine (30 ml) were added, cooled to 0 °C, and 4,4'-dimethoxytrityl chloride (4.29 g, 12.7 mmol, 1.1 eq) was added portion-wise. Nitrogen replacement was performed three times. Reaction system was stirred at 25 °C for 3 hours under nitrogen. HPLC showed no raw material. After reaction was completed, reaction solution was concentrated, extracted by adding purified water (50 ml) and ethyl acetate (50 ml). Organic phases were separated, dried over anhydrous sodium sulfate, filtered, concentrated, and subjected to column chromatography purification, to provide compound NM054-2 (2.7 g, in a yield of 41.7%). MS ESI (m/z) =563.0 $[M + H]^+$.

(7-2) Synthesis of compound NM054

To a 100 ml reaction kettle, compound NM054-2 (2.7 g, 4.8 mmol, 1.0 eq) was added, and bis(diisopropylamino)(2-cyanoethoxy)phosphine (1.74 g, 5.76 mmol, 1.2 eq) was added portion-wise, followed by addition of 4,5-dicyanoimidazole (0.45 g, 3.8 mmol, 0.8 eq, CAS No. 1122-28-7) and dichloromethane (27 ml). Nitrogen replacement was performed three times. Reaction system was stirred at 25 °C for 3 hours under nitrogen. After reaction was completed, an aqueous sodium bicarbonate solution (20 ml) was added to reaction solution. Organic phases were separated, dried over anhydrous sodium sulfate, filtered, concentrated, and subjected to reverse phase column chromatography purification (eluent: acetonitrile/water=90/10, v/v), to provide compound NM054 (3.0 g). MS ESI (m/z) = 763 $[M + H]^+$.

**Preparation Example 8: Preparation of compound CR01008 and compound CR01008Z**

(8-1) Synthesis of compound CR01008

In the present preparation example, a synthesis route of compound CR01008 was as follows:

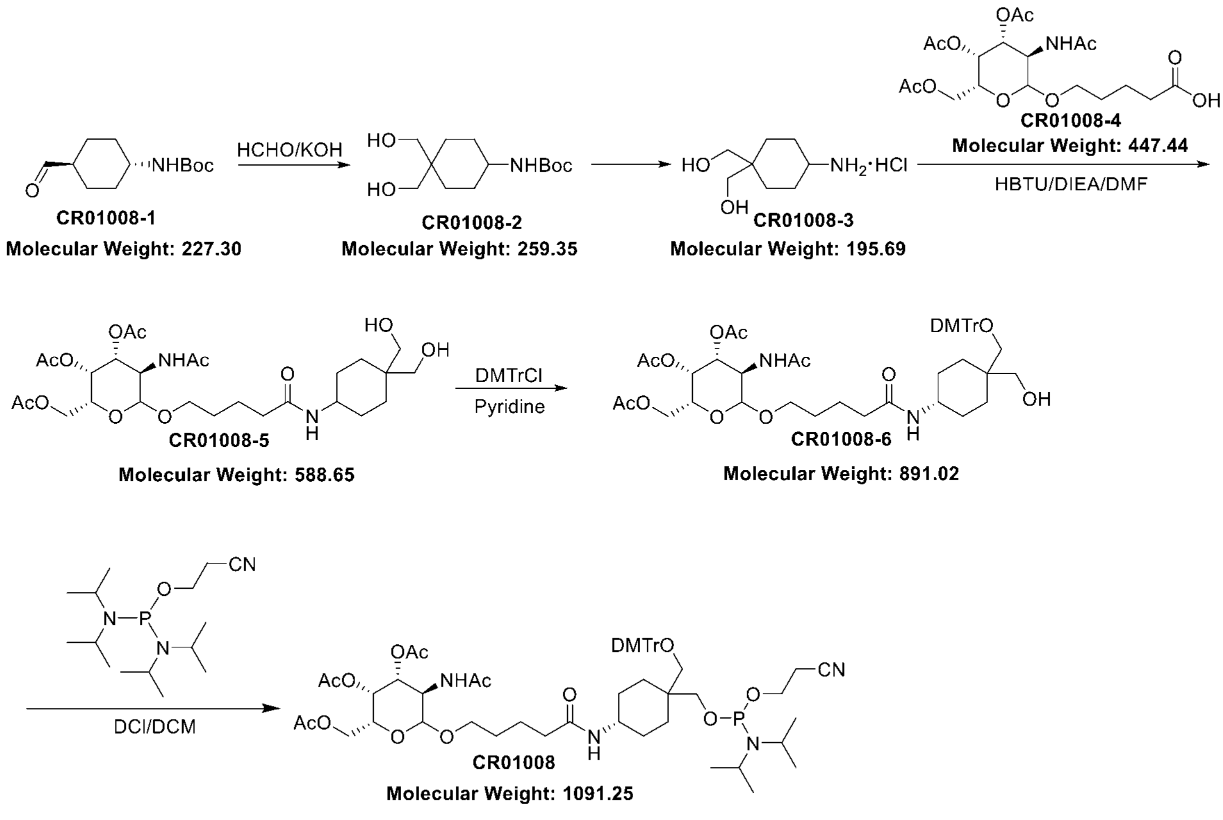


(8-1-1) Synthesis of compound CR01008-2

Compound CR01008-1 (10.0 g, 1.0 eq, trans-4-(Boc-amino)cyclohexanecarboxaldehyde, CAS No. 181308-57-6) and formaldehyde aqueous solution (8.9 g, 37 mass%, 2.4 eq) were dissolved in 33 ml of methanol. 13 ml of 45.3 mass% KOH aqueous solution was added dropwise. After the dropwise addition was completed, mixture was

stirred and reacted at 25 °C for 30 min, heated to 60 °C and subjected to reflux reaction at 60 °C for 2 hours. After reaction was ended, reaction solution was cooled to room temperature and then evaporated to dryness under reduced pressure, to yield a crude product as a white solid. The crude product was slurried by adding a small amount of water, and filtered, to provide compound CR01008-2 (9 g, in a yield of 78.9%) as a white solid. MS-ESI (*m*/*z*) = 260 [M + H]$^+$.

(8-1-2) Synthesis of compound CR01008-3

Compound CR01008-2 (9 g, 1 eq) was dissolved in 70 ml of 1,4-dioxane, followed by addition of a solution of hydrogen chloride in 1,4-dioxane solution (45 ml, 4 M), and mixture was stirred and reacted at 25 °C for 1 hour. After reaction was ended, a reaction solution was evaporated to dryness under reduced pressure, to provide compound CR01008-3 (6.8 g, in a yield of 100%) as a white solid.

(8-1-3) Synthesis of compound CR01008-5

Compound CR01008-3 (2.1 g, 2.0 eq) and compound CR01008-4 (2.1 g, 1.0 eq, 5-[[(2R,3R,4R,5R,6R)-3-acetamido-4,5-diacetoxy-6-(acetoxymethyl)-2-tetrahydropyranyl]oxy]pentanoic acid and *N,N*-diisopropylethylamine (3.5 g, 6.0 eq, abbreviated as DIEA) were dissolved in 15 ml of DMF, followed by addition of benzotriazole-*N,N,N',N'*-tetramethyluronium hexafluorophosphate (1.9 g, 1.1 eq, abbreviated as HBTU, CAS No. 94790-37-1), and mixture was stirred and reacted at 25 °C for 3 hours under nitrogen. After reaction was ended, reaction solution was evaporated to dryness under reduced pressure and subjected to reverse phase purification (eluent: 22 vol% aqueous solution of acetonitrile), to provide compound CR01008-5 (1.78 g, in a yield of 64.4%) as a white solid. MS-ESI (m/z) = 589[M + H]$^+$.

(8-1-4) Synthesis of compound CR01008-6

Compound CR01008-5 (1.54 g, 1.0 eq) was dissolved in 15 ml of pyridine, reaction system was cooled to 0 °C using an ice bath, followed by addition of 4,4'-dimethoxytrityl chloride (1.32 g, 1.5 eq, abbreviated as DMTrCl, CAS No. 40615-36-9) at 0 °C. Reaction was carried out at 25 °C for 3 hours, and 15 ml of methanol was added to reaction solution to quench the reaction. After reaction was end, the reaction solution was evaporated to dryness under reduced pressure and subjected to reverse phase purification (eluent: 60 vol% aqueous solution of acetonitrile), to provide compound CR01008-6 (1 g, in a yield of 42.7%) as a yellow solid. MS-ESI (m/z) = 891 [M + H]$^+$.

(8-1-5) Synthesis of compound CR01008

Compound CR01008-6 (1.08 g, 1.0 eq) was dissolved in 20 ml of anhydrous dichloromethane, followed by addition of 4,5-dicyanoimidazole (115 mg, 0.8 eq, abbreviated as DCI, CAS No. 1122-28-7) and bis(diisopropylamino)(2-cyanoethoxy)phosphine (732 mg, 2.1 eq, CAS No. 102691-36-1) respectively. Nitrogen replacement was performed three times. Reaction was carried out under stirring at 25 °C for 2 hours. After reaction was ended, reaction solution was added with 20 ml of saturated sodium bicarbonate aqueous solution, and extracted with 20 ml of dichloromethane three times (3×20 ml). Organic phases were combined, evaporated to dryness under reduced pressure, subjected to reverse phase purification (eluent: 72 vol% aqueous solution of acetonitrile), and then vacuum-dried for 12 hours, to provide compound CR01008 (1 g, in a yield of 76.0%) as a white powder. MS-ESI (m/z) = 1091 [M + Na]$^+$.

1H NMR (400 MHz, DMSO-d6) 08urification (eluent: aqueous solution of 72 vol% acetonitrile), and then vacuum-dried for 12 hours32 mg, 2.1 eq, CAS No. 102691-36-1) were added respectively. the reaction. S No. 94790-37-1) was added, and mixture was stirred a, 2.87 (d, J = 8.4 Hz, 1H),3.36 (s, 1H), 3.58 - 3.39 (m, 3H), 3.69 - 3.60 (m, 2H), 3.75 (s, 7H), 3.90 (dt, J = 11.2, 8.8 Hz, 1H), 4.05 (s, 3H),4.51 (d, J = 8.4 Hz, 1H), 4.99 (dd, J = 11.3, 3.4 Hz, 1H), 5.24 (d, J = 3.4 Hz, 1H), 5.78 (s, 1H), 6.93 2.1 eq(m, 4H),7.35 - 7.21 (m, 7H), 7.44 - 7.37 (m, 2H), 7.66 (d, J = 7.8 Hz, 1H), 7.84 (d, J = 9.2 Hz, 1H).

(8-2) Synthesis of compound CR01008Z

In the present preparation example, a synthesis route of compound CR01008Z was as follows:

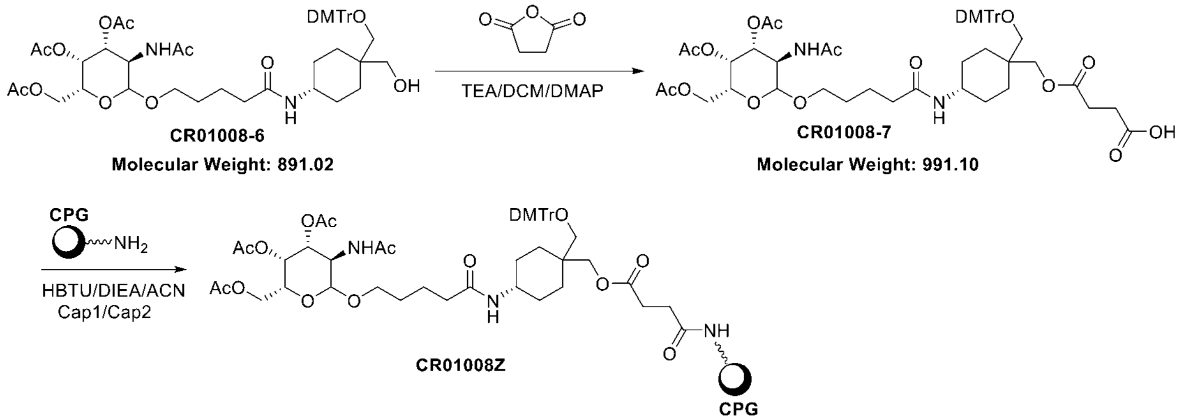


CR01008-6
Molecular Weight: 891.02

CR01008-7
Molecular Weight: 991.10

CR01008Z

(8-2-1) Synthesis of compound CR01008-7

Compound CR01008-6 (500 mg) was dissolved in 10 ml of dichloromethane, followed by addition of compound 8 (succinic anhydride, 112 mg), 4-dimethylaminopyridine (6.8 mg, abbreviated as DMAP, CAS No. 1122-58-3) and triethylamine (226.2 mg). Nitrogen replacement was performed three times. Mixture was stirred and reacted at 25 °C for 16 hours, and subjected to flash purification, to provide compound CR01008-7 (300 mg, in a yield of 53.6%). MS-ESI (m/z) = 1013 $[M + Na]^+$.

8-2-2) Synthesis of compound CR01008Z

To a 20 ml sample vial, compound CR01008-7 (50 mg, amino CPG (1.25 g, 80 μmol/g, 0.1 mmol), HBTU (27 mg), and DIEA (12 mg) were added, and mixture reacted on a shaker for 16 hours. After reaction was ended, reaction solution was filtered to render a filter cake. The filter cake was first washed with 10 ml of acetonitrile once (1×10 ml), and then vacuum-dried. To the 20 ml sample vial, the dried filter cake, DMAP (3 mg), Cap1 (10 ml) and Cap2 (1 ml) were added, and mixture reacted on the shaker for 6 hours. After reaction was ended, reaction solution was filtered to render a filter cake. The filter cake was first washed with 10 ml of acetonitrile once (1×10 ml), and then vacuum-dried, to provide compound CR01008Z (1.03 g, with a load amount of 20-30 μmol/g).

Herein, Cap1 and Cap2 were capping reagents, Cap1 was a solution of 20 volume% N-methylimidazole in pyridine/acetonitrile mixture, with a volume ratio of pyridine to acetonitrile being 3:5; and Cap2 was a solution of 20 volume% acetic anhydride in acetonitrile.

**Example 1 Synthesis of siRNA**

Synthesis of siRNA was not different from a usual phosphoramidite solid-phase synthesis method. In the synthesis of sense and antisense strands comprising a sterically bulky group structure such as TBDMS, TOM, and MOE, when it is required to introduce a nucleotide modified with a sterically bulky group, the above phosphoramidite monomer substituted the nucleotide monomer at corresponding site of the original parent sequence was substituted by the abovementioned phosphoramidite monomer.

siRNA sequence (siRNA single strand) to be synthesized was introduced to ABI394 synthesizer, and synthesis was performed according to a set synthesis program with Universal CPG/PS or compound CR01008Z as carrier. In a synthesis process, compound CR01008 was regarded as a nucleoside monomer.

Introduction of each base needed to undergo four steps of reactions of removing DMTr, coupling, oxidation and capping. Nucleoside phosphoramidite monomers modified with 2'-OMe, 2'-F, 2'-TOM, 2'-TBDMS, and 2'-MOE were all purchased from Shanghai Hongene Biotech Corporation. Compound NM062 was synthesized by the method of Preparation Example 1, and compound NM063 was synthesized by the method of Preparation Example 2. 5-Ethylthio-1*H*-tetrazole (ETT) was used as activator (0.6 M acetonitrile solution), 0.2 M xanthane hydride dissolved in a solution of acetonitrile and pyridine in a volume ratio of 1:1 (Suzhou Kroma) was used as thionating reagent, and 0.05 M iodine dissolved in a solution of pyridine and water in a volume ratio of 9:1 (Suzhou Kroma) was used as oxidant.

After synthesis was ended, the carrier was unloaded and blow-dried, deprotected with strong ammonia water (50 °C, 16 h), and cooled in -20 °C refrigerator for 10 min after reaction was ended. After centrifugation, supernatant was transferred into another centrifugation tube, concentrated to driness, and then purified by an RP-HPLC method, wherein mobile phase A was 0.1 M TEAA, and mobile phase B was acetonitrile. HPLC, MS and UV detections were performed for purification-collected fractions, and qualified fractions were collected and then lyophilized to render siRNA single strands.

The obtained siRNA single strands were annealed, to finally render siRNA double strands.

Herein, structural formula of an siRNA conjugate taking (CR01008)(CR01008)(CR01008Z) (also referred to as (CR01008×3)) as delivery vehicle is as follows:

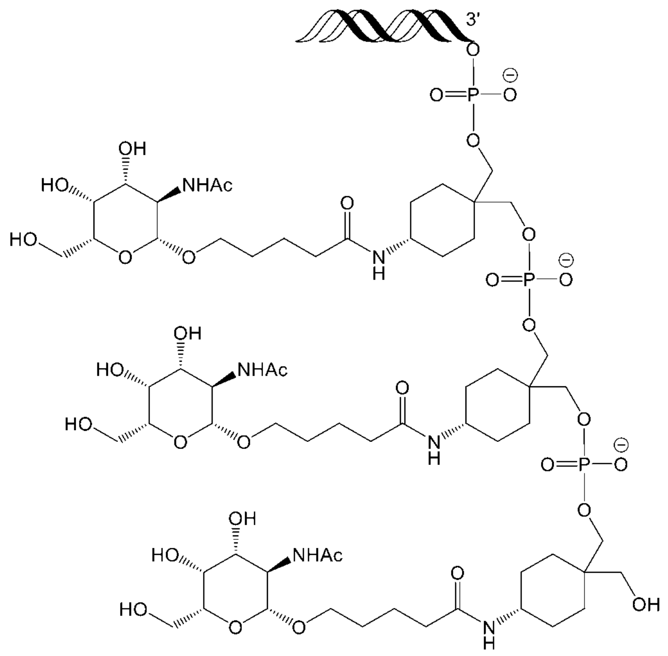

,

wherein

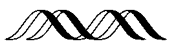

represents siRNA. (CR01008×3) were conjugated to a 3'-end of the sense strand of siRNA by phosphodiester bonds.

Structural formula of the siRNA conjugate taking L96 as delivery vehicle is as follows:

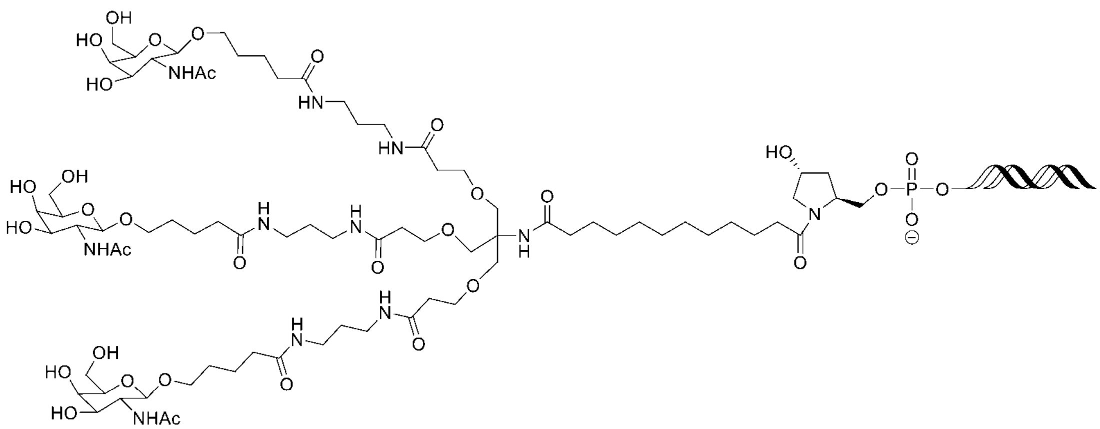

,

wherein

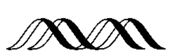

represents siRNA. L96 was conjugated to the 3'-end of the sense strand of siRNA by a phosphodiester bond.

Sequences and modification information of siRNA compounds involved in the various examples of the present disclosure are listed in Table 4.

Table 4

| Compound Serial No. | Sense Strand Sequence and Modification (5'-3') | SEQ ID NOs | Antisense Strand Sequence and Modification (5'-3') | SEQ ID NOs |
|---|---|---|---|---|
| RX502001 | CmsAmsGmAmCmAmGfAdCfAmAmGmAmCmCmAmUmCmUm | 1 | AmsGfsAmUmGmGfUmCmUmUmGmUmCmUfGmUfCmUmGmsGmsAm | 2 |
| RX502023 | C(TBDMS)sAmsGmAmCmAmGfAdCfAmAmGmAmCmCmAmUmCmUm | 3 | AmsGfsAmUmGmGfUmCmUmUmGmUmCmUfGmUfCmUmGmsGmsAm | 4 |
| RX502024 | CmsA(TBDMS)sGmAmCmAmGfAdCfAmAmGmAmCmCmAmUmCmUm | 5 | AmsGfsAmUmGmGfUmCmUmUmGmUmCmUfGmUfCmUmGmsGmsAm | 6 |
| RX502025 | CmsAmsG(TBDMS)AmCmAmGfAdCfAmAmGmAmCmCmAmUmCmUm | 7 | AmsGfsAmUmGmGfUmCmUmUmGmUmCmUfGmUfCmUmGmsGmsAm | 8 |
| RX502026 | CmsAmsGmA(TBDMS)CmAmGfAdCfAmAmGmAmCmCmAmUmCmUm | 9 | AmsGfsAmUmGmGfUmCmUmUmGmUmCmUfGmUfCmUmGmsGmsAm | 10 |
| RX502027 | CmsAmsGmAmC(TBDMS)AmGfAdCfAmAmGmAmCmCmAmUmCmUm | 11 | AmsGfsAmUmGmGfUmCmUmUmGmUmCmUfGmUfCmUmGmsGmsAm | 12 |
| RX502028 | CmsAmsGmAmCmA(TBDMS)GfAdCfAmAmGmAmCmCmAmUmCmUm | 13 | AmsGfsAmUmGmGfUmCmUmUmGmUmCmUfGmUfCmUmGmsGmsAm | 14 |
| RX502029 | CmsAmsGmAmCmAmG(TBDMS)AdCfAmAmGmAmCmCmAmUmCmUm | 15 | AmsGfsAmUmGmGfUmCmUmUmGmUmCmUfGmUfCmUmGmsGmsAm | 16 |

(continued)

| Compound Serial No. | Sense Strand Sequence and Modification (5'-3') | SEQ ID NOs | Antisense Strand Sequence and Modification (5'-3') | SEQ ID NOs |
|---|---|---|---|---|
| RX502030 | CmsAmsGmAmCmAmGfA(TBDMS)CfAmAmGmAmCmCmAmUmCmUm | 17 | AmsGfsAmUmGmGfUmCmUmUmGmUmCmUfGmUfCmUmGmsGmsAm | 18 |
| RX502031 | CmsAmsGmAmCmAmGfAdC(TBDMS)AmAmGmAmCmCmAmUmCmUm | 19 | AmsGfsAmUmGmGfUmCmUmUmGmUmCmUfGmUfCmUmGmsGmsAm | 20 |
| RX502032 | CmsAmsGmAmCmAmGfAdCfA(TBDMS)AmGmAmCmCmAmUmCmUm | 21 | AmsGfsAmUmGmGfUmCmUmUmGmUmCmUfGmUfCmUmGmsGmsAm | 22 |
| RX502033 | CmsAmsGmAmCmAmGfAdCfAmA(TBDMS)GmAmCmCmAmUmCmUm | 23 | AmsGfsAmUmGmGfUmCmUmUmGmUmCmUfGmUfCmUmGmsGmsAm | 24 |
| RX502034 | CmsAmsGmAmCmAmGfAdCfAmAmG(TBDMS)AmCmCmAmUmCmUm | 25 | AmsGfsAmUmGmGfUmCmUmUmGmUmCmUfGmUfCmUmGmsGmsAm | 26 |
| RX502035 | CmsAmsGmAmCmAmGfAdCfAmAmGmA(TBDMS)CmCmAmUmCmUm | 27 | AmsGfsAmUmGmGfUmCmUmUmGmUmCmUfGmUfCmUmGmsGmsAm | 28 |
| RX502036 | CmsAmsGmAmCmAmGfAdCfAmAmGmAmC(TBDMS)CmAmUmCmUm | 29 | AmsGfsAmUmGmGfUmCmUmUmGmUmCmUfGmUfCmUmGmsGmsAm | 30 |
| RX502037 | CmsAmsGmAmCmAmGfAdCfAmAmGmAmCmC(TBDMS)AmUmCmUm | 31 | AmsGfsAmUmGmGfUmCmUmUmGmUmCmUfGmUfCmUmGmsGmsAm | 32 |

(continued)

| Compound Serial No. | Sense Strand Sequence and Modification (5'-3') | SEQ ID NOs | Antisense Strand Sequence and Modification (5'-3') | SEQ ID NOs |
|---|---|---|---|---|
| RX502038 | CmsAmsGmAmCmAmGfAdCf AmAmGmAmCmCmA(TBDM | 33 | AmsGfsAmUmGmGfUmCmU mUmGmUmCmUfGmUfCmU | 34 |
| | S)UmCmUm | | mGmsGmsAm | |
| RX502039 | CmsAmsGmAmCmAmGfAdCf AmAmGmAmCmCmAmU(TB DMS)CmUm | 35 | AmsGfsAmUmGmGfUmCmU mUmGmUmCmUfGmUfCmU mGmsGmsAm | 36 |
| RX502040 | CmsAmsGmAmCmAmGfAdCf AmAmGmAmCmCmAmUmC( TBDMS)Um | 37 | AmsGfsAmUmGmGfUmCmU mUmGmUmCmUfGmUfCmU mGmsGmsAm | 38 |
| RX502041 | CmsAmsGmAmCmAmGfAdCf AmAmGmAmCmCmAmUmC mU(TBDMS) | 39 | AmsGfsAmUmGmGfUmCmU mUmGmUmCmUfGmUfCmU mGmsGmsAm | 40 |
| RX502002 | CmsAmsGmAmCmAmGfAdCf AmAmGmAmCmCmAmUmC mUm | 41 | A(TBDMS)sGfsAmUmGmGfU mCmUmUmGmUmCmUfGmU fCmUmGmsGmsAm | 42 |
| RX502003 | CmsAmsGmAmCmAmGfAdCf AmAmGmAmCmCmAmUmC mUm | 43 | AmsG(TBDMS)sAmUmGmGf UmCmUmUmGmUmCmUfGm UfCmUmGmsGmsAm | 44 |
| RX502004 | CmsAmsGmAmCmAmGfAdCf AmAmGmAmCmCmAmUmC mUm | 45 | AmsGfsA(TBDMS)UmGmGfU mCmUmUmGmUmCmUfGmU fCmUmGmsGmsAm | 46 |
| RX502005 | CmsAmsGmAmCmAmGfAdCf AmAmGmAmCmCmAmUmC mUm | 47 | AmsGfsAmU(TBDMS)GmGfU mCmUmUmGmUmCmUfGmU fCmUmGmsGmsAm | 48 |

(continued)

| Compound Serial No. | Sense Strand Sequence and Modification (5'-3') | SEQ ID NOs | Antisense Strand Sequence and Modification (5'-3') | SEQ ID NOs |
|---|---|---|---|---|
| RX502006 | CmsAmsGmAmCmAmGfAdCfAmAmGmAmCmCmAmUmCmUm | 49 | AmsGfsAmUmG(TBDMS)GfUmCmUmUmGmUmCmUfGmUfCmUmGmsGmsAm | 50 |
| RX502007 | CmsAmsGmAmCmAmGfAdCfAmAmGmAmCmCmAmUmCmUm | 51 | AmsGfsAmUmGmG(TBDMS)UmCmUmUmGmUmCmUfGmUfCmUmGmsGmsAm | 52 |
| RX502008 | CmsAmsGmAmCmAmGfAdCfAmAmGmAmCmCmAmUmCmUm | 53 | AmsGfsAmUmGmGfU(TBDMS)CmUmUmGmUmCmUfGmUfCmUmGmsGmsAm | 54 |
| RX502009 | CmsAmsGmAmCmAmGfAdCfAmAmGmAmCmCmAmUmCmUm | 55 | AmsGfsAmUmGmGfUmC(TBDMS)UmUmGmUmCmUfGmUfCmUmGmsGmsAm | 56 |
| RX502010 | CmsAmsGmAmCmAmGfAdCfAmAmGmAmCmCmAmUmCmUm | 57 | AmsGfsAmUmGmGfUmCmU(TBDMS)UmGmUmCmUfGmUfCmUmGmsGmsAm | 58 |
| RX502011 | CmsAmsGmAmCmAmGfAdCfAmAmGmAmCmCmAmUmCmUm | 59 | AmsGfsAmUmGmGfUmCmUmU(TBDMS)GmUmCmUfGmUfCmUmGmsGmsAm | 60 |
| RX502012 | CmsAmsGmAmCmAmGfAdCfAmAmGmAmCmCmAmUmCmUm | 61 | AmsGfsAmUmGmGfUmCmUmUmG(TBDMS)UmCmUfGmUfCmUmGmsGmsAm | 62 |
| RX502013 | CmsAmsGmAmCmAmGfAdCfAmAmGmAmCmCmAmUmCmUm | 63 | AmsGfsAmUmGmGfUmCmUmUmGmU(TBDMS)CmUfGmUfCmUmGmsGmsAm | 64 |

(continued)

| Compound Serial No. | Sense Strand Sequence and Modification (5'-3') | SEQ ID NOs | Antisense Strand Sequence and Modification (5'-3') | SEQ ID NOs |
|---|---|---|---|---|
| RX502014 | CmsAmsGmAmCmAmGfAdCfAmAmGmAmCmCmAmUmCmUm | 65 | AmsGfsAmUmGmGfUmCmUmUmGmUmC(TBDMS)UfGmUfCmUmGmsGmsAm | 66 |
| RX502015 | CmsAmsGmAmCmAmGfAdCfAmAmGmAmCmCmAmUmCmUm | 67 | AmsGfsAmUmGmGfUmCmUmUmGmUmCmU(TBDMS)GmUfCmUmGmsGmsAm | 68 |
| RX502016 | CmsAmsGmAmCmAmGfAdCfAmAmGmAmCmCmAmUmCmUm | 69 | AmsGfsAmUmGmGfUmCmUmUmGmUmCmUfG(TBDMS)UfCmUmGmsGmsAm | 70 |
| RX502017 | CmsAmsGmAmCmAmGfAdCfAmAmGmAmCmCmAmUmCmUm | 71 | AmsGfsAmUmGmGfUmCmUmUmGmUmCmUfGmU(TBDMS)CmUmGmsGmsAm | 72 |
| RX502018 | CmsAmsGmAmCmAmGfAdCfAmAmGmAmCmCmAmUmCmUm | 73 | AmsGfsAmUmGmGfUmCmUmUmGmUmCmUfGmUfC(TBDMS)UmGmsGmsAm | 74 |
| RX502019 | CmsAmsGmAmCmAmGfAdCfAmAmGmAmCmCmAmUmCmUm | 75 | AmsGfsAmUmGmGfUmCmUmUmGmUmCmUfGmUfCmU(TBDMS)GmsGmsAm | 76 |
| RX502020 | CmsAmsGmAmCmAmGfAdCfAmAmGmAmCmCmAmUmCmUm | 77 | AmsGfsAmUmGmGfUmCmUmUmGmUmCmUfGmUfCmUmG(TBDMS)sGmsAm | 78 |
| RX502021 | CmsAmsGmAmCmAmGfAdCfAmAmGmAmCmCmAmUmCmUm | 79 | AmsGfsAmUmGmGfUmCmUmUmGmUmCmUfGmUfCmUmGmsG(TBDMS)sAm | 80 |

(continued)

| Compound Serial No. | Sense Strand Sequence and Modification (5'-3') | SEQ ID NOs | Antisense Strand Sequence and Modification (5'-3') | SEQ ID NOs |
|---|---|---|---|---|
| RX502022 | CmsAmsGmAmCmAmGfAdCfAmAmGmAmCmCmAmUmCmUm | 81 | AmsGfsAmUmGmGfUmCmUmUmGmUmCmUfGmUfCmUmGmsGmsA(TBDMS) | 82 |
| RX502165 | CmsAmsGmAmC(MOE)AmGfAdCfAmAmGmAmCmCmAmUmCmUm | 83 | AmsGfsAmUmGmGfUmCmUmUmGmUmCmUfGmUfCmUmGmsGmsAm | 84 |
| RX502166 | CmsAmsGmAmCmAmG(MOE)AdCfAmAmGmAmCmCmAmUmCmUm | 85 | AmsGfsAmUmGmGfUmCmUmUmGmUmCmUfGmUfCmUmGmsGmsAm | 86 |
| RX502167 | CmsAmsGmAmCmAmGfA(MOE)CfAmAmGmAmCmCmAmUmCmUm | 87 | AmsGfsAmUmGmGfUmCmUmUmGmUmCmUfGmUfCmUmGmsGmsAm | 88 |
| RX502168 | CmsAmsGmAmCmAmGfAdC(MOE)AmAmGmAmCmCmAmUmCmUm | 89 | AmsGfsAmUmGmGfUmCmUmUmGmUmCmUfGmUfCmUmGmsGmsAm | 90 |
| RX502169 | CmsAmsGmAmCmAmGfAdCfA(MOE)AmGmAmCmCmAmUmCmUm | 91 | AmsGfsAmUmGmGfUmCmUmUmGmUmCmUfGmUfCmUmGmsGmsAm | 92 |
| RX502115 | CmsAmsGmAmCmAmGfAdCfAmAmG(MOE)AmCmCmAmUmCmUm | 93 | AmsGfsAmUmGmGfUmCmUmUmGmUmCmUfGmUfCmUmGmsGmsAm | 94 |
| RX502116 | CmsAmsGmAmCmAmGfAdCfAmAmGmAmCmCmAmUmC(MOE)Um | 95 | AmsGfsAmUmGmGfUmCmUmUmGmUmCmUfGmUfCmUmGmsGmsAm | 96 |

(continued)

| Compound Serial No. | Sense Strand Sequence and Modification (5'-3') | SEQ ID NOs | Antisense Strand Sequence and Modification (5'-3') | SEQ ID NOs |
|---|---|---|---|---|
| RX502117 | CmsAmsGmAmCmAmGfAdCfAmAmGmAmCmCmAmUmCmT(MOE) | 97 | AmsGfsAmUmGmGfUmCmUmUmGmUmCmUfGmUfCmUmGmsGmsAm | 98 |
| RX502172 | CmsAmsGmAmCmAmGfAdCfAmAmGmAmCmCmAmUmCmUm | 99 | AmsG(MOE)sAmUmGmGfUmCmUmUmGmUmCmUfGmUfCmUmGmsGmsAm | 100 |
| RX502173 | CmsAmsGmAmCmAmGfAdCfAmAmGmAmCmCmAmUmCmUm | 101 | AmsGfsA(MOE)UmGmGfUmCmUmUmGmUmCmUfGmUfCmUmGmsGmsAm | 102 |
| RX502118 | CmsAmsGmAmCmAmGfAdCfAmAmGmAmCmCmAmUmCmUm | 103 | AmsGfsAmUmGmGfUmC(MOE)UmUmGmUmCmUfGmUfCmUmGmsGmsAm | 104 |
| RX502119 | CmsAmsGmAmCmAmGfAdCfAmAmGmAmCmCmAmUmCmUm | 105 | AmsGfsAmUmGmGfUmCmUmT(MOE)GmUmCmUfGmUfCmUmGmsGmsAm | 106 |
| RX502177 | CmsAmsGmAmCmAmGfAdCfAmAmGmAmCmCmAmUmCmUm | 107 | AmsGfsAmUmGmGfUmCmUmUmGmUmCmT(MOE)GmUfCmUmGmsGmsAm | 108 |
| RX502120 | CmsAmsGmAmCmAmGfAdCfAmAmGmAmCmCmAmUmCmUm | 109 | AmsGfsAmUmGmGfUmCmUmUmGmUmCmUfG(MOE)UfCmUmGmsGmsAm | 110 |
| RX502178 | CmsAmsGmAmCmAmGfAdCfAmAmGmAmCmCmAmUmCmUm | 111 | AmsGfsAmUmGmGfUmCmUmUmGmUmCmUfGmT(MOE)CmUmGmsGmsAm | 112 |

(continued)

| Compound Serial No. | Sense Strand Sequence and Modification (5'-3') | SEQ ID NOs | Antisense Strand Sequence and Modification (5'-3') | SEQ ID NOs |
|---|---|---|---|---|
| RX502123 | CmsAmsGmAmCmA(MOE)GfAdCfAmAmG(MOE)AmCmCmAmUmCmUm | 113 | AmsGfsAmUmGmGfUmCmUmUmGmUmCmUfGmUfCmUmGmsGmsAm | 114 |
| RX502124 | CmsAmsGmAmCmAmGfAdCfAmAmGmAmCmCmAmUmC(MOE)T(MOE) | 115 | AmsGfsAmUmGmGfUmCmUmUmGmUmCmUfGmUfCmUmGmsGmsAm | 116 |
| RX502125 | CmsAmsGmAmCmA(MOE)GfAdCfAmAmGmAmCmCmAmUmC(MOE)Um | 117 | AmsGfsAmUmGmGfUmCmUmUmGmUmCmUfGmUfCmUmGmsGmsAm | 118 |
| RX502126 | CmsAmsGmAmCmAmGfAdCfAmAmG(MOE)AmCmCmAmUmC(MOE)Um | 119 | AmsGfsAmUmGmGfUmCmUmUmGmUmCmUfGmUfCmUmGmsGmsAm | 120 |
| RX502127 | CmsAmsGmAmCmA(MOE)GfAdCfAmAmG(MOE)AmCmCmAmUmC(MOE)Um | 121 | AmsGfsAmUmGmGfUmCmUmUmGmUmCmUfGmUfCmUmGmsGmsAm | 122 |
| RX502128 | CmsAmsGmAmCmA(MOE)GfAdCfAmAmG(MOE)AmCmCmAmUmC(MOE)T(MOE) | 123 | AmsGfsAmUmGmGfUmCmUmUmGmUmCmUfGmUfCmUmGmsGmsAm | 124 |
| RX502130 | CmsAmsGmAmCmAmGfAdCfAmAmGmAmCmCmAmUmCmUm | 125 | AmsGfsAmUmGmGfUmC(MOE)UmUmGmUmCmUfG(MOE)UfCmUmGmsGmsAm | 126 |
| RX502131 | CmsAmsGmAmCmAmGfAdCfAmAmGmAmCmCmAmUmCmUm | 127 | AmsGfsAmUmGmGfUmC(MOE)UmUmGmUmCmUfGmUfCmUmGmsG(MOE)sAm | 128 |

(continued)

| Compound Serial No. | Sense Strand Sequence and Modification (5'-3') | SEQ ID NOs | Antisense Strand Sequence and Modification (5'-3') | SEQ ID NOs |
|---|---|---|---|---|
| RX502132 | CmsAmsGmAmCmAmGfAdCfAmAmGmAmCmCmAmUmCmUm | 129 | AmsGfsAmUmGmGfUmC(MOE)UmUmGmUmCmUfGmUfCmUmGmsGmsA(MOE) | 130 |
| RX502134 | CmsAmsGmAmCmAmGfAdCfAmAmGmAmCmCmAmUmCmUm | 131 | AmsGfsAmUmGmGfUmCmUmT(MOE)GmUmCmUfGmUfCmUmGmsG(MOE)sAm | 132 |
| RX502135 | CmsAmsGmAmCmAmGfAdCfAmAmGmAmCmCmAmUmCmUm | 133 | AmsGfsAmUmGmGfUmCmUmT(MOE)GmUmCmUfGmUfCmUmGmsGmsA(MOE) | 134 |
| RX502136 | CmsAmsGmAmCmAmGfAdCfAmAmGmAmCmCmAmUmCmUm | 135 | AmsGfsAmUmGmGfUmCmUmUmGmUmCmUfG(MOE)UfCmUmGmsG(MOE)sAm | 136 |
| RX502138 | CmsAmsGmAmCmAmGfAdCfAmAmGmAmCmCmAmUmCmUm | 137 | AmsGfsAmUmGmGfUmC(MOE)UmT(MOE)GmUmCmUfG(MOE)UfCmUmGmsGmsAm | 138 |
| RX502139 | CmsAmsGmAmCmAmGfAdCfAmAmGmAmCmCmAmUmCmUm | 139 | AmsGfsAmUmGmGfUmCmUmT(MOE)GmUmCmUfG(MOE)UfCmUmGmsG(MOE)sAm | 140 |
| RX502140 | CmsAmsGmAmCmAmGfAdCfAmAmGmAmCmCmAmUmCmUm | 141 | AmsGfsAmUmGmGfUmC(MOE)UmT(MOE)GmUmCmUfG(MOE)UfCmUmGmsG(MOE)sA(MOE) | 142 |
| RX502146 | CmsAmsGmAmCmAmGfAdCfAmAmG(MOE)AmCmCmAmUmCmUm | 143 | AmsGfsAmUmGmGfUmC(MOE)UmUmGmUmCmUfGmUfCmUmGmsGmsAm | 144 |

(continued)

| Compound Serial No. | Sense Strand Sequence and Modification (5'-3') | SEQ ID NOs | Antisense Strand Sequence and Modification (5'-3') | SEQ ID NOs |
|---|---|---|---|---|
| RX502147 | CmsAmsGmAmCmAmGfAdCfAmAmG(MOE)AmCmCmAmUmCmUm | 145 | AmsGfsAmUmGmGfUmCmUmUmGmUmCmUfG(MOE)UfCmUmGmsGmsAm | 146 |
| RX502150 | CmsAmsGmAmCmAmGfAdCfAmAmGmAmCmCmAmUmC(MOE)Um | 147 | AmsGfsAmUmGmGfUmC(MOE)UmUmGmUmCmUfGmUfCmUmGmsGmsAm | 148 |
| RX502151 | CmsAmsGmAmCmAmGfAdCfAmAmGmAmCmCmAmUmC(MOE)Um | 149 | AmsGfsAmUmGmGfUmCmUmUmGmUmCmUfG(MOE)UfCmUmGmsGmsAm | 150 |
| RX502152 | CmsAmsGmAmCmAmGfAdCfAmAmGmAmCmCmAmUmC(MOE)Um | 151 | AmsGfsAmUmGmGfUmCmUmUmGmUmCmUfGmUfCmUmGmsGmsA(MOE) | 152 |
| RX502153 | CmsAmsGmAmCmAmGfAdCfAmAmGmAmCmCmAmUmC(MOE)Um | 153 | AmsGfsAmUmGmGfUmCmUmUmGmUmCmUfGmUfCmUmGmsG(MOE)sA(MOE) | 154 |
| RX502154 | CmsAmsGmAmCmA(MOE)GfAdCfAmAmG(MOE)AmCmCmAmUmCmUm | 155 | AmsGfsAmUmGmGfUmC(MOE)UmUmGmUmCmUfG(MOE)UfCmUmGmsGmsAm | 156 |
| RX502155 | CmsAmsGmAmCmA(MOE)GfAdCfAmAmG(MOE)AmCmCmAmUmCmUm | 157 | AmsGfsAmUmGmGfUmC(MOE)UmUmGmUmCmUfGmUfCmUmGmsGmsA(MOE) | 158 |
| RX502156 | CmsAmsGmAmCmA(MOE)GfAdCfAmAmGmAmCmCmAmUmC(MOE)Um | 159 | AmsGfsAmUmGmGfUmC(MOE)UmUmGmUmCmUfG(MOE)UfCmUmGmsGmsAm | 160 |

(continued)

| Compound Serial No. | Sense Strand Sequence and Modification (5'-3') | SEQ ID NOs | Antisense Strand Sequence and Modification (5'-3') | SEQ ID NOs |
|---|---|---|---|---|
| RX502157 | CmsAmsGmAmCmA(MOE)GfAdCfAmAmGmAmCmCmAmUmC(MOE)Um | 161 | AmsGfsAmUmGmGfUmC(MOE)UmUmGmUmCmUfGmUfCmUmGmsGmsA(MOE) | 162 |
| RX502158 | CmsAmsGmAmCmAmGfAdCfAmAmG(MOE)AmCmCmAmUmC(MOE)Um | 163 | AmsGfsAmUmGmGfUmC(MOE)UmUmGmUmCmUfG(MOE)UfCmUmGmsGmsAm | 164 |
| RX502159 | CmsAmsGmAmCmAmGfAdCfAmAmG(MOE)AmCmCmAmUmC(MOE)Um | 165 | AmsGfsAmUmGmGfUmC(MOE)UmUmGmUmCmUfGmUfCmUmGmsGmsA(MOE) | 166 |
| RX502160 | CmsAmsGmAmCmA(MOE)GfAdCfAmAmG(MOE)AmCmCmAmUmC(MOE)Um | 167 | AmsGfsAmUmGmGfUmC(MOE)UmUmGmUmCmUfG(MOE)UfCmUmGmsGmsAm | 168 |
| RX502162 | CmsAmsGmAmCmA(MOE)GfAdCfAmAmG(MOE)AmCmCmAmUmC(MOE)Um | 169 | AmsGfsAmUmGmGfUmC(MOE)UmT(MOE)GmUmCmUfG(MOE)UfCmUmGmsGmsAm | 170 |
| RX502163 | CmsAmsGmAmCmA(MOE)GfAdCfAmAmG(MOE)AmCmCmAmUmC(MOE)Um | 171 | AmsGfsAmUmGmGfUmC(MOE)UmT(MOE)GmUmCmUfG(MOE)UfCmUmGmsG(MOE)sAm | 172 |
| RX502164 | CmsAmsGmAmCmA(MOE)GfAdCfAmAmG(MOE)AmCmCmAmUmC(MOE)Um | 173 | AmsGfsAmUmGmGfUmC(MOE)UmT(MOE)GmUmCmUfG(MOE)UfCmUmGmsG(MOE)sA(MOE) | 174 |

(continued)

| Compound Serial No. | Sense Strand Sequence and Modification (5'-3') | SEQ ID NOs | Antisense Strand Sequence and Modification (5'-3') | SEQ ID NOs |
|---|---|---|---|---|
| RZ000002 | UmsUmsCmUmCmCmGfAdAfCmGmUmGmUmCmAmCmGmUm_L96 | 175 | AmsCfsGmUmGmAfCmAmCmGmUmUmCmGfGmAfGmAmAmsCmsUm | 176 |
| RZM02001 | AmsGmsAmAmGmAmAfGfAfUfAmUmUmAmUmCmUmCmUm_L96 | 177 | AmsGfsAmGmAmUfAmAmUmAmUmCmUmUfCmUfUmCmUmsGmsGm | 178 |
| RZM02004 | AmsGmsAmAmGmAmAfGfAfUfAmUmUmAmUmCmUmC(tbdms)Um_L96 | 179 | AmsGfsAmGmAmUfAmAmUmAmUmCmUmUfCmUfUmCmUmsGmsGm | 180 |
| RZM02005 | AmsGmsAmAmGmAmAfGfAfUfAmUmUmAmUmCmUmCmUm_L96 | 181 | AmsGfsAmGmAmUfAmAmUmAmUmCmUmUfC(tbdms)UfUmCmUmsGmsGm | 182 |
| RXM02006 | AmsGmsAmAmGmAmAfGfAfUfAmUmUmAmUmCmUmCmUm_L96 | 183 | AmsGfsAmGmAmUfAmAmUmAmUmCmUmUfCmUfUmCmUmsGmsG(tbdms) | 184 |
| RZ002001 | CmsAmsGmAmCmAmGfAdCfAmAmGmAmCmCmAmUmCmUm_L96 | 185 | AmsGfsAmUmGmGfUmCmUmUmGmUmCmUfGmUfCmUmGmsGmsAm | 186 |
| RZ502013 | CmsAmsGmAmCmAmGfAdCfAmAmGmAmCmCmAmUmCmU(tom)_L96 | 187 | AmsGfsAmUmGmGfUmCmUmUmGmUmCmUfGmUfCmUmGmsGmsAm | 188 |
| RZ502016 | CmsAmsGmAmCmAmGfAdCfAmAmGmAmCmCmAmUmCmUm_L96 | 189 | AmsGfsAmUmGmGfUmCmUmU(tom)GmUmCmUfGmUfCmUmGmsGmsAm | 190 |

(continued)

| Compound Serial No. | Sense Strand Sequence and Modification (5'-3') | SEQ ID NOs | Antisense Strand Sequence and Modification (5'-3') | SEQ ID NOs |
|---|---|---|---|---|
| RZ502031 | CmsAmsGmAmCmAmGfAfCfAmAmGmAmCmCmAmUmCmUm_L96 | 191 | AmsGfsAmUmGmGfUmCmUmUmGmUmCmUfGmUfCmUmGmsGmsAm | 192 |
| RZ502033 | CmsAmsGmAmCmAmGfAfCfAmAmG(MOE)AmCmCmAmUmC(MOE)Um_L96 | 193 | AmsGfsAmUmGmGfUmCmUmUmGmUmCmUfGmUfCmUmGmsGmsAm | 194 |
| RZ502034 | CmsAmsGmAmCmAmGfAfCfAmAmGmAmCmCmAmUmCmUm_L96 | 195 | AmsGfsAmUmGmGfUmC(MOE)UmUmGmUmCmUfG(MOE)UfCmUmGmsGmsAm | 196 |
| RZ502035 | CmsAmsGmAmCmAmGfAfCfAmAmG(MOE)AmCmCmAmUmCmUm_L96 | 197 | AmsGfsAmUmGmGfUmCmUmUmGmUmCmUfG(MOE)UfCmUmGmsGmsAm | 198 |
| RZ502036 | CmsAmsGmAmCmAmGfAfCfAmAmG(MOE)AmCmCmAmUmC(MOE)Um_L96 | 199 | AmsGfsAmUmGmGfUmCmUmUmGmUmCmUfG(MOE)UfCmUmGmsGmsAm | 200 |
| RZ502039 | CmsAmsGmAmCmA(MOE)GfAfCfAmAmGmAmCmCmAmUmC(MOE)Um_L96 | 201 | AmsGfsAmUmGmGfUmC(MOE)UmUmGmUmCmUfG(MOE)UfCmUmGmsGmsAm | 202 |
| RZ502049 | CmsAmsGmAmC(MOE)AmGfAfCfAmAmGmAmCmCmAmUmCmUm_L96 | 203 | AmsGfsAmUmGmGfUmCmUmUmGmUmCmUfG(MOE)UfCmUmGmsG(MOE)sAm | 204 |
| RZ502087 | CmsAmsGmAmCmAmGfAfCfAmAmGmAmCmCmAmUmCmUm_L96 | 205 | AmsGfsAmUmGmGfUmCmUmUmGmUmCmT(MOE)GmUfCmUmGmsGmsAm | 206 |

(continued)

| Compound Serial No. | Sense Strand Sequence and Modification (5'-3') | SEQ ID NOs | Antisense Strand Sequence and Modification (5'-3') | SEQ ID NOs |
|---|---|---|---|---|
| RZ597024 | CmsCmsAmAmGmAmGfCdAfCmCmAmAmGmAmAmCmUmAm_L96 | 207 | UmsAfsGmUmUmCfUmUmGmGmUmGmCmUfCmUfUmGmGmsCmsUm | 208 |
| RZ597065 | C(TBDMS)sCmsAmAmGmAmGfCdAfCmCmAmAmGmAmAmCmUmAm_L96 | 209 | UmsAfsGmUmUmCfUmUmGmGmUmGmCmUfCmUfUmGmGmsCmsUm | 210 |
| RZ597066 | CmsC(TBDMS)sAmAmGmAmGfCdAfCmCmAmAmGmAmAmCmUmAm_L96 | 211 | UmsAfsGmUmUmCfUmUmGmGmUmGmCmUfCmUfUmGmGmsCmsUm | 212 |
| RZ597067 | CmsCmsA(TBDMS)AmGmAmGfCdAfCmCmAmAmGmAmAmCmUmAm_L96 | 213 | UmsAfsGmUmUmCfUmUmGmGmUmGmCmUfCmUfUmGmGmsCmsUm | 214 |
| RZ597068 | CmsCmsAmA(TBDMS)GmAmGfCdAfCmCmAmAmGmAmAmCmUmAm_L96 | 215 | UmsAfsGmUmUmCfUmUmGmGmUmGmCmUfCmUfUmGmGmsCmsUm | 216 |
| RZ597069 | CmsCmsAmAmG(TBDMS)AmGfCdAfCmCmAmAmGmAmAmCmUmAm_L96 | 217 | UmsAfsGmUmUmCfUmUmGmGmUmGmCmUfCmUfUmGmGmsCmsUm | 218 |
| RZ597070 | CmsCmsAmAmGmA(TBDMS)GfCdAfCmCmAmAmGmAmAmCmUmAm_L96 | 219 | UmsAfsGmUmUmCfUmUmGmGmUmGmCmUfCmUfUmGmGmsCmsUm | 220 |
| RZ597071 | CmsCmsAmAmGmAmG(TBDMS)CdAfCmCmAmAmGmAmAmCmUmAm_L96 | 221 | UmsAfsGmUmUmCfUmUmGmGmUmGmCmUfCmUfUmGmGmsCmsUm | 222 |

(continued)

| Compound Serial No. | Sense Strand Sequence and Modification (5'-3') | SEQ ID NOs | Antisense Strand Sequence and Modification (5'-3') | SEQ ID NOs |
|---|---|---|---|---|
| RZ597072 | CmsCmsAmAmGmAmGfC(TBDMS)AfCmCmAmAmGmAmAmCmUmAm_L96 | 223 | UmsAfsGmUmUmCfUmUmGmGmUmGmCmUfCmUfUmGmGmsCmsUm | 224 |
| RZ597073 | CmsCmsAmAmGmAmGfCdA(TBDMS)CmCmAmAmGmAmAmCmUmAm_L96 | 225 | UmsAfsGmUmUmCfUmUmGmGmUmGmCmUfCmUfUmGmGmsCmsUm | 226 |
| RZ597074 | CmsCmsAmAmGmAmGfCdAfC(TBDMS)CmAmAmGmAmAmCmUmAm L96 | 227 | UmsAfsGmUmUmCfUmUmGmGmUmGmCmUfCmUfUmGmGmsCmsUm | 228 |
| RZ597075 | CmsCmsAmAmGmAmGfCdAfCmC(TBDMS)AmAmGmAmAmCmUmAm_L96 | 229 | UmsAfsGmUmUmCfUmUmGmGmUmGmCmUfCmUfUmGmGmsCmsUm | 230 |
| RZ597076 | CmsCmsAmAmGmAmGfCdAfCmCmA(TBDMS)AmGmAmAmCmUmAm_L96 | 231 | UmsAfsGmUmUmCfUmUmGmGmUmGmCmUfCmUfUmGmGmsCmsUm | 232 |
| RZ597077 | CmsCmsAmAmGmAmGfCdAfCmCmAmA(TBDMS)GmAmAmCmUmAm_L96 | 233 | UmsAfsGmUmUmCfUmUmGmGmUmGmCmUfCmUfUmGmGmsCmsUm | 234 |
| RZ597078 | CmsCmsAmAmGmAmGfCdAfCmCmAmAmG(TBDMS)AmAmCmUmAm_L96 | 235 | UmsAfsGmUmUmCfUmUmGmGmUmGmCmUfCmUfUmGmGmsCmsUm | 236 |
| RZ597079 | CmsCmsAmAmGmAmGfCdAfCmCmAmAmGmA(TBDMS)AmCmUmAm_L96 | 237 | UmsAfsGmUmUmCfUmUmGmGmUmGmCmUfCmUfUmGmGmsCmsUm | 238 |

(continued)

| Compound Serial No. | Sense Strand Sequence and Modification (5'-3') | SEQ ID NOs | Antisense Strand Sequence and Modification (5'-3') | SEQ ID NOs |
|---|---|---|---|---|
| RZ597080 | CmsCmsAmAmGmAmGfCdAfCmCmAmAmGmAmA(TBDMS)CmUmAm_L96 | 239 | UmsAfsGmUmUmCfUmUmGmGmUmGmCmUfCmUfUmGmGmsCmsUm | 240 |
| RZ597081 | CmsCmsAmAmGmAmGfCdAfCmCmAmAmGmAmAmC(TBDMS)UmAm_L96 | 241 | UmsAfsGmUmUmCfUmUmGmGmUmGmCmUfCmUfUmGmGmsCmsUm | 242 |
| RZ597082 | CmsCmsAmAmGmAmGfCdAfCmCmAmAmGmAmAmCmU(TBDMS)Am_L96 | 243 | UmsAfsGmUmUmCfUmUmGmGmUmGmCmUfCmUfUmGmGmsCmsUm | 244 |
| RZ597083 | CmsCmsAmAmGmAmGfCdAfCmCmAmAmGmAmAmCmUmA(TBDMS)_L96 | 245 | UmsAfsGmUmUmCfUmUmGmGmUmGmCmUfCmUfUmGmGmsCmsUm | 246 |
| RZ597084 | CmsCmsAmAmGmAmGfCdAfCmCmAmAmGmAmAmCmUmAm_L96 | 247 | U(TBDMS)sAfsGmUmUmCfUmUmGmGmUmGmCmUfCmUfUmGmGmsCmsUm | 248 |
| RZ597085 | CmsCmsAmAmGmAmGfCdAfCmCmAmAmGmAmAmCmUmAm_L96 | 249 | UmsA(TBDMS)sGmUmUmCfUmUmGmGmUmGmCmUfCmUfUmGmGmsCmsUm | 250 |
| RZ597025 | CmsCmsAmAmGmAmGfCdAfCmCmAmAmGmAmAmCmUmAm_L96 | 251 | UmsAfsG(TBDMS)UmUmCfUmUmGmGmUmGmCmUfCmUfUmGmGmsCmsUm | 252 |
| RZ597026 | CmsCmsAmAmGmAmGfCfAfCmCmAmAmGmAmAmCmUmAm_L96 | 253 | UmsAfsGmU(tbdms)UmCfUmUmGmGmUmGmCmUfCmUfUmGmGmsUmsUm | 254 |

(continued)

| Compound Serial No. | Sense Strand Sequence and Modification (5'-3') | SEQ ID NOs | Antisense Strand Sequence and Modification (5'-3') | SEQ ID NOs |
|---|---|---|---|---|
| RZ597027 | CmsCmsAmAmGmAmGfCdAfCmCmAmAmGmAmAmCmUmAm_L96 | 255 | UmsAfsGmUmU(TBDMS)CfUmUmGmGmUmGmCmUfCmUfUmGmGmsCmsUm | 256 |
| RZ597028 | CmsCmsAmAmGmAmGfCdAfCmCmAmAmGmAmAmCmUmAm_L96 | 257 | UmsAfsGmUmUmC(TBDMS)UmUmGmGmUmGmCmUfCmUfUmGmGmsCmsUm | 258 |
| RZ597029 | CmsCmsAmAmGmAmGfCfAfCmCmAmAmGmAmAmCmUmAm_L96 | 259 | UmsAfsGmUmUmCfU(tbdms)UmGmGmUmGmCmUfCmUfUmGmGmsUmsUm | 260 |
| RZ597030 | CmsCmsAmAmGmAmGfCfAfCmCmAmAmGmAmAmCmUmAm_L96 | 261 | UmsAfsGmUmUmCfUmU(tbdms)GmGmUmGmCmUfCmUfUmGmGmsUmsUm | 262 |
| RZ597031 | CmsCmsAmAmGmAmGfCdAfCmCmAmAmGmAmAmCmUmAm_L96 | 263 | UmsAfsGmUmUmCfUmUmG(TBDMS)GmUmGmCmUfCmUfUmGmGmsCmsUm | 264 |
| RZ597086 | CmsCmsAmAmGmAmGfCdAfCmCmAmAmGmAmAmCmUmAm_L96 | 265 | UmsAfsGmUmUmCfUmUmGmG(TBDMS)UmGmCmUfCmUfUmGmGmsCmsUm | 266 |
| RZ597087 | CmsCmsAmAmGmAmGfCdAfCmCmAmAmGmAmAmCmUmAm_L96 | 267 | UmsAfsGmUmUmCfUmUmGmGmU(TBDMS)GmCmUfCmUfUmGmGmsCmsUm | 268 |
| RZ597088 | CmsCmsAmAmGmAmGfCdAfCmCmAmAmGmAmAmCmUmAm_L96 | 269 | UmsAfsGmUmUmCfUmUmGmGmUmG(TBDMS)CmUfCmUfUmGmGmsCmsUm | 270 |

(continued)

| Compound Serial No. | Sense Strand Sequence and Modification (5'-3') | SEQ ID NOs | Antisense Strand Sequence and Modification (5'-3') | SEQ ID NOs |
|---|---|---|---|---|
| RZ597089 | CmsCmsAmAmGmAmGfCdAfCmCmAmAmGmAmAmCmUmAm_L96 | 271 | UmsAfsGmUmUmCfUmUmGmGmUmGmC(TBDMS)UfCmUfUmGmGmsCmsUm | 272 |
| RZ597090 | CmsCmsAmAmGmAmGfCdAfCmCmAmAmGmAmAmCmUmAm_L96 | 273 | UmsAfsGmUmUmCfUmUmGmGmUmGmCmU(TBDMS)CmUfUmGmGmsCmsUm | 274 |
| RZ597091 | CmsCmsAmAmGmAmGfCdAfCmCmAmAmGmAmAmCmUmAm_L96 | 275 | UmsAfsGmUmUmCfUmUmGmGmUmGmCmUfC(TBDMS)UfUmGmGmsCmsUm | 276 |
| RZ597092 | CmsCmsAmAmGmAmGfCdAfCmCmAmAmGmAmAmCmUmAm_L96 | 277 | UmsAfsGmUmUmCfUmUmGmGmUmGmCmUfCmU(TBDMS)UmGmGmsCmsUm | 278 |
| RZ597093 | CmsCmsAmAmGmAmGfCdAfCmCmAmAmGmAmAmCmUmAm_L96 | 279 | UmsAfsGmUmUmCfUmUmGmGmUmGmCmUfCmUfU(TBDMS)GmGmsCmsUm | 280 |
| RZ597094 | CmsCmsAmAmGmAmGfCdAfCmCmAmAmGmAmAmCmUmAm_L96 | 281 | UmsAfsGmUmUmCfUmUmGmGmUmGmCmUfCmUfUmG(TBDMS)GmsCmsUm | 282 |
| RZ597095 | CmsCmsAmAmGmAmGfCdAfCmCmAmAmGmAmAmCmUmAm_L96 | 283 | UmsAfsGmUmUmCfUmUmGmGmUmGmCmUfCmUfUmGmG(TBDMS)sCmsUm | 284 |
| RZ597096 | CmsCmsAmAmGmAmGfCdAfCmCmAmAmGmAmAmCmUmAm_L96 | 285 | UmsAfsGmUmUmCfUmUmGmGmUmGmCmUfCmUfUmGmGmsC(TBDMS)sUm | 286 |

(continued)

| Compound Serial No. | Sense Strand Sequence and Modification (5'-3') | SEQ ID NOs | Antisense Strand Sequence and Modification (5'-3') | SEQ ID NOs |
|---|---|---|---|---|
| RZ597097 | CmsCmsAmAmGmAmGfCdAfCmCmAmAmGmAmAmCmUmAm_L96 | 287 | UmsAfsGmUmUmCfUmUmGmGmUmGmCmUfCmUfUmGmGmsCmsU(TBDMS) | 288 |
| RZ594001 | GmsUmsAmCmGmUmGfGdAfCmUmGmGmAmUmUmCmUmGm_L96 | 289 | CmsAfsGmAmAmUfCmCmAmGmUmCmCmAfCmGfUmAmCmsUmsUm | 290 |
| RZ594007 | G(TOM)sUmsAmCmGmUmGfGdAfCmUmGmGmAmUmUmCmUmGm_L96 | 291 | CmsAfsGmAmAmUfCmCmAmGmUmCmCmAfCmGfUmAmCmsUmsUm | 292 |
| RZ594008 | GmsU(TOM)sAmCmGmUmGfGdAfCmUmGmGmAmUmUmCmUmGm_L96 | 293 | CmsAfsGmAmAmUfCmCmAmGmUmCmCmAfCmGfUmAmCmsUmsUm | 294 |
| RZ594009 | GmsUmsA(TOM)CmGmUmGfGdAfCmUmGmGmAmUmUmCmUmGm_L96 | 295 | CmsAfsGmAmAmUfCmCmAmGmUmCmCmAfCmGfUmAmCmsUmsUm | 296 |
| RZ594010 | GmsUmsAmC(TOM)GmUmGfGdAfCmUmGmGmAmUmUmCmUmGm_L96 | 297 | CmsAfsGmAmAmUfCmCmAmGmUmCmCmAfCmGfUmAmCmsUmsUm | 298 |
| RZ594011 | GmsUmsAmCmG(TOM)UmGfGdAfCmUmGmGmAmUmUmCmUmGm_L96 | 299 | CmsAfsGmAmAmUfCmCmAmGmUmCmCmAfCmGfUmAmCmsUmsUm | 300 |
| RZ594012 | GmsUmsAmCmGmU(TOM)GfGdAfCmUmGmGmAmUmUmCmUmGm_L96 | 301 | CmsAfsGmAmAmUfCmCmAmGmUmCmCmAfCmGfUmAmCmsUmsUm | 302 |

(continued)

| Compound Serial No. | Sense Strand Sequence and Modification (5'-3') | SEQ ID NOs | Antisense Strand Sequence and Modification (5'-3') | SEQ ID NOs |
|---|---|---|---|---|
| RZ594013 | GmsUmsAmCmGmUmG(TOM)GdAfCmUmGmGmAmUmUmCmUmGm_L96 | 303 | CmsAfsGmAmAmUfCmCmAmGmUmCmCmAfCmGfUmAmCmsUmsUm | 304 |
| RZ594014 | GmsUmsAmCmGmUmGfG(TOM)AfCmUmGmGmAmUmUmCmUmGm_L96 | 305 | CmsAfsGmAmAmUfCmCmAmGmUmCmCmAfCmGfUmAmCmsUmsUm | 306 |
| RZ594015 | GmsUmsAmCmGmUmGfGdA(TOM)CmUmGmGmAmUmUmCmUmGm_L96 | 307 | CmsAfsGmAmAmUfCmCmAmGmUmCmCmAfCmGfUmAmCmsUmsUm | 308 |
| RZ594016 | GmsUmsAmCmGmUmGfGdAfC(TOM)UmGmGmAmUmUmCmUmGm_L96 | 309 | CmsAfsGmAmAmUfCmCmAmGmUmCmCmAfCmGfUmAmCmsUmsUm | 310 |
| RZ594017 | GmsUmsAmCmGmUmGfGdAfCmU(TOM)GmGmAmUmUmCmUmGm_L96 | 311 | CmsAfsGmAmAmUfCmCmAmGmUmCmCmAfCmGfUmAmCmsUmsUm | 312 |
| RZ594018 | GmsUmsAmCmGmUmGfGdAfCmUmG(TOM)GmAmUmUmCmUmGm_L96 | 313 | CmsAfsGmAmAmUfCmCmAmGmUmCmCmAfCmGfUmAmCmsUmsUm | 314 |
| RZ594019 | GmsUmsAmCmGmUmGfGdAfCmUmGmG(TOM)AmUmUmCmUmGm_L96 | 315 | CmsAfsGmAmAmUfCmCmAmGmUmCmCmAfCmGfUmAmCmsUmsUm | 316 |
| RZ594020 | GmsUmsAmCmGmUmGfGdAfCmUmGmGmA(TOM)UmUmCmUmGm_L96 | 317 | CmsAfsGmAmAmUfCmCmAmGmUmCmCmAfCmGfUmAmCmsUmsUm | 318 |

(continued)

| Compound Serial No. | Sense Strand Sequence and Modification (5'-3') | SEQ ID NOs | Antisense Strand Sequence and Modification (5'-3') | SEQ ID NOs |
|---|---|---|---|---|
| RZ594021 | GmsUmsAmCmGmUmGfGdAfCmUmGmGmAmU(TOM)UmCmUmGm_L96 | 319 | CmsAfsGmAmAmUfCmCmAmGmUmCmCmAfCmGfUmAmCmsUmsUm | 320 |
| RZ594022 | GmsUmsAmCmGmUmGfGdAfCmUmGmGmAmUmU(TOM)CmUmGm_L96 | 321 | CmsAfsGmAmAmUfCmCmAmGmUmCmCmAfCmGfUmAmCmsUmsUm | 322 |
| RZ594023 | GmsUmsAmCmGmUmGfGdAfCmUmGmGmAmUmUmC(TOM)UmGm_L96 | 323 | CmsAfsGmAmAmUfCmCmAmGmUmCmCmAfCmGfUmAmCmsUmsUm | 324 |
| RZ594024 | GmsUmsAmCmGmUmGfGdAfCmUmGmGmAmUmUmCmU(TOM)Gm_L96 | 325 | CmsAfsGmAmAmUfCmCmAmGmUmCmCmAfCmGfUmAmCmsUmsUm | 326 |
| RZ594025 | GmsUmsAmCmGmUmGfGdAfCmUmGmGmAmUmUmCmUmG(TOM)_L96 | 327 | CmsAfsGmAmAmUfCmCmAmGmUmCmCmAfCmGfUmAmCmsUmsUm | 328 |
| RZ594026 | GmsUmsAmCmGmUmGfGdAfCmUmGmGmAmUmUmCmUmGm_L96 | 329 | C(TOM)sAfsGmAmAmUfCmCmAmGmUmCmCmAfCmGfUmAmCmsUmsUm | 330 |
| RZ594027 | GmsUmsAmCmGmUmGfGdAfCmUmGmGmAmUmUmCmUmGm_L96 | 331 | CmsA(TOM)sGmAmAmUfCmCmAmGmUmCmCmAfCmGfUmAmCmsUmsUm | 332 |
| RZ594028 | GmsUmsAmCmGmUmGfGdAfCmUmGmGmAmUmUmCmUmGm_L96 | 333 | CmsAfsG(TOM)AmAmUfCmCmAmGmUmCmCmAfCmGfUmAmCmsUmsUm | 334 |

(continued)

| Compound Serial No. | Sense Strand Sequence and Modification (5'-3') | SEQ ID NOs | Antisense Strand Sequence and Modification (5'-3') | SEQ ID NOs |
|---|---|---|---|---|
| RZ594029 | GmsUmsAmCmGmUmGfGdAfCmUmGmGmAmUmUmCmUmGm_L96 | 335 | CmsAfsGmA(TOM)AmUfCmCmAmGmUmCmCmAfCmGfUmAmCmsUmsUm | 336 |
| RZ594002 | GmsUmsAmCmGmUmGfGdAfCmUmGmGmAmUmUmCmUmGm_L96 | 337 | CmsAfsGmAmA(TOM)UfCmCmAmGmUmCmCmAfCmGfUmAmCmsUmsUm | 338 |
| RZ594003 | GmsUmsAmCmGmUmGfGdAfCmUmGmGmAmUmUmCmUmGm_L96 | 339 | CmsAfsGmAmAmU(TOM)CmCmAmGmUmCmCmAfCmGfUmAmCmsUmsUm | 340 |
| RZ594004 | GmsUmsAmCmGmUmGfGdAfCmUmGmGmAmUmUmCmUmGm_L96 | 341 | CmsAfsGmAmAmUfC(TOM)CmAmGmUmCmCmAfCmGfUmAmCmsUmsUm | 342 |
| RZ594005 | GmsUmsAmCmGmUmGfGdAfCmUmGmGmAmUmUmCmUmGm_L96 | 343 | CmsAfsGmAmAmUfCmC(TOM)AmGmUmCmCmAfCmGfUmAmCmsUmsUm | 344 |
| RZ594006 | GmsUmsAmCmGmUmGfGdAfCmUmGmGmAmUmUmCmUmGm_L96 | 345 | CmsAfsGmAmAmUfCmCmA(TOM)GmUmCmCmAfCmGfUmAmCmsUmsUm | 346 |
| RZ594030 | GmsUmsAmCmGmUmGfGdAfCmUmGmGmAmUmUmCmUmGm_L96 | 347 | CmsAfsGmAmAmUfCmCmAmG(TOM)UmCmCmAfCmGfUmAmCmsUmsUm | 348 |
| RZ594031 | GmsUmsAmCmGmUmGfGdAfCmUmGmGmAmUmUmCmUmGm_L96 | 349 | CmsAfsGmAmAmUfCmCmAmGmU(TOM)CmCmAfCmGfUmAmCmsUmsUm | 350 |

(continued)

| Compound Serial No. | Sense Strand Sequence and Modification (5'-3') | SEQ ID NOs | Antisense Strand Sequence and Modification (5'-3') | SEQ ID NOs |
|---|---|---|---|---|
| RZ594032 | GmsUmsAmCmGmUmGfGdAfCmUmGmGmAmUmUmCmUmGm_L96 | 351 | CmsAfsGmAmAmUfCmCmAmGmUmC(TOM)CmAfCmGfUmAmCmsUmsUm | 352 |
| RZ594033 | GmsUmsAmCmGmUmGfGdAfCmUmGmGmAmUmUmCmUmGm_L96 | 353 | CmsAfsGmAmAmUfCmCmAmGmUmCmC(TOM)AfCmGfUmAmCmsUmsUm | 354 |
| RZ594034 | GmsUmsAmCmGmUmGfGdAfCmUmGmGmAmUmUmCmUmGm_L96 | 355 | CmsAfsGmAmAmUfCmCmAmGmUmCmCmA(TOM)CmGfUmAmCmsUmsUm | 356 |
| RZ594035 | GmsUmsAmCmGmUmGfGdAfCmUmGmGmAmUmUmCmUmGm_L96 | 357 | CmsAfsGmAmAmUfCmCmAmGmUmCmCmAfC(TOM)GfUmAmCmsUmsUm | 358 |
| RZ594036 | GmsUmsAmCmGmUmGfGdAfCmUmGmGmAmUmUmCmUmGm_L96 | 359 | CmsAfsGmAmAmUfCmCmAmGmUmCmCmAfCmG(TOM)UmAmCmsUmsUm | 360 |
| RZ594037 | GmsUmsAmCmGmUmGfGdAfCmUmGmGmAmUmUmCmUmGm_L96 | 361 | CmsAfsGmAmAmUfCmCmAmGmUmCmCmAfCmGfU(TOM)AmCmsUmsUm | 362 |
| RZ594038 | GmsUmsAmCmGmUmGfGdAfCmUmGmGmAmUmUmCmUmGm_L96 | 363 | CmsAfsGmAmAmUfCmCmAmGmUmCmCmAfCmGfUmA(TOM)CmsUmsUm | 364 |
| RZ594039 | GmsUmsAmCmGmUmGfGdAfCmUmGmGmAmUmUmCmUmGm_L96 | 365 | CmsAfsGmAmAmUfCmCmAmGmUmCmCmAfCmGfUmAmC(TOM)sUmsUm | 366 |

(continued)

| Compound Serial No. | Sense Strand Sequence and Modification (5'-3') | SEQ ID NOs | Antisense Strand Sequence and Modification (5'-3') | SEQ ID NOs |
|---|---|---|---|---|
| RZ594040 | GmsUmsAmCmGmUmGfGdAfCmUmGmGmAmUmUmCmUmGm_L96 | 367 | CmsAfsGmAmAmUfCmCmAmGmUmCmCmAfCmGfUmAmCmsU(TOM)sUm | 368 |
| RZ594041 | GmsUmsAmCmGmUmGfGdAfCmUmGmGmAmUmUmCmUmGm_L96 | 369 | CmsAfsGmAmAmUfCmCmAmGmUmCmCmAfCmGfUmAmCmsUmsU(TOM) | 370 |
| RZ599001 | UmsUmsUmUmAmAmUfCfCfUmCmAmCmUmCmUmAmAmAm_L96 | 371 | UmsUfsUmAmGmAfGmUmGmAmGmGmAmUfUmAfAmAmAmsUmsGm | 372 |
| RZ599015 | UmsUmsUmUmA(MOE)AmUfCfCfUmCmAmCmUmCmUmAmAmAm_L96 | 373 | UmsUfsUmAmGmAfGmUmGmAmGmGmAmUfU(MOE)AfAmAmAmsUmsGm | 374 |
| RZ599016 | UmsUmsUmUmAmAmUfCfCfUmCmA(MOE)CmUmCmUmAmA(MOE)Am_L96 | 375 | UmsUfsUmAmGmAfGmUmGmAmGmGmAmUfU(MOE)AfAmAmAmsUmsGm | 376 |
| RZ599017 | UmsUmsUmUmA(MOE)AmUfCfCfUmCmA(MOE)CmUmCmUmAmA(MOE)Am_L96 | 377 | UmsUfsUmAmGmAfGmUmGmAmGmGmAmUfU(MOE)AfAmAmAmsUmsGm | 378 |
| RZ599018 | UmsUmsUmUmA(MOE)AmUfCfCfUmCmAmCmUmCmUmAmAmAm_L96 | 379 | UmsUfsUmAmGmAfGmU(MOE)GmAmGmGmAmUfU(MOE)AfAmAmAmsUmsGm | 380 |
| RZ599019 | UmsUmsUmUmA(MOE)AmUfCfCfUmCmAmCmUmCmUmAmAmAm_L96 | 381 | UmsUfsUmAmGmAfGmUmGmAmGmGmAmUfU(MOE)AfAmAmAmsU(MOE)sGm | 382 |

(continued)

| Compound Serial No. | Sense Strand Sequence and Modification (5'-3') | SEQ ID NOs | Antisense Strand Sequence and Modification (5'-3') | SEQ ID NOs |
|---|---|---|---|---|
| RZ599020 | UmsUmsUmUmAmAmUfCfCfUmCmA(MOE)CmUmCmUmAmA(MOE)Am_L96 | 383 | UmsUfsUmAmGmAfGmUmGmAmGmGmAmUfU(MOE)AfAmAmAmsU(MOE)sGm | 384 |
| RZ599026 | UmsUmsUmUmAmAmUfCfCfUfCmAmCmUmCmUmAmAmAm_L96 | 385 | UmsUfsUmAmGmAfGmUmGfAmGmGmAmUfUmAfAmAmAmsUmsGm | 386 |
| RZ599027 | UmsUmsUmUmAmAmUfCfCfUfCmAmCmUmCmUmAmAmAm_L96 | 387 | UmsUfsUmAmGmAfGm(NM062)GfAmGmGmAmUfUmAfAmAmAmsUmsGm | 388 |
| RZ599028 | UmsUmsUmUmAmAmUfCfCfUfCmAmCmUmCmUmAmAmAm_L96 | 389 | UmsUfsUmAmGmAfGmUmGfAmGmGmAmUf(NM062)AfAmAmAmsUmsGm | 390 |
| RZ599029 | UmsUmsUmUmAmAmUfCfCfUfCmAmCmUmCmUmAmAmAm_L96 | 391 | UmsUfsUmAmGmAfGm(NM063)GfAmGmGmAmUfUmAfAmAmAmsUmsGm | 392 |
| RZ599030 | UmsUmsUmUmAmAmUfCfCfUfCmAmCmUmCmUmAmAmAm_L96 | 393 | UmsUfsUmAmGmAfGmUmGfAmGmGmAmUf(NM063)AfAmAmAmsUmsGm | 394 |
| RZ002003 | CmsAmsGmAmGmAmAfAdUfUmCmUmAmCmUmAmCmAmUm_L96 | 407 | AmsUfsGmUmAmGfUmAmGmAmAmUmUmUfCmUfCmUmGmsUmsAm | 408 |
| RZ002050 | CmsAmsGmAmGmAmAfAfUfUfCmUmAmCmUmAmCmAmUm_L96 | 409 | AmsUfsGmUmAmGfUmAmGfAmAmUmUmUfC(moe)UfCmUmGmsUmsAm | 410 |

(continued)

| Compound Serial No. | Sense Strand Sequence and Modification (5'-3') | SEQ ID NOs | Antisense Strand Sequence and Modification (5'-3') | SEQ ID NOs |
|---|---|---|---|---|
| RZ002051 | CmsAmsGmAmGmAmAfAfUfUfCmUmAmCmUmAmCmAmUm_L96 | 411 | AmsUfsGmUmAmGfUmAmGmAfAmUmUmUfC(moe)UfCmUmGmsUmsAm | 412 |
| RZ002052 | CmsAmsGmAmGmAmAfAfUfUfCmUmAmCmUmAmCmAmUm_L96 | 413 | AmsUfsGmUmAmGfUmAmGmAmAfUmUmUfC(moe)UfCmUmGmsUmsAm | 414 |
| RZ002053 | CmsAmsGmAmG(moe)AmAfAfUfUfCmUmAmCmUmAmCmAmUm_L96 | 415 | AmsUfsGmUmAmGfUmAmGmAfAmUmUmUfC(moe)UfCmUmGmsUmsAm | 416 |
| 416RZ0020 55 | CmsAmsGmAmGmAmAfAfUfUfCmUmA(moe)CmUmAmCmAmUm_L96 | 417 | AmsUfsGmUmAmGfUmAmGmAfAmUmUmUfC(moe)UfCmUmGmsUmsAm | 418 |
| RZ002058 | CmsAmsGmAmGmAmAfAfUfUfCmT(moe)AmCmUmAmCmAmUm_L96 | 419 | AmsUfsGmUmAmGfUmA(moe)GmAfAmUmUmUfCmUfCmUmGmsUmsAm | 420 |
| RZ002059 | CmsAmsGmAmGmAmAfAfUfUfCmUmA(moe)CmUmAmCmAmUm_L96 | 421 | AmsUfsGmUmAmGfUmA(moe)GmAfAmUmUmUfCmUfCmUmGmsUmsAm | 422 |
| RZ002060 | CmsAmsGmAmGmAmAfAfUfUfCmUmAmCmUmAmCmA(moe)Um_L96 | 423 | AmsUfsGmUmAmGfUmA(moe)GmAfAmUmUmUfCmUfCmUmGmsUmsAm | 424 |
| RZ002006 | CmsGmsAmAmGmCmUfCdAfUmGmAmAmUmAmUmAmUmUm_L96 | 425 | AmsAfsUmAmUmAfUmUmCmAmUmGmAmGfCmUfUmCmGmsUmsAm | 426 |

(continued)

| Compound Serial No. | Sense Strand Sequence and Modification (5'-3') | SEQ ID NOs | Antisense Strand Sequence and Modification (5'-3') | SEQ ID NOs |
|---|---|---|---|---|
| RZ002033 | CmsGmsAmAmGmCmUfCfAfUfGmAmAmUmAmUmAmUmUm_L96 | 427 | AmsAfsUmAmUmAfUmUmCfAmUmGmAmGfC(moe)UfUmCmGmsUmsAm | 428 |
| RZ002034 | CmsGmsAmAmGmCmUfCfAfUfGmAmAmUmAmUmAmUmUm_L96 | 429 | AmsAfsUmAmUmAfUmUmCmAfUmGmAmGfC(moe)UfUmCmGmsUmsAm | 430 |
| RZ002035 | CmsGmsAmAmGmCmUfCfAfUfGmAmAmUmAmUmAmUmUm_L96 | 431 | AmsAfsUmAmUmAfUmUmCmAmUfGmAmGfC(moe)UfUmCmGmsUmsAm | 432 |
| RZ002036 | CmsGmsAmAmG(moe)CmUfCfAfUfGmAmAmUmAmUmAmUmUm_L96 | 433 | AmsAfsUmAmUmAfUmUmCmAfUmGmAmGfC(moe)UfUmCmGmsUmsAm | 434 |
| RZ002037 | CmsGmsAmAmGmCmUfCfAfUfGmA(moe)AmUmAmUmAmUmUm_L96 | 435 | AmsAfsUmAmUmAfUmUmCmAfUmGmAmGfC(moe)UfUmCmGmsUmsAm | 436 |
| RZ002038 | CmsGmsAmAmGmCmUfCfAfUfGmAmA(moe)UmAmUmAmUmUm_L96 | 437 | AmsAfsUmAmUmAfUmUmCmAfUmGmAmGfC(moe)UfUmCmGmsUmsAm | 438 |
| RZ002041 | CmsGmsAmAmGmCmUfCfAfUfGmA(moe)AmUmAmUmAmUmUm_L96 | 439 | AmsAfsUmAmUmAfUmT(moe)CmAfUmGmAmGfCmUfUmCmGmsUmsAm | 440 |
| RZ002042 | CmsGmsAmAmGmCmUfCfAfUfGmAmA(moe)UmAmUmAmUmUm_L96 | 441 | AmsAfsUmAmUmAfUmT(moe)CmAfUmGmAmGfCmUfUmCmGmsUmsAm | 442 |

(continued)

| Compound Serial No. | Sense Strand Sequence and Modification (5'-3') | SEQ ID NOs | Antisense Strand Sequence and Modification (5'-3') | SEQ ID NOs |
|---|---|---|---|---|
| RZ002043 | CmsGmsAmAmGmCmUfCfAfUfGmAmAmUmAmUmAmT(moe)Um_L96 | 443 | AmsAfsUmAmUmAfUmT(moe)CmAfUmGmAmGfCmUfUmCmGmsUmsAm | 444 |
| RZ002031 | GmsAmsGmAmAmUmUfGdCfUmUmCmAmUmAmCmAmAmAm_L96 | 445 | UmsUfsUmGmUmAfUmGmAmAmGmCmAmAfUmUfCmUmCmsCmsUm | 446 |
| RZ002067 | GmsAmsGmAmAmUmUfGfCfUfUmCmAmUmAmCmAmAmAm_L96 | 447 | UmsUfsUmGmUmAfUmGmAmAfGmCmAmAfT(moe)UfCmUmCmsCmsUm | 448 |
| RZ002068 | GmsAmsGmAmAmUmUfGfCfUfUmCmAmUmAmCmAmAmAm_L96 | 449 | UmsUfsUmGmUmAfUmGmAmAmGfCmAmAfT(moe)UfCmUmCmsCmsUm | 450 |
| RZ002069 | GmsAmsGmAmA(moe)UmUfGfCfUfUmCmAmUmAmCmAmAmAm_L96 | 451 | UmsUfsUmGmUmAfUmGmAmAfGmCmAmAfT(moe)UfCmUmCmsCmsUm | 452 |
| RZ002070 | GmsAmsGmAmAmUmUfGfCfUfUmC(moe)AmUmAmCmAmAmAm_L96 | 453 | UmsUfsUmGmUmAfUmGmAmAfGmCmAmAfT(moe)UfCmUmCmsCmsUm | 454 |
| RZ002071 | GmsAmsGmAmAmUmUfGfCfUfUmCmA(moe)UmAmCmAmAmAm_L96 | 455 | UmsUfsUmGmUmAfUmGmAmAfGmCmAmAfT(moe)UfCmUmCmsCmsUm | 456 |
| RZ002011 | CmsAmsAmCmUmCmAfCdCfUmGmUmAmAmUmAmAmAmUm_L96 | 457 | AmsUfsUmUmAmUfUmAmCmAmGmGmUmGfAmGfUmUmGmsAmsUm | 458 |

(continued)

| Compound Serial No. | Sense Strand Sequence and Modification (5'-3') | SEQ ID NOs | Antisense Strand Sequence and Modification (5'-3') | SEQ ID NOs |
|---|---|---|---|---|
| RZ002082 | CmsAmsAmCmUmCmAfCfCfUfGmUmAmAmUmAmAmAmUm_L96 | 459 | AmsUfsUmUmAmUfUmAmCfAmGmGmUmGfA(moe)GfUmUmGmsAmsUm | 460 |
| RZ002083 | CmsAmsAmCmUmCmAfCfCfUfGmUmAmAmUmAmAmAmUm_L96 | 461 | AmsUfsUmUmAmUfUmAmCmAfGmGmUmGfA(moe)GfUmUmGmsAmsUm | 462 |
| RZ002084 | CmsAmsAmCmUmCmAfCfCfUfGmUmAmAmUmAmAmAmUm_L96 | 463 | AmsUfsUmUmAmUfUmAmCmAmGfGmUmGfA(moe)GfUmUmGmsAmsUm | 464 |
| RZ002085 | CmsAmsAmCmT(moe)CmAfCfCfUfGmUmAmAmUmAmAmAmUm_L96 | 465 | AmsUfsUmUmAmUfUmAmCmAfGmGmUmGfA(moe)GfUmUmGmsAmsUm | 466 |
| RZ002086 | CmsAmsAmCmUmCmAfCfCfUfGmT(moe)AmAmUmAmAmAmUm_L96 | 467 | AmsUfsUmUmAmUfUmAmCmAfGmGmUmGfA(moe)GfUmUmGmsAmsUm | 468 |
| RZ002087 | CmsAmsAmCmUmCmAfCfCfUfGmUmA(moe)AmUmAmAmAmUm_L96 | 469 | AmsUfsUmUmAmUfUmAmCmAfGmGmUmGfA(moe)GfUmUmGmsAmsUm | 470 |
| RZ002091 | CmsAmsAmCmUmCmAfCfCfUfGmUmA(moe)AmUmAmAmAmUm_L96 | 471 | AmsUfsUmUmAmUfUmA(moe)CmAfGmGmUmGfAmGfUmUmGmsAmsUm | 472 |
| RZ002092 | CmsAmsAmCmUmCmAfCfCfUfGmUmAmAmUmAmAmA(moe)Um_L96 | 473 | AmsUfsUmUmAmUfUmA(moe)CmAfGmGmUmGfAmGfUmUmGmsAmsUm | 474 |

(continued)

| Compound Serial No. | Sense Strand Sequence and Modification (5'-3') | SEQ ID NOs | Antisense Strand Sequence and Modification (5'-3') | SEQ ID NOs |
|---|---|---|---|---|
| RZ002099 | CmsGmsAmAmGmCmUfCfAfUfGmAmAmUmAmUmAmUmUm_(CR01008×3) | 475 | AmsAfsUmAmUmAfUmUmCmAmUfGmAmGfC(moe)UfUmCmGmsUmsAm | 476 |
| RZ002101 | CmsGmsAmAmGmCmUfCfAfUfGmAmA(moe)UmAmUmAmUmUm_(CR01008×3) | 477 | AmsAfsUmAmUmAfUmUmCmAmUfGmAmGfC(moe)UfUmCmGmsUmsAm | 478 |
| RZ002106 | CmsAmsGmAmGmAmAfAfUfUfCmUmAmCmUmAmCmAmUm_(CR01008×3) | 479 | AmsUfsGmUmAmGfUmAmGmAmAfUmUmUfC(moe)UfCmUmGmsUmsAm | 480 |
| RZ002113 | CmsAmsAmCmUmCmAfCfCfUfGmUmAmAmUmAmAmA(moe)Um_(CR01008×3) | 481 | AmsUfsUmUmAmUfUmA(moe)CmAmGfGmUmGfAmGfUmUmGmsAmsUm | 482 |
| RZ003017 | CmsAmsAmGmUmUmGfAdGfAmAmCmAmAmAmAmAmUmUm_L96 | 483 | AmsAfsUmUmUmUfUmGmUmUmCmUmCmAfAmCfUmUmGmsAmsAm | 484 |
| RZ003028 | CmsAmsAmGmUmUmGfAfGfAfAmC(moe)AmAmAmAmAmUmUm_L96 | 485 | AmsAfsUmUmUmUfUmGmUmUfCmUmCmAfA(moe)CfUmUmGmsAmsAm | 486 |
| RZ003029 | CmsAmsAmGmUmUmGfAfGfAfAmC(moe)AmAmAmAmAmUmUm_L96 | 487 | AmsAfsUmUmUmUfUmGmUmUmCfUmCmAfA(moe)CfUmUmGmsAmsAm | 488 |
| RZ003031 | CmsAmsAmGmUmUmGfAfGfAfAmC(moe)AmAmAmAmAmT(moe)Um_L96 | 489 | AmsAfsUmUmUmUfUmGmUmUfCmUmCmAfA(moe)CfUmUmGmsAmsAm | 490 |

(continued)

| Compound Serial No. | Sense Strand Sequence and Modification (5'-3') | SEQ ID NOs | Antisense Strand Sequence and Modification (5'-3') | SEQ ID NOs |
|---|---|---|---|---|
| RZ003032 | CmsAmsAmGmUmUmGfAfGfAfAmCmAmAmAmAmAmT(moe)Um_L96 | 491 | AmsAfsUmUmUmUfUmG(moe)UmUfCmUmCmAfAmCfUmUmGmsAmsAm | 492 |
| RZ003020 | GmsUmsUmUmUmAmAfAdAfUmUmAmAmAmGmUmAmUmAm_L96 | 493 | UmsAfsUmAmCmUfUmUmAmAmUmUmUmUfAmAfAmAmCmsCmsCm | 494 |
| RZ003041 | GmsUmsUmUmUmAmAfAfAfUfUmA(moe)AmAmGmUmAmUmAm_L96 | 495 | UmsAfsUmAmCmUfUmUmAmAfUmUmUmUfA(moe)AfAmAmCmsCmsCm | 496 |
| RZ003042 | GmsUmsUmUmUmAmAfAfAfUfUmA(moe)AmAmGmUmAmUmAm_L96 | 497 | UmsAfsUmAmCmUfUmUmAmAmUfUmUmUfA(moe)AfAmAmCmsCmsCm | 498 |
| RZ003021 | AmsAmsGmUmAmUmAfCdAfUmUmUmUmUmGmCmAmUmUm_L96 | 499 | AmsAfsUmGmCmAfAmAmAmAmUmGmUmAfUmAfCmUmUmsUmsAm | 500 |
| RZ003065 | AmsAmsGmUmAmUmAfCfAfUfUmUmUmUmGmCmAmUmUm_(CR01008×3) | 501 | AmsAfsUmGmCmAfAmAmAmAfUmGmUmAfUmAfCmUmUmsUmsAm | 502 |
| RZ003066 | AmsAmsGmUmAmUmAfCfAfUfUmUmUmUmGmCmAmUmUm_(CR01008×3) | 503 | AmsAfsUmGmCmAfAmAmAmAfUmGmUmAfT(moe)AfCmUmUmsUmsAm | 504 |
| RZ003069 | GmsUmsUmUmUmAmAfAfAfUfUmAmAmAmGmUmAmUmAm_(CR01008×3) | 505 | UmsAfsUmAmCmUfUmUmAfAmUmUmUmUfA(moe)AfAmAmCmsCmsCm | 506 |

(continued)

| Compound Serial No. | Sense Strand Sequence and Modification (5'-3') | SEQ ID NOs | Antisense Strand Sequence and Modification (5'-3') | SEQ ID NOs |
|---|---|---|---|---|
| RZ001003 | GmsGmsCmUmUmGmAfdUCfAmUmGmAmAmCmUmAmCmUm_L96 | 507 | AmsGfsUmAmGmUfUmCmAmUmGmAmUmCfAmAfGmCmCmsAmsGm | 508 |
| RZ001005 | GmsCmsAmGmCmUmCfdCUfUmAmUmUmGmUmUmAmUmAm_L96 | 509 | UmsAfsUmAmAmCfAmAmUmAmAmGmGmAfGmCfUmGmCmsCmsAm | 510 |
| RZ001008 | CmsGmsGmUmAmAmUfdGGfAmCmAmGmAmGmUmUmAmUm_L96 | 511 | AmsUfsAmAmCmUfCmUmGmUmCmCmAmUfUmAfCmCmGmsUmsGm | 512 |
| RZ001012 | CmsUmsGmGmCmUmUfdGAfUmCmAmAmGmAmAmCmUmAm_L96 | 513 | UmsAfsGmUmUmCfUmUmGmAmUmCmAmAfGmCfCmAmGmsCmsAm | 514 |
| RZ001026 | CmsUmsGmAmCmAmCfdAAfUmGmCmUmCmAmGmAmAmAm_L96 | 515 | UmsUfsUmCmUmGfAmGmCmAmUmUmGmUfGmUfCmAmGmsAmsUm | 516 |
| RZ001032 | GmsGmsCmUmUmGmAfUfCfAfUmGmAmAmCmUmAmCmUm_(CR01008×3) | 517 | AmsGfsUmAmGmUfUmCmAmUmGfAmUmCfA(moe)AfGmCmCmsAmsGm | 518 |
| RZ001034 | GmsCmsAmGmCmUmCfCfUfUfAmUmUmGmUmUmAmUmAm_(CR01008×3) | 519 | UmsAfsUmAmAmCfAmAmUmAmAfGmGmAfG(moe)CfUmGmCmsCmsAm | 520 |
| RZ001037 | CmsGmsGmUmAmAmUfGfGfAfCmAmGmAmGmUmUmAmUm_(CR01008×3) | 521 | AmsUfsAmAmCmUfCmUmGmUmCfCmAmUfT(moe)AfCmCmGmsUmsGm | 522 |

(continued)

| Compound Serial No. | Sense Strand Sequence and Modification (5'-3') | SEQ ID NOs | Antisense Strand Sequence and Modification (5'-3') | SEQ ID NOs |
|---|---|---|---|---|
| RZ001039 | CmsUmsGmGmCmUmUfGfAfUfCmAmAmGmAmAmCmUmAm_(CR01008×3) | 523 | UmsAfsGmUmUmCfUmUmGmAmUfCmAmAfG(moe)CfCmAmGmsCmsAm | 524 |
| RZ001044 | CmsUmsGmAmCmAmCfAfAfUfGmCmUmCmAmGmAmAmAm_(CR01008×3) | 525 | UmsUfsUmCmUmGfAmGmCmAmUfUmGmUfG(moe)UfCmAmGmsAmsUm | 526 |
| RZ599062 | UmsUmsUmUmAmAmUfCfCfUfCmAmCmUmCmUmAmAmAm_L96 | 527 | UmsUfsUmAmGmAfGmUmGfAmGmGmAmUfU(moe)AfAmAmAmsUmsGm | 528 |
| RZ599136 | UmsUmsUmUmAmAmUfCfCfUfCmAmCmUmCmUmAmAmAm_L96 | 529 | UmsUfsUmAmGmAfGmUmGfAmGmGmAmUf(NM096)AfAmAmAmsUmsGm | 530 |
| RZ599137 | UmsUmsUmUmAmAmUfCfCfUfCmAmCmUmCmUmAmAmAm_L96 | 531 | UmsUfsUmAmGmAfGmUmGfAmGmGmAmUf(NM097)AfAmAmAmsUmsGm | 532 |
| RZ599138 | UmsUmsUmUmAmAmUfCfCfUfCmAmCmUmCmUmAmAmAm_L96 | 533 | UmsUfsUmAmGmAfGmUmGfAmGmGmAmUf(NM098)AfAmAmAmsUmsGm | 534 |
| RZ599139 | UmsUmsUmUmAmAmUfCfCfUfCmAmCmUmCmUmAmAmAm_L96 | 535 | UmsUfsUmAmGmAfGmUmGfAmGmGmAmUf(NM099)AfAmAmAmsUmsGm | 536 |
| RZ599051 | UmsUmsUmUmAmAmUf(Gemcitabine)CfUmCmAmCmUmCmUmAmAmAm_L96 | 537 | UmsUfsUmAmGmAfGmUmGmAmGmGmAmUfUmAfAmAmAmsUmsGm | 538 |

(continued)

| Compound Serial No. | Sense Strand Sequence and Modification (5'-3') | SEQ ID NOs | Antisense Strand Sequence and Modification (5'-3') | SEQ ID NOs |
|---|---|---|---|---|
| RZ599055 | UmsUmsUmUmAmAmUf(Gem citabine)CfUfCmAmCmUmCm UmAmAmAm_L96 | 539 | UmsUfsUmAmGmAfGmUmG mAmGmGfAmUfUmAfAmAm AmsUmsGm | 540 |
| RZ599056 | UmsUmsUmUmAmAmUfCf(G emcitabine)UfCmAmCmUmCm UmAmAmAm_L96 | 541 | UmsUfsUmAmGmAfGmUmG mAmGmGfAmUfUmAfAmAm AmsUmsGm | 542 |
| RZ597002 | CmsCmsAmAmGmAmGfCfAf CmCmAmAmGmAmAmCmU mAm_L96 | 543 | UmsAfsGmUmUmCfUmUmG mGmUmGmCmUfCmUfUmG mGmsUmsUm | 544 |
| RZ597060 | CmsCmsAmAmGmAmGfCfAf CmCmAmAmGmAmAmCmU mAm_L96 | 545 | UmsAfsGmUmUm(Gemcitabin e)UmUmGmGmUmGmCmUfC mUfUmGmGmsUmsUm | 546 |
| RZ597101 | CmsCmsAmAmGmAmGf(Gem citabine)AfCfCmAmAmGmAm AmCmUmAm_L96 | 547 | UmsAfsGmUmUmCfUmUmG mGmUmGfCmUfC(moe)UfUm GmGmsCmsUm | 548 |
| RZM11015 | GmsCmsUmCmAmAmCfCfAf AmAmUmCmAmGmUmUmA mUm_L96 | 649 | AmsUfsAmAmCmUfGmAmU mUmUmGmGmUfUmGfAmG mCmsUmsUm | 550 |
| RZM11508 | GmsCmsUmCmAmAmCfCfAf AfAmUmCmAmGmUmUmAm Um_L96 | 551 | AmsUfsAmAmCmUfGmAmU mUmUmGfGmUfUmGfAmGm CmsUmsUm | 552 |
| RZM11509 | GmsCmsUmCmAmAmCfCfAf AfAmUmCmAmGmUmUmAm Um_L96 | 553 | AmsUfsAmAmCmUfGmAmU mUmUmGfGmUfT(moe)GfAm GmCmsUmsUm | 554 |

(continued)

| Compound Serial No. | Sense Strand Sequence and Modification (5'-3') | SEQ ID NOs | Antisense Strand Sequence and Modification (5'-3') | SEQ ID NOs |
|---|---|---|---|---|
| RZM11510 | GmsCmsUmCmAmAm(Gemcitabine)CfAfAfAmUmCmAmGmUmUmAmUm_L96 | 555 | AmsUfsAmAmCmUfGmAmUmUmUmGfGmUfT(moe)GfAmGmCmsUmsUm | 556 |
| RZM11001 | GmsGmsAmGmGmGmAfGfUfAmGmAmGmAmUmCmAmAmAm_L96 | 557 | UmsUfsUmGmAmUfCmUmCmUmAmCmUmCfCmCfUmCmCmsAmsGm | 558 |
| RZM11502 | GmsGmsAmGmGmGmAfGfUfAfGmAmGmAmUmCmAmAmAm_L96 | 559 | UmsUfsUmGmAmUfCmUmCmUmAmCfUmCfC(moe)CfUmCmCmsAmsGm | 560 |
| RZM11504 | GmsGmsAmGmGmGmAfGfUfAfGmAmGmAmUmCmAmAmAm_L96 | 561 | UmsUfsUmGmAmUfCmUmCmUmAmCfUm(Gemcitabine)CmCfUmCmCmsAmsGm | 562 |
| RZM11505 | GmsGmsAmGmGmGmAfGfUfAfGmAmGmAmUmCmAmAmAm_L96 | 563 | UmsUfsUmGmAmUfCmUmCmUmAmCfUmCfCm(Gemcitabine)UmCmCmsAmsGm | 564 |
| RZM11507 | GmsGmsAmGmGmGmAfGfUfAfGmAmGmAmUmCmAmAmAm_L96 | 565 | UmsUfsUmGmAmUfCmUmCmUmAmCfUm(Gemcitabine)C(moe)CfUmCmCmsAmsGm | 566 |
| RZM12501 | CmsAmsAmCmAmGmUfGfAfUfGmAmAmAmUmCmCmAmAm_(CR01008)(CR01008)(CR01008Z) | 567 | UmsUfsGmGmAmUfUmUmCfAmUmCmAmCfU(moe)GfUmUmGmsCmsUm | 568 |

(continued)

| Compound Serial No. | Sense Strand Sequence and Modification (5'-3') | SEQ ID NOs | Antisense Strand Sequence and Modification (5'-3') | SEQ ID NOs |
|---|---|---|---|---|
| RZM12503 | CmsAmsAmCmAmGmUfGfAfUfGmAmAmAmUmCmCmAmAm_(CR01008)(CR01008)(CR01008Z) | 569 | UmsUfsGmGmAmUfUmUm(Gemcitabine)AmUmCmAmCfU(moe)GfUmUmGmsCmsUm | 570 |
| RZ597114 | CmsCmsAmAmGmAmGfCfAfCfCmAmAmGmAmAmCmUmAm_L96 | 571 | UmsAfsGmUmUmCfUmUmGfGmUmGmCmUfC(moe)UfUmGmGmsUmsUm | 572 |
| RZ597115 | CmsCmsAmAmGmAmGfCfAfCfCmAmAmGmAmAmCmUmAm_L96 | 573 | UmsAfsGmUmUmCfUmUmGfGmUmGmCmUfC(moe)UfUmGmGms(NM054)sUm | 574 |
| RZ597116 | CmsCmsAmAmGmAmGfCfAfCfCmAmAmGmAmAmCmUmAm_L96 | 575 | UmsAfsGmUmUmCfUmUmGfGmUmGmCmUfC(moe)UfUmGmGms(NM054)s(NM054) | 576 |
| RZ599060 | UmsUmsUmUmAmAmUfCfCfUfCmAmCmUmCmUmAmAmAm_L96 | 577 | UmsUfsUmAmGmAfGmUmGmAmGmGfAmUfUmAfAmAmAms(NM054)sGm | 578 |
| RZ599061 | UmsUmsUmUmAmAmUfCfCfUfCmAmCmUmCmUmAmAmAm_L96 | 579 | UmsUfsUmAmGmAfGmUmGmAmGmGfAmUfUmAfAmAmAms(NM054)s(NM054) | 580 |

(Gemcitabine) represents a nucleotide in siRNA, with structural formula

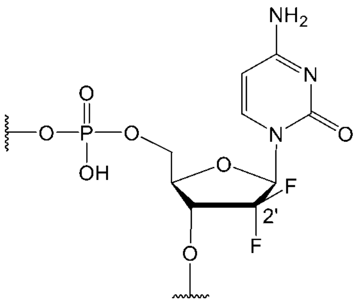

(Gemcitabine)

.

(NM054) represents a nucleotide in siRNA, with structural formula

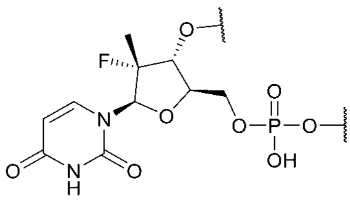

(NM054)

.

(NM096) represents a nucleotide in siRNA, with structural formula

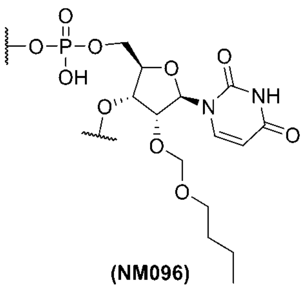

(NM096)

.

(NM097) represents a nucleotide in siRNA, with structural formula

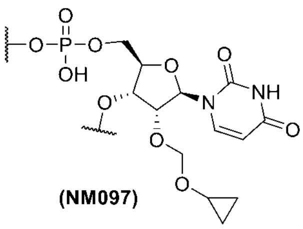

(NM097)

.

(NM098) represents a nucleotide in siRNA, with structural formula

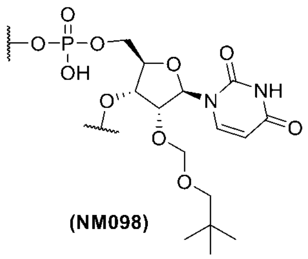

(NM098)

.

(NM099) represents a nucleotide in siRNA, with structural formula

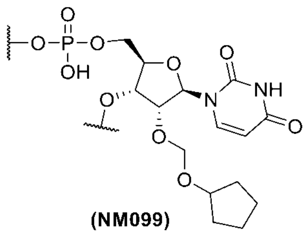

(NM099)

.

**Example 2 *In vitro* inhibitory activities of siRNAs modified with TBDMS at different sites against complement component 3 (CC3) mRNA**

The present example evaluated inhibitory activities of compounds RX502002-RX502041 modified with TBDMS at different sites of sense strand and antisense strand, and reference compound RX502001 without TBDMS modification against target gene CC3 in HepG2 cells by an *in vitro* cell screening method.

1. Formulation of tested articles

Each of the above siRNA tested articles was centrifuged, and then dissolved by adding an appropriate amount of 1× PBS according to specification of each vial, to prepare a 20 μM stock solution, which was then further diluted with 1× PBS to render a 5 μM working solution.

2. Cell culturing and plating

HepG2 cells were cultured and proliferated in DMEM medium comprising 10% FBS in a 37 °C, 5% $CO_2$ incubator. Before plating, the medium was discarded, and rinsing was performed with 0.25% pancreatin. After the cells were digested by pancreatin, digestion of the medium was terminated. The cells were resuspended, centrifuged at 800 rpm for 5 min, resuspended with fresh DMEM medium comprising 10% FBS, counted, diluted to cell density of $1\times10^5$ cells/mL, thoroughly mixed, plated in a 24-well plate, 500 μL/well, and subjected to a transfection operation after 24 h of culturing.

3. Cell transfection

1) Formulation of solution 1-siRNA mixed solution

49.4 μL of Opti-MEM medium and 0.6 μL of siRNA test substance working solution (5 μM) per cell well were uniformly mixed. Each siRNA tested article was prepared with 3 cell replicate wells.

2) Formulation of solution 2-Lipofectamine 3000 mixed solution:

A transfection reagent was gently mixed upside down, 2 μL of Lipo3000 reagent was diluted according to 48 μL of

Opti-MEM medium per cell well, gently pipetted 3-5 times, uniformly mixed, and stood for 5 min. Each siRNA tested article was prepared with 3 cell replicate wells.

3) Formulation of solution 3-mixture of solution 1 and solution 2:

One part (50 μL) of the solution 1 was gently mixed with one part (50 μL) of the solution 2 to prepare a solution 3 (100 μL) transfection complex, followed by incubation at room temperature for 20 min.

A cell culture plate to be transfected was taken out, original medium was discarded, and 500 μL of Opti-MEM medium was added. The solution 3 (100 μL) having undergone the incubation was added dropwise into cell culture wells to render concentration of about 5 nM of the siRNA test substance in each cell transfection well. The cell culture plate was further cultured in the 37 °C, 5% $CO_2$ incubator for 4 hours, and 1 mL of DMEM medium comprising 20% FBS was supplemented to each well. Further culturing was carried out in the 37 °C, 5% $CO_2$ incubator for 24 hours.

HepG2 cells were normally cultured without any transfection procedure in Blank control group; and Mock control group was a control group in which no siRNA was added to the transfection complex, and only Lipofectamine 3000 transfection reagent was added.

4. Detection

RNA extraction: total RNAs in cells of various wells were extracted using full-automatic nucleic acid extractor and a nucleic acid extraction kit from Zhejiang Hanwei Science and Technology Co. Ltd. according to a method described in instructions.

Reverse transcription reaction: 1 μg of total RNAs extracted from cells in each well were taken, and configured into 20 μL of reverse transcription system according to a method described in kit instructions and the reverse transcription reaction was completed by using a reverse transcription kit (Reverse Transcription System, A3500) from Promega company and selecting Oligo $(dT)_{15}$ reverse transcription primer. After the reaction was ended, 80 μL of RNase-Free water was added into the reverse transcription system, to yield cDNA solution for Real-time PCR detection.

Real-time PCR detection: 20 μL of Real-time PCR reaction system per PCR detection well was configured according to a method described in kit instructions using SYBR™ Select Master Mix (Catalog number, 4472908) reagent from ABI company. Each detection system comprised 5 μL of cDNA template obtained from the above reverse transcription reaction, 10 μL of SYBR™ Select Master Mix, 0.5 μL of 10 μM upstream primers, 0.5 μL of 10 μM downstream primers (see Table 5 for primer information), and 4 μL of RNase-Free $H_2O$. The configured reaction system was subjected to Real-time PCR amplification on ABI StepOnePlus PCR instrument by a three-step method, wherein an amplification procedure was pre-denaturation at 95 °C for 10 min, then denaturation at 95 °C for 30 s, annealing at 60 °C for 30 s, and extension at 72 °C for 30 s; and processes of denaturation, annealing, and extension were repeated for 40 cycles. Gene expression differences were calculated by the above ΔΔCt method after completion of the procedure.

Table 5

| Target Point | Primer Type | Primer Sequence (5'-3') | SEQ ID NO: |
|---|---|---|---|
| CC3 | upstream primer | AAGCGCATTCCGATTGAGGA | 395 |
| | downstream primer | CCTGAGTGCAAGATGACGGT | 396 |
| GAPDH | upstream primer | GGTCGGAGTCAACGGATTT | 397 |
| | downstream primer | CCAGCATCGCCCCACTTGA | 398 |

Statistics was carried out respectively on relative expression levels of target gene CC3 mRNA (FIG. 1) and residual activity of target gene CC3 mRNA (Table 6) after HepG2 was transfected by siRNAs modified with TBDMS at different sites of the sense strand. Results show that compared with the reference sequence (RX502001) without TBDMS modification, siRNA sequences modified with TBDMS at position 5 (RX502027), position 6 (RX502028), position 12 (RX502034), position 13 (RX502035), and position 18 (RX502040) of the sense strand had elevated *in vitro* activity; and TBDMS modification at position 7 (RX502029), position 8 (RX502030), position 9 (RX502031), and position 10 (RX502032) of the sense strand significantly weakened the *in vitro* activity of the siRNA sequences.

TABLE 6

| Group | Residual Activity% | ±STDEV Value |
|---|---|---|
| Blank | 95.12% | 4.13 |

(continued)

| Group | Residual Activity% | ±STDEV Value |
|---|---|---|
| Mock | 100.00% | 23.29 |
| RX502001 | 37.87% | 8.02 |
| RX502023 | 46.85% | 5.43 |
| RX502024 | 41.97% | 1.77 |
| RX502025 | 42.35% | 6.33 |
| RX502026 | 36.67% | 0.35 |
| RX502027 | 32.59% | 5.10 |
| RX502028 | 34.93% | 0.24 |
| RX502029 | 60.14% | 7.73 |
| RX502030 | 68.75% | 3.51 |
| RX502031 | 59.94% | 8.31 |
| RX502032 | 51.88% | 5.43 |
| RX502033 | 40.68% | 6.38 |
| RX502034 | 31.97% | 0.45 |
| RX502035 | 28.28% | 9.63 |
| RX502036 | 42.11% | 6.72 |
| RX502037 | 41.44% | 9.96 |
| RX502038 | 39.42% | 1.68 |
| RX502039 | 34.18% | 5.43 |
| RX502040 | 30.57% | 6.01 |
| RX502041 | 35.45% | 4.59 |

Statistics was carried out respectively on relative expression levels of target gene CC3 mRNA (FIG. 2) and residual activity of target gene CC3 mRNA (Table 7) after HepG2 was transfected by siRNAs modified with TBDMS at different sites of the antisense strand. Compared with the reference sequence (RX502001) without TBDMS modification, siRNA sequences modified with TBDMS at position 8 (RX502009) and position 15 (RX502016) on the antisense strand further elevated the *in vitro* activity of the siRNA sequences.

TABLE 7

| Group | Residual Activity% | ±STDEV Value |
|---|---|---|
| Blank | 84.73% | 21.89 |
| Mock | 100.00% | 2.00 |
| RX502001 | 31.03% | 0.30 |
| RX502002 | 46.41% | 5.90 |
| RX502003 | 59.23% | 3.38 |
| RX502004 | 52.15% | 4.46 |
| RX502005 | 64.90% | 5.35 |
| RX502006 | 59.99% | 3.59 |
| RX502007 | 49.94% | 0.78 |
| RX502008 | 41.48% | 3.09 |
| RX502009 | 29.52% | 2.44 |

(continued)

| Group | Residual Activity% | ±STDEV Value |
|---|---|---|
| RX502010 | 33.34% | 2.50 |
| RX502011 | 33.05% | 0.70 |
| RX502012 | 73.19% | 1.73 |
| RX502013 | 47.10% | 7.03 |
| RX502014 | 75.17% | 1.13 |
| RX502015 | 63.59% | 6.45 |
| RX502016 | 23.91% | 10.26 |
| RX502017 | 70.65% | 7.34 |
| RX502018 | 39.76% | 11.36 |
| RX502019 | 31.94% | 0.36 |
| RX502020 | 34.41% | 5.13 |
| RX502021 | 37.34% | 1.72 |
| RX502022 | 41.58% | 4.38 |

The above results show that the effect of TBDMS modification on siRNA sequence activity is associated with modification site, and that TBDMS bulky group modification at a specific site does not affect siRNA activity, and even may further elevate the activity.

**Example 3. Inhibitory activities of siRNA conjugates modified with TBDMS at different sites against mouse serum CC3 protein**

The present example evaluated inhibitory activities of compounds RZM02004, RZM02005 and RZM02006 modified with TBDMS at different sites of an antisense strand and reference compound RZM02001 without TBDMS modification against serum CC3 protein level in mice based on Elisa detection method and principle.

1. Animal grouping, administration and blood sampling

6-8-week-old C57BL/6j mice were randomly divided into 5 groups in total based on body weight, 5 mice per group. Each group of mice received the above siRNA conjugates via abdominal subcutaneous administration, at a dose of 3 mg/kg per mouse, and an administration volume of 5 mL/kg. PBS control group received the same volume of siRNA conjugate-free PBS solution. Pre-administration blood collection was recorded as pre-dose, the day of administration was recorded as the first day (D0), and blood collection was performed on each mouse from retro-orbital plexus using a clot activator tube (purchased from KONSFI, 220518) respectively on D14, D28, D42, D56, D70 and D91 post-administration, with a blood collection volume of about 0.1 mL per mouse per time. Blood collection was performed using the clot activator tube (purchased from KONSFI, 220518). Blood samples were stored at 4 °C overnight, and centrifuged at 1800 g at 4 °C for 10 min. Serum was separated and stored at -80 °C.

2. Serum CC3 protein detection

Serum samples were diluted 1:10000 in a 10-fold gradient, and a content of CC3 protein in each mouse serum was measured using a mouse Complement C3 ELISA kit (purchased from abcam, ab157711). Operations such as preparation of a standard working solution and subsequent sample addition were performed according to a method described in kit instructions, and finally, Elisa plate was placed in a multimode microplate reader (purchased from Biotek, Biotek Synergy H1) to detect a light absorption value at 450 nm. Four-parameter fitting was performed using Graphpad prism 8.0 (Equation: Y= Bottom + (X^Hillslope) * (Top-Bottom) / (X^HillSlope + EC50^HillSlope)) to draw a standard curve and calculate R squared. The protein content of each sample was calculated by substituting OD450 values into the standard curve followed by multiplication with respective dilution factors, and normalized against Pre-dose detection values.

3. Analysis of Results

Statistics was carried out on expression level of serum CC3 protein (FIG. 3) and residual activity of target gene CC3 mRNA (Table 8) in mice post-administration. Results show that siRNAs with TBDMS at position 18 of the sense strand (RZM02004), position 15 of the antisense strand (RZM02005), and position 21 of the antisense strand (RXM02006) had comparable activity to the reference sequence (RZM02001) without TBDMS modification, and they all could produce a potent sustained inhibitory effect on the serum CC3 protein content. By D91, the inhibitory effect of TBDMS modified RZM02004 (residual level 61.69%), RZM02005 (residual level 45.29%) and RXM02006 (residual level 56.75%) on serum CC3 protein level was significantly higher than that of the reference sequence RZM02001 (residual level 72.75%), suggesting that the TBDMS bulky group modification at specific site of siRNA does not affect sequence activity, and even may further elevate sequence activity.

Table 8

| | PBS | | RZM02001 | | RZM02004 | | RZM02005 | | RZM02006 | |
|---|---|---|---|---|---|---|---|---|---|---|
| Collection Time | residual activity% | ±STDE V value | residual activity% | ±STDEV value | residual activity% | ±STDEV value | residual activity% | ±STDEV value | residual activity% | ±STDEV value |
| pre-dose | 100.00% | 0.00 | 100.00% | 0.00 | 100.00% | 0.00 | 100.00% | 0.00 | 100.00% | 0.00 |
| D14 | 79.34% | 9.75 | 14.02% | 2.15 | 11.37% | 6.27 | 15.25% | 2.21 | 15.12% | 1.52 |
| D28 | 70.79% | 10.18 | 14.43% | 0.38 | 13.88% | 7.12 | 16.94% | 3.96 | 14.18% | 1.59 |
| D42 | 75.15% | 11.76 | 18.76% | 4.43 | 15.82% | 3.37 | 27.84% | 14.33 | 18.77% | 3.53 |
| D56 | 79.89% | 14.34 | 20.65% | 4.89 | 23.84% | 5.41 | 26.89% | 10.88 | 24.27% | 2.60 |
| D70 | 68.66% | 8.32 | 22.56% | 4.79 | 34.40% | 5.78 | 25.63% | 6.09 | 30.97% | 6.26 |
| D91 | 86.35% | 28.75 | 72.75% | 25.57 | 61.69% | 9.05 | 45.29% | 9.90 | 56.75% | 12.21 |

**Example 4. *In vitro* inhibitory activities of siRNA conjugates modified with TBDMS at different sites against angiopoietin-like 3 (ANGPTL3) mRNA in mouse primary hepatocytes**

The present example evaluated inhibitory activities of RZ597065, etc. modified with TBDMS at different sites of a sense strand and RZ597084, etc. modified with TBDMS at different sites of an antisense strand, reference compound RZ597024 without TBDMS modification, and negative control compound RZ000002 without any known gene targeting against target gene ANGPTL3 in mouse primary hepatocytes by an *in vitro* mouse primary hepatocyte screening method.

1. Formulation of tested articles

Each of the above siRNA conjugates was centrifuged, and then dissolved by adding an appropriate amount of 1× PBS according to specification of each vial, to prepare a 20 μM stock solution, which was then further diluted with 1× PBS to render a 5 μM working solution.

2. Isolation of mouse primary hepatocytes

(1) Anesthesia: C57BL/6j mice were intraperitoneally injected with 10% chloral hydrate solution at 0.04 mL/10g.
(2) Perfusion: animals were secured and disinfected with 75% ethanol for abdomen and chest thereof. Abdomen was opened to find hepatic portal veins and inferior vena cavas. Indwelling needle was introduced from a lower end of the inferior vena cavas, and HBSS solution started to be perfused at a rate of 120 drops/min. The hepatic portal veins were cut, to confirm that perfusate flowed out of the cut hepatic portal veins. Infusion apparatus was well secured, and perfusion was continued for 4 minutes.
(3) Digestion of hepatocytes: the HBSS solution was changed into HBSS comprising 0.08% type IV collagenase (Collagenase from Clostridium histolyticum, Sigma), which was further perfused at a rate of 120 drops/min for 8 minutes.
(4) Isolation of primary hepatocytes: perfused livers were removed from the animals and shredded in sterile petri dish comprising 10 mL DMEM. Undigested hepatic tissues and connective tissues were filtered and removed by cytoscreener (75 μm). Cells were centrifuged, and resuspended and washed twice with DMEM. 10 mL of DMEM medium comprising 10% FBS was added for resuspension, and counting, to wait for an operation of free drug uptake.

3. Free uptake of siRNA conjugates:

Freshly isolated mouse primary hepatocytes were plated into type I collagen-coated 12-well cell culture plate (Corning Incorporated), comprising $5\times10^5$ viable cells and 2 mL of DMEM medium comprising 10% FBS in each well.
2 μL of the above 5 μM siRNA conjugate working solution was added into each cell well, and gently mixed to render a final concentration of 5 nM of the siRNA conjugate in each cell well. Blank control group was original mouse primary hepatocytes without any operation of free uptake of siRNA conjugate. Mouse primary hepatocytes having undergone free uptake treatment were further cultured in a 37 °C, 5% $CO_2$ incubator for 24 h.

4. Detection

RNA extraction: total RNAs in cells of various wells were extracted using full-automatic nucleic acid extractor and a nucleic acid extraction kit from Zhejiang Hanwei Science and Technology Co. Ltd. according to a method described in instructions.
Reverse transcription reaction: 1 μg of the total RNAs extracted from cells in each well were taken, and configured into 20 μL of reverse transcription system according to a method described in kit instructions and the reverse transcription reaction was completed by using a reverse transcription kit (Reverse Transcription System, A3500) from Promega company and selecting Oligo $(dT)_{15}$ reverse transcription primer. After the reaction was ended, 80 μL of RNase-Free water was added into the reverse transcription system to yield cDNA solution for Real-time PCR detection.
Real-time PCR detection: 20 μL of Real-time PCR reaction system per PCR detection well was configured according to a method described in kit instructions using SYBR™ Select Master Mix (Catalog number, 4472908) from ABI company. Each detection system comprised 5 μL of cDNA template obtained from the above reverse transcription reaction, 10 μL of SYBR™ Select Master Mix, 0.5 μL of 10 μM upstream primers, 0.5 μL of 10 μM downstream primers (see Table 9 for primer information), and 4 μL of RNase-Free $H_2O$. The configured reaction system was subjected to Real-time PCR amplification on ABI StepOnePlus PCR instrument by a three-step method, wherein an amplification procedure was pre-denaturation at 95 °C for 10 min, then denaturation at 95 °C for 30 s, annealing at 60 °C for 30 s, and extension at 72 °C

for 30 s; and processes of denaturation, annealing, and extension were repeated for 40 cycles. Gene expression differences were calculated by the above ΔΔCt method after completion of the procedure.

Table 9

| Target Point | Primer Type | Primer Sequence (5'-3') | SEQ ID NO: |
|---|---|---|---|
| ANGPTL3 | upstream primer | GAGGAGCAGCTAACCAACTTAAT | 399 |
| | downstream primer | TCTGCATGTGCTGTTGACTTAAT | 400 |
| GAPDH | upstream primer | TGCACCACCAACTGCTTAG | 401 |
| | downstream primer | GGATGCAGGGATGATGTTC | 402 |

5. Analysis of Results

Statistics was carried out respectively on relative expression levels (FIG. 4) and residual activity (Table 10) of target gene ANGPTL3 mRNA following free uptake of siRNAs modified with TBDMS at different sites of a sense strand by mouse primary hepatocytes. Results show that compared with the reference sequence (RZ597024) without TBDMS modification, TBDMS modification at position 5 (RZ597069), position 6 (RZ597070), position 12 (RZ597076), position 13 (RZ597077), and position 18 (RZ597082) of the sense strand was conducive to enhancing *in vitro* activity of siRNA sequences; and TBDMS modification at position 2 (RZ597066), position 3 (RZ597067), position 7 (RZ597071), position 8 (RZ597072), position 9 (RZ597073), and position 10 (RZ597074) of the sense strand significantly weakened the *in vitro* activity of the siRNA sequences.

Table 10

| Group | Residual Activity | ±STDEV Value |
|---|---|---|
| Blank | 100.00% | 8.83 |
| RZ000002 | 104.09% | 14.13 |
| RZ597024 | 18.26% | 4.41 |
| RZ597065 | 22.05% | 2.97 |
| RZ597066 | 37.16% | 9.95 |
| RZ597067 | 36.97% | 2.21 |
| RZ597068 | 20.50% | 2.85 |
| RZ597069 | 12.93% | 2.56 |
| RZ597070 | 16.55% | 5.90 |
| RZ597071 | 68.28% | 13.33 |
| RZ597072 | 45.95% | 9.77 |
| RZ597073 | 72.55% | 28.40 |
| RZ597074 | 52.65% | 7.89 |
| RZ597075 | 19.44% | 0.53 |
| RZ597076 | 15.12% | 0.68 |
| RZ597077 | 12.81% | 1.88 |
| RZ597078 | 23.03% | 3.75 |
| RZ597079 | 27.89% | 11.77 |
| RZ597080 | 24.63% | 14.70 |
| RZ597081 | 21.84% | 0.99 |
| RZ597082 | 12.96% | 1.60 |
| RZ597083 | 11.53% | 2.93 |

Statistics was carried out respectively on relative expression levels (FIG. 5) and residual activity (Table 11) of target gene ANGPTL3 mRNA following free uptake of siRNAs modified with TBDMS at different sites of an antisense strand by mouse primary hepatocytes. Results show that compared with the reference sequence (RZ597024) without TBDMS modification, position 8 (RZ597030), position 9 (RZ597031), position 10 (RZ597086), position 15 (RZ597091), position 20 (RZ597096), and position 21 (RZ597097) of the antisense strand was conducive to enhancing *in vitro* activity of siRNA sequences.

Table 11

| Group | Residual Activity | ±STDEV Value |
|---|---|---|
| Blank | 100.00% | 33.38 |
| RZ000002 | 95.70% | 34.93 |
| RZ597024 | 28.92% | 4.17 |
| RZ597084 | 44.74% | 14.86 |
| RZ597085 | 74.01% | 31.38 |
| RZ597025 | 57.95% | 1.85 |
| RZ597026 | 61.39% | 0.84 |
| RZ597027 | 34.93% | 14.14 |
| RZ597028 | 36.04% | 7.30 |
| RZ597029 | 28.29% | 10.81 |
| RZ597030 | 17.00% | 0.94 |
| RZ597031 | 17.00% | 0.52 |
| RZ597086 | 17.30% | 5.41 |
| RZ597087 | 30.07% | 9.39 |
| RZ597088 | 42.58% | 7.60 |
| RZ597089 | 49.49% | 4.79 |
| RZ597090 | 50.68% | 11.91 |
| RZ597091 | 22.68% | 3.81 |
| RZ597092 | 34.85% | 7.27 |
| RZ597093 | 36.96% | 10.01 |
| RZ597094 | 29.13% | 1.22 |
| RZ597095 | 33.20% | 14.25 |
| RZ597096 | 23.81% | 6.40 |
| RZ597097 | 23.80% | 9.93 |

Experimental conclusions suggested by experimental results in Example 4 are highly similar to those in Example 2, both indicating that the effect of TBDMS modification on siRNA sequence activity is associated with modification site, and that TBDMS bulky group modification at a specific site does not affect siRNA activity, and even may further elevate the activity.

**Example 5. Inhibitory activities of siRNA conjugates modified with TBDMS at different sites against ANGPTL3 mRNA in mice**

The present example evaluated inhibitory activities of siRNA conjugates RZ597026 and RZ597030 modified with TBDMS at different sites of an antisense strand and reference conjugate RZ597024 without TBDMS modification against a target gene ANGPTL3 in mice by an evaluation method for inhibitory activity against a target gene in mice.

6-8-week-old C57BL/6j mice were randomly divided into 4 groups in total based on body weight, 25 mice per group. Each group of mice received the above siRNA conjugates via abdominal subcutaneous administration, at a dose of

3 mg/kg per mouse, and an administration volume of 5 mL/kg. PBS control group received the same volume of siRNA conjugate-free PBS solution. The day of administration was recorded as the first day (D0). Five mice in each group were sacrificed respectively on D7, D14, D28 and D56 post-administration. The animals were subjected to gross anatomy, and liver tissues were collected and cut into a plurality of 2 $mm^3$ pieces which were stored in RNAlater. An appropriate amount of liver tissue samples were taken out from RNAlater, fragmented for 60 s in a Tissuelyser type II full-automatic tissue homogenizer, and subjected to RNA extraction, reverse transcription reaction and Real-time PCR detection according to the detection method described in Example 4. Relative quantitative calculation was performed for the target gene mRNA in various test groups by the preceding ΔΔCt method.

Statistics was carried out respectively on expression level of ANGPTL3 mRNA (FIG. 6) and residual activity of ANGPTL3 mRNA (Table 12) in mice post-administration. Experimental conclusion was similar to that of *in vitro* primary hepatocytes in Example 4. position 8 (RZ597030) of the antisense strand had good tolerance to TBDMS modification, and RZ597030 exhibited superior *in vivo* activity and sustained pharmacodynamic efficacy to the reference sequence (RZ597024) without TBDMS modification, while TBDMS modification at position 4 (RZ597026) of the antisense strand had a relatively great influence on *in vivo* activity of the sequence.

Table 12

| Collection Time | PBS | | RZ597024 | | RZ597026 | | RZ597030 | |
|---|---|---|---|---|---|---|---|---|
| | residual activity% | ±STDEV value | residual activity% | ±STDEV value | residual activity% | ±STDEV value | residual activity% | ±STDEV value |
| D0 | 100.00% | 0.00 | 100.00% | 0.00 | 100.00% | 0.00 | 100.00% | 0.00 |
| D7 | 99.29% | 22.56 | 17.73% | 10.91 | 55.68% | 27.88 | 2.98% | 1.30 |
| D14 | 97.35% | 14.70 | 18.08% | 9.52 | 63.15% | 24.32 | 3.11% | 1.20 |
| D28 | 115.32% | 25.16 | 17.54% | 9.43 | 63.42% | 31.54 | 4.44% | 0.82 |
| D56 | 117.02% | 64.52 | 103.98% | 26.72 | 77.70% | 43.25 | 32.97% | 6.13 |

The *in vivo* experimental results of Example 5 further verify that TBDMS modification on siRNA sequence activity is associated with modification site, and that TBDMS bulky group modification at a specific site does not affect siRNA activity, and even may further elevate the activity.

**Example 6. *In vitro* inhibitory activities of siRNA conjugates modified with TOM at different sites against coagulation factor XI (Factor XI, FXI) mRNA in mouse primary hepatocytes**

The inhibitory activities of the siRNA conjugates modified with TOM at different sites of an antisense strand against a target gene FXI in mouse primary hepatocytes were evaluated by the same experimental method as that in Example 4. Differences lie in that the siRNA conjugates modified with TOM at different sites of an antisense strand were used in the present example to replace the siRNA conjugates used in Example 4, and that target gene FXI detection primers listed in Table 13 were used in the present example to replace target gene detection primers used in Example 4. Relative quantitative calculation was performed for the target gene mRNA in various test groups by the preceding ΔΔCt method.

Table 13

| Target Point | Primer Type | Primer Sequence (5'-3') | SEQ ID NO: |
|---|---|---|---|
| FXI | upstream primer | GCCCTGTTAAAACTGGAATCAGC | 403 |
| | downstream primer | GCCCTGTTAAAACTGGAATCAGC | 404 |
| GAPDH | upstream primer | TGCACCACCAACTGCTTAG | 405 |
| | downstream primer | GGATGCAGGGATGATGTTC | 406 |

Statistics was carried out respectively on relative expression levels of the target gene FXI mRNA (FIG. 7) and residual activity of FXI mRNA (Table 14) following free uptake of siRNAs modified with TOM at different sites of a sense strand by mouse primary hepatocytes. Results show that compared with the reference sequence (RZ594001) without TOM modification, TOM modification at sense strand position 5 (RZ594011), position 6 (RZ594012), position 12 (RZ594018), position 13 (RZ594019), position 18 (RZ594024), and position 19 (RZ594025) was conducive to enhancing

*in vitro* activity of siRNA sequences; and TOM modification at sense strand position 2 (RZ594008), position 3 (RZ594009), position 7 (RZ594013), position 8 (RZ594014), position 9 (RZ594015), and position 10 (RZ594016) significantly weakened the *in vitro* activity of the siRNA sequences.

Table 14

| Group | Residual Activity | ±STDEV Value |
|---|---|---|
| Blank | 98.20% | 0.05 |
| RZ000002 | 100.00% | 0.11 |
| RZ594001 | 45.06% | 0.10 |
| RZ594007 | 58.21% | 0.11 |
| RZ594008 | 67.51% | 0.00 |
| RZ594009 | 74.34% | 0.40 |
| RZ594010 | 49.30% | 0.21 |
| RZ594011 | 38.92% | 0.06 |
| RZ594012 | 19.02% | 0.07 |
| RZ594013 | 72.04% | 0.11 |
| RZ594014 | 88.06% | 0.05 |
| RZ594015 | 91.68% | 0.19 |
| RZ594016 | 95.24% | 0.13 |
| RZ594017 | 46.44% | 0.03 |
| RZ594018 | 29.60% | 0.03 |
| RZ594019 | 35.99% | 0.06 |
| RZ594020 | 58.41% | 0.07 |
| RZ594021 | 50.19% | 0.03 |
| RZ594022 | 77.93% | 0.14 |
| RZ594023 | 66.24% | 0.22 |
| RZ594024 | 31.93% | 0.07 |
| RZ594025 | 40.17% | 0.14 |

Statistics was carried out respectively on relative expression levels of the target gene FXI mRNA (FIG. 8) and residual activity of FXI mRNA (Table 15) following free uptake of siRNAs modified with TOM at different sites of the antisense strand by mouse primary hepatocytes. Compared with the reference sequence (RZ594001) without TOM modification, antisenses trand position 7 (RZ594004), position 8 (RZ594005), position 9 (RZ594006), position 10 (RZ594030), position 15 (RZ594035), position 20 (RZ594040), and position 21 (RZ594041) were conducive to enhancing *in vitro* activity of siRNA sequences.

Table 15

| Group | Residual Activity | ±STDEV Value |
|---|---|---|
| Blank | 112.17% | 0.15 |
| RZ000002 | 100.00% | 0.16 |
| RZ594001 | 44.17% | 0.08 |
| RZ594026 | 57.92% | 0.04 |
| RZ594027 | 73.82% | 0.17 |
| RZ594028 | 57.92% | 0.04 |
| RZ594029 | 73.82% | 0.17 |

(continued)

| Group | Residual Activity | ±STDEV Value |
|---|---|---|
| RZ594002 | 62.97% | 0.08 |
| RZ594003 | 65.46% | 0.09 |
| RZ594004 | 37.32% | 0.01 |
| RZ594005 | 31.60% | 0.07 |
| RZ594006 | 41.14% | 0.05 |
| RZ594030 | 35.51% | 0.06 |
| RZ594031 | 59.62% | 0.01 |
| RZ594032 | 59.37% | 0.07 |
| RZ594033 | 74.85% | 0.18 |
| RZ594034 | 83.68% | 0.09 |
| RZ594035 | 31.89% | 0.02 |
| RZ594036 | 73.49% | 0.07 |
| RZ594037 | 62.44% | 0.12 |
| RZ594038 | 61.86% | 0.09 |
| RZ594039 | 43.47% | 0.02 |
| RZ594040 | 39.27% | 0.02 |
| RZ594041 | 37.18% | 0.03 |

Results of Example 6 indicate that the effect of TOM modification on siRNA sequence activity is associated with modification site, and that TOM bulky group modification at a specific site does not affect siRNA activity, and even may further elevate the activity, with the effect mode highly similar to that of the TOM modification.

**Example 7. Inhibitory activities of siRNA conjugates modified with TOM at different sites against CC3 mRNA in mice**

The present example evaluated inhibitory activities of conjugate RZRZ502013 modified with TOM at position 19 of a sense strand, conjugate RZ502016 modified with TOM at position 10 of an antisense strand, and reference conjugate RZ002001 without TOM modification against a target gene CC3 in mice by an evaluation method for inhibitory activity against a target gene in mice.

6-8-week-old C57BL/6j mice were randomly divided into 4 groups in total based on body weight, 5 mice per group. Each group of mice received the above siRNA conjugates via abdominal subcutaneous administration, at a dose of 3 mg/kg per mouse, and an administration of 5 mL/kg. PBS control group received the same volume of siRNA conjugate-free PBS solution. The day of administration was recorded as the first day (D0), and the mice were sacrificed on D7 post-administration. The animals were subjected to gross anatomy, and liver tissues were collected and cut into a plurality of 2 $mm^3$ pieces which were stored in RNAlater. An appropriate amount of liver tissue samples were taken out from RNAlater, fragmented for 60 s in a Tissuelyser type II full-automatic tissue homogenizer, and subjected to RNA extraction, reverse transcription reaction and Real-time PCR detection by the detection method described in Example 2. Relative quantitative calculation was performed for the target gene mRNA in various test groups by the preceding $\Delta\Delta$Ct method. A difference lies in that the siRNA conjugates modified with TOM were used in the present example to replace the siRNA conjugates used in Example 4. Relative quantitative calculation was performed for the target gene mRNA in various test groups by the preceding $\Delta\Delta$Ct method.

Statistics was carried out respectively on expression level of CC3 mRNA (FIG. 9) and residual activity of CC3 mRNA (Table 16) in mice post-administration. Results show that siRNA conjugate RZ502013 modified with TOM at position 19 on the siRNA sense strand exhibited higher *in vivo* activity (D7) than the reference conjugate RZ002001, and RZ502016 modified with TOM at position 10 of the antisense strand had comparable *in vivo* activity to the reference conjugate RZ002001.

Table 16

| Group | Residual Activity | ±STDEV Value |
|---|---|---|
| PBS | 100.00% | 20.66 |
| RZ002001 | 27.97% | 4.37 |
| RZ502013 | 17.28% | 5.63 |
| RZ502016 | 27.05% | 2.54 |

The *in vivo* results of Example 7 indicate that TOM bulky group modification at specific site does not affect siRNA activity, and even may further elevate the activity.

**Example 8. Inhibitory activities of siRNA conjugates modified with MOE at different sites against CC3 mRNA in HepG2 cells**

*In vitro* activities of siRNA compounds, such as RX502165 modified with MOE at different sites of a sense strand and RX502172 modified with MOE at different sites of an antisense strand, and a reference compound RX502001 without MOE modification on HepG2 cells were evaluated using the same experimental method as that in Example 2. A difference lies in that the siRNA compounds modified with MOE at different sites of the sense and antisense strands were used in the present example to replace the siRNA compounds used in Example 2. Relative quantitative calculation was performed for the target gene mRNA in various test groups by the preceding ΔΔCt method.
Results of Example 8 indicate that the effect of MOE modification on siRNA sequence activity is associated with modification site, and that MOE group modification at specific site does not affect siRNA activity, and even may further elevate the activity, with an effect mode highly similar to those of TBDMS modification and TOM modification.
Statistics was carried out respectively on relative expression levels of target gene CC3 mRNA (FIG. 10) and residual activity of CC3 mRNA (Table 17) after HepG2 was transfected by siRNAs modified with MOE at different sites of the sense strand. Results show that compared with the reference sequence (RX502001) without MOE modification, MOE modification at sense strand position 5 (RZ594011), position 12 (RX502115), position 18 (RX502116), and position 19 (RX502117) was conducive to elevating the *in vitro* activity of siRNA sequences; and MOE modification at sense strand position 7 (RX502166), position 8 (RX502167), position 9 (RX502168), and position 10 (RX502169) significantly weakened the *in vitro* activity of the siRNA sequences.

Table 17

| Group | Residual Activity | ±STDEV Value |
|---|---|---|
| BLANK | 123.72% | 27.50 |
| MOCK | 100.00% | 3.26 |
| RX502001 | 32.73% | 10.04 |
| RX502165 | 28.12% | 0.16 |
| RX502166 | 56.98% | 1.16 |
| RX502167 | 71.83% | 14.09 |
| RX502168 | 50.68% | 17.54 |
| RX502169 | 38.27% | 0.70 |
| RX502115 | 30.07% | 1.56 |
| RX502116 | 27.65% | 0.90 |
| RX502117 | 27.81% | 0.86 |

Statistics was carried out respectively on relative expression levels of target gene CC3 mRNA (FIG. 11) and residual activity of CC3 mRNA (Table 18) after HepG2 was transfected by siRNAs modified with MOE at different sites of the antisense strand. Results show that compared with the reference sequence without MOE modification (RX502001), antisense strand position 8 (RX502118), position 10 (RX502119), and position 15 (RX502120) were conducive to elevating the *in vitro* activity of the siRNA sequences.

Table 18

| Group | Residual Activity | ±STDEV Value |
|---|---|---|
| BLANK | 123.72% | 27.50 |
| MOCK | 100.00% | 3.26 |
| RX502001 | 37.72% | 2.97 |
| RX502172 | 80.25% | 6.90 |
| RX502173 | 50.83% | 26.06 |
| RX502118 | 28.05% | 1.96 |
| RX502119 | 31.74% | 2.80 |
| RX502177 | 72.15% | 2.17 |
| RX502120 | 27.73% | 2.84 |
| RX502178 | 42.07% | 5.89 |

Results of Example 8 indicate that the effect of MOE modification on the siRNA sequence activity is associated with modification site, and that MOE group modification at specific site does not affect the siRNA activity, and even may further elevate the activity, with an effect mode highly similar to those of TBDMS modification and TOM modification.

**Example 9. Inhibitory activities of conjugates modified with MOE at multiple sites against CC3 mRNA in HepG2 cells**

*In vitro* activities of siRNA compounds modified with MOE at multiple sites of a sense strand such as RX502123, siRNA compounds modified with MOE at multiple sites of an antisense strand such as RX502130, compounds modified with MOE an both sense strand and antisense strand such as RX502146, a reference compound RX502001 without MOE modification, and a negative control compound RZ000002 not targeting any known target point on HepG2 cells were evaluated using the same experimental method as that in Example 2. A difference lies in that the above siRNA compounds modified with MOE on the sense strand and/or antisense strand were used in the present example to replace the siRNA compounds used in Example 2. Relative quantitative calculation was performed for the target gene mRNA in various test groups by the preceding $\Delta\Delta$Ct method.

Statistics was carried out respectively on relative expression levels of target gene CC3 mRNA (FIG. 12) and residual activity of CC3 mRNA (Table 19) after HepG2 was transfected by siRNAs modified with MOE at multiple sites of the sense strand. Results show that combined MOE modification at multiple sites of position 6, position 12, position 18, and position 19 of the sense strand exhibited comparable *in vitro* activity to the siRNA reference sequence (RX502001), wherein the compound RX502126 modified with MOE at both position 12 and position 18 of the sense strand had slightly higher *in vitro* activity than the referece sequence RX502001.

Table 19

| Group | Residual Activity | ±STDEV Value |
|---|---|---|
| Blank | 109.24% | 14.81 |
| Mock | 100.00% | 4.74 |
| RZ000002 | 101.95% | 7.63 |
| RX502001 | 50.48% | 0.58 |
| RX502123 | 49.14% | 3.34 |
| RX502124 | 48.82% | 1.50 |
| RX502125 | 49.56% | 5.91 |
| RX502126 | 39.57% | 3.84 |
| RX502127 | 51.46% | 3.02 |
| RX502128 | 47.60% | 2.17 |

Statistics was carried out respectively on relative expression levels of target gene CC3 mRNA (FIG. 13) and residual activity of CC3 mRNA (Table 20) after HepG2 was transfected by siRNAs modified with MOE at multiple sites of the antisense strand. Combined MOE modification at multiple sites of antisense strand position 8, position 10, position 15, position 20, and position 21 exhibited comparable *in vitro* activity to the siRNA reference sequence (RX502001) without MOE modification, wherein multiple modified sequences such as the compound RX502130 modified with MOE at both position 8 and position 15 of the sense strand had slightly higher *in vitro* activity than the referece sequence RX502001.

Table 20

| Group | Residual Activity | ±STDEV Value |
|---|---|---|
| Blank | 110.50% | 10.43 |
| Mock | 100.00% | 6.61 |
| RZ000002 | 107.76% | 1.18 |
| RX502001 | 42.58% | 3.88 |
| RX502130 | 36.09% | 5.96 |
| RX502131 | 38.52% | 0.38 |
| RX502132 | 46.57% | 2.73 |
| RX502134 | 41.29% | 3.98 |
| RX502135 | 46.41% | 5.68 |
| RX502136 | 36.99% | 6.09 |
| RX502138 | 40.82% | 0.30 |
| RX502139 | 39.03% | 5.04 |
| RX502140 | 42.50% | 11.02 |

Statistics was carried out respectively on relative expression levels of target gene CC3 mRNA (FIG. 14) and residual activity of CC3 mRNA (Table 21) after HepG2 was transfected by siRNAs modified with MOE at multiple sites of the sense strand and the antisense strand. Results show that compounds modified with MOE at sense strand position 6, position 12, and position 18 and antisense strand position 8, position 10, position 15, position 20, and position 21 in combination exhibited comparable *in vitro* activity to the siRNA reference sequence (RX502001) without MOE modification, wherein multiple modified sequences, such as compound RX502151 modified with MOE at both position 18 of the sense strand and position 15 of the antisense strand, compound RX502154 modified with MOE simultaneously at position 6 and position 12 of the sense strand and position 8 and position 15 of the antisense strand, compound RX502156 modified with MOE simultaneously at position 6 and position 18 of the sense strand and position 8 and position 15 of the antisense strand, compound RX502158 modified with MOE simultaneously at position 12 and position 18 of the sense strand and position 8 and position 15 of the antisense strand, had slightly higher *in vitro* activity than the referece sequence RX502001. Even MOE modification (RX502164) simultaneously at 8 sites, i.e., position 6, position 12, and position 18 of the sense strand and position 8, position 10, position 15, position 20, and position 21 of the antisense strand, still can well maintain sequence *in vitro* activity.

Table 21

| Group | Residual Activity | ±STDEV Value |
|---|---|---|
| Blank | 116.68% | 22.21 |
| Mock | 100.00% | 8.37 |
| RZ000002 | 101.23% | 0.18 |
| RX502001 | 52.76% | 3.37 |
| RX502146 | 55.37% | 2.44 |
| RX502147 | 49.71% | 9.00 |
| RX502150 | 46.92% | 8.12 |
| RX502151 | 44.70% | 0.10 |

(continued)

| Group | Residual Activity | ±STDEV Value |
|---|---|---|
| RX502152 | 53.10% | 2.55 |
| RX502153 | 60.77% | 2.75 |
| RX502154 | 42.19% | 1.00 |
| RX502155 | 58.49% | 5.70 |
| RX502156 | 39.78% | 0.68 |
| RX502157 | 58.00% | 3.86 |
| RX502158 | 43.32% | 0.36 |
| RX502159 | 54.69% | 2.06 |
| RX502160 | 48.33% | 1.47 |
| RX502162 | 52.14% | 4.40 |
| RX502163 | 49.89% | 4.75 |
| RX502164 | 54.06% | 0.41 |

Results of Example 9 indicate that the effect of MOE modification on siRNA sequence activity is associated with modification site, and that MOE modification at single or multiple sites among specific sites of the sense strand and/or antisense strand can have good tolerance, and still can well maintain or even further elevate the siRNA sequence activity.

**Example 10. Inhibitory activities of siRNA conjugates modified with MOE at different sites of sense strand and/or antisense strand against CC3 mRNA in mice**

Inhibitory activities of siRNA conjugates modified with MOE at different sites of a sense strand and/or an antisense strand, and a reference conjugate RZ502031 without MOE modification against target gene CC3 in mice were evaluated using the same experimental method as that in Example 7. A difference lies in that the siRNA compounds modified with MOE on the sense strand and/or the antisense strand were used in the present example to replace the siRNA compounds used in Example 7. Relative quantitative calculation was performed for the target gene mRNA in various test groups by the preceding ΔΔCt method. RNA extraction, reverse transcription reaction and Real-time PCR detection were completed according to the detection method described in Example 2, and relative quantitative calculation was performed for the target gene mRNA in various test groups by the preceding ΔΔCt method. Relative quantitative calculation was performed for the target gene mRNA in various test groups by the preceding ΔΔCt method.

Statistics was carried out respectively on expression levels of the CC3 mRNA (FIGS. 15 and 16) and residual activity of CC3 mRNA (Tables 22 and 23) in mice post-administration (the siRNA conjugates of the present example). Results show that multi-site MOE modification on specific sites of the sense strand and/or antisense strand can still well maintain or even further elevate the *in vivo* activity of siRNA sequences. The siRNA conjugate RZ502039 modified with MOE simultaneously at position 6 and position 18 of the siRNA sense strand and position 8 and position 15 of the antisense strand, and the siRNA conjugate RZ502036 with modified MOE at position 12 and position 18 of the sense strand and position 15 of the antisense strand had higher *in vivo* activity (D21) than the reference conjugate RZ502031. However, MOE modification at position 14 of the antisense strand (RZ502087) remarkably affected the *in vivo* activity of the siRNA.

Table 22

| Group | Residual Activity | ±STDEV Value |
|---|---|---|
| PBS | 100.00% | 38.33 |
| RZ502031 | 37.42% | 11.14 |
| RZ502033 | 32.38% | 9.28 |
| RZ502034 | 33.83% | 5.89 |
| RZ502035 | 34.73% | 2.74 |
| RZ502039 | 30.36% | 13.58 |

Table 23

| Group | Residual Activity | ±STDEV Value |
|---|---|---|
| PBS | 100.00% | 18.16 |
| RZ502031 | 50.99% | 9.23 |
| RZ502036 | 37.73% | 5.66 |
| RZ502049 | 59.94% | 6.06 |
| RZ502087 | 127.94% | 25.65 |

Results of Example 10 indicate that the effect of MOE modification on siRNA sequence activity is associated with modification site, and that MOE modification at single or multiple sites among specific sites of the sense strand and/or antisense strand can have good tolerance, and still can well maintain or even further elevate the siRNA sequence activity. But for some other sites, even the MOE modification at a single site will remarkably affect the siRNA sequence activity.

**Example 11. Inhibitory activities of siRNA conjugates modified with MOE at different sites of sense strand and/or antisense strand against SOD1 mRNA in mice**

Inhibitory activities of siRNA conjugates modified with MOE at different sites of a sense strand and/or an antisense strand, and a reference conjugate RZ599001 without MOE modification against target gene SOD1 in mice were evaluated using the same experimental method as that in Example 7. A difference lies in that the siRNA compounds modified with MOE on the sense strand and/or the antisense strand were used in the present example to replace the siRNA compounds used in Example 7. Relative quantitative calculation was performed for the target gene mRNA in various test groups by the preceding ΔΔCt method. RNA extraction, reverse transcription reaction and Real-time PCR detection were completed according to the detection method described in Example 2, and relative quantitative calculation was performed for the target gene mRNA in various test groups by the preceding ΔΔCt method. Relative quantitative calculation was performed for the target gene mRNA in various test groups by the preceding ΔΔCt method.

Statistics was carried out respectively on expression levels (FIG. 17) and residual activity (Table 24) of SOD1 mRNA in mice on the 7$^{th}$ day and the 21$^{st}$ day post-administration. Results indicate that the effect of MOE modification on siRNA sequence activity is associated with modification site, and that MOE modification at single or multiple sites among specific sites of the sense strand and/or antisense strand can have good tolerance, and still can well maintain or even further elevate the siRNA sequence activity.

Table 24

| Group | D7 | | D21 | |
|---|---|---|---|---|
| | residual activity | ±STDEV value | residual activity | ±STDEV value |
| PBS | 100.00% | 13.67 | 100.00% | 10.42 |
| RZ599001 | 30.51% | 11.31 | 36.61% | 7.30 |
| RZ599015 | 23.39% | 4.39 | 27.50% | 1.98 |
| RZ599016 | 26.46% | 6.31 | 38.86% | 9.78 |
| RZ599017 | 23.49% | 3.28 | 25.19% | 7.93 |
| RZ599018 | 16.60% | 4.29 | 30.27% | 4.74 |
| RZ599019 | 32.79% | 9.07 | 47.61% | 10.13 |
| RZ599020 | 32.00% | 6.06 | 37.10% | 5.48 |

**Example 12. Evaluation of *in vitro* activity of siRNA conjugates of different modification schemes against target gene C3 in HepG2 cells**

The present example comparatively evaluated inhibitory activities of various siRNA conjugates derived from different modification schemes of RZ002003, RZ002006, RZ002011 and RZ002031 on target gene C3 mRNA in cells of human hepatocarcinoma cell line HepG2.

Formulation of tested articles:

Each of the above siRNA tested articles was centrifuged, and then dissolved by adding an appropriate amount of PBS according to specification of each vial, to prepare a 20 μM stock solution, which was then further subjected to gradient dilution with PBS into 0.5 μM and 0.05 μM working solutions. Dose experiments were performed at final duplex concentrations 5 nM and 0.5 nM.

96-well transfection and detection:

HepG2 cells grown to near confluency were digested with trypsin, and the cells were washed to prepare a cell suspension. 100 μL of the cell suspension was added to each well of a 96-well plate, in which the number of cells per well was 12,000, and the cells were cultured in a 37 °C, 5% $CO_2$ incubator. When the cells were adhered to walls for 24 h, DMEM medium in the 96-well plate was aspirated and discarded, 80 μL of Opti-MEM™ medium was added to each well, and then the 96-well plate was placed in the incubator to continue culturing. 1 μL of 0.1 μM and 0.01 μM working solutions were dispersed in 9 μL of Opti-MEM to form siRNA mixtures, 0.3 μL of RNAiMAX was dispersed in 9.7 μL of Opti-MEM, and well mixed with each siRNA mixture to form transfection complexes. The transfection complexes were incubated at room temperature for 10 minutes, and then the transfection complexes were added to the 96-well plate, at 20 μL per well. After culturing for 4 h, each well was supplemented with 100 μL of DMEM medium comprising 20% FBS, and the 96-well plate was placed in the incubator to continue culturing for 24 h. Herein, for Mock control group: 0.3 μL of RNAiMAX was dispersed in 9.7 μL of Opti-MEM, and then added with 10 μL of Opti-MEM, followed by incubation at room temperature for 10 minutes; then resultant was added to the 96-well plate, at 20 μL per well. After culturing for 4 h, each well was supplemented with 100 μL of DMEM medium containing 20% FBS, and the 96-well plate was placed in the incubator to continue culturing for 24 h.

The 96-well plate was taken out, and total RNAs were extracted using a full-automatic nucleic acid extractor (purchased from Zhejiang Hanwei Science and Technology Co. Ltd.) and a nucleic acid extraction kit (purchased from Zhejiang Hanwei Science and Technology Co. Ltd., GO-MNTR-100) according to standard operation steps of total RNA extraction.

A reverse transcription kit (Thermo Fisher Scientific company, RevertAid First Strand cDNA Synthesis Kit, K1622) was used and Oligo (dT)18 reverse transcription primer was selected, to configure 20 μL of reverse transcription system according to a method described in kit instructions and complete the reverse transcription reaction. Next, expression of target gene mRNA in HepG2 cells was detected on a fluorescence quantitative PCR instrument (Bio-Rad, CFX Opus 384) using a real-time fluorescent quantitative PCR kit (Thermo Fisher Scientific, TaqMan Fast Advanced Master Mix, 4444557). In the real-time fluorescence quantitative PCR method, a glyceraldehyde-3-phosphate dehydrogenase (GAPDH) gene was taken as an internal reference gene, and primers for the target gene and primers for the GAPDH internal reference gene were used for detecting the target gene and the GAPDH internal reference gene, respectively. Sequences of the detection primers were listed in Table 25.

Table 25

| Gene | | Primer Type | Primer Sequence | Fluorescent Group | SEQ ID NO: |
|---|---|---|---|---|---|
| Target gene | C3 | upstream primer | 5'-AGTCTCCTGCTTTAGTGATGC-3' | / | 581 |
| | | downstream primer | 5'-GCCTTTGTTCTCATCTCGCT-3' | / | 582 |
| | | probe primer | 5'-CTGTTGACCTGCTCCTCGCAAATATCT-3' | 5'FAM; 3'MGB | 583 |
| Internal Reference Gene | GAPDH | upstream primer | 5'-AAGAAGGTGGTGAAGCAGG-3' | / | 584 |
| | | downstream primer | 5'-CAAAGTGGTCGTTGAGGG-3' | / | 585 |
| | | probe primer | 5'-CAACAGCGACACCCACTC-3' | 5'VIC; 3'MGB | 586 |

10 μL Real-time PCR reaction system was configured in each PCR detection well according to the method described in instructions of the real-time fluorescence quantitative PCR kit. Each reaction system comprised 4 μL of cDNA solution obtained by the above reverse transcription reaction, 5 μL of TaqMan™ Fast Advanced Master Mix (2×), 0.15 μL of 10 μM upstream primer, 0.15 μL of 10 μM downstream primer, 0.15 μL of 10 μM probe primer, and 0.55 μL of RNase-Free H2O. The configured reaction system was subjected to Real-time PCR amplification on a real-time fluorescence quantitative PCR instrument (Bio-Rad, CFX Opus 384) by a two-step method, wherein an amplification procedure was 50 °C for 2 min, then pre-denaturation at 95 °C for 20 s, denaturation at 95 °C for 3 s, annealing at 60 °C and extension for 30 s, and processes of denaturation, annealing and extension were repeated for 40 cycles. In the real-time fluorescence quantitative PCR method, relative quantitative calculation of expression levels and inhibition rates of target gene C3 mRNA in various test groups were performed by the preceding ΔΔCt method.

Results of Example 12 indicate that at doses of 5 nM and 0.5 nM, siRNAs derived from different modification schemes of RZ002003, RZ002006, RZ002011 and RZ002031 could significantly inhibit the expression of C3 gene mRNA in HepG2 cells and had higher *in vitro* activity than original sequences RZ002003, RZ002006, RZ002011 and RZ002031 (FIGS. 18a, 18b, 18c, and 18d; and Tables 26a, 26b, 26c, and 26d).

Table 26a

| Group | 5 nM | | 0.5 nM | |
|---|---|---|---|---|
| | residual activity% | ±STDEV value | residual activity% | ±STDEV value |
| Mock | 100.08 | 5.72 | N/A | N/A |
| RZ002003 | 17.22 | 2.77 | 54.38 | 3.56 |
| RZ002050 | 17.66 | 2.29 | 36.57 | 2.38 |
| RZ002051 | 16.28 | 2.17 | 27.45 | 6.33 |
| RZ002052 | 13.93 | 1.18 | 30.06 | 0.72 |
| RZ002053 | 14.89 | 4.02 | 26.02 | 0.90 |
| RZ002055 | 15.59 | 2.72 | 24.55 | 2.19 |
| RZ002058 | 14.76 | 0.11 | 31.74 | 1.35 |
| RZ002059 | 11.29 | 1.47 | 26.65 | 4.85 |
| RZ002060 | 10.29 | 1.48 | 25.95 | 0.70 |

Table 26b

| Group | 5 nM | | 0.5 nM | |
|---|---|---|---|---|
| | residual activity% | ±STDEV value | residual activity% | ±STDEV value |
| biaoMock | 100.02 | 3.12 | N/A | N/A |
| RZ002006 | 12.47 | 0.29 | 44.20 | 7.28 |
| RZ002033 | 9.16 | 0.94 | 31.05 | 0.71 |
| RZ002034 | 8.80 | 0.76 | 24.49 | 6.22 |
| RZ002035 | 7.65 | 1.92 | 32.11 | 1.97 |
| RZ002036 | 11.99 | 0.18 | 24.01 | 1.03 |
| RZ002037 | 11.26 | 0.61 | 27.13 | 0.60 |
| RZ002038 | 9.04 | 1.21 | 30.72 | 0.51 |
| RZ002041 | 10.48 | 1.47 | 27.69 | 6.13 |
| RZ002042 | 9.07 | 0.71 | 26.13 | 1.61 |
| RZ002043 | 8.04 | 0.71 | 30.69 | 0.16 |

Table 26c

| Group | 5 nM | | 0.5 nM | |
|---|---|---|---|---|
| | residual activity% | ±STDEV value | residual activity% | ±STDEV value |
| Mock | 100.08 | 5.65 | N/A | N/A |
| RZ002011 | 27.09 | 1.06 | 47.46 | 6.82 |
| RZ002082 | 21.94 | 5.23 | 28.47 | 2.52 |
| RZ002083 | 19.50 | 8.69 | 25.41 | 1.19 |
| RZ002084 | 15.72 | 8.18 | 27.87 | 2.26 |
| RZ002085 | 23.29 | 5.75 | 33.23 | 2.55 |
| RZ002086 | 21.13 | 8.66 | 34.13 | 6.23 |
| RZ002087 | 21.79 | 0.85 | 36.56 | 3.15 |
| RZ002091 | 11.74 | 0.65 | 36.05 | 0.00 |
| RZ002092 | 11.68 | 1.28 | 37.75 | 0.36 |

Table 26d

| Group | 5 nM | | 0.5 nM | |
|---|---|---|---|---|
| | residual activity% | ±STDEV value | residual activity% | ±STDEV value |
| Mock | 100.02 | 2.98 | N/A | N/A |
| RZ002031 | 28.18 | 0.90 | 54.75 | 6.16 |
| RZ002067 | 16.90 | 4.88 | 27.45 | 1.50 |
| RZ002068 | 15.99 | 9.04 | 38.28 | 2.66 |
| RZ002069 | 18.45 | 5.73 | 31.23 | 2.43 |
| RZ002070 | 16.78 | 8.11 | 34.34 | 1.93 |
| RZ002071 | 16.24 | 5.09 | 32.75 | 1.91 |

**Example 13. Evaluation of *in vitro* activity of siRNA conjugates of different modification schemes against target gene angiotensinogen (AGT) in Huh7 cells**

The present example comparatively evaluated inhibitory activities of various siRNA conjugates derived from different modification schemes of RZ003017 and RZ003020 against target gene AGT mRNA in cells of human hepatocarcinoma cell line Huh7.

Formulation of tested articles:

Each of the above siRNA tested articles was centrifuged, and then dissolved by adding an appropriate amount of PBS according to specification of each vial, to prepare a 20 μM stock solution, which was then further subjected to gradient dilution with PBS into 0.1 μM and 0.01 μM working solutions. Dose experiments were performed at final duplex concentrations 1 nM and 0.1 nM.

96-well transfection and detection:

Huh7 cells grown to near confluency were digested with trypsin, and the cells were washed to prepare a cell suspension. 100 μL of the cell suspension was added to each well of a 96-well plate, in which the number of cells per well was 12,000, and the cells were cultured in a 37 °C, 5% $CO_2$ incubator. When the cells were adhered to walls for 24 h, DMEM medium in the 96-well plate was aspirated and discarded, 80 μL of Opti-MEM™ medium was added to each well, and then the 96-well plate was placed in the incubator to continue culturing. 1 μL of 0.1 μM and 0.01 μM working solutions were dispersed in 9 μL of Opti-MEM to form siRNA mixtures, 0.3 μL of RNAiMAX was dispersed in 9.7 μL of Opti-MEM, and well mixed with each siRNA mixture to form transfection complexes. The transfection complexes were incubated at room

temperature for 10 minutes, and then the transfection complexes were added to the 96-well plate, at 20 μL per well. After culturing for 4 h, each well was supplemented with 100 μL of DMEM medium containing 20% FBS, and the 96-well plate was placed in the incubator to continue culturing for 24 h. Herein, for Mock control group: 0.3 μL of RNAiMAX was dispersed in 9.7 μL of Opti-MEM, and then added with 10 μL of Opti-MEM, followed by incubation at room temperature for 10 minutes; then resultant was added to the 96-well plate, at 20 μL per well. After culturing for 4 h, each well was supplemented with 100 μL of DMEM medium containing 20% FBS, and the 96-well plate was placed in the incubator to continue culturing for 24 h. RNA extraction, reverse transcription reaction, and fluorescence quantitative PCR operations were as described in Example 12. In the real-time fluorescence quantitative PCR method, relative quantitative calculation of expression levels and inhibition rates of the target gene mRNA in various test groups was performed by the preceding ΔΔCt method. Sequences of detection primers were listed in Table 27.

Table 27

| Gene | | Primer Type | Primer Sequence | Fluorescent Group | SEQ ID NO: |
|---|---|---|---|---|---|
| **Target gene** | AGT | upstream primer | 5'-GTATGTACACCCGGTCACC-3' | / | 587 |
| | | downstream primer | 5'-GTTGTTCTGGGTACTACAGCA-3' | / | 588 |
| | | probe primer | 5'-CATCCTCTGCCTCCTGGCCTG-3' | 5'FAM; 3'MGB | 589 |
| **Internal Reference Gene** | GAPDH | upstream primer | 5'-AAGAAGGTGGTGAAGCAGG-3' | / | 590 |
| | | downstream primer | 5'-CAAAGTGGTCGTTGAGGG-3' | / | 591 |
| | | probe primer | 5'-CAACAGCGACACCCACTC-3' | 5'VIC; 3'MGB | 592 |

Results of Example 13 indicate that at doses of 1 nM and 0.1 nM, siRNAs derived from different modification schemes of RZ003017 and RZ003020 could significantly inhibit the expression of AGT gene mRNA in HepG2 cells and had comparable or higher *in vitro* activity compared with original sequences RZ003017 and RZ003020 (FIG. 19 and Table 28).

Table 28

| Group | 1 nM | | 0.1 nM | |
|---|---|---|---|---|
| | residual activity% | ±STDEV value | residual activity% | ±STDEV value |
| Mock | 100.07 | 4.01 | | |
| RZ003017 | 3.52 | 1.39 | 18.74 | 4.23 |
| RZ003028 | 4.17 | 1.35 | 9.08 | 0.69 |
| RZ003029 | 3.98 | 0.27 | 15.75 | 0.34 |
| RZ003031 | 3.19 | 0.21 | 17.32 | 0.18 |
| RZ003032 | 4.17 | 1.15 | 15.35 | 1.91 |
| RZ003020 | 6.55 | 0.77 | 31.73 | 5.79 |
| RZ003041 | 4.77 | 0.27 | 20.14 | 1.25 |
| RZ003042 | 3.92 | 0.84 | 20.76 | 0.43 |

**Example 14. Evaluation of *in vivo* protein-lowering effect of siRNA conjugates of different modification schemes in hREN×hAGT hypertensive mice**

The present example measured effect of reference siRNA conjugates RZ003021, RZ003065, and RZ 003066 on AGT protein expression in serum of hREN×hAGT hypertensive mice at different time points after a single administration using an enzyme-linked immunosorbent assay (ELISA).

Animal grouping, administration and tissue sample collection:

9-11-week-old hREN×hAGT hypertensive mice (purchased from Cyagen Biosciences (Suzhou) Inc.) were grouped based on systolic pressure levels (all males). Each test group received a pre-determined dose of a drug conjugate and a PBS control group was added. All mice received a single subcutaneous abdominal injection at a dose calculated according to body weight. Each drug conjugate was administered as a PBS solution at 0.3 mg/mL (based on siRNA), and an administration volume was 10 mL/kg mouse body weight, that is, each drug conjugate was administered at a dose of 3 mg/kg mouse body weight (based on siRNA). PBS control group was administered the same volume of PBS solution (without drug conjugate). Serum of all groups of mice was collected before administration (recorded as pre-dose), on the day of administration recorded as the 0$^{th}$ Day (recorded as D0), and on the 7$^{th}$ day (recorded as D7), the 14$^{th}$ day (recorded as D14), the 21$^{st}$ day (recorded as D21), the 28$^{th}$ day (recorded as D28), and the 56$^{th}$ day (recorded as D56) post-administration. An AGT protein expression assay was performed using a human angiotensinogen kit (IBL, 27412).

Results of Example 14 indicate that, at the single administration of the dose 3 mg/kg, all of RZ003021, RZ003065 and RZ003066, can effectively reduce the AGT protein level in hREN×hAGT hypertensive mice, in which RZ003066 had superior activity and duration of efficacy to RZ003021 and RZ003065 (FIG. 20, Table 29).

Table 29

| **Group** | **Pre-dose** | | **7$^{th}$ Day** | | **14$^{th}$ Day** | | **21st Day** | |
|---|---|---|---|---|---|---|---|---|
| | residual activity% | ±STDEV value | residual activity% | ±STDEV value | residual activity% | ±STDEV value | residual activity% | ±STDEV value |
| **PBS** | 100.00 | 0.00 | 85.92 | 31.19 | 102.09 | 32.50 | 99.64 | 21.27 |
| **RZ003021** | 100.00 | 0.00 | 10.85 | 1.92 | 10.02 | 1.34 | 14.22 | 2.68 |
| **RZ003065** | 100.00 | 0.00 | 9.94 | 1.79 | 10.35 | 1.46 | 18.01 | 2.87 |
| **RZ003066** | 100.00 | 0.00 | 9.94 | 0.68 | 10.46 | 1.06 | 9.01 | 0.97 |
| | | | | | | | | |
| **Group** | 28$^{th}$ **Day** | | 42$^{nd}$ **Day** | | 56$^{th}$ **Day** | | | |
| | residual activity% | ±STDEV value | residual activity% | ±STDEV value | residual activity% | ±STDEV value | | |
| **PBS** | 102.10 | 20.61 | 87.40 | 6.38 | 80.16 | 7.82 | | |
| **RZ003021** | 21.05 | 4.70 | 44.95 | 4.53 | 78.19 | 7.97 | | |
| **RZ003065** | 25.15 | 6.48 | 38.05 | 7.26 | 55.51 | 10.75 | | |
| **RZ003066** | 11.96 | 1.11 | 21.75 | 2.95 | 32.93 | 1.76 | | |

**Example 15. Effect of siRNA conjugates on *in vivo* activity of lipoprotein(A) (lipoprotein(a), LPA) mRNA in HDI mouse models**

The present example comparatively evaluated the *in vivo* activity of LPA-targeting siRNA when using different modification schemes and GalNAc conjugation structures using BALB/c mouse hydrodynamic injection models.

Plasmid construction:

LPA gene sequence (NM_005577.4) was constructed into the pcDNA-CMV vector plasmid by Sangon Biotech (Shanghai) Co., Ltd., yielding pcDNA-CMV-RG001 expression plasmid.

Construction of mouse models:

BALB/c mouse hydrodynamic injection models were models established by rapidly injecting pcDNA-CMV-RG001 plasmid solution into mice through mouse tail vein under high pressure. On the 3rd day of experiment, the mice received hydrodynamic tail vein injection of 10 μg of pcDNA-CMV-RG001 within 5 seconds, at an injection volume of 8% of the mouse body weight. The plasmid DNA for injection was diluted in normal saline, and the solution was prepared before injection and stored at 4 °C.

Animal grouping, administration and tissue sample collection:

6-8-week-old BALB/c mice were randomly divided into groups based on body weight (all females), 5 mice per group. Each test group was administered a pre-determined dose of a drug conjugate and a PBS control group was added. All mice received a single subcutaneous abdominal injection at a dose calculated according to body weight. Each drug conjugate was administered as a PBS solution at 0.1 mg/mL (based on siRNA), at an administration volume of 10 mL/kg mouse body weight, that is, each drug conjugate was administered at a dose of 1 mg/kg mouse body weight (based on siRNA). PBS control group was administered the same volume of PBS solution (without drug conjugate). The day of administration was recorded as the 0th Day (recorded as D0), plasmid injection was performed on the 3rd day (recorded as D3) post-administration, and 5 mice per group were sacrificed on the 4th day (recorded as D4). The sacrificed mice were subjected to gross anatomy, and liver tissues of each sacrificed mouse were collected, and cut into a plurality of 2 $mm^3$ pieces which were stored in RNAlater.

For each mouse, an appropriate amount of liver tissue sample was taken from the RNAlater, the liver tissue sample was fragmented in a Tissuelyser type II full-automatic tissue homogenizer for 60 s, and then total RNAs were extracted using a full-automatic nucleic acid extractor (purchased from Zhejiang Hanwei Science and Technology Co. Ltd.) and a nucleic acid extraction kit (purchased from Zhejiang Hanwei Science and Technology Co. Ltd., GO-MNTR-100) according to standard operation steps of total RNA extraction. Reverse transcription reaction, and fluorescence quantitative PCR were as described in Example 12. In the real-time fluorescence quantitative PCR method, Nero gene on plasmid backbone was taken as an internal reference gene, and primers for the target gene and primers for the Nero internal reference gene were used for detecting the target gene and the Nero internal reference gene, respectively. Sequences of the detection primers were listed in Table 30.

Table 30

| **Gene** | | **Primer Type** | **Primer Sequence** | **Fluorescent Group** | **SEQ ID NO:** |
|---|---|---|---|---|---|
| **Target gene** | LPA | upstream primer | 5'-GACACGATGCTCAGATGC-3' | / | 593 |
| | | downstream primer | 5'-GGGGTTTCCTCAGTCAGT-3' | / | 594 |
| | | probe primer | 5'-CGTCCCTCCGAATGTTA-3' | 5'FAM; 3'MGB | 595 |
| **Internal Reference Gene** | Nero | upstream primer | 5'-CGTTGGCTACCCGTGATATT-3' | / | 596 |
| | | downstream primer | 5'-CTCGTCAAGAAGGCGATAGAAG-3' | / | 597 |
| | | probe primer | 5'-CCGCTTCCTCGTGCTTTACGGTAT-3' | 5'VIC; 3'MGB | 598 |

10 μL Real-time PCR reaction system was configured in each PCR detection well according to the method described in instructions of the real-time fluorescence quantitative PCR kit. Each reaction system comprised 4 μL of cDNA solution obtained by the above reverse transcription reaction, 5 μL of TaqMan™ Fast Advanced Master Mix (2×), 0.15 μL of 10 μM upstream primer, 0.15 μL of 10 μM downstream primer (see Table 11 for primer information), 0.15 μL of 10 μM probe primer, and 0.55 μL of RNase-Free H2O. The configured reaction system was subjected to Real-time PCR amplification on a real-time fluorescence quantitative PCR instrument (Bio-Rad, CFX Opus 384) by a two-step method,

wherein an amplification procedure was 50 °C for 2 min, then pre-denaturation at 95 °C for 20 s, denaturation at 95 °C for 3 s, annealing at 60 °C and extension for 30 s, and processes of denaturation, annealing and extension were repeated for 40 cycles. In the real-time fluorescence quantitative PCR method, relative quantitative calculation of expression levels and inhibition rates of target gene mRNA in various test groups was performed by the ΔΔCt method according to the technical method in the embodiment.

Results of Example 15 indicate that, at the dose of 1 mg/kg, RZ001032 had superior activity to RZ001003, RZ001034 had superior activity to RZ001005, RZ001037 had superior activity to RZ001008, RZ001039 had superior activity to RZ001012, and RZ001044 had superior activity to RZ001026 (FIG. 21, Table 31).

Table 31

| Group | Residual Activity% | ±STDEV Value |
|---|---|---|
| PBS | 100.00 | 10.71 |
| RZ001003 | 26.75 | 4.97 |
| RZ001032 | 19.61 | 5.91 |
| RZ001005 | 28.18 | 5.83 |
| RZ001034 | 18.39 | 1.92 |
| RZ001008 | 58.28 | 23.49 |
| RZ001037 | 52.72 | 14.26 |
| RZ001012 | 31.77 | 9.31 |
| RZ001039 | 30.85 | 5.25 |
| RZ001026 | 41.66 | 9.10 |
| RZ001044 | 28.08 | 14.91 |

**Example 16 Evaluation of inhibitory activities of siRNA conjugates against target gene LPA in hApo(a) transgenic mice**

hApo(a) transgenic mice were conditional knock-in human Apo(a) transgenic mice constructed by Cyagen Biosciences (Suzhou) Inc. based on its proprietary TurboKnockout® platform. A construction process comprised the following:

(1) design and construction of targeting vectors: the human LPA gene was constructed into vectors to yield CAG promoter-loxP-PGK-Neo-6*SV40 pA-loxP-Kozak-Human LPA CDS-rBG pA targeting vectors. The vectors were capable of inserting human LPA gene (NM_005577.4) into an intergenic region between Eif4enif1 and Drg1 genes at Hipp11 locus on mouse chromosome 11 *in vivo.* The targeting vectors were validated through enzyme digestion, PCR and sequencing, and plasmid extraction and linearization were performed after the targeting vectors were validated to be correct;
(2) electroporation of targeting vectors to embryonic stem cells, and screening of positive clones: the targeting vectors were electroporated into mouse embryonic stem cells (ES), followed by positive selection using Neo and negative selection using DTA. Drug-resistant clones were picked out, and ES cells were validated through PCR, karyotype analysis and Southern blot;
(3) preparation of TurboKnockout® mice: positive ES cells were microinjected into blastocysts, which were then transfered into pseudopregnant female mice. Postnatal mice were genotyped, to obtain TurboKnockout mice with germline transmission ability; and
(4) derivation of hApo(a) transgenic mice: mice with liver-specific Cre-expressing were hybridized with TurboKnockout® mice, to remove a resistant gene flanked by loxP sites, thereby activating the human LPA gene expression, and obtaining the hApo(a) transgenic mice.

The present example measured the Apo(a) protein expression levels in serum of the hApo(a) transgenic mice at different time points after a single administration of the siRNA conjugates RZ001032 and RZ001034 using an enzyme-linked immunosorbent assay (ELISA).

Animal grouping, administration and tissue sample collection:

The Apo(a) transgenic mice (constructed by Cyagen Biosciences (Suzhou) Inc.) were divided into groups based on the Apo(a) levels in serum. Each test group received a pre-determined dose of a drug conjugate, and a PBS control group was added. All mice received a single subcutaneous abdominal injection at a dose calculated according to body weight. Each drug conjugate was administered as a PBS solution at 0.1 mg/mL (based on siRNA), with an administration volume of 10 mL/kg mouse body weight. That is, each drug conjugate was administered at a dose of 1 mg/kg mouse body weight (based on siRNA). PBS control group was administered the same volume of PBS). Serum of all groups of mice was collected on the 28$^{th}$ day (recorded as D28), the 35$^{th}$ day (recorded as D35), the 42$^{nd}$ day (recorded as D42), the 49$^{th}$ day (recorded as D49), and the 56$^{th}$ day (recorded as D56). An Apo(a) protein expression assay was performed using a human Lipoprotein A ELISA kit (Abcam, ab212165).

Results of Example 16 indicate that, at the single administration of the dose of 1 mg/kg, RZ001032 and RZ001034 both can remarkably reduce the Apo(a) protein level in the Apo(a) transgenic mice (FIG. 22, Table 32).

Table 32

| Group | PBS | | RZ001032 | | RZ001034 | |
|---|---|---|---|---|---|---|
| | residual activity% | ±STDEV value | residual activity% | ±STDEV value | residual activity% | ±STDEV value |
| D0 | 100.00 | 0.00 | 100.00 | 0.00 | 100.00 | 0.00 |
| D7 | 132.20 | 13.61 | 12.13 | 10.64 | 16.02 | 3.49 |
| D14 | 88.66 | 7.47 | 10.11 | 10.34 | 15.28 | 3.50 |
| D21 | 152.29 | 70.19 | 11.57 | 8.02 | 35.24 | 6.30 |
| D28 | 150.80 | 66.65 | 18.70 | 12.82 | 36.14 | 5.41 |
| D35 | 112.43 | 46.49 | 25.73 | 19.46 | 60.75 | 10.34 |
| D42 | 129.76 | 53.86 | 34.36 | 20.33 | 68.89 | 14.37 |
| D49 | 93.20 | 37.30 | 44.10 | 17.83 | 90.20 | 39.00 |
| D56 | 104.02 | 44.87 | 51.24 | 15.28 | 103.90 | 36.74 |

**Example 17. Inhibitory activities of siRNA conjugates modified with NM062 and NM063 at different sites of antisense strand against SOD1 mRNA in mice**

The present example evaluated inhibitory activities of siRNA conjugates modified with NM062 and NM063 at different sites of an antisense strand and reference conjugates RZ599001 and RZ599015 without NM062 and NM063 modifications against a target gene SOD1 in mice by the *in vivo* mouse evaluation method as shown in Example 7.

6-8-week-old C57BL/6j mice were randomly divided into 6 groups based on body weight, 15 mice per group. Each group of mice received the above siRNA conjugates via abdominal subcutaneous administration. Herein, each mouse in the PBS control group received an administration volume of 5 mL/kg, and each mouse in the siRNA conjugate experimental groups was administered at a dose of 3 mg/kg (based on siRNA), with an administration volume of 5 mL/kg. The day of administration was recorded as the 0$^{th}$ day (D0). 5 mice per group were sacrificed respectively on the 7$^{th}$ day (D7), the 28$^{th}$ day (D28), and the 56$^{th}$ day (D56) post-administration. The animals were subjected to gross anatomy, and liver tissues were collected and cut into a plurality of 2 mm$^3$ pieces which were stored in RNAlater. RNA extraction, reverse transcription reaction, and fluorescence quantitative PCR operations were as described in the above, and gene expression differences were calculated by the $\Delta\Delta$Ct method. Primers are as listed in Table 33.

Table 33

| Target Point | | Primer Type | Primer Sequence (5'-3') |
|---|---|---|---|
| Target gene | SOD1 | upstream primer | GGGTTCCACGTCCATCAGTA (SEQ ID NO.599) |
| | | downstream primer | ACACCGTCCTTTCCAGCAGT (SEQ ID NO.600) |
| Internal Reference Gene | GAPDH | upstream primer | TGCACCACCAACTGCTTAG (SEQ ID NO.601) |
| | | downstream primer | GGATGCAGGGATGATGTTC (SEQ ID NO.602) |

Statistics was carried out respectively on expression levels (FIG. 23) and residual activity (Table 34) of SOD1 mRNA in mice on D7, D28 and D56 post-administration. Results indicate that NM062 and NM063 modifications at specific sites of the antisense strand of siRNA can have good tolerance, and NM062 and NM063 modifications at position 8 and position 15 of the antisense strand can further elevate the siRNA sequence activity.

Table 34

| Group | D7 | | D28 | | D56 | |
|---|---|---|---|---|---|---|
| | residual activity% | ±STDEV value | residual activity% | ±STDEV value | residual activity% | ±STDEV value |
| PBS | 100.00 | 7.86 | 100.00 | 9.66 | 100.00 | 13.29 |
| RZ599026 | 5.84 | 2.05 | 15.75 | 9.46 | 67.77 | 4.77 |
| RZ599027 | 5.09 | 1.38 | 8.43 | 0.65 | 50.57 | 6.49 |
| RZ599028 | 3.56 | 1.49 | 8.27 | 2.29 | 46.79 | 7.06 |
| RZ599029 | 6.80 | 3.19 | 7.88 | 2.21 | 62.49 | 9.51 |
| RZ599030 | 4.72 | 1.05 | 10.77 | 1.21 | 63.93 | 6.51 |

**Example 18. Inhibitory activities of siRNA conjugates modified with NM096, NM098 and NM099 on antisense strand against SOD1 mRNA in mouse primary hepatocytes**

The inhibitory activities of siRNA conjugate RZ599136 modified with NM096 at position 15 of an antisense strand, siRNA conjugate RZ599138 modified with NM098, siRNA conjugate RZ599139 modified with NM099, and control RZ599062 against a target gene SOD1 in mouse primary hepatocytes were evaluated by the above experimental method for inhibitory activity evaluation against a target gene in mouse primary hepatocytes.

Isolation of mouse primary hepatocytes, cell culturing and other operations were as described in the preceding. The mouse primary hepatocytes were extracted from fresh liver tissues of C56BL/6j mice, and seeded in a 12-well culture plate at a cell amount of $2\times10^5$ cells/well. Various groups of double-stranded siRNA conjugates were gradiently diluted with PBS to 10 μM working solutions (based on siRNA). The working solutions of the siRNA conjugates at respective concentrations were respectively added to the above 12-well culture plate at 1 μL/well, equivalent to that an siRNA conjugate transfection final concentration was 10 nM (based on siRNA), and the siRNAs at each concentration were set with 2 culture wells. Another 2 culture wells were added with 1 μL of PBS per well as blank control wells (BLANK). The culture plate was shaken to mix solutions uniformly. Further culturing was performed in the 37 °C, 5% $CO_2$ incubator for 24 h. Relative quantitative calculation was performed for the target gene mRNA in various test groups by the preceding ΔΔCt method. Primers used in the present example are as listed in Table 33. Relative quantitative calculation was performed for the target gene mRNA in various test groups by the preceding ΔΔCt method.

Results of Example 18 indicate that the siRNA conjugates modified with NM096, NM098 and NM099 at position 15 of the antisense strand had comparable inhibitory activity on mouse primary hepatocytes, compared with the control RZ599062. (FIG. 24, Table 35).

Table 35

| Group | % Mean Value of Inhibition Rate | ±STDEV Value |
|---|---|---|
| BLANK | 0.00 | 3.70 |
| RZ599062 | 96.47 | 0.09 |
| RZ599136 | 96.28 | 0.06 |
| RZ599138 | 96.04 | 0.39 |
| RZ599139 | 96.30 | 0.14 |

**Example 19. Evaluation of inhibitory activities of 2'-difluoro substituted siRNA conjugates at specific sites of sense strand against target gene SOD1 in mouse primary hepatocytes**

Example 19 evaluated inhibitory activities of siRNA conjugate RZ599051 with 2'-difluoro at position 8 of the sense strand and siRNA reference conjugate RZ599001 modified with F at position 8 against a target gene SOD1 in mouse

primary hepatocytes by the above experimental method for inhibitory activity evaluation against a target gene in mouse primary hepatocytes. Isolation of the mouse primary hepatocytes, cell culturing and other operations were as described in the preceding. The mouse primary hepatocytes were extracted from fresh liver tissues of C56BL/6j mice, and seeded in a 12-well culture plate at a cell amount of $2\times10^5$ cells/well. Various groups of double-stranded siRNA conjugates were gradiently diluted with PBS to 5 μM working solutions (based on siRNA). The working solutions of the siRNA conjugates at respective concentrations were respectively added to the above 12-well culture plate at 1 μL/well, equivalent to that an siRNA conjugate transfection final concentration was 5 nM (based on siRNA), and the siRNAs at each concentration were set with 2 culture wells. Another 2 culture wells were added with 1 μL of PBS per well as blank control wells (BLANK). The culture plate was shaken to mix solutions uniformly. Further culturing was performed in the 37 °C, 5% $CO_2$ incubator for 24 h. Relative quantitative calculation was performed for the target gene mRNA in various test groups by the preceding ΔΔCt method. Primers used in the present example are as listed in Table 34.

Results of Example 19 indicate that the siRNA conjugate RZ599051 modified with 2'-difluoro at nucleotide of position 8 of the sense strand had comparable inhibitory activity to the reference conjugate RZ599001. (FIG. 25, Table 36).

Table 36

| Group | % Mean Value of Inhibition Rate | ±STDEV Value |
|---|---|---|
| BLANK | 0.00 | 1.37 |
| RZ599001 | 86.53 | 2.06 |
| RZ599051 | 87.41 | 1.27 |

**Example 20. Evaluation of inhibitory activities of 2'-difluoro substituted siRNA conjugates at specific sites of sense strand against target gene SOD1 in mouse primary hepatocytes**

Example 20 evaluated inhibitory activities of siRNA conjugate RZ599055 modified with 2'-difluoro at nucleotide of position 8 of the sense strand, siRNA conjugate RZ599056 modified with 2'-difluoro at position 9 of the sense strand, and reference conjugate RZ599001 against the target gene SOD1 in mouse primary hepatocytes by the above experimental method for inhibitory activity evaluation against a target gene in mouse primary hepatocytes. RZ599055 differs from RZ599051 in Example 19 in that nucleotide modifications at position 10 of the sense strand and position 12 of the antisense strand were replaced by fluoro modifications instead of methoxyl modifications.

The mouse primary hepatocytes were seeded in a 12-well culture plate at a cell amount of $2\times10^5$ cells/well. Various groups of double-stranded siRNA conjugates were gradiently diluted with PBS to 5 μM working solutions (based on siRNA). The working solutions of the siRNA conjugates at respective concentrations were respectively added to the above 12-well culture plate at 1 μL/well, equivalent to that an siRNA conjugate transfection final concentration was 5 nM (based on siRNA), and the siRNAs at each concentration were set with 2 culture wells. Another 2 culture wells were added with 1 μL of PBS per well as blank control wells (BLANK). Primers in the present example are as listed in Table 34.

Results of Example 20 indicate that the siRNA conjugate RZ599055 modified with 2'-difluoro at nucleotide of position 8 of the sense strand and the siRNA conjugate RZ599056 modified with 2'-difluoro at nucleotide of position 9 of the sense strand had comparable or superior inhibitory activity compared with the reference conjugate RZ599001. (FIG. 26, Table 37).

Table 37

| Group | % Mean Value of Inhibition Rate | ±STDEV Value |
|---|---|---|
| BLANK | 0.00 | 47.92 |
| RZ599001 | 91.25 | 1.10 |
| RZ599055 | 93.13 | 0.83 |
| RZ599056 | 92.83 | 1.08 |

**Example 21. Evaluation of inhibitory activities of 2'-difluoro substituted siRNA conjugates at specific sites of sense strand against target gene ANGPTL3 in mouse primary hepatocytes**

Example 21 evaluated inhibitory activities of siRNA conjugate RZ597101 modified with 2'-difluoro at nucleotide of position 8 of the sense strand and reference conjugate RZ597002 against the target gene ANGPTL3 in mouse primary hepatocytes by the above experimental method for inhibitory activity evaluation against a target gene in mouse primary

hepatocytes. RZ597101 differs from RZ597002 in that nucleotide modifications at position 10 of the sense strand and position 12 of the antisense strand were replaced by fluoro modifications instead of methoxyl modifications, and nucleotide modification at position 15 of the antisense strand was replaced by moe modification instead of 2'-methoxyl.

The mouse primary hepatocytes were seeded in a 12-well culture plate at a cell amount of $2\times10^5$ cells/well. Various groups of double-stranded siRNA conjugates were gradiently diluted with PBS to 10 μM working solutions (based on siRNA). The working solutions of the siRNA conjugates at respective concentrations were respectively added to the above 12-well culture plate at 1 μL/well, equivalent to that an siRNA conjugate transfection final concentration was 10 nM (based on siRNA), and the siRNAs at each concentration were set with 2 culture wells. Another 2 culture wells were added with 1 μL of PBS per well as blank control wells (BLANK). Primers in the present example are as listed in Table 9.

Results of Example 21 indicate that the siRNA conjugate RZ597101 had superior inhibitory activity to the reference conjugate RZ597002. (FIG. 27, Table 38).

Table 38

| Group | % Mean Value of Inhibition Rate | ±STDEV Value |
|---|---|---|
| BLANK | 0.00 | 49.64 |
| RZ597002 | 79.61 | 3.00 |
| RZ597101 | 82.64 | 3.59 |

**Example 22. Evaluation of inhibitory activities of 2'-difluoro substituted siRNA conjugates at specific sites of sense strand against target gene complement factor B (CFB) in mouse primary hepatocytes**

Example 22 evaluated inhibitory activities of siRNA conjugates RZM11508, RZM11509, and RZM11510 and reference conjugate RZM11015 against the target gene CFB in mouse primary hepatocytes by the above experimental method for inhibitory activity evaluation against a target gene in mouse primary hepatocytes. Differences lie in that compared with the reference conjugate RZM11015, nucleotide modifications at position 10 of the sense strand and position 12 of the antisense strand in RZM11508 were replaced by fluoro modifications instead of methoxyl modifications; on the basis of modification mode of RZM11508, nucleotide modification at position 15 of the antisense strand in RZM11509 was replaced by moe modification instead of 2'-methoxyl; and RZM11510 and RZM11509 are different in that nucleotide at position 7 of the sense strand was replaced by nucleotide modified with 2'-difluoro.

The mouse primary hepatocytes were seeded in a 12-well culture plate at a cell amount of $2\times10^5$ cells/well. Various groups of double-stranded siRNA conjugates were gradiently diluted with PBS to 10 μM working solutions (based on siRNA). The working solutions of the siRNA conjugates at respective concentrations were respectively added to the above 12-well culture plate at 1 μL/well, equivalent to that an siRNA conjugate transfection final concentration was 10 nM (based on siRNA), and the siRNAs at each concentration were set with 2 culture wells. Another 2 culture wells were added with 1 μL of PBS per well as blank control wells (BLANK).

Table 39

| Target Point | | Primer Type | Primer Sequence (5'-3') |
|---|---|---|---|
| Target gene | CFB | probe | CGGCATCGCTACTCCTCTCATCAGA(SEQ ID NO.603) |
| | | upstream primer | GCCACGATATGGTCTCCTGA(SEQ ID NO.604) |
| | | downstream primer | GAGCTTCTCTGTGACCCAGT(SEQ ID NO.605) |
| Internal Reference Gene | GAPDH | probe | TCTCGGCCTTGACTGTGCCGT(SEQ ID NO.606) |
| | | upstream primer | TATGACTCCACTCACGGCAA(SEQ ID NO.607) |
| | | downstream primer | TGGAAGATGGTGATGGGCTT(SEQ ID NO.608) |

Results of Example 22 indicate that the siRNA conjugate RZM11508, siRNA conjugate RZM11509, and siRNA conjugate RZM11510 had superior inhibitory activity to the reference conjugate RZ011015. (FIG. 28, Table 40).

Table 40

| Group | % Mean Value of Inhibition Rate | ±STDEV Value |
|---|---|---|
| BLANK | 0.00 | 5.01 |

(continued)

| Group | % Mean Value of Inhibition Rate | ±STDEV Value |
|---|---|---|
| RZM11015 | 73.36 | 1.13 |
| RZM11508 | 79.68 | 2.23 |
| RZM11509 | 82.52 | 1.16 |
| RZM11510 | 82.65 | 2.21 |

**Example 23. Evaluation of inhibitory activities of 2'-difluoro substituted siRNA conjugates at specific sites of sense strand against target gene CFB in mice**

The present example evaluated inhibitory activities of siRNA conjugate RZM11509 and RZM11510 and reference conjugate RZM11015 against the target gene CFB in mice by the above evaluation method for inhibitory activity against a target gene in mice.

6-8-week-old C57BL/6j mice were randomly divided into 4 groups based on body weight, 10 mice per group. Each group of mice received the above siRNA conjugates via abdominal subcutaneous administration. Herein, each mouse in the PBS control group was administered at an administration volume of 5 mL/kg, and each mouse in the siRNA conjugate experimental groups was administered at a dose of 3 mg/kg (based on siRNA), with an administration volume of 5 mL/kg. The day of administration was recorded as D0. 5 mice per group were sacrificed respectively on the $7^{th}$ day (D7) and the $28^{th}$ day (D28) post-administration. The animals were subjected to gross anatomy, and liver tissues were collected and cut into a plurality of 2 $mm^3$ pieces which were stored in RNAlater. RNA extraction, reverse transcription reaction and Real-time PCR detection methods were as described in the preceding. Relative quantitative calculation was performed for the target gene mRNA of interest in various test groups by the preceding $\Delta\Delta$Ct method. Primers are as listed in Table 39.

Results of Example 23 indicate that the siRNA conjugate RZM11509 and the siRNA conjugate RZM11510 had substantially comparable inhibitory activity against the target gene on D7 compared with the reference conjugate RZM11015, and exhibited superior inhibitory activity to the reference conjugate on D28. (FIG. 29, Table 41).

Table 41

| Group | D7 | | D28 | |
|---|---|---|---|---|
| | % mean value of inhibition rate | ±STDEV value | % mean value of inhibition rate | ±STDEV value |
| BLANK | 0.00 | 11.63 | 0.00 | 4.85 |
| RZM11015 | 83.63 | 4.03 | 59.92 | 7.98 |
| RZM11509 | 84.39 | 3.44 | 65.11 | 6.63 |
| RZM11510 | 83.22 | 3.93 | 67.39 | 7.63 |

**Example 24. Evaluation of inhibitory activities of 2'-difluoro substituted siRNA conjugates at specific sites of antisense strand against target gene ANGPTL3 in mouse primary hepatocytes**

Example 24 evaluated inhibitory activities of siRNA conjugate RZ597060 modified with 2'-difluoro at nucleotide of position 6 of the antisense strand and reference conjugate RZ597002 against a target gene ANGPTL3 in mouse primary hepatocytes by the above experimental method for inhibitory activity evaluation against a target gene in mouse primary hepatocytes. The mouse primary hepatocytes were seeded in a 12-well culture plate at a cell amount of $2\times10^5$ cells/well. Various groups of double-stranded siRNA conjugates were gradiently diluted with PBS to 10 $\mu$M working solutions (based on siRNA). The working solutions of the siRNA conjugates at respective concentrations were respectively added to the above 12-well culture plate at 1 $\mu$L/well, equivalent to that an siRNA conjugate transfection final concentration was 10 nM (based on siRNA), and the siRNAs at each concentration were set with 2 culture wells. Another 2 culture wells were added with 1 $\mu$L of PBS per well as blank control wells (BLANK). RNA extraction, reverse transcription reaction and Real-time PCR detection methods were as described in the preceding. Relative quantitative calculation was performed for the target gene mRNA in various test groups by the preceding $\Delta\Delta$Ct method. Primers are as listed in Table 9.

Results of Example 24 indicate that the siRNA conjugate RZ597060 modified with 2'-difluoro at nucleotide of position 6 of the antisense strand had comparable inhibitory activity to the reference conjugate RZ597002. (FIG. 30, Table 42).

Table 42

| Group | % Mean Value of Inhibition Rate | ±STDEV Value |
|---|---|---|
| BLANK | 0.00 | 30.17 |
| RZ597002 | 87.36 | 2.52 |
| RZ597060 | 88.53 | 1.65 |

**Example 25. Evaluation of inhibitory activities of 2'-difluoro substituted siRNA conjugates at specific sites of antisense strand against target gene CFB in mouse primary hepatocytes**

Example 25 evaluated inhibitory activities of siRNA conjugate RZM11504 modified with 2'-difluoro at nucleotide of position 14 of the antisense strand, siRNA conjugate RZM11505 modified with 2'-difluoro at nucleotide of position 16 of the antisense strand and reference conjugate RZM11001 against a target target gene in mouse primary hepatocytes by the above experimental method for inhibitory activity evaluation against a target gene in mouse primary hepatocytes. The mouse primary hepatocytes were seeded in a 12-well culture plate at a cell amount of $2\times10^5$ cells/well. Various groups of double-stranded siRNA conjugates were gradiently diluted with PBS to 10 μM working solutions (based on siRNA). The working solutions of the siRNA conjugates at respective concentrations were respectively added to the above 12-well culture plate at 1 μL/well, equivalent to that an siRNA conjugate transfection final concentration was 10 nM (based on siRNA), and the siRNAs at each concentration were set with 2 culture wells. Another 2 culture wells were added with 1 μL of PBS per well as blank control wells (BLANK). RNA extraction, reverse transcription reaction and Real-time PCR detection methods were as described in the preceding. Relative quantitative calculation was performed for the target gene mRNA in various test groups by the preceding ΔΔCt method. Primers are as listed in Table 39.

Results of Example 25 indicate that the siRNA conjugate RZM11504 modified with 2'-difluoro at nucleotide of position 14 of the antisense strand and the siRNA conjugate RZM11505 modified with 2'-difluoro at nucleotide of position 16 of the antisense strand had superior inhibitory activities to the reference conjugate RZM11001. (FIG. 31, Table 43).

Table 43

| Group | %Mean Value of Inhibition Rate | ±STDEV Value |
|---|---|---|
| BLANK | 0.00 | 5.01 |
| RZM11001 | 85.97 | 0.13 |
| RZM11504 | 88.21 | 0.35 |
| RZM11505 | 88.50 | 0.12 |

**Example 26. Evaluation of inhibitory activities of 2'-difluoro substituted siRNA conjugates at specific sites of antisense strand against target gene CFB in mouse primary hepatocytes**

Example 26 evaluated inhibitory activities of siRNA conjugate RZM11507 modified with 2'-difluoro at nucleotide of position 14 of the antisense strand and a reference conjugate RZM11502 against a target gene CFB in mouse primary hepatocytes by the above experimental method for inhibitory activity evaluation against a target gene in mouse primary hepatocytes. It is different from Example 25 in that nucleotide modification at position 15 of the antisense strand was replaced by moe modification instead of 2'-methoxy.

The mouse primary hepatocytes were seeded in a 12-well culture plate at a cell amount of $2\times10^5$ cells/well. Various groups of double-stranded siRNA conjugates were gradiently diluted with PBS to 10 μM working solutions (based on siRNA). The working solutions of the siRNA conjugates at respective concentrations were respectively added to the above 12-well culture plate at 1 μL/well, equivalent to that an siRNA conjugate transfection final concentration was 10 nM (based on siRNA), and the siRNAs at each concentration were set with 2 culture wells. Another 2 culture wells were added with 1 μL of PBS per well as blank control wells (BLANK). RNA extraction, reverse transcription reaction and Real-time PCR detection methods were as described in the preceding. Relative quantitative calculation was performed for the target gene mRNA in various test groups by the preceding ΔΔCt method. Primers are as listed in Table 39.

Results of Example 26 indicate that the siRNA conjugate RZM11507 modified with 2'-difluoro at nucleotide of position 14 of the antisense strand had superior inhibitory activity to the reference conjugate RZM11502. (FIG. 32, Table 44).

Table 44

| Group | %Mean Value of Inhibition Rate | ±STDEV Value |
|---|---|---|
| BLANK | 0.00 | 5.01 |
| RZM11502 | 81.51 | 0.32 |
| RZM11507 | 90.44 | 1.24 |

**Example 27. Evaluation of inhibitory activities of 2'-difluoro substituted siRNA conjugates at specific sites of antisense strand against target gene CFB in mice**

The present example evaluated inhibitory activities of CFB-targeting siRNA conjugates RZM11502 and RZM11507 and a reference conjugate RZM11001 against the target gene CFB in mice by the above evaluation method for inhibitory activity against a target gene in mice. Differences lie in that compared with the reference conjugate RZM11001, nucleotide modifications at position 10 of the sense strand and position 12 of the antisense strand of RZM11502 were replaced by fluoro modifications instead of methoxyl modifications, nucleotide modification at position 15 of the antisense strand was replaced by moe modification instead of 2'-methoxyl; and RZM11507 is different from RZM11502 in that nucleotide at position 14 of the antisense strand was nucleotide modified with 2'-difluoro.

6-8-week-old C57BL/6j mice were randomly divided into 4 groups based on body weight, 15 mice per group. Each group of mice received the above siRNA conjugates via abdominal subcutaneous administration. Herein, each mouse in the PBS control group was administered at an administration volume of 5 mL/kg, and each mouse in the siRNA conjugate experimental groups was administered at a dose of 3 mg/kg (based on siRNA), at an administration volume of 5 mL/kg. The day of administration was recorded as D0. 5 mice per group were sacrificed respectively on D7, D28, and D56 post-administration. The animals were subjected to gross anatomy, and liver tissues were collected and cut into a plurality of 2 $mm^3$ pieces which were stored in RNAlater. RNA extraction, reverse transcription reaction and Real-time PCR detection methods were as described in the preceding. Relative quantitative calculation was performed for the target gene mRNA in various test groups by the preceding $\Delta\Delta$Ct method. Primers are as listed in Table 39.

Results of Example 27 indicate that the siRNA conjugate RZM11502 had superior inhibitory activity on D7 amd D28 compared with the reference conjugate RZM11001; and the siRNA conjugate RZM11507 had higher inhibitory effect and longer duration of pharmacodynamic efficacy on D7, D28, and D56 than the reference conjugate RZM11001. (FIG. 33, Table 45).

Table 45

| Group | D7 | | D28 | | D56 | |
|---|---|---|---|---|---|---|
| | % mean value of inhibition rate | ±STDEV value | % mean value of inhibition rate | ±STDEV value | % mean value of inhibition rate | ±STDEV value |
| PBS | 0.00 | 11.63 | 0.00 | 4.85 | 0.00 | 9.54 |
| RZM11001 | 83.25 | 6.31 | 63.17 | 10.22 | 13.34 | 16.52 |
| RZM11502 | 91.27 | 2.02 | 76.37 | 3.80 | 10.14 | 14.72 |
| RZM11507 | 90.68 | 4.18 | 91.84 | 1.00 | 44.46 | 6.84 |

**Example 28. Evaluation of inhibitory activities of 2'-difluoro substituted siRNA conjugates at specific sites of antisense strand against target gene complement component (Complement C4B) in mice**

The present example evaluated inhibitory activities of siRNA conjugate RZM12503 modified with 2'-difluoro at position 9 of the antisense strand and a reference conjugate RZM12501 against the target gene C4B in mice by the above evaluation method for inhibitory activity against a target gene in mice. 6-8-week-old C57BL/6j mice were randomly divided into 3 groups based on body weight, 10 mice per group. Each group of mice received the above siRNA conjugates via abdominal subcutaneous administration. Herein, each mouse in the PBS control group was administered at an administration volume of 5 mL/kg, and each mouse in the siRNA conjugate experimental groups was administered at a dose of 3 mg/kg (based on siRNA), at an administration volume of 5 mL/kg. The day of administration was recorded as D0. 5 mice per group were sacrificed respectively on D7 and D28 post-administration. The animals were subjected to gross anatomy, and liver tissues were collected and cut into a plurality of 2 $mm^3$ pieces which were stored in RNAlater. RNA extraction, reverse transcription reaction and Real-time PCR detection methods were as described in the preceding. Relative quantitative calculation was performed for the target gene mRNA in various test groups by the preceding $\Delta\Delta$Ct method.

Table 46

| Target Point | | Primer Type | Primer Sequence (5'-3') |
|---|---|---|---|
| Target gene | C4B | probe | TGGCAGAGCTGCCTTCCGTCT (SEQ ID NO.609) |
| | | upstream primer | TGGAGTATGGCTTCACGGTT (SEQ ID NO.610) |
| | | downstream primer | TGCAGGACTTGGGTGATCTT (SEQ ID NO.611) |
| Internal Reference Gene | GAPDH | probe | TCTCGGCCTTGACTGTGCCGT (SEQ ID NO.612) |
| | | upstream primer | TATGACTCCACTCACGGCAA (SEQ ID NO.613) |
| | | downstream primer | TGGAAGATGGTGATGGGCTT (SEQ ID NO.614) |

Results of Example 28 indicate that the siRNA conjugate RZM12503 modified with 2'-difluoro at position 9 of the antisense strand had comparable inhibitory effect and duration of pharmacodynamic efficacy compared with the reference conjugate RZM12501. (FIG. 34, Table 47).

Table 47

| Group | D7 | | D28 | |
|---|---|---|---|---|
| | % mean value of inhibition rate | ±STDEV value | % mean value of inhibition rate | ±STDEV value |
| BLANK | 0.00 | 16.71 | 0.00 | 19.34 |
| RZM12501 | 80.75 | 1.73 | 81.30 | 2.10 |
| RZM12503 | 81.88 | 1.05 | 82.62 | 1.25 |

**Example 29. Evaluation of inhibitory activities of siRNA conjugates comprising NM054 group in antisense strand against target gene SOD1 in mice**

The present example evaluated inhibitory activities of SOD1-targeting conjugate RZ599060 comprising NM054 group at position 20 of siRNA antisense strand and conjugate RZ599061 comprising NM054 group at positions 20 and 21, and conjugate RZ599001 without the group against the target gene SOD1 in mice by the evaluation method for inhibitory activity against a target gene in mice.

6-8-week-old C57BL/6j mice were randomly divided into 4 groups based on body weight, 15 mice per group. Each group of mice received the above siRNA conjugates via abdominal subcutaneous administration. Herein, each mouse in the PBS control group was administered at an administration volume of 5 mL/kg, and each mouse in the siRNA conjugate experimental groups was administered at a dose of 3 mg/kg (based on siRNA), at an administration volume of 5 mL/kg. The day of administration was recorded as the $0^{th}$ day (D0). 5 mice per group were sacrificed respectively on the $7^{th}$ day (D7), the $28^{th}$ day (D28), and the $49^{th}$ day (D49) post-administration. The animals were subjected to gross anatomy, and liver tissues were collected and cut into a plurality of 2 $mm^3$ pieces which were stored in RNAlater. RNA extraction, reverse transcription reaction, and fluorescence quantitative PCR operations were as described in the above, and gene expression differences were calculated by the $\Delta\Delta$Ct method. Primers are as listed in Table 34.

Results of Example 29 indicate that compared with the control conjugate RZ599001 without NM054 group, both conjugates RZ599060 and RZ599061 comprising NM054 group had higher inhibitory activity on D7, D28 and D49, and the inhibitory activity of RZ599060 and RZ599061 on D49 was about 15% higher than that of the control conjugate RZ599001. (FIG. 35, Table 48).

Table 48

| Group | D7 | | D28 | | D49 | |
|---|---|---|---|---|---|---|
| | mean value of inhibition rate | ±SD value | mean value of inhibition rate | ±SD value | mean value of inhibition rate | ±SD value |
| PBS | 0.00 | 18.73 | 0.00 | 9.79 | 0.00 | 24.49 |
| RZ599001 | 95.08 | 1.18 | 88.01 | 1.97 | 49.68 | 16.94 |
| RZ599060 | 96.29 | 1.82 | 88.42 | 8.87 | 66.12 | 10.66 |
| RZ599061 | 96.76 | 0.53 | 93.00 | 1.11 | 64.98 | 7.23 |

**Example 30. Evaluation of inhibitory activities of siRNA conjugates comprising NM054 group in antisense strand against target gene** ANGPTL3 **in mice**

The present example evaluated inhibitory activities of ANGPTL3-targeting conjugate RZ597115 comprising NM054 group at position 20 of siRNA antisense strand and conjugate RZ597116 comprising NM054 group at positions 20 and 21, and conjugate RZ597114 without the group against the target gene ANGPTL3 in mice by the evaluation method for inhibitory activity against a target gene in mice.

6-8-week-old C57BL/6j mice were randomly divided into 4 groups based on body weight, 15 mice per group. Each group of mice received the above siRNA conjugates via abdominal subcutaneous administration. Herein, each mouse in the PBS control group was administered at an administration volume of 5 mL/kg, and each mouse in the siRNA conjugate experimental groups was administered at a dose of 3 mg/kg (based on siRNA), at an administration volume of 5 mL/kg. The day of administration was recorded as D0. 5 mice per group were sacrificed respectively on D7, D28, and D56 post-administration. The animals were subjected to gross anatomy, and liver tissues were collected and cut into a plurality of 2 $mm^3$ pieces which were stored in RNAlater. RNA extraction, reverse transcription reaction, and Real-time PCR detection were as described in the above, and gene expression differences were calculated by the $\Delta\Delta$Ct method. Primers are as listed in Table 9.

Results of Example 30 indicate that compared with the control conjugate RZ597114 without NM054 group, both conjugates RZ597115 and RZ597116 comprising NM054 group had higher inhibitory activity on D7, D28 and D56. On D56, the inhibitory activity of the conjugate RZ597115 comprising NM054 group at position 20 of the antisense strand was about 25% higher than that of the control conjugate RZ597114; and the inhibitory activity of the conjugate RZ597116 comprising NM054 at positions 20 and 21 of the antisense strand was about 35% higher than that of the control conjugate RZ597114. (FIG. 36, Table 49).

Table 49

| Group | D7 | | D28 | | D56 | |
|---|---|---|---|---|---|---|
| | mean value of inhibition rate | ±SD | mean value of inhibition rate | ±SD value | mean value of inhibition rate | ±SD value |
| PBS | 0.00 | 10.67 | 0.00 | 16.36 | 0.00 | 30.50 |
| RZ597114 | 86.86 | 4.31 | 47.55 | 7.59 | 3.55 | 24.81 |
| RZ597115 | 90.55 | 2.96 | 60.37 | 16.05 | 29.12 | 20.77 |
| RZ597116 | 92.30 | 3.23 | 60.63 | 13.28 | 38.44 | 14.64 |

**Example 31. Evaluation of *in vivo* C3 protein-lowering effects of siRNA conjugates in cynomolgus monkeys**

The present example measured expression of C3 protein in cynomolgus monkey serum at different time points following a single administration of (CR01008)×3 vector conjugates RZ002099, RZ002101, RZ002106 and RZ002113 by ELISA.

Animal grouping, administration and tissue sample collection:

Healthy cynomolgus monkeys with a body weight of 3-5 kg were grouped based on serum C3 protein level, 4 in each group, half females and half males. Each test group received a pre-determined dose of a drug conjugate, and a PBS control group was added. All animals received a single subcutaneous abdominal injection at a dose calculated according to body weight. Each drug conjugate was administered as a PBS solution at 9 mg/mL, at an injection volume of 1 mL/kg (cynomolgus monkey body weight). That is, each drug conjugate was administered at a dose of 9 mg (based on siRNA)/kg (cynomolgus monkey body weight). PBS control group received siRNA conjugate-free PBS solution 1 mL/kg (cynomolgus monkeybody weight). Cynomolgus monkey serum was collected before administration (recorded as pre-dose), on the day of administration recorded as the $0^{th}$ Day (recorded as D0), on the $7^{th}$ day (recorded as D7), the $14^{th}$ day (recorded as D14), the $21^{st}$ day (recorded as D21), the $28^{th}$ day (recorded as D28), the $35^{th}$ day (recorded as D35), the $42^{nd}$ day (recorded as D42), the $49^{th}$ day (recorded as D49), and the $56^{th}$ day (recorded as D56) post-administration. C3 protein expression assay was performed using a human Complement C3 ELISA kit (Hycult Biotech, HK366-01).

Results of Example 31 indicate that, at the single administration of the dose of 9 mg/kg, all of RZ002099, RZ002101, RZ002106 and RZ002113 could significantly effectively reduce the C3 protein level in cynomolgus monkey serum, in which RZ002106 could reduce the C3 protein level by 90% or higher. (FIG. 37, Table 50).

Table 50

| Group | pre-dose | | D7 | | D14 | | D21 | | D28 | |
|---|---|---|---|---|---|---|---|---|---|---|
| | residual activity% | ±STDEV value | residual activity% | ±STDEV value | residual activity% | ±STDEV value | residual activity% | ±STDEV value | residual activity% | ±STDEV value |
| PBS | 100.00 | 0.00 | 100.20 | 6.80 | 95.70 | 14.00 | 104.10 | 18.10 | 82.90 | 10.00 |
| RZ002099 | 100.00 | 0.00 | 48.57 | 12.93 | 27.63 | 11.82 | 30.95 | 14.81 | 24.25 | 11.70 |
| RZ002101 | 100.00 | 0.00 | 47.87 | 15.67 | 26.08 | 8.71 | 22.75 | 10.48 | 19.11 | 6.12 |
| RZ002106 | 100.00 | 0.00 | 44.31 | 6.21 | 21.03 | 5.24 | 11.88 | 3.48 | 13.34 | 6.63 |
| RZ002113 | 100.00 | 0.00 | 41.55 | 13.82 | 21.54 | 9.85 | 17.12 | 8.27 | 16.91 | 5.24 |
| | | | | | | | | | | |
| Group | D35 | | D42 | | D49 | | D56 | | | |
| | residual activity% | ±STDEV value | residual activity% | ±STDEV value | residual activity% | ±STDEV value | residual activity% | ±STDEV value | | |
| PBS | 79.30 | 7.90 | 80.60 | 4.50 | 88.10 | 8.40 | 79.41 | 7.84 | | |
| RZ002099 | 26.75 | 8.80 | 30.29 | 15.16 | 37.84 | 24.22 | 34.34 | 26.33 | | |
| RZ002101 | 20.99 | 4.49 | 23.77 | 6.44 | 21.76 | 4.33 | 25.18 | 3.66 | | |
| RZ002106 | 12.15 | 4.03 | 11.87 | 4.67 | 9.01 | 1.95 | 9.51 | 2.59 | | |
| RZ002113 | 15.70 | 5.49 | 15.73 | 5.48 | 14.45 | 5.65 | 19.25 | 8.15 | | |

**Example 32. Evaluation of *in vivo* AGT protein-lowering effects of siRNA conjugates in cynomolgus monkeys**

The present example measured expression of AGT protein in cynomolgus monkey serum at different time points following a single administration of (CR01008)×3 vector conjugate RZ003069 by ELISA.

Animal grouping, administration and tissue sample collection

Healthy cynomolgus monkeys with a body weight of 3-5 kg were grouped based on serum AGT protein level, 4 in each group, half females and half males. Each test group received a pre-determined dose of a drug conjugate, and a PBS control group was added. All animals received a single subcutaneous abdominal injection at a dose calculated according to body weight. Each drug conjugate was administered as a PBS solution at 3 mg/mL, at an injection volume of 1 mL/kg (cynomolgus monkey body weight). That is, each drug conjugate was administered at a dose of 3 mg/kg (cynomolgus monkey body weight). PBS control group received siRNA conjugate-free PBS solution 1 mL/kg. Cynomolgus monkey serum was collected on the day of administration recorded as the $0^{th}$ Day (recorded as D0), and on the $7^{th}$ day (recorded as D7), the $14^{th}$ day (recorded as D14), and the $21^{st}$ day (recorded as D21) post-administration. AGT protein expression assay was performed using a human angiotensinogen ELISA kit (Immuno-Biological, 27412).

Results of Example 32 indicate that at a single administration of the dose of 3 mg/kg, RZ003069 could significantly reduce the AGT protein level in the cynomolgus monkey serum (FIG. 38, Table 51).

Table 51

| Group | D0 | | D7 | | D14 | | D21 | |
|---|---|---|---|---|---|---|---|---|
| | residual activity% | ±STDEV value | residual activity% | ±STDEV value | residual activity% | ±STDEV value | residual activity% | ±STDEV value |
| 0.9% normal saline | 100.00 | 0.00 | 111.29 | 9.08 | 108.39 | 19.07 | 120.02 | 29.31 |
| RZ003069 | 100.00 | 0.00 | 51.03 | 11.86 | 33.68 | 11.86 | 30.01 | 12.18 |

Through repeated researches and experiments, the inventors found that specific modification at single or multiple sites among specific sites of the sense strand and/or the antisense strand of the oligonucleotide enables the modified oligonucleotide to have good tolerance, and even further improves the activity of the oligonucleotide molecule, boasting a promising application prospect in the development of RNA interference (RNAi)-based drugs.

(1) The present disclosure demonstrates that the effect of TBDMS modification on the siRNA sequence activity is associated with the modification site, and that TBDMS bulky group modification at specific site does not affect the siRNA activity, and even may further elevate the activity.

(2) Experimental results of the present disclosure prove that the effect of TOM modification on the siRNA sequence activity is associated with the modification site, and that TOM group modification at specific site does not affect the siRNA activity, with an effect mode highly similar to those of TBDMS modification and the TOM modification.

Taking an oligonucleotide with a double-stranded region having 19 nucleotides (nt) in length as an example: compared with a reference sequence without MOE modification, MOE modification at one or more sites of position 5, position 12, position 18, and position 19 of the sense strand contributes to the improvement on the *in vitro* activity of the siRNA sequence. Compared with the reference sequence without MOE modification, MOE modification at one or more sites of position 8, position 10, and position 15 of the antisense strand contributes to the improvement on the *in vitro* activity of the siRNA sequence.

(3) The present disclosure demonstrates that the effect of MOE modification on the siRNA sequence activity is associated with the modification site, and that the MOE modification at single or multiple sites among specific sites of the sense strand and/or antisense strand can have good tolerance, and still can well maintain or even further elevate the siRNA sequence activity.

In the description of the present description, description with reference to the terms such as "an example", "some examples", "example", "specific example", "some embodiments" or "some instances" mean that specific features, structures, materials or characteristics described in conjunction with this embodiment or example are comprised in at least one embodiment or example of the present disclose. In the present description, exemplary expressions of the above terms do not necessarily refer to the same embodiment or example. Moreover, specific features, structures, materials or characteristics described can be combined in any appropriate manner in any one or more embodiments or examples. Furthermore, those skilled in the art could integrate and combine different embodiments or examples as well as features of

the different embodiments or examples described in the description, without conflict.

Although the embodiments of the present disclosure have been shown and described in the above, it could be understood that the above embodiments are exemplary, and should not be construed as limitation to the present disclosure. Those ordinarily skilled in the art could change, modify, substitute and vary the above embodiments within the scope of the present disclosure.

**INDUSTRIAL APPLICABILITY**

The present disclosure provides a modified double-stranded oligonucleotide and an oligonucleotide conjugate. The double-stranded oligonucleotide and/or oligonucleotide conjugate of the present disclosure have good tolerance through specific modification at single or multiple sites of the sense strand and/or antisense strand, can well maintain the pharmaceutical activity of the oligonucleotide, and have relatively high activity of regulating expression of target genes.

## Claims

**1.** A double-stranded oligonucleotide, comprising a sense strand and an antisense strand, each having 17 to 35 nucleotides, wherein each nucleotide is a modified or unmodified nucleotide; the sense strand and the antisense strand are at least partially reversely complementary to form a double-stranded region, and the double-stranded region comprises a nucleotide with sterically bulky modification and/or a nucleotide with disubstitution modification, wherein

the double-stranded region is as represented by following Formula (I):

SS: $5'\text{-}(N)_{a'}\text{-}(X)_{p'}\text{-}(N)_{b'}\text{-}(X)_{q'}\text{-}(N)_{c'}\text{-}(X)_{r'}\text{-}(N)_{d'}\text{-}3'$
AS:

$$3'\text{-}(N)_a\text{-}(X)_p\text{-}(N)_b\text{-}(X)_q\text{-}(N)_c\text{-}5' \quad \text{(I).}$$

wherein SS represents the sense strand, and AS represents the antisense strand;
each X independently represents a nucleotide in which 2'-hydroxyl group of ribose is substituted with a sterically bulky group, or a nucleotide in which 2'-position is substituted with disubstituent,
wherein the sterically bulky group is independently selected from $2'\text{-}(O)_{m1}(CH_2)_n(O)_{m2}R_1$, wherein each of m1 and m2 is independently 0 or 1, and n is an integer selected from 0 to 6,
wherein $R_1$ is substituted or unsubstituted $C_1$-$C_6$ alkyl or $-Si(R_2)_3$, wherein each $R_2$ is independently selected from substituted or unsubstituted $C_1$-$C_6$ alkyl or $C_1$-$C_6$ alkoxy, wherein the substitution comprises one or more substituents selected from the group consisting of halogen, $C_1$-$C_6$ alkyl, $C_1$-$C_6$ alkoxy, hydroxyl group, amino group, cycloalkyl having up to 6 carbon atoms, aryl having up to 12 carbon atoms, and heteroaryl having up to 12 carbon atoms,
wherein each substituent in the disubstitution is independently selected from $C_1$-$C_6$ alkyl or halogen;
each N independently represents a modified or unmodified nucleotide, wherein when N represents a modified nucleotide, 2'-position of ribose thereof is modified and is not X, and each N is a nucleotide with modification independently selected from one or more of the following: 2'-O-alkyl, 2'-alkyl, 2'-substituted alkyl, 2'-halo, 2'-deoxy, or is a nucleotide analog, wherein the alkyl has 1-6 carbon atoms, and the nucleotide analog is selected from one or more of ENA, BNA, LNA, GNA and UNA;
each of a, a', p, p', b, b', q, q', c, c', r', and d' independently represents the number of nucleotides, wherein a' is an integer selected from 3 to 8; p' is an integer selected from 0 to 3; b' is an integer selected from 4 to 13; q' is an integer selected from 0 to 4; c' is an integer selected from 3 to 9; r' is an integer selected from 0 to 3; d' is an integer selected from 0 to 9; a is an integer selected from 4 to 7; p is an integer selected from 0 to 1; b is an integer selected from 4 to 8; q is an integer selected from 0 to 4; and c is an integer selected from 6 to 10; and
p', q', r', p and q are not simultaneously 0, and $0 \le q'+r' \le 4$.

**2.** The double-stranded oligonucleotide according to claim 1, wherein each X is a nucleotide with modification independently selected from $2'\text{-}O(CH_2)_nOR_1$ or $2'\text{-}R_3\text{-}2'\text{-}R_4$, wherein n is 1 or 2, $R_1$ is substituted or unsubstituted $C_1$-$C_6$ alkyl, or $-Si(R_2)_3$, and
each of $R_3$ and $R_4$ is independently selected from $C_1$-$C_6$ alkyl or halogen.

3. The double-stranded oligonucleotide according to claim 1 or 2, wherein each N is independently selected from nucleotide with 2'-OMe modification, nucleotide with 2'-F modification, nucleotide with 2°-H modification, or unmodified nucleotide;

optionally, each X is a nucleotide with modification independently selected from 2'-O-methoxyethyl, 2'-O-TBDMS, 2'-O-TOM, or 2'-O-$CH_2$-O-$R_5$, wherein $R_5$ is unsubstituted or substituted $C_1$-$C_3$ alkyl; optionally, the substituent is independently selected from one or more of halogen, $C_1$-$C_3$ alkyl, $C_1$-$C_3$ alkoxy, and amino group; and
optionally, the halogen is fluorine.

4. The double-stranded oligonucleotide according to any one of claims 1-3, wherein in a direction from 5'-end to 3'-end, at least one of nucleotides at positions 8, 9, 10 and 15 of the antisense strand of the Formula (I) is a modified nucleotide X, and/or a modified nucleotide X is present or absent in the sense strand;

optionally, when the sense strand comprises the modified nucleotide X, at least one nucleotide X is located at a site opposite to any of positions 2, 8 and 15 of the antisense strand;
optionally, the modified nucleotide X is located at at least one of positions 10 and 15 of the antisense strand;
optionally, each X is a nucleotide with modification independently selected from 2'-O-methoxyethyl, 2'-O-TBDMS, 2'-O-TOM, 2'-O-$CH_2$-O-$CH_2$-$CH_3$, or 2'-O-$CH_2$-O-$CH_2$-$CF_3$.

5. The double-stranded oligonucleotide according to any one of claims 1-4, wherein

q'+c'+r'+d'=9, and 0≤q'+r'≤4;
optionally, when p=0, a=5-7;
optionally, when p=1, in a direction from 5'-end to 3'-end, X is located at position 15 of the antisense strand;
optionally, when q=0, b+c=14;
optionally, in a direction from the 5'-end to the 3'-end, at least one X in (X)p' is located at a site of the sense strand opposite to position 15 of the antisense strand;
optionally, when p'=1, in the direction from the 5'-end to the 3'-end, X in (X)p' is located at a site opposite to position 15 of the antisense strand;
optionally, in the direction from the 5'-end to the 3'-end, the first four nucleotides of (N)b' on the sense strand comprise at least two fluoro-modified nucleotides;
optionally, q' is 0-2;
optionally, q' is 0 or 1;
optionally, when r'=1 and q'=1, b' and c' are 5, and d' is 1;
optionally, when p'=0, a'=6;
optionally, when p'=0, a'=8;
optionally, when q' and r' are not simultaneously 0, in the direction from the 5'-end to the 3'-end, at least one X on the sense strand is located at a position opposite to positions 1-8 of the antisense strand; and
optionally, d'=1-9.

6. The double-stranded oligonucleotide according to any one of claims 1-5, wherein

a' is an integer selected from 3 to 8; p' is an integer selected from 0 to 2; b' is an integer selected from 4 to 12; q' is an integer selected from 0 to 2; c' is an integer selected from 3 to 8; r' is an integer selected from 0 to 2; d' is an integer selected from 1 to 9; a is an integer selected from 4 to 7; p is an integer selected from 0 or 1; b is an integer selected from 4 to 8; q is an integer selected from 0 to 2; and c is an integer selected from 6 to 10;
optionally, each of (N)a and (N)b comprises at least one fluoro-modified nucleotide, and (N)c comprises at least two fluoro-modified nucleotides;
optionally, q'+c'+r'+d'=9, and 0≤q'+r'≤4; and
optionally, counting from 5'-end, the first four nucleotides of (N)b' comprise at least two fluoro-modified nucleotides.

7. The double-stranded oligonucleotide according to any one of claims 1-6, wherein

a' is an integer selected from 3 to 8; p' is 0 or 1; b' is an integer selected from 4 to 13; q' is 0 or 1; c' is an integer selected from 3 to 9; r' is 0 or 1; d' is an integer selected from 1 to 8; a is an integer selected from 4 to 7; p is 1; b is an integer selected from 4 to 8; q is selected from 0 or 1; and c is an integer selected from 6 to 10;

optionally, (N)a comprises at least one fluoro-modified nucleotide, and counting from 5'-end, position 16 of the antisense strand is a fluoro-modified nucleotide;
optionally, (N)b comprises at least one fluoro-modified nucleotide, and counting from the 5'-end, position 14 of the antisense strand is a fluoro-modified nucleotide;
optionally, (N)c comprises at least two fluoro-modified nucleotides, and counting from the 5'-end, positions 2 and 6 of the antisense strand are both fluoro-modified nucleotides;
optionally, $q'+c'+r'+d'=9$, and $0 \leq q'+r' \leq 2$;
optionally, the sense strand comprises at least one X at a position opposite to a seed region of the antisense strand, wherein the seed region of the antisense strand is locatd at positions 1-8, counting from the 5'-end; and
optionally, counting from the 5'-end, the first four nucleotides of (N)b' are located at sites opposite to positions 10-13 of the antisense strand, and the first four nucleotides comprise at least two fluoro-modified nucleotides.

**8.** The double-stranded oligonucleotide according to any one of claims 1-7, wherein counting from 5'-end, X in Formula (I) is located at any of positions 8-10 and 15 of the antisense strand;

optionally, X in the sense strand is located at a position opposite to any of positions 2, 8 and 15 of the antisense strand; and
optionally, counting from the 5'-end, X is located at position 15 of the antisense strand, and the antisense strand comprises at least one fluoro modification at position 9 or position 12, and/or a position of the sense strand opposite to position 10 of the antisense strand comprises a fluoro modification.

**9.** The double-stranded oligonucleotide according to any one of claims 1-8, wherein in addition to the double-stranded region, the double-stranded oligonucleotide further comprises one or more overhang regions, wherein the overhang region has a length of 1-6 nucleotides.

**10.** The double-stranded oligonucleotide according to claim 9, wherein the double-stranded oligonucleotide comprising the overhang region is represented by following Formula (II):

SS: $5'\text{-}(T)_{t1}\text{-}(N)_{a'}\text{-}(X)_{p'}\text{-}(N)_{b'}\text{-}(X)_{q'}\text{-}(N)_{c'}\text{-}(X)_{r'}\text{-}(N)_{d'}\text{-}(T)_{t2}\text{-}3'$
AS:

$$3'\text{-}(T)_{t1}\text{-}(N)_{a}\text{-}(X)_{p}\text{-}(N)_{b}\text{-}(X)_{q}\text{-}(N)_{c}\text{-}(T)_{t2}\text{-}5' \qquad \text{(II)},$$

wherein T represents a nucleotide of the overhang, each of t1 and t2 is an integer independently selected from 0 to 6, and cannot be simultaneously 0; and remaining substituents are as defined for Formula (I).

**11.** The double-stranded oligonucleotide according to claim 10, wherein each nucleotide T of the overhang is independently a modified or unmodified nucleotide, wherein the modified nucleotide is selected from one or more of 2'-O-alkyl, 2'-alkyl, 2'-substituted alkyl, 2'-halogen, 2'-deoxy, abasic nucleotide, and inverted nucleotide.

**12.** The double-stranded oligonucleotide according to any one of claims 1-8, wherein the antisense strand of the double-stranded oligonucleotide comprises a nucleotide overhang at 3'-end, and the overhang comprises 1-3 nucleotides; and the double-stranded oligonucleotide molecule is represented by following Formula (IIa):

SS: $5'\text{-}(N)_{a'}\text{-}(X)_{p'}\text{-}(N)_{b'}\text{-}(X)_{q'}\text{-}(N)_{c'}\text{-}(X)_{r'}\text{-}(N)_{d'}\text{-}3'$
AS:

$$3'\text{-}(T)_{t1}\text{-}(N)_{a}\text{-}(X)_{p}\text{-}(N)_{b}\text{-}(X)_{q}\text{-}(N)_{c}\text{-}5' \qquad \text{(IIa)}$$

,wherein t1 is an integer selected from 1 to 3.

**13.** The double-stranded oligonucleotide according to any one of claims 9-12, wherein the overhang region further comprises a phosphorothioate modification.

**14.** The double-stranded oligonucleotide according to any one of claims 1-13, wherein each strand of the double-stranded oligonucleotide comprises 17-35 nucleotides;

optionally, each strand comprises 17-23 nucleotides;

optionally, each strand comprises 17-21 nucleotides;
optionally, each strand comprises 17-29 nucleotides;
optionally, each strand comprises 19-25 nucleotides;
optionally, each strand comprises 19-23 nucleotides;
optionally, each strand comprises 19-21 nucleotides; and
optionally, each strand comprises 21-23 nucleotides.

**15.** The double-stranded oligonucleotide according to any one of claims 1-12, wherein the double-stranded oligonucleotide has at least one of features as follows:

optionally, the sense strand comprises at least one nucleotide X; and counting from 5'-end, at least one X is located at a position opposite to any of position 15, position 8 and position 2 of the antisense strand;
optionally, counting from the 5'-end, four nucleotides on the sense strand located at positions opposite to positions 10-13 of the antisense strand comprise at least two fluoro-modified nucleotides;
optionally, counting from the 5'-end, position 2, position 6, position 14 and position 16 on the antisense strand are all fluoro-modified nucleotides;
optionally, counting from the 5'-end, at least one of position 8, position 9, position 10 and position 15 on the antisense strand is nucleotide X;
optionally, counting from the 5'-end, the antisense strand comprises at least one X at a position opposite to positions 1-8 on the antisense strand; and
optionally, counting from the 5'-end, position 15 on the antisense strand is nucleotide X.

**16.** The double-stranded oligonucleotide according to claim 15, wherein the nucleotide X is a nucleotide with modification independently selected from 2'-O-MOE, 2'-O-TBDMS, 2'-O-TOM, 2'-O-$CH_2$-O-$CH_2$-$CH_3$, and 2'-O-$CH_2$-O-$CH_2$-$CF_3$; and
optionally, each of the sense strand and the antisense strand comprises at least one phosphorothioate group, and the phosphorothioate group is located in a terminal region of each single strand.

**17.** The double-stranded oligonucleotide according to claim 12, wherein an overhang $(T)_{t1}$ of the AS strand comprises at least one nucleotide with substitution of 2'-F-2'-Me.

**18.** The double-stranded oligonucleotide according to claim 1 or 2, wherein the double-stranded oligonucleotide further comprises at least one ligand;

optionally, the ligand comprises liver-targeting ligands and non-liver-targeting ligands;
optionally, the ligand is ASGPR ligand; and
optionally, the ASGPR ligand comprises GalNAc attached by a branched linker or a derivative thereof.

**19.** A double-stranded oligonucleotide, comprising a sense strand and an antisense strand, each having 17 to 35 nucleotides, wherein each nucleotide is a modified or unmodified nucleotide, the sense strand and the antisense strand are at least partially reversely complementary to form a double-stranded region, wherein the double-stranded oligonucleotide comprises at least one nucleotide with 2'- disubstitution modification, and each substituent in the 2'-disubstitution is independently selected from methyl or fluoro, wherein
when the double-stranded region of the double-stranded oligonucleotide comprises a nucleotide with 2'-disubstitution modification, the nucleotide with 2'-disubstitution modification is selected from 2'-difluoro substituted nucleotides; or, when the double-stranded oligonucleotide comprises a nucleotide modified with 2'-bisubstituent at positions outside the double-stranded region, the nucleotide with 2'-disubstitution modification is selected from nucleotides with substitution of 2'-F-2'-Me.

**20.** The double-stranded oligonucleotide according to any one of claims 1-19, having one or more of features as follows:

(1) when the double-stranded oligonucleotide comprises a 2'-difluoro substituted nucleotide, the 2'-difluoro substituted nucleotide is located at at least one of positions 7, 8, 9, and 10 of the sense strand counting from a 5'-end;
optionally, the 2'-difluoro substituted nucleotide is located at at least two or at least three of positions 7, 8, 9 and 10 of the sense strand counting from the 5'-end;
(2) when the double-stranded oligonucleotide comprises a 2'-difluoro substituted nucleotide, the 2'-difluoro substituted nucleotide is located at at least one of positions 2, 4, 6, 8, 9, 10, 12, 14 and 16 of the antisense strand

counting from the 5'-end;

optionally, the 2'-difluoro substituted nucleotide is located at at least two of positions 2, 4, 6, 8, 9, 10, 12, 14 and 16 of the antisense strand counting from the 5'-end;

(3) when the double-stranded oligonucleotide comprises a nucleotide with substitution of 2'-F-2'-Me, the nucleotide with substitution of 2'-F-2'-Me is located at a position outside the double-stranded region of the oligonucleotide; and

(4) when the double-stranded oligonucleotide comprises a nucleotide with substitution of 2'-F-2'-Me, the nucleotide with substitution of 2'-F-2'-Me is located at an overhang at 3'-end of the antisense strand.

**21.** An oligonucleotide conjugate, comprising the double-stranded oligonucleotide according to any one of claims 1-20, and a conjugating group conjugated to the double-stranded oligonucleotide; and
optionally, the conjugating group comprises a pharmaceutically acceptable targeting group and/or a delivery assisting group.

**22.** A pharmaceutical composition, comprising the double-stranded oligonucleotide according to any one of claims 1-20 or the oligonucleotide conjugate according to claim 21.

**23.** Use of the double-stranded oligonucleotide according to any one of claims 1-20, the oligonucleotide conjugate according to claim 21, or the pharmaceutical composition according to claim 22 in the preparation of a medicament for treating and/or preventing diseases or conditions associated with level of mRNA expressed by a target gene.

**24.** A method for treating and/or preventing diseases or conditions associated with level of mRNA expressed by a target gene, comprising administering the double-stranded oligonucleotide according to any one of claims 1-20, the oligonucleotide conjugate according to claim 21, or the pharmaceutical composition according to claim 22 to a subject in need thereof.

**25.** A method for regulating expression level of a target gene in cells, comprising contacting an effective amount of the double-stranded oligonucleotide according to any one of claims 1-20, the oligonucleotide conjugate according to claim 21, or the pharmaceutical composition according to claim 22 with the cells.

**26.** A kit, comprising at least one of the double-stranded oligonucleotide according to any one of claims 1-20, the oligonucleotide conjugate according to claim 21, and the pharmaceutical composition according to claim 22.

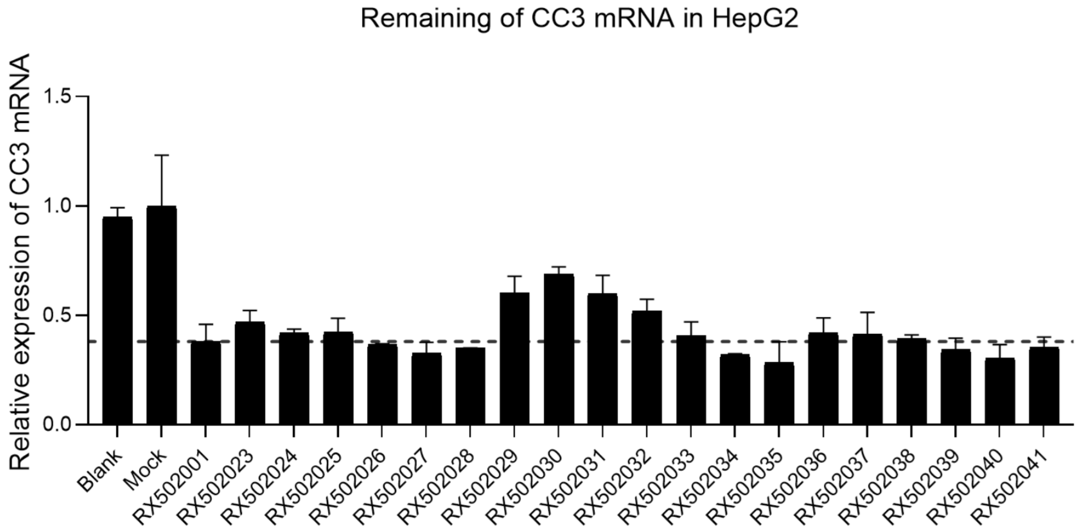
Remaining of CC3 mRNA in HepG2
Relative expression of CC3 mRNA
1.5
1.0
0.5
0.0
Blank
Mock
RX502001
RX502023
RX502024
RX502025
RX502026
RX502027
RX502028
RX502029
RX502030
RX502031
RX502032
RX502033
RX502034
RX502035
RX502036
RX502037
RX502038
RX502039
RX502040
RX502041

FIG. 1

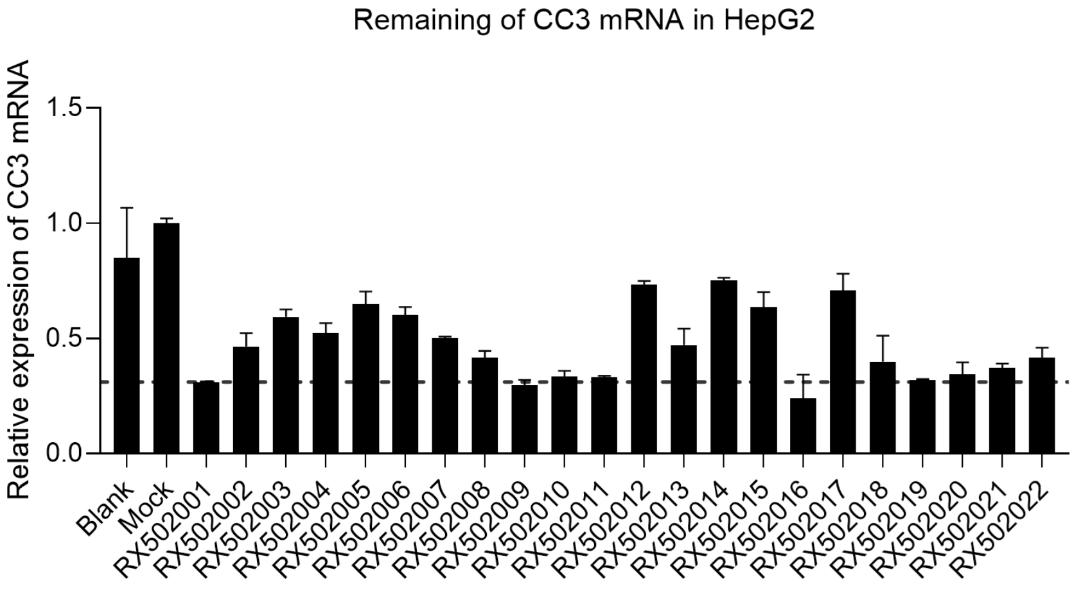
Remaining of CC3 mRNA in HepG2
Relative expression of CC3 mRNA
1.5
1.0
0.5
0.0
Blank
Mock
RX502001
RX502002
RX502003
RX502004
RX502005
RX502006
RX502007
RX502008
RX502009
RX502010
RX502011
RX502012
RX502013
RX502014
RX502015
RX502016
RX502017
RX502018
RX502019
RX502020
RX502021
RX502022

FIG. 2

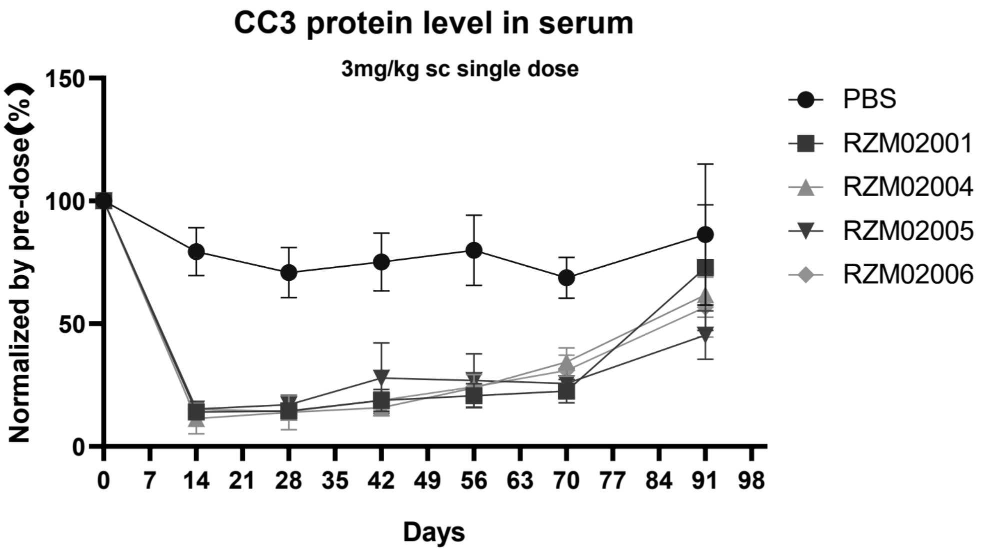
CC3 protein level in serum
3mg/kg sc single dose
Normalized by pre-dose(%)
150
100
50
0
0 7 14 21 28 35 42 49 56 63 70 77 84 91 98
Days
PBS
RZM02001
RZM02004
RZM02005
RZM02006

FIG. 3

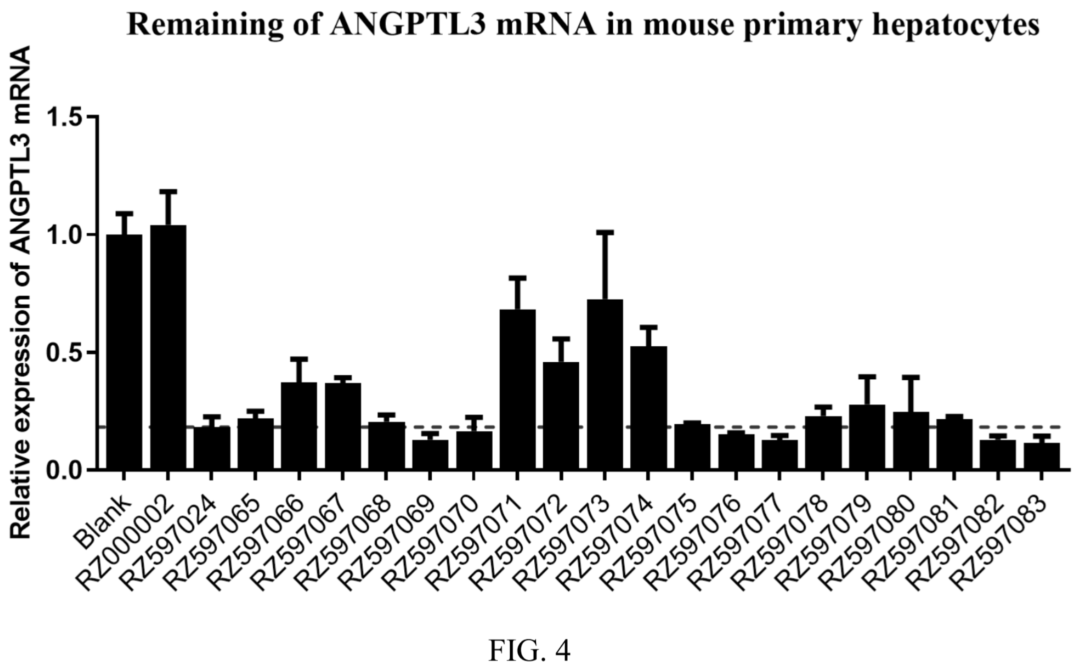
Remaining of ANGPTL3 mRNA in mouse primary hepatocytes
Relative expression of ANGPTL3 mRNA
1.5
1.0
0.5
0.0
Blank
RZ000002
RZ597024
RZ597065
RZ597066
RZ597067
RZ597068
RZ597069
RZ597070
RZ597071
RZ597072
RZ597073
RZ597074
RZ597075
RZ597076
RZ597077
RZ597078
RZ597079
RZ597080
RZ597081
RZ597082
RZ597083

FIG. 4

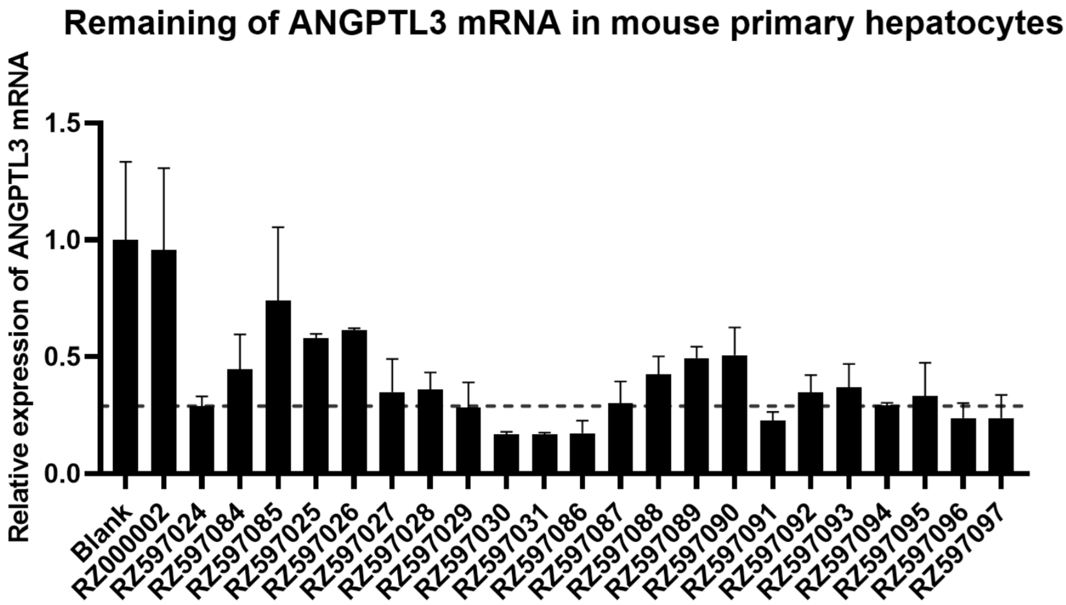
Remaining of ANGPTL3 mRNA in mouse primary hepatocytes
Relative expression of ANGPTL3 mRNA
1.5
1.0
0.5
0.0
Blank
RZ000002
RZ597024
RZ597084
RZ597085
RZ597025
RZ597026
RZ597027
RZ597028
RZ597029
RZ597030
RZ597031
RZ597086
RZ597087
RZ597088
RZ597089
RZ597090
RZ597091
RZ597092
RZ597093
RZ597094
RZ597095
RZ597096
RZ597097

FIG. 5

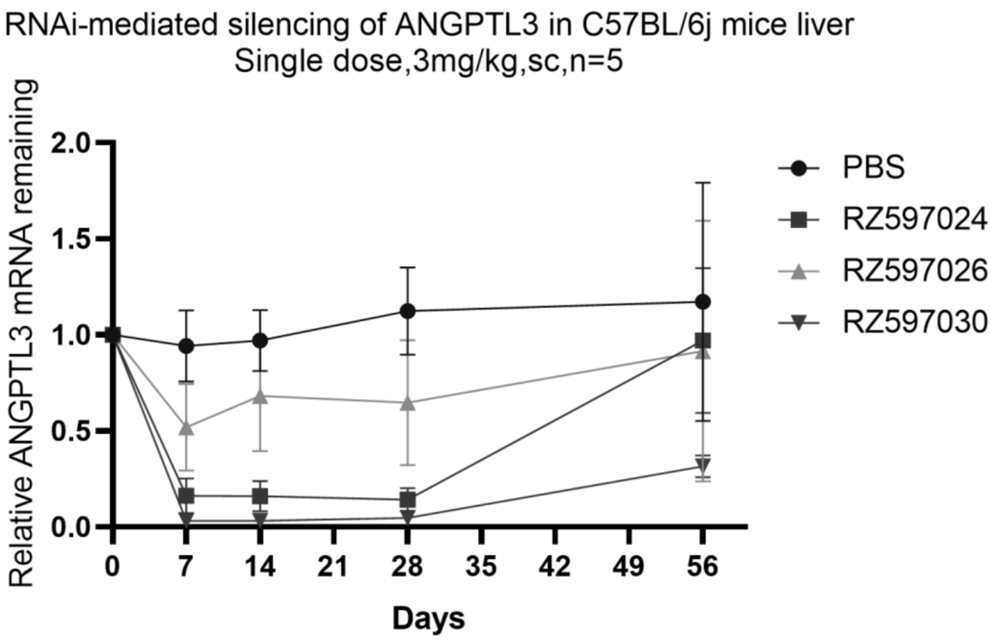
RNAi-mediated silencing of ANGPTL3 in C57BL/6j mice liver
Single dose,3mg/kg,sc,n=5
Relative ANGPTL3 mRNA remaining
2.0
1.5
1.0
0.5
0.0
0
7
14
21
28
35
42
49
56
Days
PBS
RZ597024
RZ597026
RZ597030

FIG. 6

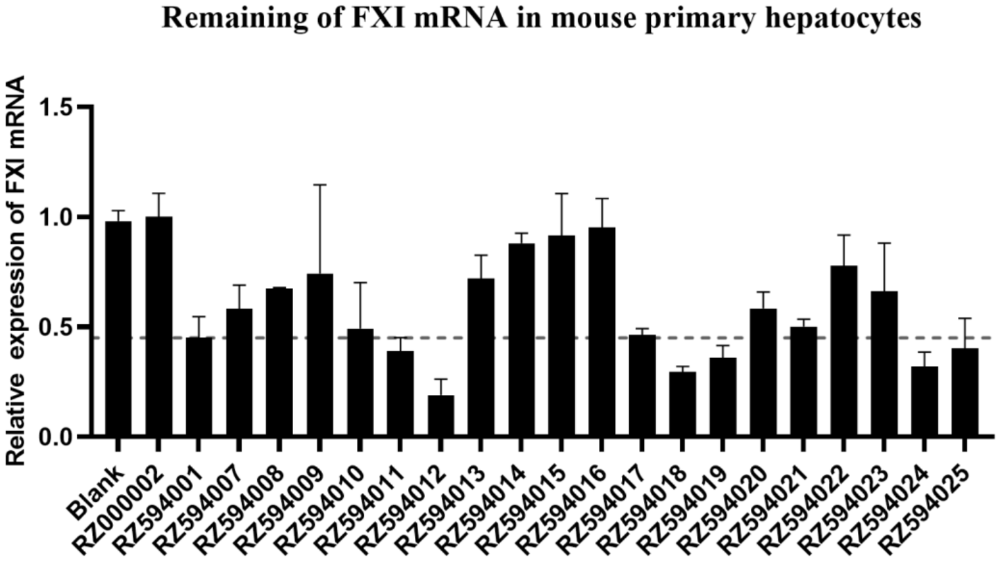
Remaining of FXI mRNA in mouse primary hepatocytes
Relative expression of FXI mRNA
1.5
1.0
0.5
0.0
Blank
RZ000002
RZ594001
RZ594007
RZ594008
RZ594009
RZ594010
RZ594011
RZ594012
RZ594013
RZ594014
RZ594015
RZ594016
RZ594017
RZ594018
RZ594019
RZ594020
RZ594021
RZ594022
RZ594023
RZ594024
RZ594025

FIG. 7

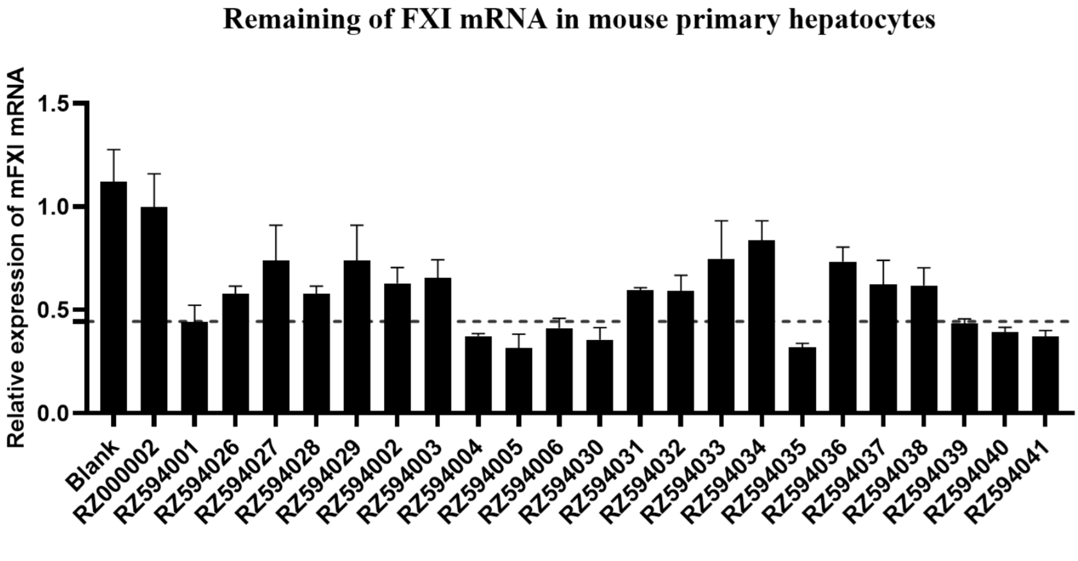
Remaining of FXI mRNA in mouse primary hepatocytes
Relative expression of mFXI mRNA
1.5
1.0
0.5
0.0
Blank
RZ000002
RZ594001
RZ594026
RZ594027
RZ594028
RZ594029
RZ594002
RZ594003
RZ594004
RZ594005
RZ594006
RZ594030
RZ594031
RZ594032
RZ594033
RZ594034
RZ594035
RZ594036
RZ594037
RZ594038
RZ594039
RZ594040
RZ594041

FIG. 8

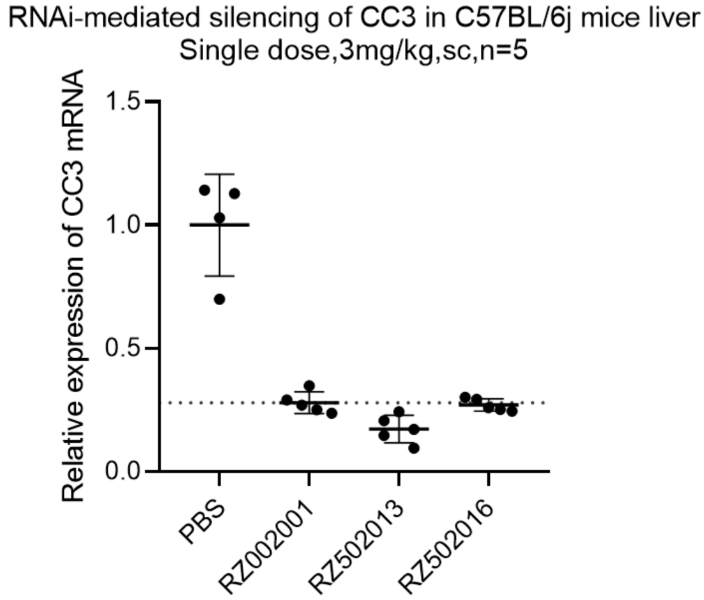
RNAi-mediated silencing of CC3 in C57BL/6j mice liver
Single dose,3mg/kg,sc,n=5
Relative expression of CC3 mRNA
1.5
1.0
0.5
0.0
PBS
RZ002001
RZ502013
RZ502016

FIG. 9

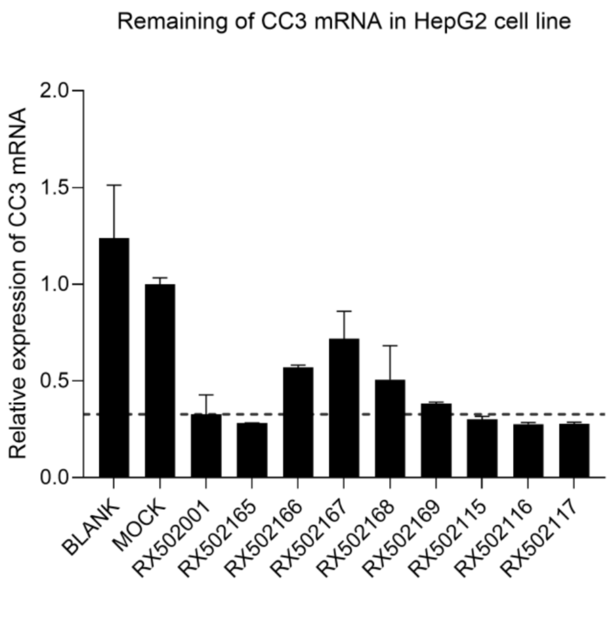
Remaining of CC3 mRNA in HepG2 cell line
Relative expression of CC3 mRNA
2.0
1.5
1.0
0.5
0.0
BLANK
MOCK
RX502001
RX502165
RX502166
RX502167
RX502168
RX502169
RX502115
RX502116
RX502117

FIG. 10

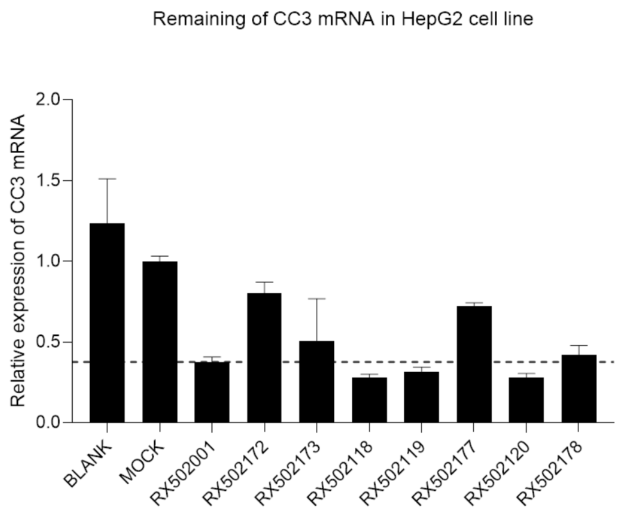


FIG. 11

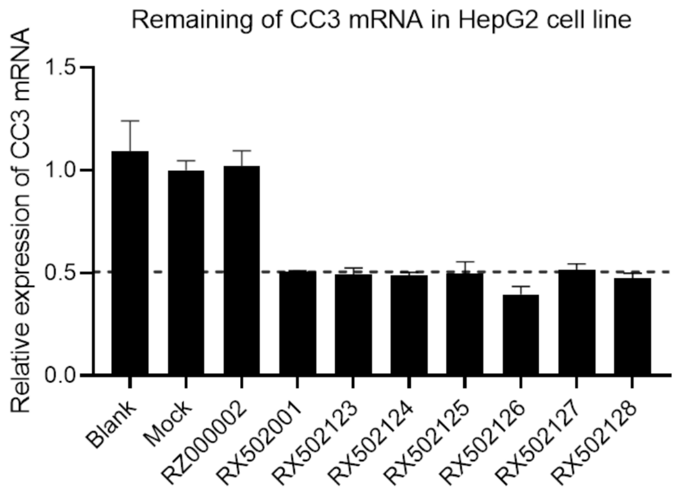


FIG. 12

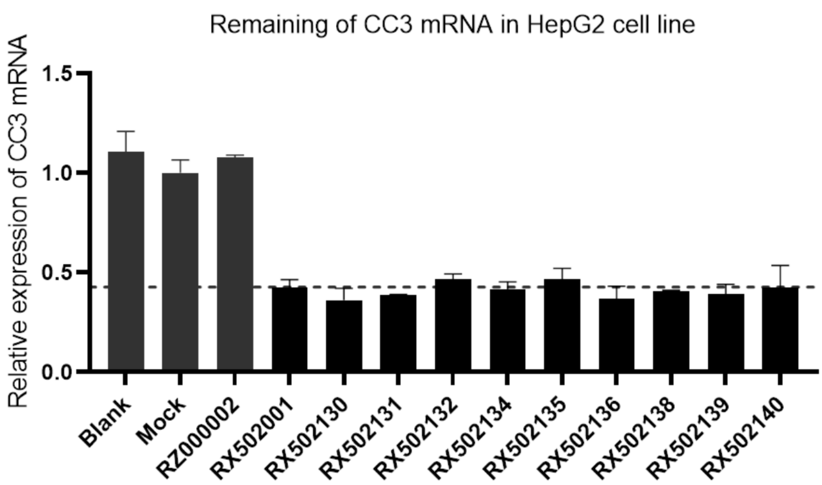


FIG. 13

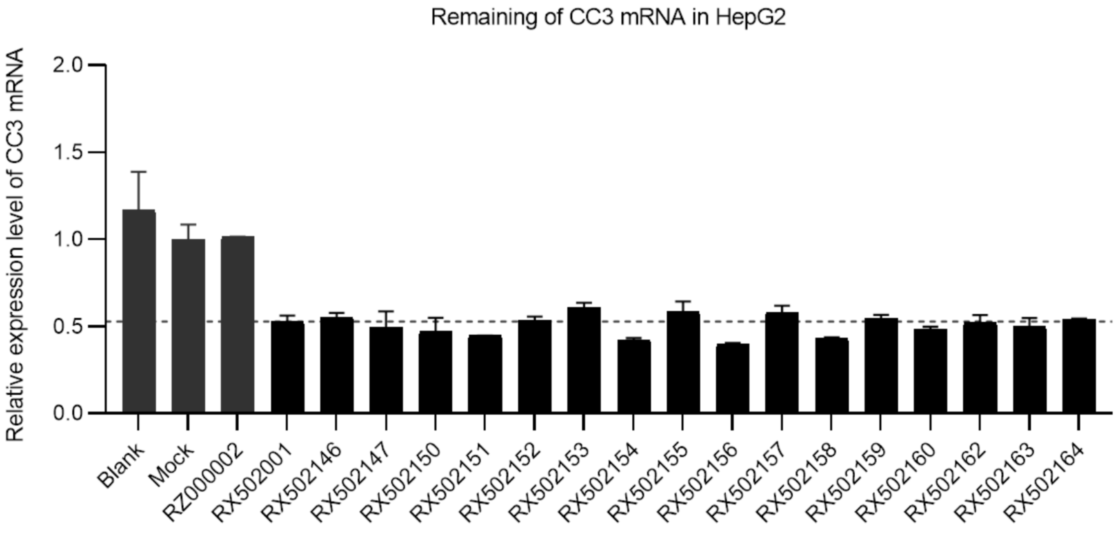
Remaining of CC3 mRNA in HepG2
Relative expression level of CC3 mRNA
2.0
1.5
1.0
0.5
0.0
Blank
Mock
RZ000002
RX502001
RX502146
RX502147
RX502150
RX502151
RX502152
RX502153
RX502154
RX502155
RX502156
RX502157
RX502158
RX502159
RX502160
RX502162
RX502163
RX502164

FIG. 14

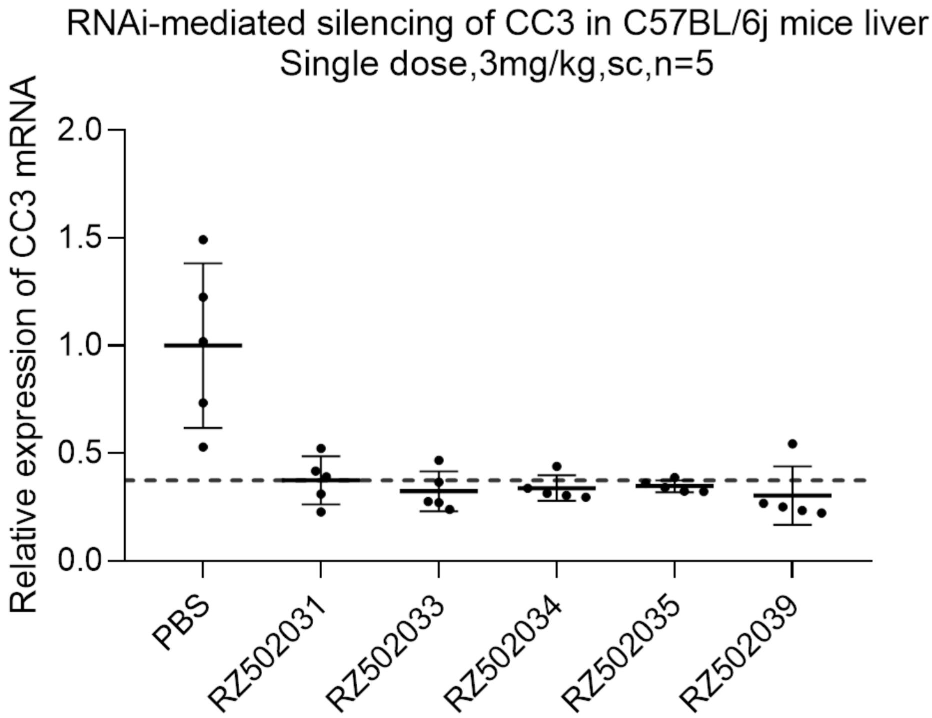
RNAi-mediated silencing of CC3 in C57BL/6j mice liver
Single dose,3mg/kg,sc,n=5
Relative expression of CC3 mRNA
2.0
1.5
1.0
0.5
0.0
PBS
RZ502031
RZ502033
RZ502034
RZ502035
RZ502039

FIG. 15

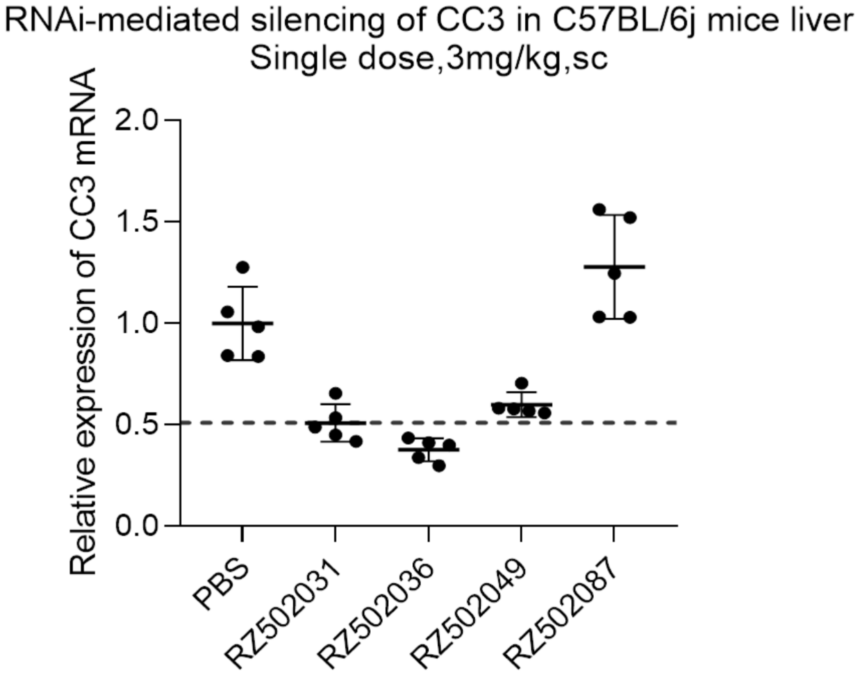
RNAi-mediated silencing of CC3 in C57BL/6j mice liver
Single dose,3mg/kg,sc
Relative expression of CC3 mRNA
2.0
1.5
1.0
0.5
0.0
PBS
RZ502031
RZ502036
RZ502049
RZ502087

FIG. 16

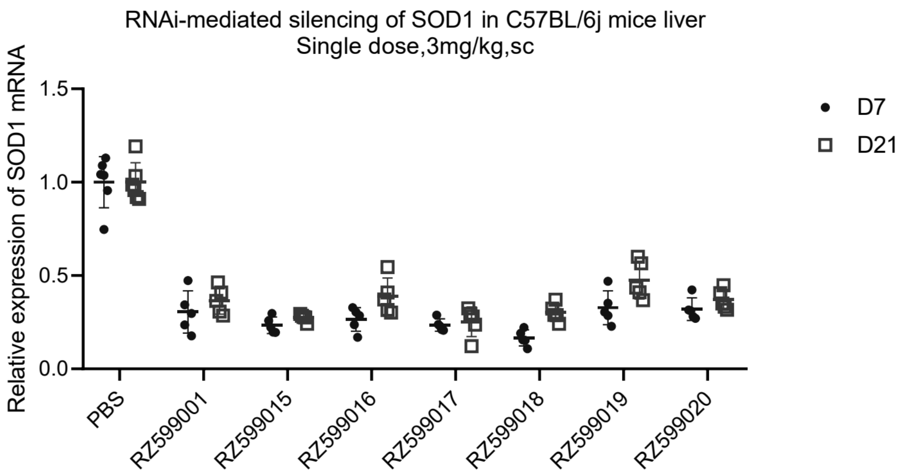
RNAi-mediated silencing of SOD1 in C57BL/6j mice liver
Single dose,3mg/kg,sc
Relative expression of SOD1 mRNA
1.5
1.0
0.5
0.0
D7
D21
PBS
RZ599001
RZ599015
RZ599016
RZ599017
RZ599018
RZ599019
RZ599020

FIG. 17

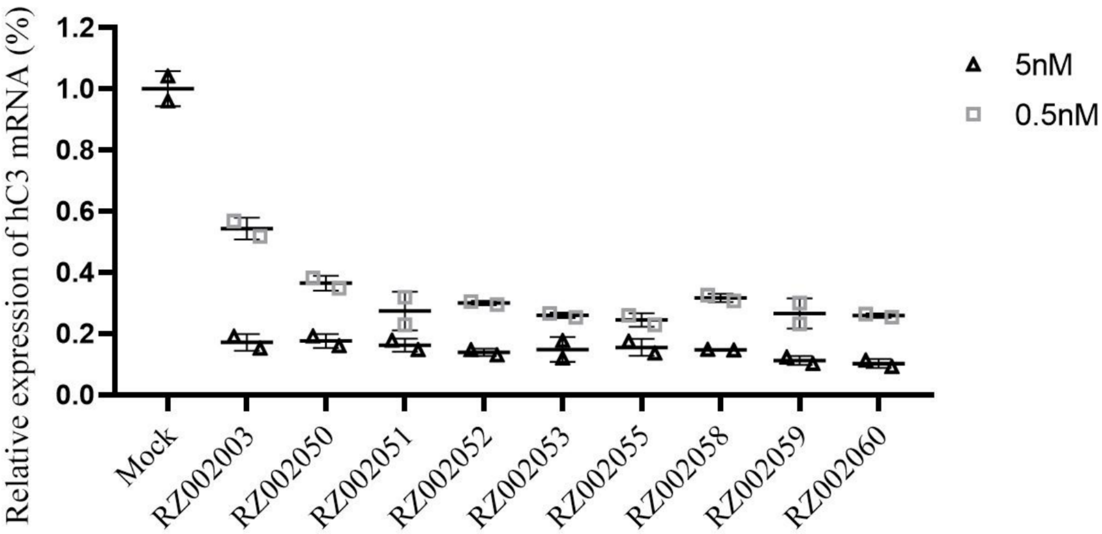


FIG. 18a

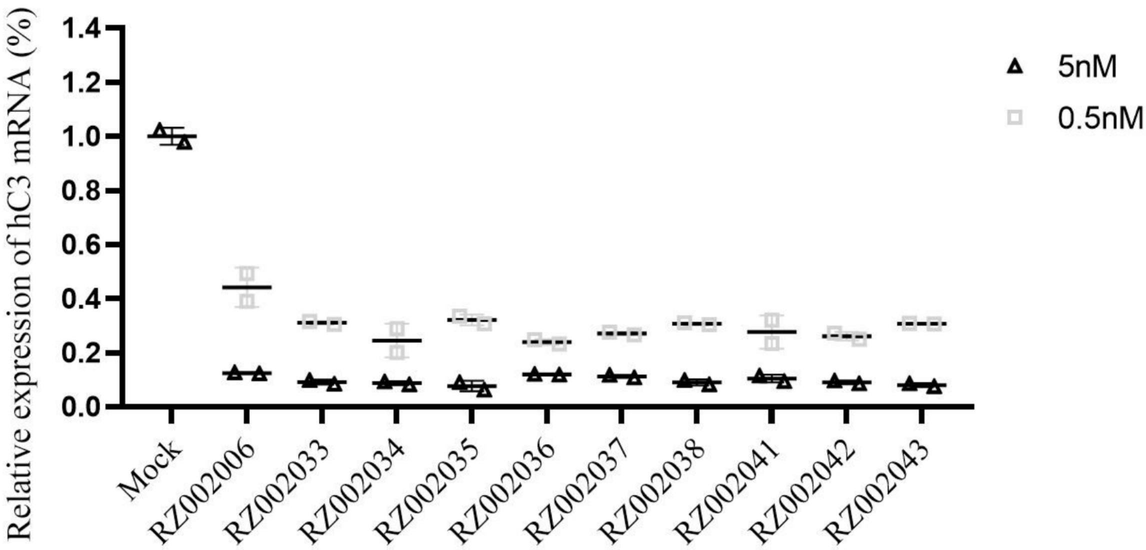


FIG. 18b

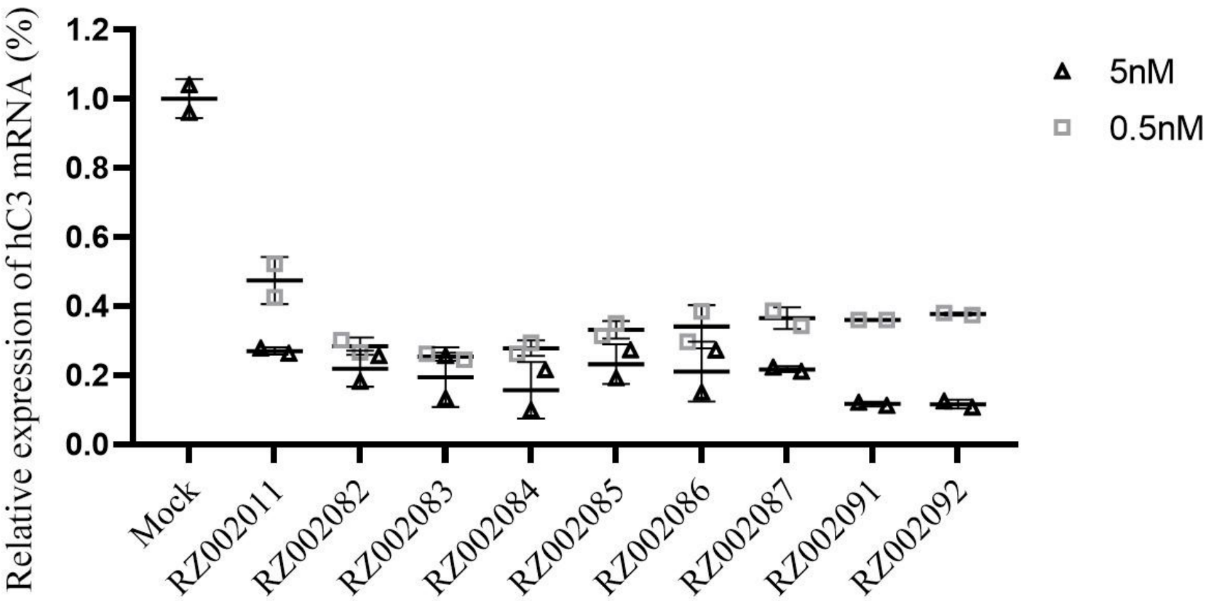


FIG. 18c

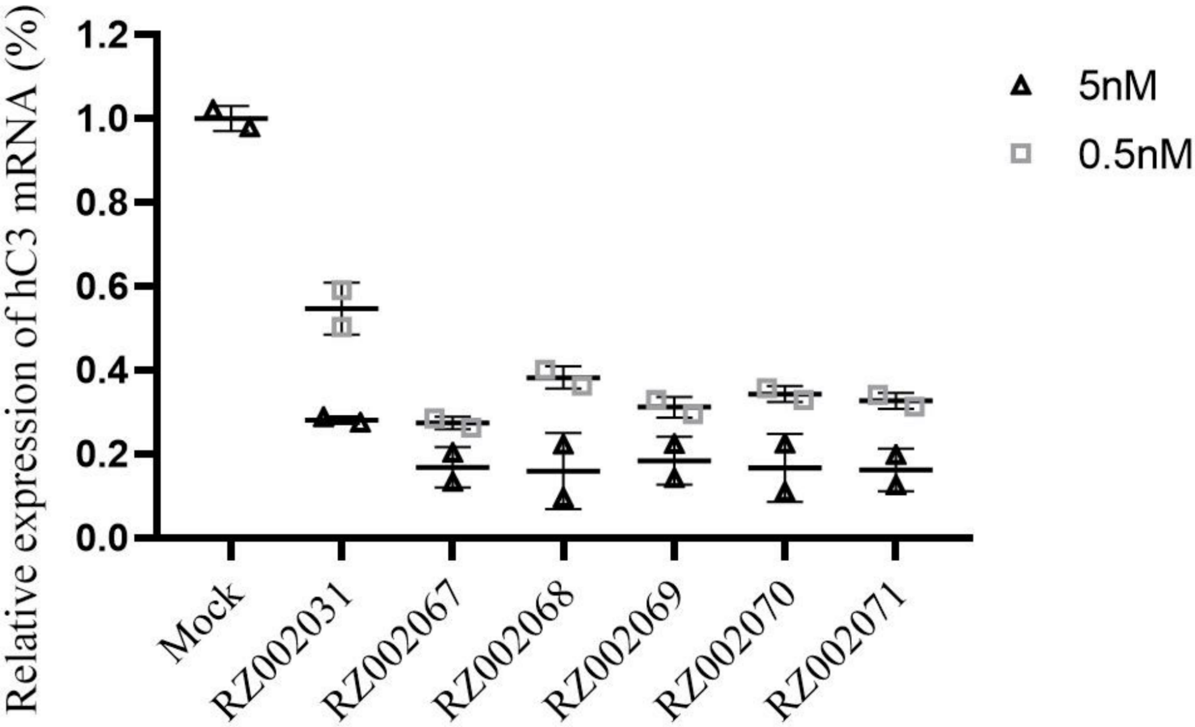


FIG. 18d

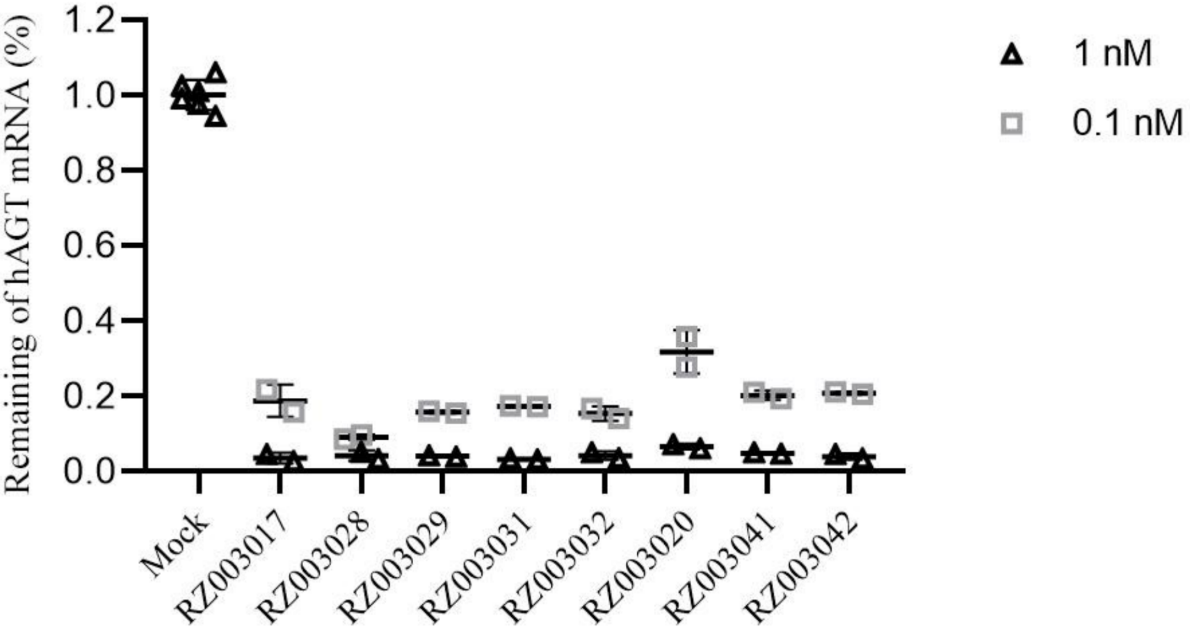
Remaining of hAGT mRNA (%)
1.2
1.0
0.8
0.6
0.4
0.2
0.0
Mock
RZ003017
RZ003028
RZ003029
RZ003031
RZ003032
RZ003020
RZ003041
RZ003042
1 nM
0.1 nM

FIG. 19

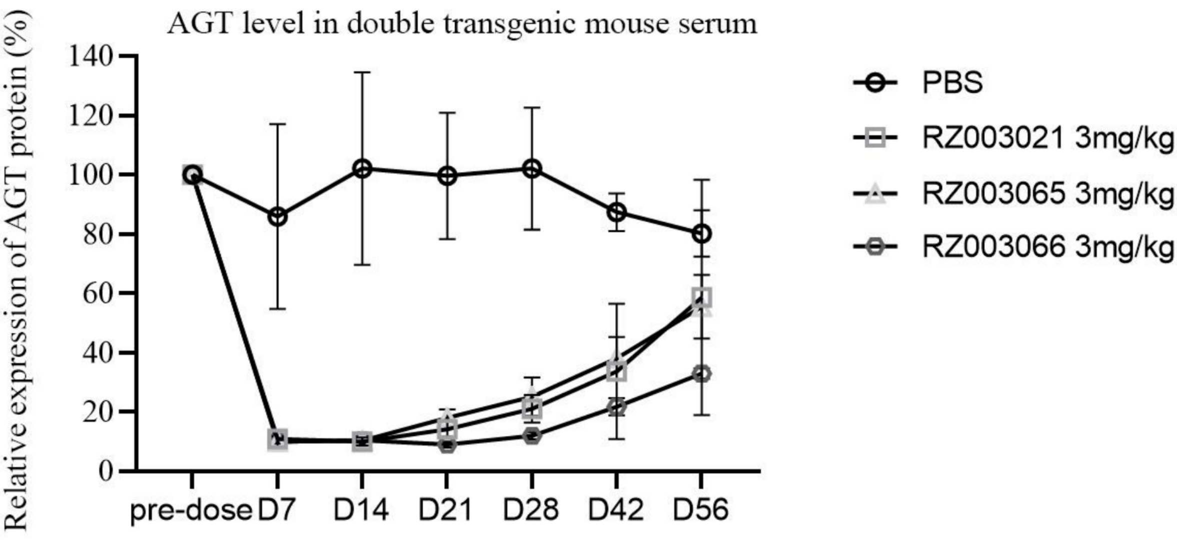
AGT level in double transgenic mouse serum
Relative expression of AGT protein (%)
140
120
100
80
60
40
20
0
pre-dose
D7
D14
D21
D28
D42
D56
PBS
RZ003021 3mg/kg
RZ003065 3mg/kg
RZ003066 3mg/kg

FIG. 20

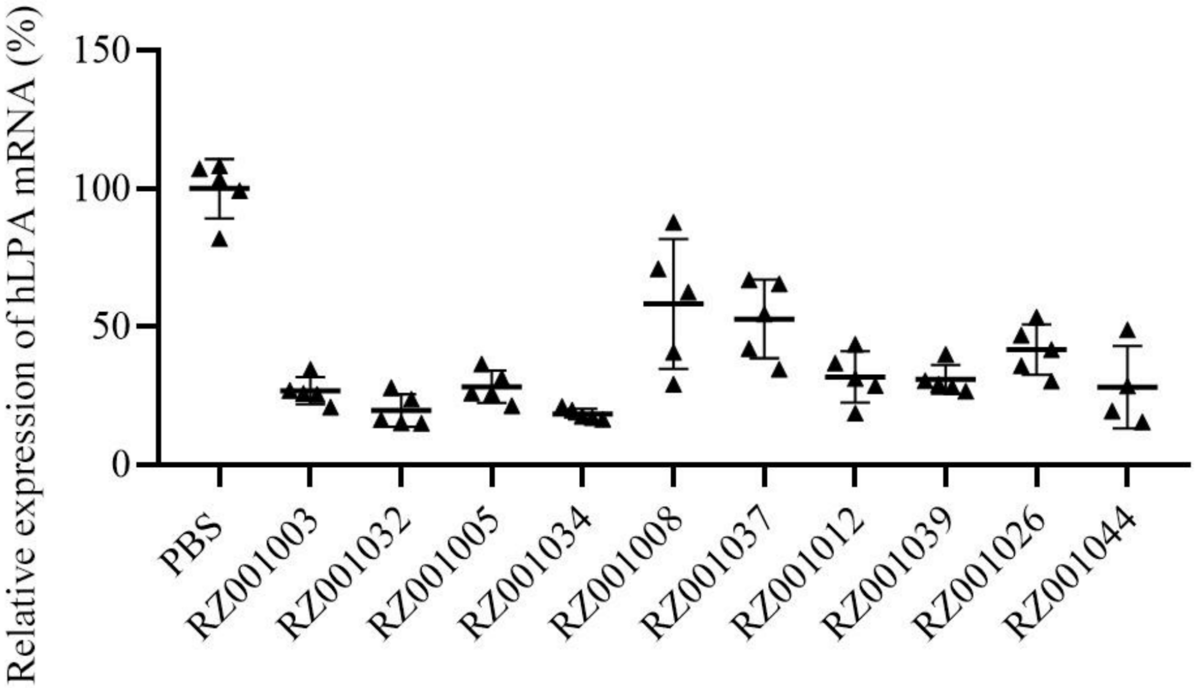
Relative expression of hLPA mRNA (%)
150
100
50
0
PBS
RZ001003
RZ001032
RZ001005
RZ001034
RZ001008
RZ001037
RZ001012
RZ001039
RZ001026
RZ001044

FIG. 21

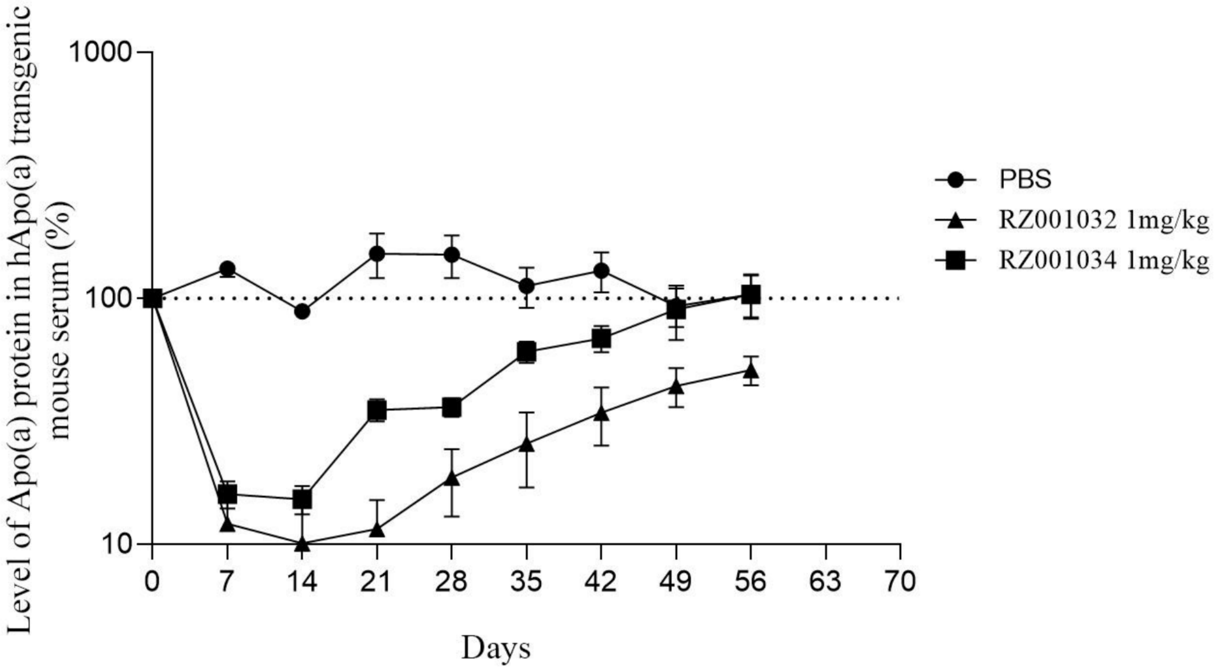
Level of Apo(a) protein in hApo(a) transgenic mouse serum (%)
1000
100
10
0
7
14
21
28
35
42
49
56
63
70
Days
PBS
RZ001032 1mg/kg
RZ001034 1mg/kg

FIG. 22

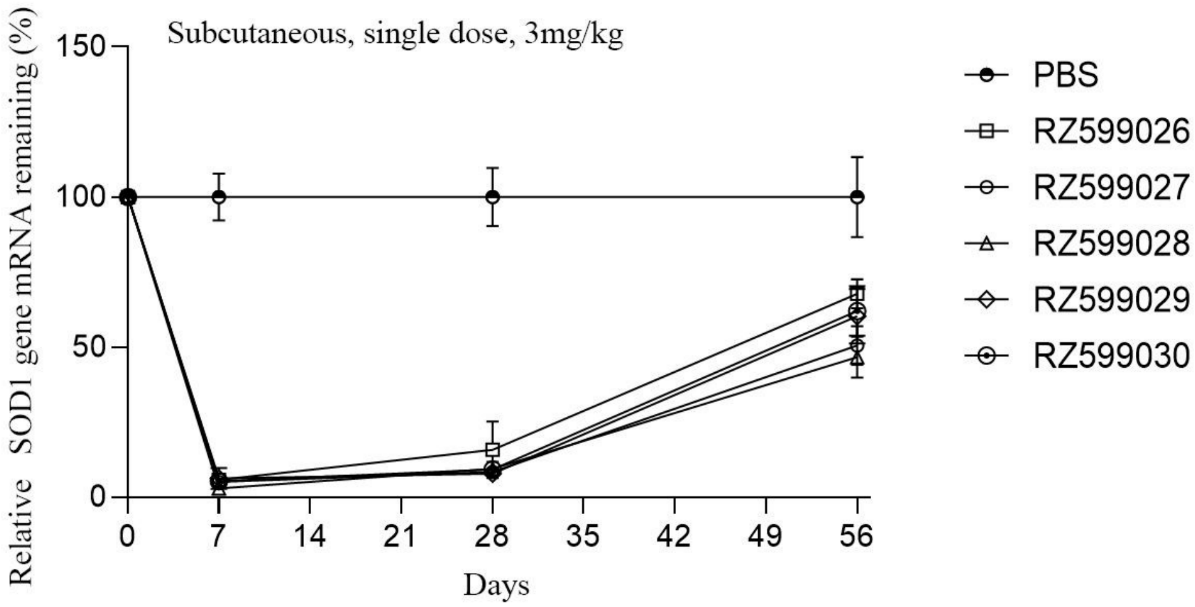
Subcutaneous, single dose, 3mg/kg
Relative SOD1 gene mRNA remaining (%)
150
100
50
0
0
7
14
21
28
35
42
49
56
Days
PBS
RZ599026
RZ599027
RZ599028
RZ599029
RZ599030

FIG. 23

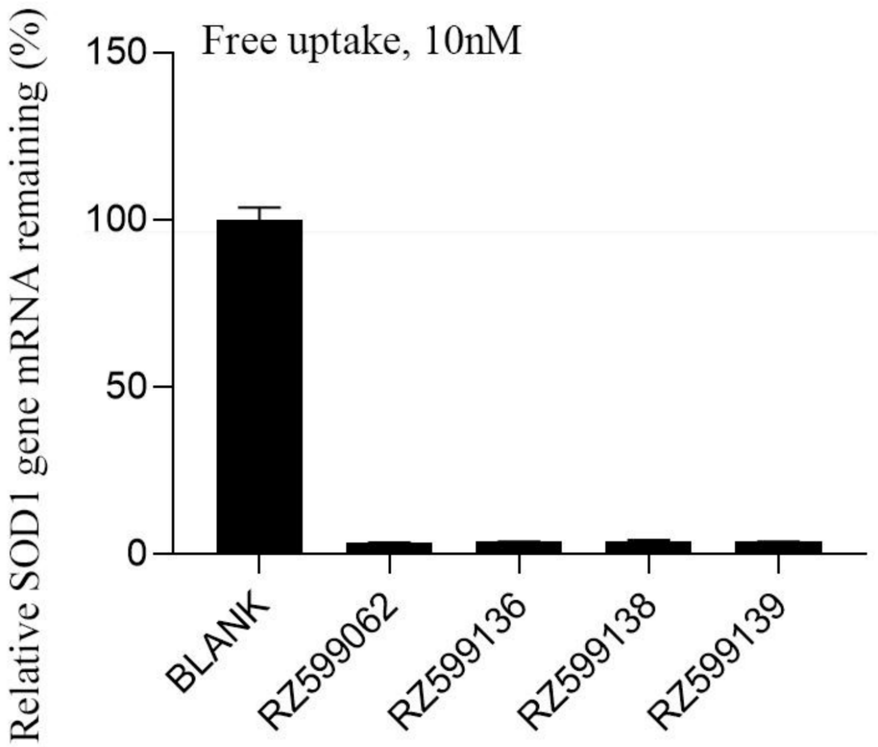
Free uptake, 10nM
Relative SOD1 gene mRNA remaining (%)
150
100
50
0
BLANK
RZ599062
RZ599136
RZ599138
RZ599139

FIG. 24

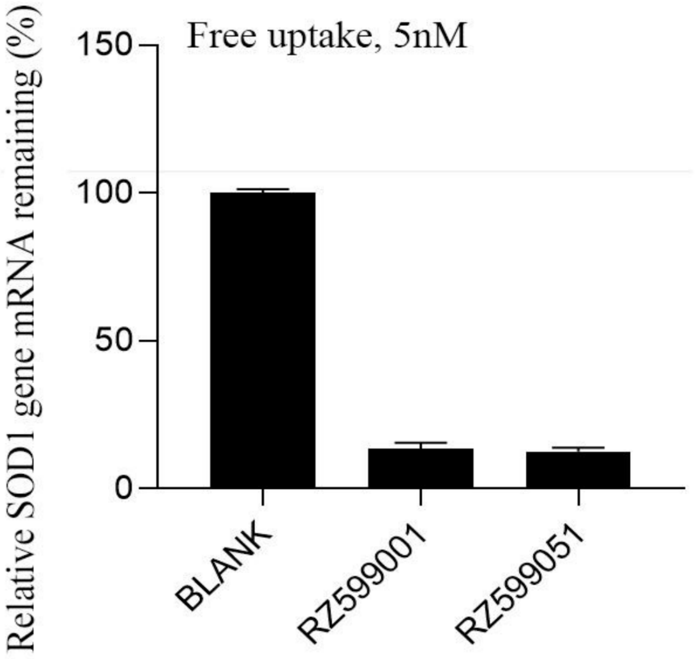
Free uptake, 5nM
Relative SOD1 gene mRNA remaining (%)
150
100
50
0
BLANK
RZ599001
RZ599051

FIG. 25

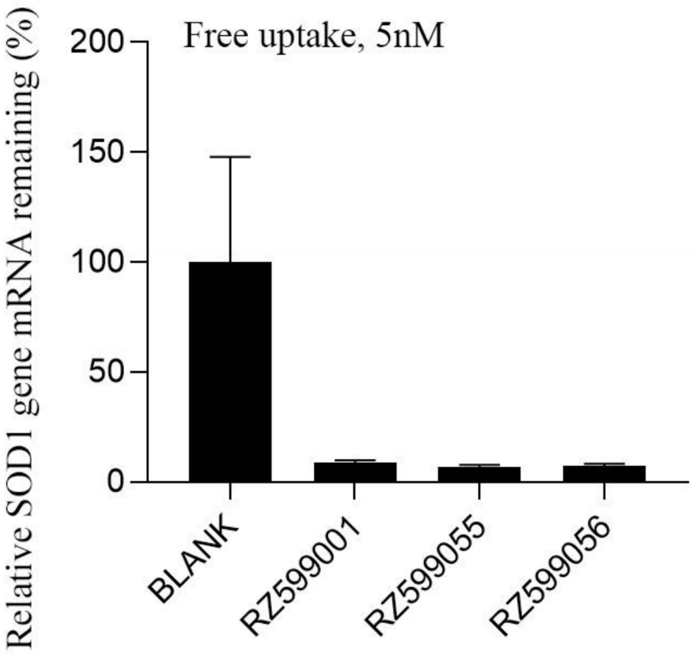
Free uptake, 5nM
Relative SOD1 gene mRNA remaining (%)
200
150
100
50
0
BLANK
RZ599001
RZ599055
RZ599056

FIG. 26

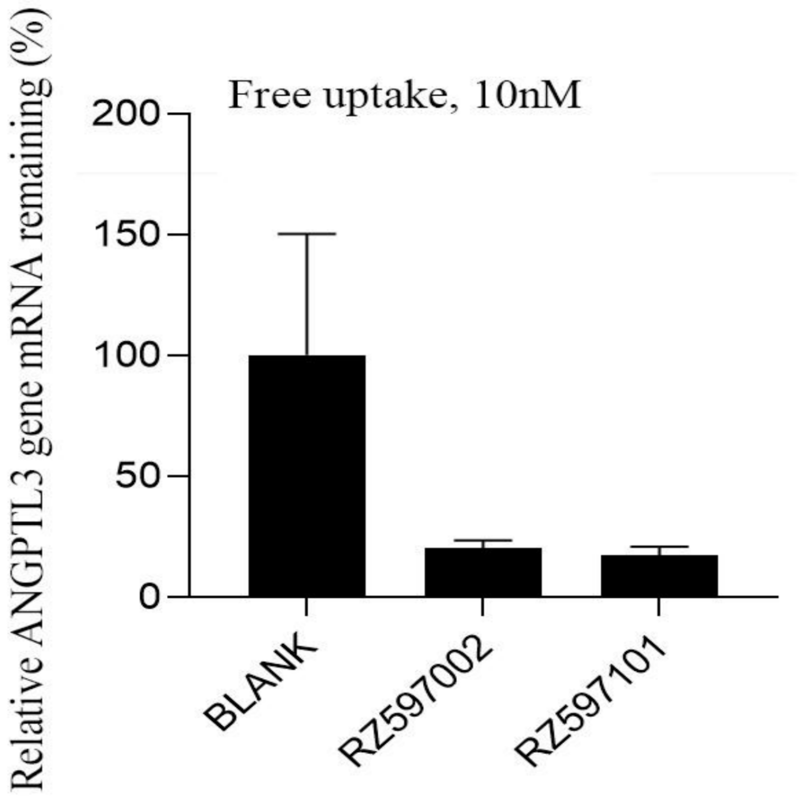
Free uptake, 10nM
Relative ANGPTL3 gene mRNA remaining (%)
200
150
100
50
0
BLANK
RZ597002
RZ597101

FIG. 27

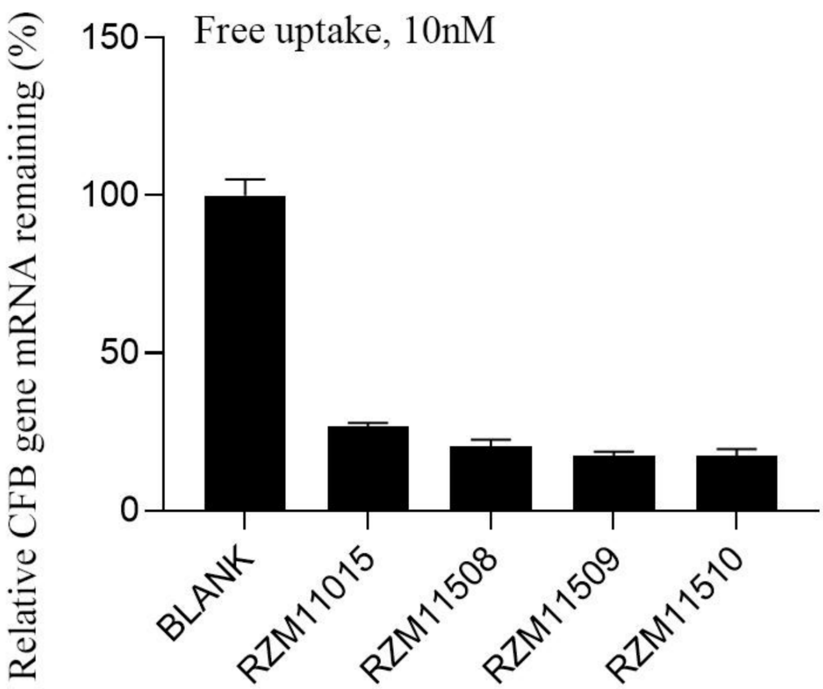
Free uptake, 10nM
Relative CFB gene mRNA remaining (%)
150
100
50
0
BLANK
RZM11015
RZM11508
RZM11509
RZM11510

FIG. 28

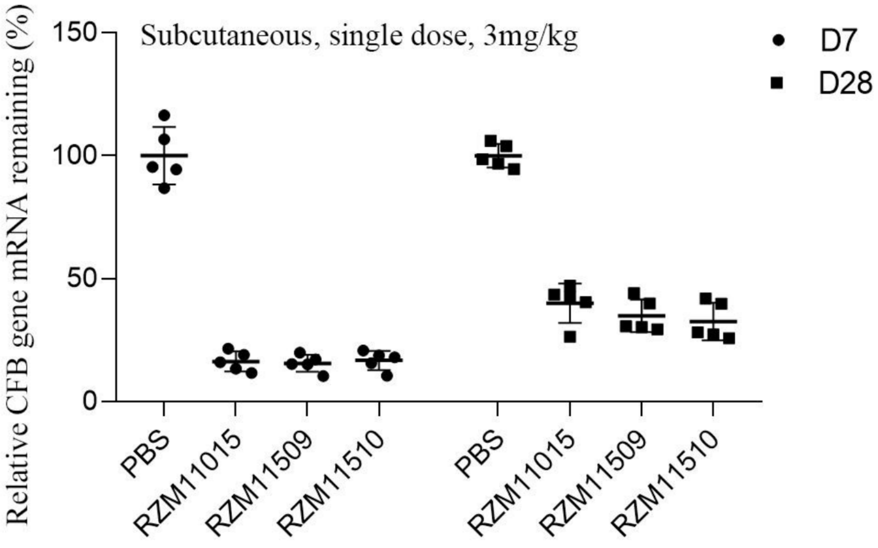
Subcutaneous, single dose, 3mg/kg
D7
D28
Relative CFB gene mRNA remaining (%)
150
100
50
0
PBS
RZM11015
RZM11509
RZM11510
PBS
RZM11015
RZM11509
RZM11510

FIG. 29

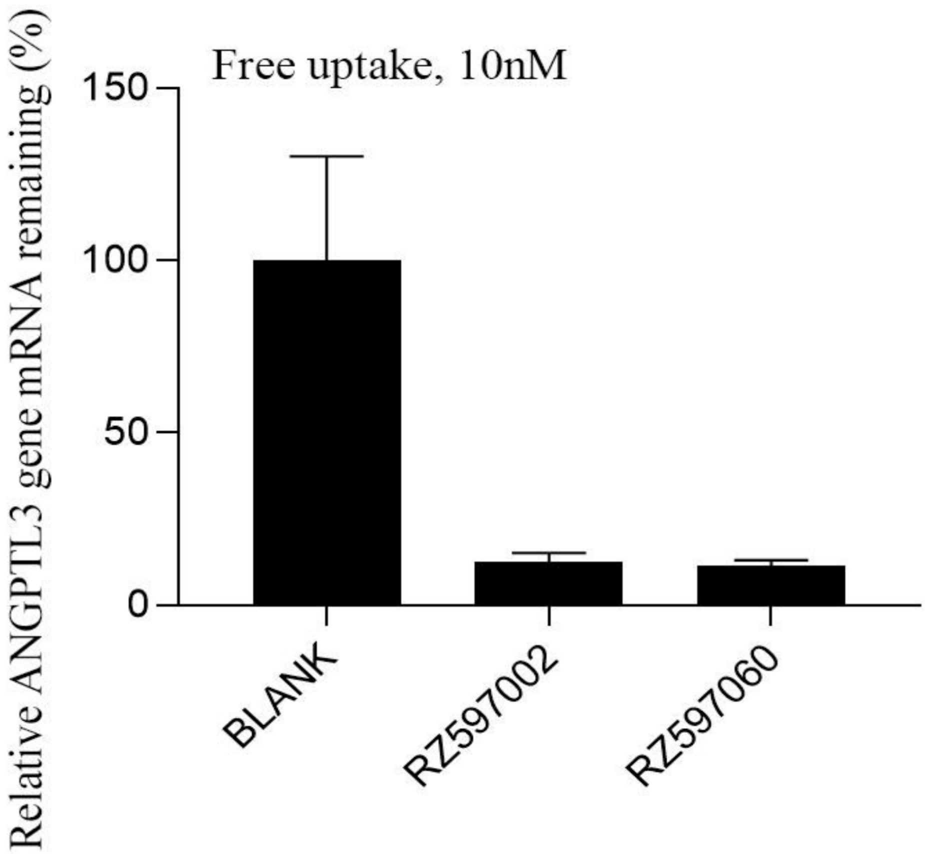
Free uptake, 10nM
Relative ANGPTL3 gene mRNA remaining (%)
150
100
50
0
BLANK
RZ597002
RZ597060

FIG. 30

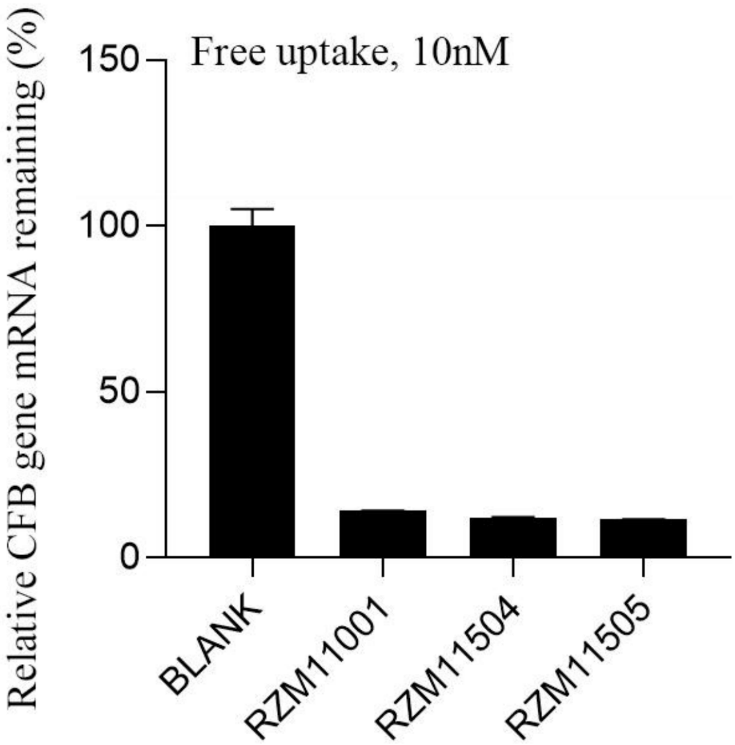
Free uptake, 10nM
Relative CFB gene mRNA remaining (%)
150
100
50
0
BLANK
RZM11001
RZM11504
RZM11505

FIG. 31

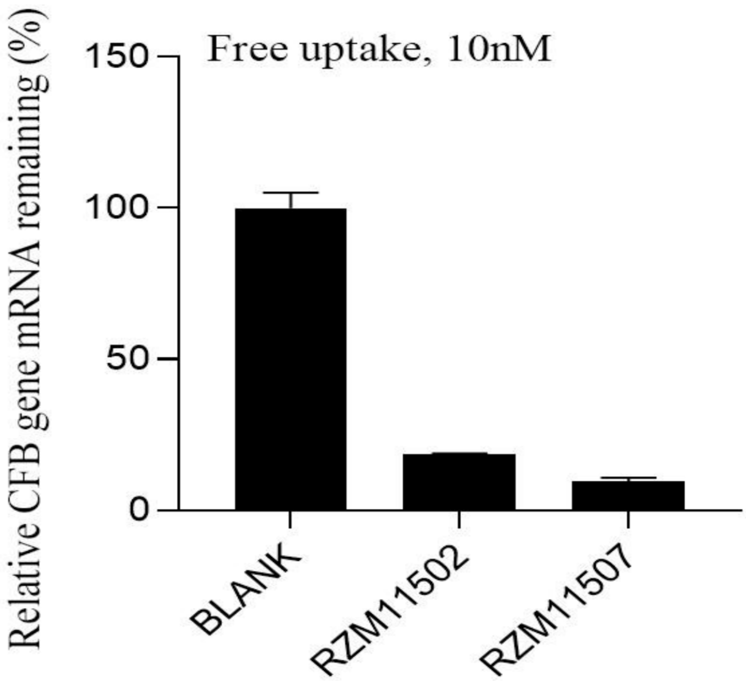
Free uptake, 10nM
Relative CFB gene mRNA remaining (%)
150
100
50
0
BLANK
RZM11502
RZM11507

FIG. 32

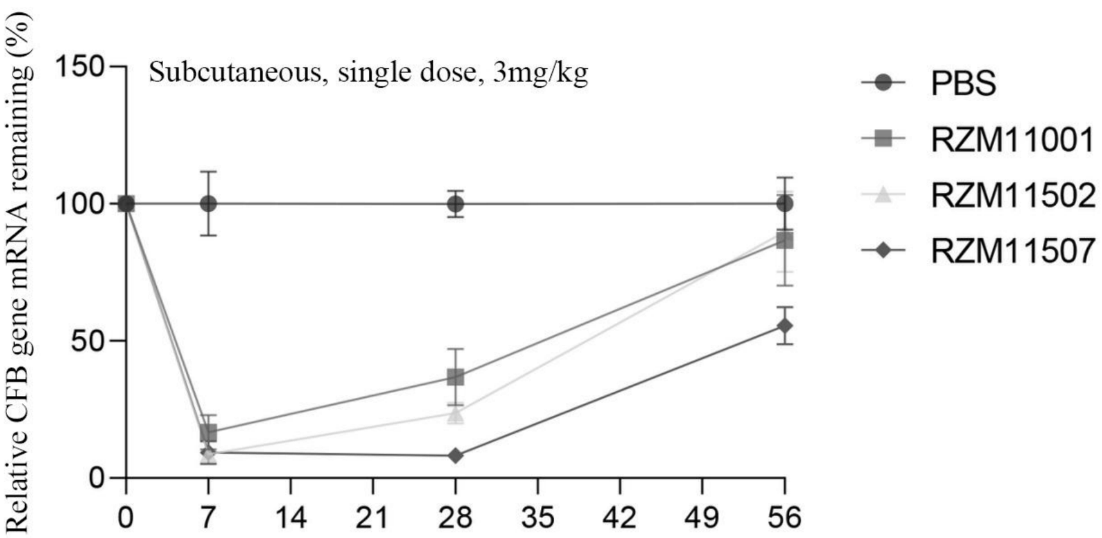
Relative CFB gene mRNA remaining (%)
Subcutaneous, single dose, 3mg/kg
150
100
50
0
0
7
14
21
28
35
42
49
56
PBS
RZM11001
RZM11502
RZM11507

FIG. 33

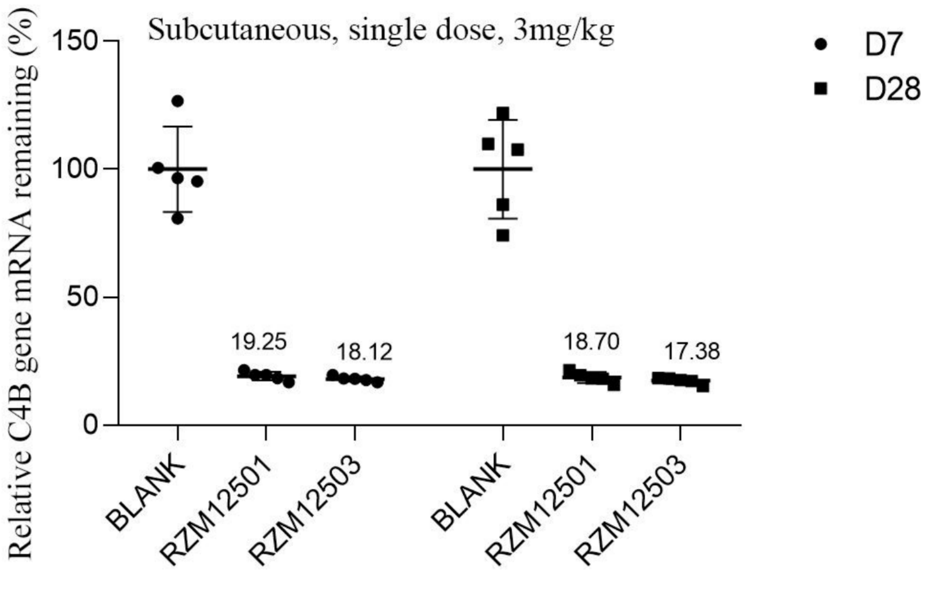
Relative C4B gene mRNA remaining (%)
Subcutaneous, single dose, 3mg/kg
150
100
50
0
19.25
18.12
18.70
17.38
BLANK
RZM12501
RZM12503
BLANK
RZM12501
RZM12503
D7
D28

FIG. 34

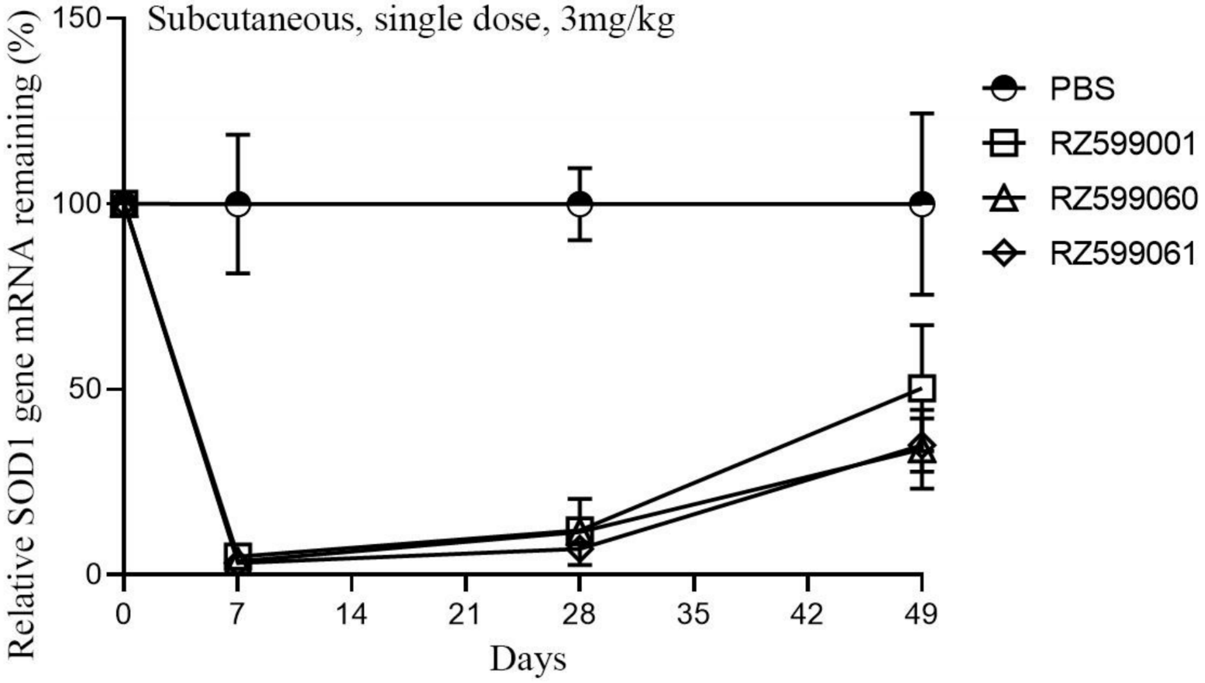
Subcutaneous, single dose, 3mg/kg
Relative SOD1 gene mRNA remaining (%)
150
100
50
0
0
7
14
21
28
35
42
49
Days
PBS
RZ599001
RZ599060
RZ599061

FIG. 35

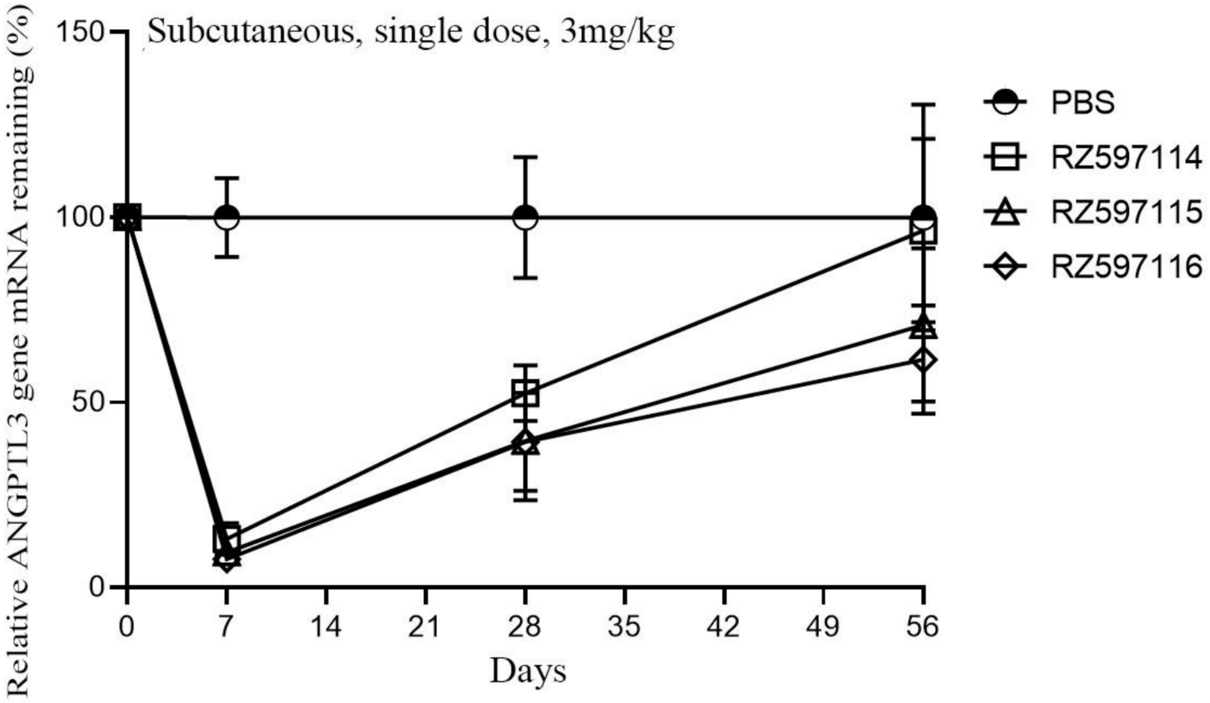
Subcutaneous, single dose, 3mg/kg
Relative ANGPTL3 gene mRNA remaining (%)
150
100
50
0
0
7
14
21
28
35
42
49
56
Days
PBS
RZ597114
RZ597115
RZ597116

FIG. 36

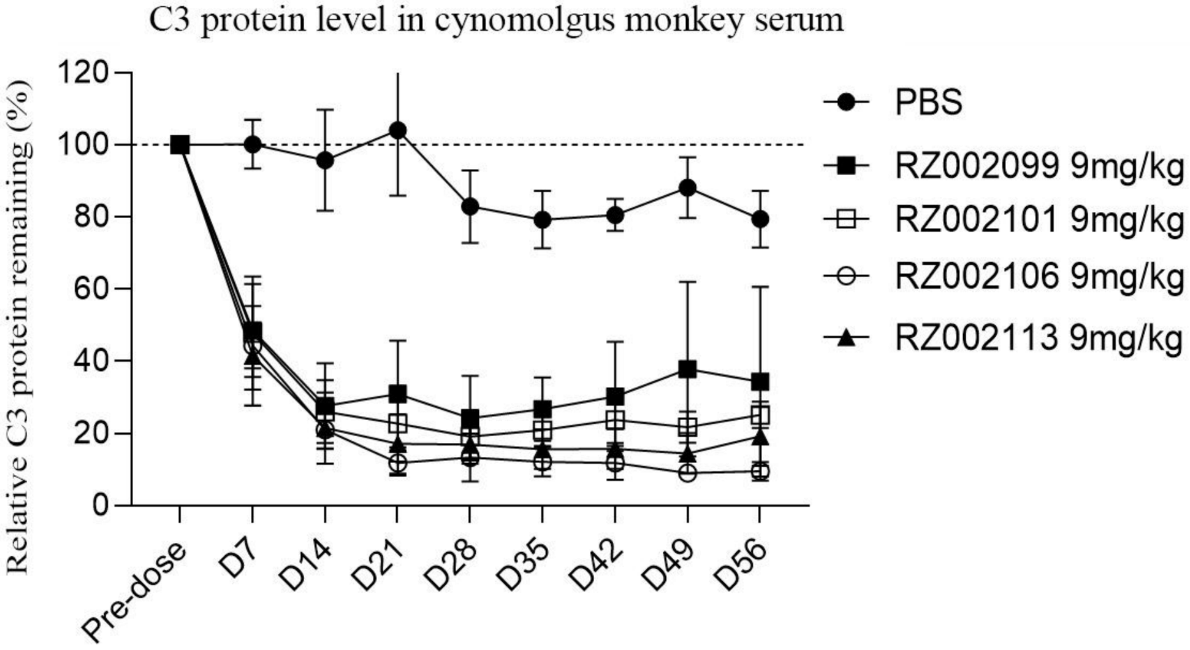
C3 protein level in cynomolgus monkey serum
Relative C3 protein remaining (%)
120
100
80
60
40
20
0
Pre-dose
D7
D14
D21
D28
D35
D42
D49
D56
PBS
RZ002099 9mg/kg
RZ002101 9mg/kg
RZ002106 9mg/kg
RZ002113 9mg/kg

FIG. 37

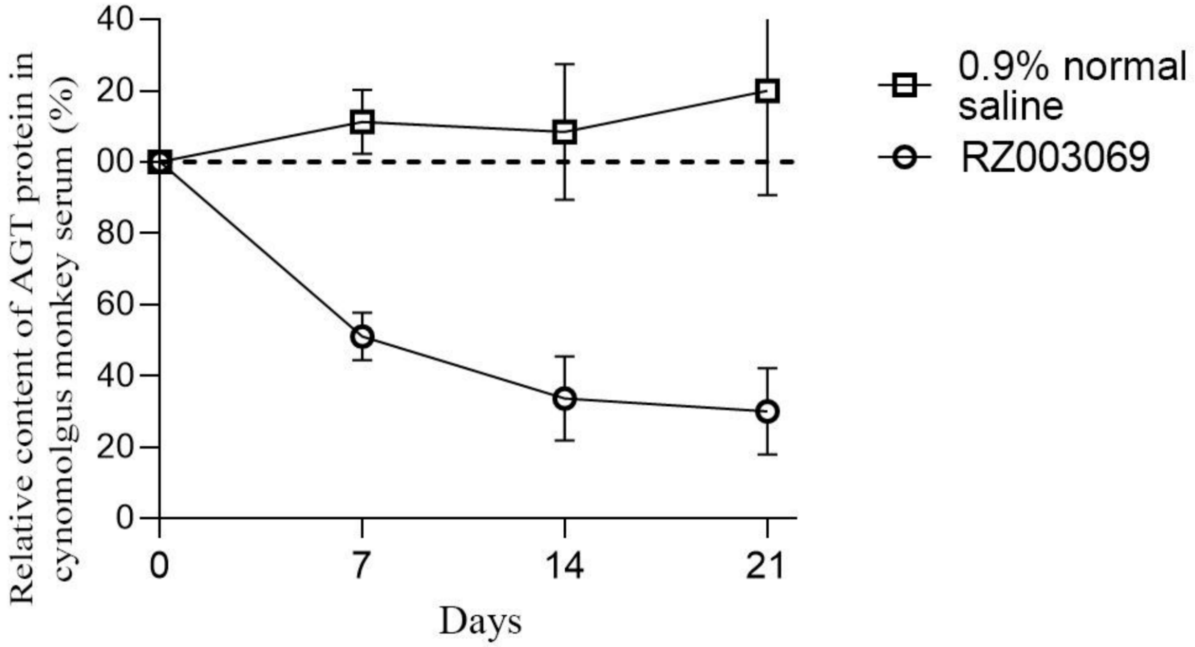
Relative content of AGT protein in cynomolgus monkey serum (%)
40
20
00
80
60
40
20
0
0
7
14
21
Days
0.9% normal saline
RZ003069

FIG. 38

## INTERNATIONAL SEARCH REPORT

International application No.
**PCT/CN2024/093907**

**A. CLASSIFICATION OF SUBJECT MATTER**

C12N15/113(2010.01)i; A61K31/713(2006.01)i; A61K31/712(2006.01)i; C07H21/02(2006.01)i

According to International Patent Classification (IPC) or to both national classification and IPC

**B. FIELDS SEARCHED**

Minimum documentation searched (classification system followed by classification symbols)

IPC: C12N A61K C07H

Documentation searched other than minimum documentation to the extent that such documents are included in the fields searched

Electronic data base consulted during the international search (name of data base and, where practicable, search terms used)

CNTXT, ENTXTC, VEN, DWPI, SIPOABS, CNKI, 万方数据库, WANFANG DATABASE, 百度, Baidu, Pubmed, EBI, NCBI, STN: 双链寡核苷酸, 正义链, 反义链, sense strand, antisense strand, sirna, 体积, 位阻, 替代, 替换, 修饰, 核糖, 2', TBDMS, TOM, 三异丙基甲氧基硅烷基, 叔丁基二甲基氯硅烷基, 2'-O-MOE, 2'-O-TOM, 2'-O-CH2-O-CH2-CH3, 2'-O-CH2-O-CH2-CF3, 2'-O-甲氧基乙基, 氟, 甲基, 两个取代, 双取代, 双"F"取代, 双氟取代, Methyl, sirna

**C. DOCUMENTS CONSIDERED TO BE RELEVANT**

| Category* | Citation of document, with indication, where appropriate, of the relevant passages | Relevant to claim No. |
|---|---|---|
| X | WO 2023284763 A1 (SUZHOU RIBO LIFE SCIENCE CO., LTD.) 19 January 2023 (2023-01-19)<br>claims 1-28, 50, 55, 59 and 62, and description, page 18, table 1 to page 20, line 19, and page 44, lines 22-24 | 1-18, 21-26 |
| Y | WO 2023284763 A1 (SUZHOU RIBO LIFE SCIENCE CO., LTD.) 19 January 2023 (2023-01-19)<br>claims 1-28, 50, 55, 59 and 62, and description, page 18, table 1 to page 20, line 19, and page 44, lines 22-24 | 1-26 |
| Y | CN 109952378 A (JANSSEN BIOPHARMA, INC.) 28 June 2019 (2019-06-28)<br>description, paragraphs 17-21 | 1-26 |
| X | WO 2023284559 A1 (SUZHOU RIBO LIFE SCIENCE CO., LTD.) 19 January 2023 (2023-01-19)<br>description, page 11, line 2 to page 12, line 24, and pages 54-58, tables 1a-1c | 1-18, 21-26 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **19 August 2024** | **23 August 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2024/093907**

**C. DOCUMENTS CONSIDERED TO BE RELEVANT**

| Category* | Citation of document, with indication, where appropriate, of the relevant passages | Relevant to claim No. |
|---|---|---|
| Y | WO 2023284559 A1 (SUZHOU RIBO LIFE SCIENCE CO., LTD.) 19 January 2023 (2023-01-19)<br>description, page 11, line 2 to page 12, line 24, and pages 54-58, tables 1a-1c | 1-26 |
| A | CN 102264374 A (ISIS PHARMACEUTICALS INC.) 30 November 2011 (2011-11-30)<br>description, paragraphs 19-44 | 1-26 |
| A | CN 104884618 A (ROCHE INNOVATION CENTER COPENHAGEN A/S) 02 September 2015 (2015-09-02)<br>claims 1-28 | 1-26 |
| A | CN 107075516 A (ALNYLAM PHARMACEUTICALS INC.) 18 August 2017 (2017-08-18)<br>claims 1-63 | 1-26 |
| A | CN 110831605 A (THOMAS I. KALMAN) 21 February 2020 (2020-02-21)<br>description, example 5 | 1-26 |
| A | CN 114685585 A (SUZHOU RIBO LIFE SCIENCE CO., LTD.) 01 July 2022 (2022-07-01)<br>claims 1-37 | 1-26 |
| A | CN 115927337 A (SANORI BIOMEDICAL TECHNOLOGY (WUXI) CO., LTD.) 07 April 2023 (2023-04-07)<br>claims 1-34 | 1-26 |
| A | US 2018256729 A1 (IONIS PHARMACEUTICALS, INC.) 13 September 2018 (2018-09-13)<br>claims 1-115 | 1-26 |
| A | 孙莉萍 等 (SUN, Liping et al.). "siRNA的化学修饰和临床应用 (Chemical Modification of siRNA and Clinical Applications)"<br>*生命的化学 (Chemistry of Life)*, Vol. 25, No. (4), 31 December 2005 (2005-12-31),<br>pp. 339-342 | 1-26 |
| A | LIN, X. et al. "Advance of Structural Modification of Nucleosides Scaffold"<br>*European Journal of Medicinal Chemistry*, Vol. vol. 214, 30 January 2021 (2021-01-30),<br>Article Number 113233, pages 1-21 | 1-26 |

Form PCT/ISA/210 (second sheet) (July 2022)

**INTERNATIONAL SEARCH REPORT**

International application No.
**PCT/CN2024/093907**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**

International application No.
**PCT/CN2024/093907**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **24, 25 (in part)**
because they relate to subject matter not required to be searched by this Authority, namely:

Claim 24 relates to a method for treating and/or preventing diseases or symptoms related to the level of mRNA expressed by a target gene, and claim 25 (in part) relates to a method for regulating the expression level of a target gene associated with a disease in cells in a body, that is, claims 24 and 25 (in part) relate to subject matter for which no search is required by the International Searching Authority as defined in PCT Rule 39.1: (4) methods for treatment of the human or animal body by surgery or therapy, as well as diagnostic methods. The international search is carried out on the basis of the use of the double-stranded oligonucleotide, the oligonucleotide conjugate and the pharmaceutical composition in the preparation of a drug for treating diseases or symptoms related to the level of mRNA expressed by a target gene.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2024/093907**

| Patent document cited in search report | Publication date (day/month/year) | Patent family member(s) | Publication date (day/month/year) |
|---|---|---|---|
| WO 2023284763 A1 | 19 January 2023 | None | |
| CN 109952378 A | 28 June 2019 | CA 3037042 A1 | 22 March 2018 |
| | | MX 2023014145 A | 13 December 2023 |
| | | TW 201825103 A | 16 July 2018 |
| | | TWI 769177 B | 01 July 2022 |
| | | WO 2018053185 A1 | 22 March 2018 |
| | | BR 112019004911 A2 | 25 June 2019 |
| | | RU 2019111020 A | 15 October 2020 |
| | | RU 2019111020 A3 | 22 June 2021 |
| | | EP 3512949 A1 | 24 July 2019 |
| | | AU 2024201485 A1 | 28 March 2024 |
| | | KR 20230033736 A | 08 March 2023 |
| | | KR 102627418 B1 | 19 January 2024 |
| | | US 2018135054 A1 | 17 May 2018 |
| | | US 10793859 B2 | 06 October 2020 |
| | | MX 2019002960 A | 18 September 2019 |
| | | KR 20240010761 A | 24 January 2024 |
| | | JP 2022023936 A | 08 February 2022 |
| | | JP 7170820 B2 | 14 November 2022 |
| | | MA 46422 A | 24 July 2019 |
| | | AU 2017326372 A1 | 04 April 2019 |
| | | AU 2017326372 B2 | 07 December 2023 |
| | | US 2021040483 A1 | 11 February 2021 |
| | | JP 2023011793 A | 24 January 2023 |
| | | JP 7478210 B2 | 02 May 2024 |
| | | JP 2019529406 A | 17 October 2019 |
| | | JP 6970187 B2 | 24 November 2021 |
| | | KR 20190047025 A | 07 May 2019 |
| | | KR 102533038 B1 | 17 May 2023 |
| | | IL 265359 A | 30 May 2019 |
| WO 2023284559 A1 | 19 January 2023 | None | |
| CN 102264374 A | 30 November 2011 | WO 2010048552 A2 | 29 April 2010 |
| | | WO 2010048552 A3 | 15 July 2010 |
| | | JP 2012506701 A | 22 March 2012 |
| | | JP 5763539 B2 | 12 August 2015 |
| | | US 2015126725 A1 | 07 May 2015 |
| | | EP 2358397 A2 | 24 August 2011 |
| | | EP 2358397 B1 | 01 January 2020 |
| | | AU 2016202427 A1 | 12 May 2016 |
| | | AU 2009308217 A1 | 29 April 2010 |
| | | AU 2009308217 B2 | 21 January 2016 |
| | | US 2012059045 A1 | 08 March 2012 |
| | | CA 2741294 A1 | 29 April 2010 |
| | | CA 2741294 C | 24 April 2018 |
| | | WO 2010048549 A2 | 29 April 2010 |
| | | WO 2010048549 A3 | 10 September 2010 |
| | | US 2011269821 A1 | 03 November 2011 |
| | | US 8883752 B2 | 11 November 2014 |
| CN 104884618 A | 02 September 2015 | JP 2016501195 A | 18 January 2016 |
| | | JP 6452614 B2 | 16 January 2019 |

Form PCT/ISA/210 (patent family annex) (July 2022)

**INTERNATIONAL SEARCH REPORT**
**Information on patent family members**

International application No.
**PCT/CN2024/093907**

| Patent document cited in search report | Publication date (day/month/year) | Patent family member(s) | | | Publication date (day/month/year) |
|---|---|---|---|---|---|
| | | SG | 10201804331 | TA | 30 July 2018 |
| | | JP | 2019055995 | A | 11 April 2019 |
| | | MX | 2015005794 | A | 12 January 2016 |
| | | MX | 363068 | B | 07 March 2019 |
| | | WO | 2014076195 | A1 | 22 May 2014 |
| | | BR | 112015010116 | A2 | 22 August 2017 |
| | | WO | 2014076196 | A1 | 22 May 2014 |
| | | AU | 2019202288 | A1 | 18 April 2019 |
| | | MX | 2015005792 | A | 17 December 2015 |
| | | RU | 2015119411 | A | 10 January 2017 |
| | | HK | 1210213 | A1 | 15 April 2016 |
| | | US | 2015275212 | A1 | 01 October 2015 |
| | | US | 10077443 | B2 | 18 September 2018 |
| | | HRP | 20190826 | T1 | 28 June 2019 |
| | | DK | 2920307 | T3 | 16 July 2018 |
| | | EP | 2920304 | A1 | 23 September 2015 |
| | | EP | 2920304 | B1 | 06 March 2019 |
| | | US | 2015291958 | A1 | 15 October 2015 |
| | | IL | 268420 | A | 26 September 2019 |
| | | EP | 3406718 | A1 | 28 November 2018 |
| | | RU | 2015119409 | A | 10 January 2017 |
| | | RU | 2653438 | C2 | 08 May 2018 |
| | | ES | 2724853 | T3 | 16 September 2019 |
| | | CA | 2889044 | A1 | 22 May 2014 |
| | | US | 2018251764 | A1 | 06 September 2018 |
| | | US | 11155816 | B2 | 26 October 2021 |
| | | NZ | 708171 | A | 29 November 2019 |
| | | JP | 2016503300 | A | 04 February 2016 |
| | | SI | 2920304 | T1 | 28 June 2019 |
| | | CA | 2889596 | A1 | 22 May 2014 |
| | | CA | 2889596 | C | 23 August 2022 |
| | | ZA | 201503375 | B | 24 February 2016 |
| | | MY | 173826 | A | 24 February 2020 |
| | | HK | 1214297 | A1 | 22 July 2016 |
| | | IL | 238448 | A0 | 30 June 2015 |
| | | IL | 238448 | B | 26 September 2019 |
| | | SG | 11201503821 | YA | 29 June 2015 |
| | | US | 2021238601 | A1 | 05 August 2021 |
| | | DK | 2920304 | T3 | 13 May 2019 |
| | | AU | 2013346767 | A1 | 28 May 2015 |
| | | AU | 2013346767 | A2 | 18 June 2015 |
| | | AU | 2013346767 | B2 | 11 April 2019 |
| | | KR | 20150083920 | A | 20 July 2015 |
| | | EP | 2920307 | A1 | 23 September 2015 |
| | | EP | 2920307 | B1 | 02 May 2018 |
| | | PL | 2920304 | T3 | 31 July 2019 |
| | | KR | 20150082643 | A | 15 July 2015 |
| | | KR | 102112892 | B1 | 19 May 2020 |
| | | BR | 112015011112 | A2 | 06 November 2018 |
| | | RU | 2018112970 | A | 01 March 2019 |

Form PCT/ISA/210 (patent family annex) (July 2022)

**INTERNATIONAL SEARCH REPORT**
**Information on patent family members**

International application No.
**PCT/CN2024/093907**

| Patent document cited in search report | Publication date (day/month/year) | Patent family member(s) | Publication date (day/month/year) |
|---|---|---|---|
| | | RU 2018112970 A3 | 27 May 2020 |
| CN 107075516 A | 18 August 2017 | US 2022002727 A1 | 06 January 2022 |
| | | NZ 767118 A | 23 February 2024 |
| | | SG 11201701166 UA | 30 March 2017 |
| | | US 2022056448 A1 | 24 February 2022 |
| | | US 11427822 B2 | 30 August 2022 |
| | | EP 3812462 A1 | 28 April 2021 |
| | | US 2017275626 A1 | 28 September 2017 |
| | | US 10233448 B2 | 19 March 2019 |
| | | JP 2021073182 A | 13 May 2021 |
| | | JP 7370311 B2 | 27 October 2023 |
| | | KR 20230019498 A | 08 February 2023 |
| | | KR 102630289 B1 | 31 January 2024 |
| | | IL 250448 A0 | 30 March 2017 |
| | | IL 250448 B | 25 March 2021 |
| | | JP 2017525705 A | 07 September 2017 |
| | | JP 7289610 B2 | 12 June 2023 |
| | | US 2019241891 A1 | 08 August 2019 |
| | | US 10612024 B2 | 07 April 2020 |
| | | US 2023110876 A1 | 13 April 2023 |
| | | US 2019241893 A1 | 08 August 2019 |
| | | US 10612027 B2 | 07 April 2020 |
| | | IL 280941 A | 29 April 2021 |
| | | US 2021017519 A1 | 21 January 2021 |
| | | US 11401517 B2 | 02 August 2022 |
| | | NZ 730296 A | 29 September 2023 |
| | | US 2023183701 A1 | 15 June 2023 |
| | | MX 2017002144 A | 15 August 2017 |
| | | SG 10201903290 YA | 30 May 2019 |
| | | SG 10201913791 QA | 30 March 2020 |
| | | EP 3186377 A1 | 05 July 2017 |
| | | JP 2021073183 A | 13 May 2021 |
| | | KR 20170099832 A | 01 September 2017 |
| | | KR 102494171 B1 | 02 February 2023 |
| | | EA 201790420 A1 | 31 July 2017 |
| | | EP 3808846 A1 | 21 April 2021 |
| | | CA 2958758 A1 | 25 February 2016 |
| | | WO 2016028649 A1 | 25 February 2016 |
| | | KR 20240010762 A | 24 January 2024 |
| | | JP 2024020209 A | 14 February 2024 |
| | | US 2022389424 A1 | 08 December 2022 |
| | | US 11549109 B2 | 10 January 2023 |
| CN 110831605 A | 21 February 2020 | EP 3618837 A1 | 11 March 2020 |
| | | EP 3618837 A4 | 04 November 2020 |
| | | US 2020054661 A1 | 20 February 2020 |
| | | US 10751358 B2 | 25 August 2020 |
| | | JP 2020518657 A | 25 June 2020 |
| | | JP 7173613 B2 | 16 November 2022 |
| | | BR 112019022470 A2 | 12 May 2020 |
| | | CA 3061621 A1 | 01 November 2018 |

Form PCT/ISA/210 (patent family annex) (July 2022)

**INTERNATIONAL SEARCH REPORT**
**Information on patent family members**

International application No.
**PCT/CN2024/093907**

| Patent document cited in search report | | | Publication date (day/month/year) | Patent family member(s) | | | Publication date (day/month/year) |
|---|---|---|---|---|---|---|---|
| | | | | WO | 2018200859 | A1 | 01 November 2018 |
| CN | 114685585 | A | 01 July 2022 | None | | | |
| CN | 115927337 | A | 07 April 2023 | None | | | |
| US | 2018256729 | A1 | 13 September 2018 | JP | 2018528783 | A | 04 October 2018 |
| | | | | EP | 3353306 | A1 | 01 August 2018 |
| | | | | EP | 3353306 | A4 | 05 June 2019 |
| | | | | WO | 2017053999 | A1 | 30 March 2017 |
| | | | | US | 11116843 | B2 | 14 September 2021 |
| | | | | US | 2022023429 | A1 | 27 January 2022 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 202310574400
- WO 2015006740 A2

### Non-patent literature cited in the description

- **TAI, W.** Chemical modulation of siRNA lipophilicity for efficient delivery.. *J Control Release*, vol. 307, 98-107
- *CHEMICAL ABSTRACTS*, 429-41-4
- *CHEMICAL ABSTRACTS*, 40615-36-9
- *CHEMICAL ABSTRACTS*, 102691-36-1
- *CHEMICAL ABSTRACTS*, 1122-28-7
- *CHEMICAL ABSTRACTS*, 75-84-3
- *CHEMICAL ABSTRACTS*, 863329-66-2
- *CHEMICAL ABSTRACTS*, 181308-57-6
- *CHEMICAL ABSTRACTS*, 94790-37-1
- *CHEMICAL ABSTRACTS*, 1122-58-3